# EUROPEAN PATENT APPLICATION

(11) **EP 4 620 952 A1**
(43) Date of publication of application: **24.09.2025**
(21) Application number: 23891585.4
(22) Date of filing: 14.11.2023
(51) Int. Cl.: C07D 213/40, A01N 53/12, A01P 3/00, C07D 213/42

(54) **AMIDE COMPOUNDS OR SALTS THEREOF, BACTERICIDAL AGENTS FOR AGRICULTURE AND HORTICULTURE CONTAINING SAID COMPOUNDS, AND METHOD OF USING SAME**

(30) Priority: 15.11.2022 JP 2022182306; 31.03.2023 JP 2023057006
(71) Applicant: Nihon Nohyaku Co., Ltd., Tokyo 104-8386 (JP)
(72) Inventor: MATSUI, Shunsuke, Kawachinagano-shi, Osaka 586-0094 (JP); NAKAUCHI, Ayuko, Chuo-ku, Tokyo 104-8386 (JP); OSADA, Rumi, Kawachinagano-shi, Osaka 586-0094 (JP); HARAYAMA, Hiroto, Kawachinagano-shi, Osaka 586-0094 (JP); ANDO, Akihiro, Kawachinagano-shi, Osaka 586-0094 (JP); YAMASHITA, Masao, Chuo-ku, Tokyo 104-8386 (JP)
(74) Representative: J A Kemp LLP
(86) International application number: PCT/JP2023/040959
(87) International publication number: WO 2024/106438

(57) **Abstract**

The present invention provides an excellent new agricultural and horticultural pest control agent.

The present invention relates to compounds represented by the following general formula [I]

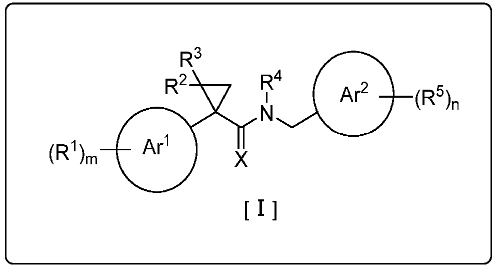

wherein each symbol is as described in the description,
or salts thereof, agricultural and horticultural fungicides comprising them as active ingredients, and methods of use thereof.

## Description

### [Technical Field]

The present invention relates to amide compounds or salts thereof, agricultural and horticultural fungicides containing the compounds as active ingredients, and methods of use thereof.

### [Background Art]

Various compounds have been studied as agricultural and horticultural fungicides, and it has been reported that certain cycloalkyl-containing amide compounds are useful as insecticides, fungicides and immunomodulators (for example, see Patent Literatures 1 to 4 and Non-Patent Literature 1).

The literatures, however, do not disclose the amide compound of the present invention.

### [Citation List]

### [Patent Literature]

[Patent Literature 1]
   DE 3422077 pamphlet
[Patent Literature 2]
   WO 2000/024720 pamphlet
[Patent Literature 3]
   WO 2019/099314 pamphlet
[Patent Literature 4]
   WO 2008/005964 pamphlet

### [Non-Patent Literature]

[Non-Patent Literature 1]
Huaxue Shiji, 34(2), 154-156, 2012

### [Summary of Invention]

### [Technical Problem]

In crop production in the fields of agriculture, horticulture and the like, the damage caused by various diseases etc. is still immense, and diseases resistant to existing fungicides have emerged. Under such circumstances, the development of novel agricultural and horticultural fungicides is desired. In addition, laborsaving and automated application is a recent trend in these fields, and from this viewpoint, there is a need for the development of agricultural and horticultural fungicides having properties suitable for various application methods, such as seed treatment, small-volume application, and topical application. That is, there is a need for agricultural and horticultural fungicides having moderate environmental and thermal stability and adequate translocation and diffusibility throughout crop plants or the vicinity thereof. Meanwhile, in the context of a growing demand for sustainable agriculture, agrochemical toxicity and impact on surrounding living organisms and surrounding environment are assessed from various viewpoints, and there is a need for fungicides having safety from various aspects, moderate persistence, biodegradability, environmental degradability, etc.

### [Solution to Problem]

The present inventors conducted extensive research to solve the above-described problems. As a result, the present inventors found that an amide compound represented by the general formula [I] or salts thereof has an excellent control effect on agricultural and horticultural diseases. The present inventors conducted further research and thus found that the amide compound or salts thereof is suitable for use in various agrochemical applications and excellent in physicochemical and biochemical properties. Based on these findings, the present invention has been completed.

That is, the present invention relates to the following.
[1] A compound represented by the general formula [I] 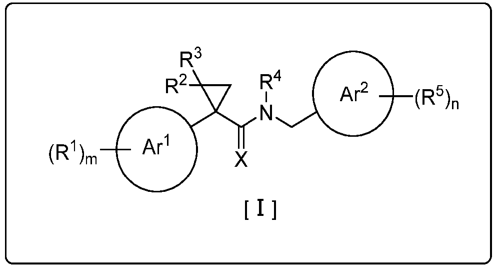 wherein
   R¹ and R⁵ may be the same or different and each represents
      (a1) a hydrogen atom;
      (a2) a halogen atom;
      (a3) a cyano group;
      (a4) a nitro group;
      (a5) a hydroxy group;
      (a6) a pentafluorosulfanyl group;
      (a7) a (C1-C6) alkyl group;
      (a8) a halo (C1-C6) alkyl group;
      (a9) a (C2-C6) alkenyl group;
      (a10) a halo (C2-C6) alkenyl group;
      (all) a (C2-C6) alkynyl group;
      (a12) a halo (C2-C6) alkynyl group;
      (a13) a (C3-C6) cycloalkyl group;
      (a14) a (C3-C6) cycloalkenyl group;
      (a15) a halo (C3-C6) cycloalkyl group;
      (a16) a (C3-C6) cycloalkyl (C1-C6) alkyl group;
      (a17) a (C3-C6) cycloalkyl (C3-C6) cycloalkyl group;
      (a18) a halo (C3-C6) cycloalkyl (C1-C6) alkyl group;
      (a19) a halo (C3-C6) cycloalkyl halo (C1-C6) alkyl group;
      (a20) a cyano (C1-C6) alkyl group;
      (a21) a cyano (C3-C6) cycloalkyl group;
      (a22) a hydroxy (C1-C6) alkyl group;
      (a23) a (C1-C6) alkoxy (C1-C6) alkyl group;
      (a24) a halo (C1-C6) alkoxy (C1-C6) alkyl group;
      (a25) a (C3-C6) cycloalkoxy (C1-C6) alkyl group;
      (a26) a halo (C3-C6) cycloalkoxy (C1-C6) alkyl group;
      (a27) a (C1-C6) alkylsulfanyl (C1-C6) alkyl group;
      (a28) a halo (C1-C6) alkylsulfanyl (C1-C6) alkyl group;
      (a29) a (C1-C6) alkylsulfanyl halo (C1-C6) alkyl group;
      (a30) a (C1-C6) alkylsulfinyl (C1-C6) alkyl group;
      (a31) a halo (C1-C6) alkylsulfinyl (C1-C6) alkyl group;
      (a32) a (C1-C6) alkylsulfinyl halo (C1-C6) alkyl group;
      (a33) a (C1-C6) alkylsulfonyl (C1-C6) alkyl group;
      (a34) a halo (C1-C6) alkylsulfonyl (C1-C6) alkyl group;
      (a35) a (C1-C6) alkylsulfonyl halo (C1-C6) alkyl group;
      (a36) a (C1-C6) alkoxy (C2-C6) alkenyl group;
      (a37) a (C1-C6) alkoxy (C2-C6) alkynyl group;
      (a38) a (C1-C10) alkoxy group;
      (a39) a halo (C1-C10) alkoxy group;
      (a40) a (C2-C6) alkenyloxy group;
      (a41) a halo (C2-C6) alkenyloxy group;
      (a42) a (C2-C6) alkynyloxy group;
      (a43) a halo (C2-C6) alkynyloxy group;
      (a44) a (C3-C7) cycloalkoxy group;
      (a45) a (C3-C6) cycloalkyl (C1-C6) alkoxy group;
      (a46) a (C1-C6) alkoxy (C1-C6) alkoxy group;
      (a47) a (C1-C6) alkylcarbonyloxy group;
      (a48) a (C3-C6) cycloalkylcarbonyloxy group;
      (a49) a (C3-C6) cycloalkenyl group;
      (a50) a (C1-C6) alkylsulfanyl group;
      (a51) a halo (C1-C6) alkylsulfanyl group;
      (a52) a (C1-C6) alkylsulfinyl group;
      (a53) a halo (C1-C6) alkylsulfinyl group;
      (a54) a (C1-C6) alkylsulfonyl group;
      (a55) a halo (C1-C6) alkylsulfonyl group;
      (a56) a Z group;
      (a57) a Z (C1-C6) alkyl group;
      (a58) a Z (C1-C6) alkoxy group;
      (a59) a Z halo (C1-C6) alkoxy group;
      (a60) a Z (C2-C6) alkenyl group;
      (a61) a Z halo (C2-C6) alkenyl group;
      (a62) a Z (C2-C6) alkynyl group;
      (a63) a Z halo (C2-C6) alkynyl group;
      (a64) a Z (C3-C6) cycloalkyl group;
      (a65) a Z halo (C3-C6) cycloalkyl group;
      (a66) a Z carbonyloxy group;
      (a67) a Z (C1-C6) alkylsulfanyl group;
      (a68) an N-mono-Z (C1-C6) alkylamino group;
      (a69) an N-(C1-C6) alkyl-N-Z (C1-C6) alkylamino group;
      (a70) an N-Z (C1-C6) alkyl-N-Z (C1-C6) alkylamino group;
      (a71) a Z oxy group;
      (a72) a Z sulfonylamino group;
      (a73) a Z sulfanyl group;
      (a74) a Z sulfinyl group;
      (a75) a Z sulfonyl group;
      (a76) a Z carbonylamino group;
      (a77) an N-((C1-C6) alkyl)-N-(Z carbonyl)amino group;
      (a78) an N-mono-(C1-C6) alkoxycarbonylamino group;
      (a79) an N-((C1-C6) alkoxycarbonyl) -N-((C1-C6) alkyl) amino group;
      (a80) an N-(C1-C6) alkyl-N-Z aminocarbonyl group;
      (a81) a Z carbonyl group;
      (a82) a Z (C1-C6) alkylcarbonylamino group;
      (a83) a Z amino group;
      (a84) an amino group;
      (a85) an N-mono-(C1-C6) alkylamino group;
      (a86) an N-(C1-C6) alkyl-N-(C1-C6) alkylamino group;
      (a87) an N-mono-(C2-C6) alkenylamino group;
      (a88) an N-(C2-C6) alkenyl-N-(C2-C6) alkenylamino group;
      (a89) an N-mono-(C2-C6) alkynylamino group;
      (a90) an N-(C2-C6) alkynyl-N-(C2-C6) alkynylamino group;
      (a91) a (C1-C6) alkylcarbonylamino group;
      (a92) a (C3-C6) cycloalkylcarbonylamino group;
      (a93) an aminosulfanyl group;
      (a94) an aminosulfinyl group;
      (a95) an aminosulfonyl group;
      (a96) an N-mono-(C1-C6) alkylaminosulfanyl group;
      (a97) an N-(C1-C6) alkyl-N-(C1-C6) alkylaminosulfanyl group;
      (a98) an N-mono-(C1-C6) alkylaminosulfinyl group;
      (a99) an N-(C1-C6) alkyl-N-(C1-C6) alkylaminosulfinyl group;
      (a100) an N-mono-(C1-C6) alkylaminosulfonyl group;
      (a101) an N-(C1-C6) alkyl-N-(C1-C6) alkylaminosulfonyl group;
      (a102) an aminocarbonyl group;
      (a103) an N-mono-(C1-C6) alkylaminocarbonyl group;
      (a104) an N-(C1-C6) alkyl-N-(C1-C6) alkylaminocarbonyl group;
      (a105) a (C1-C6) alkylcarbonyl group;
      (a106) a (C3-C6) cycloalkylcarbonyl group;
      (a107) a (C1-C6) alkoxycarbonyl group;
      (a108) an aminooxy group;
      (a109) a hydroxyamino group;
      (a110) a (C3-C6) cycloalkoxycarbonyl group;
      (a111) a formyl group;
      (a112) a carboxy group;
      (a113) an oxido group;
      (a114) a (C1-C12) alkylsilyl group;
      (a115) a halo (C3-C6) cycloalkoxy group;
      (a116) a Z halo (C1-C6) alkyl group;
      (a117) an N-Z aminosulfonyl group;
      (a118) a Z,Z (C1-C6) alkoxy group;
      (a119) a (C1-C6) alkoxy (C1-C6) alkoxy (C1-C6) alkoxy group;
      (a120) an N-((C1-C6) alkoxycarbonyl)-N-Z amino group;
      (a121) a (C3-C6) cycloalkoxy group;
      (a122) a halo (C1-C6) alkylcarbonylamino group;
      (a123) a (C3-C6) cycloalkylamino group;
      (a124) a Z aminocarbonyl group;
      (a125) a (C3-C6) cycloalkylaminocarbonyl group; or
      (a126) a group represented by the following formula A1, A2, A3, A4 or A5 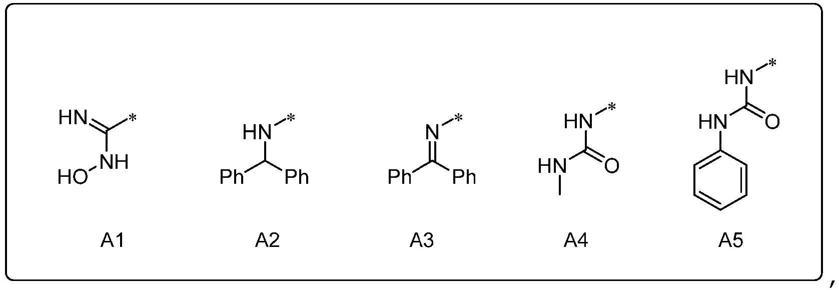
   Z represents
      (b1) a phenyl group;
      (b2) a substituted phenyl group having, on a ring, 1 to 5 substituents independently selected from the group consisting of a halogen atom, a nitro group, a cyano group, a hydroxy group, an amino group, a (C1-C6) alkyl group, a halo (C1-C6) alkyl group, a (C1-C6) alkoxy group, a halo (C1-C6) alkoxy group, a (C2-C6) alkenyl group, a halo (C2-C6) alkenyl group, a (C2-C6) alkynyl group, a (C3-C6) cycloalkyl group, a (C2-C6) alkenyloxy group, a halo (C2-C6) alkenyloxy group, a (C1-C6) alkylsulfanyl group, a halo (C1-C6) alkylsulfanyl group, a (C1-C6) alkylsulfonyloxy group, a halo (C1-C6) alkylsulfonyloxy group, a (C1-C6) alkylsulfinyl group, a halo (C1-C6) alkylsulfinyl group, a (C1-C6) alkylsulfonyl group, a halo (C1-C6) alkylsulfonyl group, a (C2-C6) alkenylsulfanyl group, a halo (C2-C6) alkenylsulfanyl group, a (C2-C6) alkenylsulfinyl group, a halo (C2-C6) alkenylsulfinyl group, a (C2-C6) alkenylsulfonyl group, a halo (C2-C6) alkenylsulfonyl group, an N-mono-(C1-C6) alkylamino group, an N,N-di-(C1-C6) alkylamino group in which the alkyl groups may be the same or different, a (C1-C6) alkylsulfonylamino group, a halo (C1-C6) alkylsulfonylamino group, a (C1-C6) alkylcarbonyl group, a formyl group, a halo (C1-C6) alkylcarbonyl group, a hydroxy (C1-C6) alkyl group, a (C1-C6) alkoxycarbonyl group, an aminocarbonyl group, a (C1-C12) alkylsilyl group, a phenoxy group and a phenyl group;
      (b3) a heterocyclic group;
      (b4) a heterocyclic group having, on a ring, 1 to 3 substituents independently selected from the group consisting of a halogen atom, a hydroxy group, a (C1-C6) alkyl group, a halo (C1-C6) alkyl group, a (C1-C6) alkoxy group, a halo (C1-C6) alkoxy group, a (C1-C6) alkylsulfanyl group, a halo (C1-C6) alkylsulfanyl group, a (C1-C6) alkylsulfinyl group, a halo (C1-C6) alkylsulfinyl group, a (C1-C6) alkylsulfonyl group, a halo (C1-C6) alkylsulfonyl group and a (C1-C6) alkoxycarbonyl group;
      (b5) a naphthyl group;
      (b6) a dihydroindenyl group; or
      (b7) an indenyl group,
   R² and R³ may be the same or different and each represents
      (c1) a (C1-C6) alkyl group; or
      (c2) a halogen atom,
   R₄ represents
      (d1) a hydrogen atom;
      (d2) a (C1-C6) alkyl group;
      (d3) a halo (C1-C6) alkyl group;
      (d4) a (C1-C6) alkoxy group;
      (d5) a halo (C1-C6) alkoxy group;
      (d6) a (C2-C6) alkenyl group;
      (d7) a (C2-C6) alkynyl group;
      (d8) a (C3-C6) cycloalkyl group;
      (d9) a (C3-C6) cycloalkyl (C1-C6) alkyl group;
      (d10) a (C1-C6) alkoxy (C1-C6) alkyl group;
      (d11) a (C1-C6) alkylcarbonyl group;
      (d12) a (C1-C6) alkoxycarbonyl group;
      (d13) a (C3-C6) cycloalkylcarbonyl group;
      (d14) a (C1-C6) alkoxy (C1-C6) alkylcarbonyl group;
      (d15) a halo (C1-C6) alkylcarbonyl group;
      (d16) a (C1-C6) alkoxycarbonyl (C1-C6) alkyl group;
      (d17) an amino (C1-C6) alkylcarbonyl group;
      (d18) an N-mono-(C1-C6) alkylamino (C1-C6) alkylcarbonyl group;
      (d19) an N,N-di-(C1-C6) alkylamino (C1-C6) alkylcarbonyl group;
      (d20) a (C1-C6) alkylsulfanyl group;
      (d21) a halo (C1-C6) alkylsulfanyl group;
      (d22) a (C1-C6) alkylsulfinyl group;
      (d23) a halo (C1-C6) alkylsulfinyl group;
      (d24) a (C1-C6) alkylsulfonyl group;
      (d25) a halo (C1-C6) alkylsulfonyl group;
      (d26) a (C3-C6) cycloalkylsulfanyl group;
      (d27) a (C3-C6) cycloalkylsulfinyl group;
      (d28) a (C3-C6) cycloalkylsulfonyl group;
      (d29) an amino (C1-C6) alkyl group;
      (d30) an N-mono-(C1-C6) alkylamino (C1-C6) alkyl group;
      (d31) an N,N-di-(C1-C6) alkylamino (C1-C6) alkyl group;
      (d32) a Z (C1-C6) alkyl group;
      (d33) a Z group;
      (d34) a Z oxy group;
      (d35) a Z carbonyl group;
      (d36) a (C2-C6) alkenyloxycarbonyl group;
      (d37) a (C2-C6) alkynyloxycarbonyl group;
      (d38) a (C2-C6) alkenylcarbonyl group;
      (d39) an amino (C1-C6) alkylcarbonyl group;
      (d40) an N-mono-(C1-C6) alkylamino (C1-C6) alkylcarbonyl group;
      (d41) an N,N-di-(C1-C6) alkylamino (C1-C6) alkylcarbonyl group;
      (d42) a Z (C1-C6) alkylcarbonyl group; or
      (d43) a Z (C1-C6) alkoxycarbonyl group,
   two R¹ may be bonded to each other to form a ring structure represented by the following formula R¹⁻¹, R¹⁻², R¹⁻³ or R¹⁻⁴, each containing the carbon atoms to which they are bonded 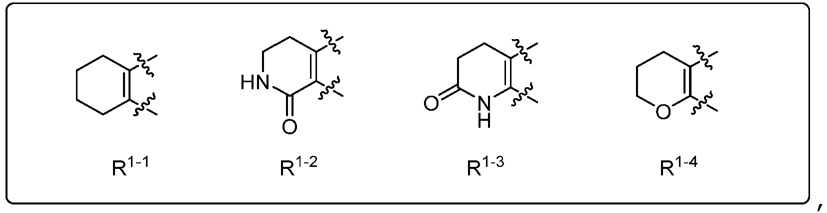
   two R⁵ may be bonded to each other to form a ring structure represented by the following formula R⁵⁻¹ or R⁵⁻², each containing the carbon atoms to which they are bonded 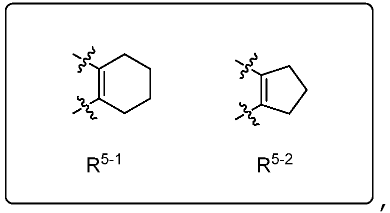
   R⁴ and R⁵ may be bonded to each other to form a ring structure containing a partial structure represented by the following formula R⁴⁻⁵⁻¹ or R⁴⁻⁵⁻² and the carbon atom and the nitrogen atom to which they are bonded 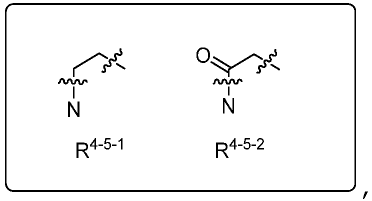
   X represents an oxygen atom or a sulfur atom,
   Ar¹ represents phenyl, pyridyl, pyridazinyl, pyrimidinyl, pyrazinyl, thienyl, thiazolyl or a group represented by the following formula #1, #2 or #3 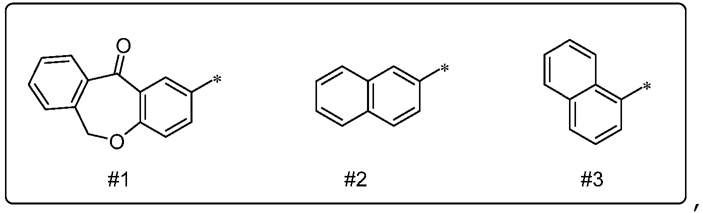
   Ar² represents a group represented by the following formula Ar²⁻¹, Ar²⁻², Ar²⁻³, Ar²⁻⁴, Ar²⁻⁵, Ar²⁻⁶, Ar²⁻⁷, Ar²⁻⁸, Ar²⁻⁹, Ar²⁻¹⁰, Ar²⁻¹¹, Ar²⁻¹², Ar²⁻¹³, Ar²⁻¹⁴, Ar²⁻¹⁵, Ar²⁻¹⁶, Ar²⁻¹⁷, Ar²⁻¹⁸, Ar²⁻¹⁹, Ar²⁻²⁰, Ar²⁻²¹, Ar²⁻²² or Ar²⁻²³ 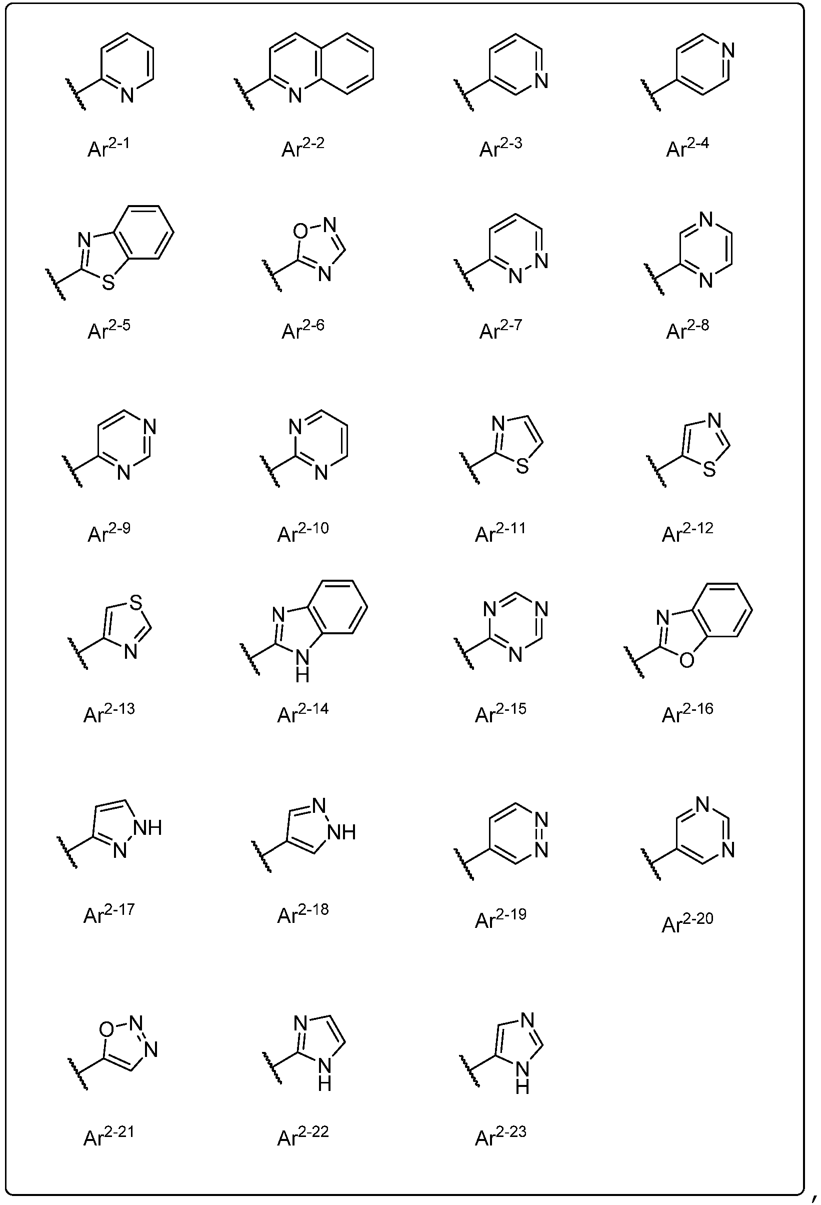
   m represents 1, 2, 3, 4 or 5, and
   n represents 1, 2, 3, 4 or 5,
      or a salt thereof,
      provided that a compound represented by the following formula DC1 and a salt thereof are excluded 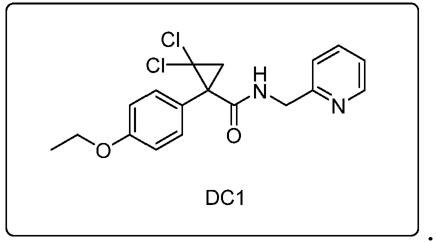
[2] The compound or salt thereof of [1], wherein
   R¹ and R⁵ may be the same or different and each is
      (a1) a hydrogen atom;
      (a2) a halogen atom;
      (a3) a cyano group;
      (a4) a nitro group;
      (a5) a hydroxy group;
      (a7) a (C1-C6) alkyl group;
      (a8) a halo (C1-C6) alkyl group;
      (a9) a (C2-C6) alkenyl group;
      (a10) a halo (C2-C6) alkenyl group;
      (a11) a (C2-C6) alkynyl group;
      (a12) a halo (C2-C6) alkynyl group;
      (a13) a (C3-C6) cycloalkyl group;
      (a14) a (C3-C6) cycloalkenyl group;
      (a15) a halo (C3-C6) cycloalkyl group;
      (a16) a (C3-C6) cycloalkyl (C1-C6) alkyl group;
      (a17) a (C3-C6) cycloalkyl (C3-C6) cycloalkyl group;
      (a22) a hydroxy (C1-C6) alkyl group;
      (a23) a (C1-C6) alkoxy (C1-C6) alkyl group;
      (a24) a halo (C1-C6) alkoxy (C1-C6) alkyl group;
      (a25) a (C3-C6) cycloalkoxy (C1-C6) alkyl group;
      (a26) a halo (C3-C6) cycloalkoxy (C1-C6) alkyl group;
      (a27) a (C1-C6) alkylsulfanyl (C1-C6) alkyl group;
      (a36) a (C1-C6) alkoxy (C2-C6) alkenyl group;
      (a37) a (C1-C6) alkoxy (C2-C6) alkynyl group;
      (a38) a (C1-C10) alkoxy group;
      (a39) a halo (C1-C10) alkoxy group;
      (a40) a (C2-C6) alkenyloxy group;
      (a41) a halo (C2-C6) alkenyloxy group;
      (a42) a (C2-C6) alkynyloxy group;
      (a43) a halo (C2-C6) alkynyloxy group;
      (a44) a (C3-C7) cycloalkoxy group;
      (a45) a (C3-C6) cycloalkyl (C1-C6) alkoxy group;
      (a46) a (C1-C6) alkoxy (C1-C6) alkoxy group;
      (a47) a (C1-C6) alkylcarbonyloxy group;
      (a48) a (C3-C6) cycloalkylcarbonyloxy group;
      (a49) a (C3-C6) cycloalkenyl group;
      (a50) a (C1-C6) alkylsulfanyl group;
      (a51) a halo (C1-C6) alkylsulfanyl group;
      (a52) a (C1-C6) alkylsulfinyl group;
      (a53) a halo (C1-C6) alkylsulfinyl group;
      (a54) a (C1-C6) alkylsulfonyl group;
      (a55) a halo (C1-C6) alkylsulfonyl group;
      (a56) a Z group;
      (a57) a Z (C1-C6) alkyl group;
      (a58) a Z (C1-C6) alkoxy group;
      (a59) a Z halo (C1-C6) alkoxy group;
      (a62) a Z (C2-C6) alkynyl group;
      (a66) a Z carbonyloxy group;
      (a67) a Z (C1-C6) alkylsulfanyl group;
      (a68) an N-mono-Z (C1-C6) alkylamino group;
      (a69) an N-(C1-C6) alkyl-N-Z (C1-C6) alkylamino group;
      (a70) an N-Z (C1-C6) alkyl-N-Z (C1-C6) alkylamino group;
      (a71) a Z oxy group;
      (a72) a Z sulfonylamino group;
      (a73) a Z sulfanyl group;
      (a74) a Z sulfinyl group;
      (a75) a Z sulfonyl group;
      (a76) a Z carbonylamino group;
      (a77) an N-((C1-C6) alkyl)-N-(Z carbonyl)amino group;
      (a78) an N-mono-(C1-C6) alkoxycarbonylamino group;
      (a79) an N-((C1-C6) alkoxycarbonyl) -N-((C1-C6) alkyl) amino group;
      (a80) an N-(C1-C6) alkyl-N-Z aminocarbonyl group;
      (a81) a Z carbonyl group;
      (a82) a Z (C1-C6) alkylcarbonylamino group;
      (a83) a Z amino group;
      (a84) an amino group;
      (a85) an N-mono-(C1-C6) alkylamino group;
      (a86) an N-(C1-C6) alkyl-N-(C1-C6) alkylamino group;
      (a87) an N-mono-(C2-C6) alkenylamino group;
      (a88) an N-(C2-C6) alkenyl-N-(C2-C6) alkenylamino group;
      (a89) an N-mono-(C2-C6) alkynylamino group;
      (a90) an N-(C2-C6) alkynyl-N-(C2-C6) alkynylamino group;
      (a91) a (C1-C6) alkylcarbonylamino group;
      (a92) a (C3-C6) cycloalkylcarbonylamino group;
      (a93) an aminosulfanyl group;
      (a94) an aminosulfinyl group;
      (a95) an aminosulfonyl group;
      (a96) an N-mono-(C1-C6) alkylaminosulfanyl group;
      (a97) an N-(C1-C6) alkyl-N-(C1-C6) alkylaminosulfanyl group;
      (a98) an N-mono-(C1-C6) alkylaminosulfinyl group;
      (a99) an N-(C1-C6) alkyl-N-(C1-C6) alkylaminosulfinyl group;
      (a100) an N-mono-(C1-C6) alkylaminosulfonyl group;
      (a101) an N-(C1-C6) alkyl-N-(C1-C6) alkylaminosulfonyl group;
      (a102) an aminocarbonyl group;
      (a103) an N-mono-(C1-C6) alkylaminocarbonyl group;
      (a104) an N-(C1-C6) alkyl-N-(C1-C6) alkylaminocarbonyl group;
      (a105) a (C1-C6) alkylcarbonyl group;
      (a106) a (C3-C6) cycloalkylcarbonyl group;
      (a107) a (C1-C6) alkoxycarbonyl group;
      (a108) an aminooxy group;
      (a109) a hydroxyamino group;
      (a110) a (C3-C6) cycloalkoxycarbonyl group;
      (a111) a formyl group;
      (a112) a carboxy group;
      (a113) an oxido group;
      (a114) a (C1-C12) alkylsilyl group;
      (a120) an N-((C1-C6) alkoxycarbonyl)-N-Z amino group;
      (a121) a (C3-C6) cycloalkoxy group;
      (a122) a halo (C1-C6) alkylcarbonylamino group;
      (a123) a (C3-C6) cycloalkylamino group;
      (a124) a Z aminocarbonyl group;
      (a125) a (C3-C6) cycloalkylaminocarbonyl group; or
      (a126) a group represented by the following formula A1, A2, A3, A4 or A5 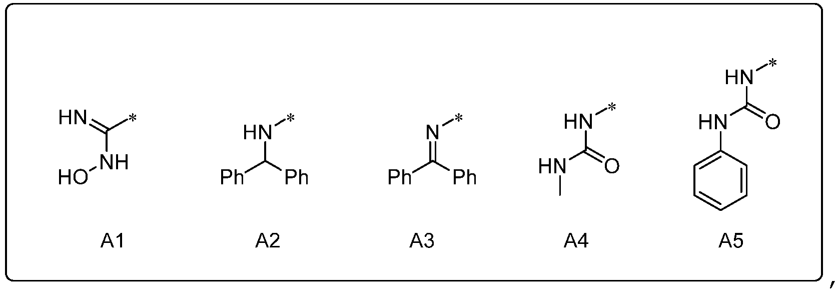
   R² and R³ may be the same or different and each is a halogen atom,
   R⁴ is
      (d1) a hydrogen atom;
      (d2) a (C1-C6) alkyl group;
      (d4) a (C1-C6) alkoxy group;
      (d6) a (C2-C6) alkenyl group;
      (d9) a (C3-C6) cycloalkyl (C1-C6) alkyl group;
      (d10) a (C1-C6) alkoxy (C1-C6) alkyl group;
      (d11) a (C1-C6) alkylcarbonyl group;
      (d12) a (C1-C6) alkoxycarbonyl group;
      (d14) a (C1-C6) alkoxy (C1-C6) alkylcarbonyl group;
      (d16) a (C1-C6) alkoxycarbonyl (C1-C6) alkyl group;
      (d20) a (C1-C6) alkylsulfanyl group;
      (d22) a (C1-C6) alkylsulfinyl group;
      (d24) a (C1-C6) alkylsulfonyl group;
      (d28) a (C3-C6) cycloalkylsulfonyl group;
      (d29) an amino (C1-C6) alkyl group;
      (d30) an N-mono-(C1-C6) alkylamino (C1-C6) alkyl group;
      (d31) an N,N-di-(C1-C6) alkylamino (C1-C6) alkyl group;
      (d32) a Z (C1-C6) alkyl group;
      (d33) a Z group;
      (d35) a Z carbonyl group;
      (d36) a (C2-C6) alkenyloxycarbonyl group;
      (d37) a (C2-C6) alkynyloxycarbonyl group;
      (d38) a (C2-C6) alkenylcarbonyl group;
      (d39) an amino (C1-C6) alkylcarbonyl group;
      (d40) an N-mono-(C1-C6) alkylamino (C1-C6) alkylcarbonyl group; or
      (d41) an N,N-di-(C1-C6) alkylamino (C1-C6) alkylcarbonyl group,
   R⁴ and R⁵ may be bonded to each other to form a ring structure containing a partial structure represented by the following formula R⁴⁻⁵⁻¹ and the carbon atom and the nitrogen atom to which they are bonded 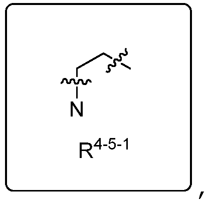 and
   Ar² is a group represented by the following formula Ar²⁻¹, Ar²⁻², Ar²⁻³, Ar²⁻⁴, Ar²⁻⁵, Ar²⁻⁶, Ar²⁻⁷, Ar²⁻⁸, Ar²⁻⁹, Ar²⁻¹⁰, Ar²⁻¹¹, Ar²⁻¹², Ar²⁻¹³, Ar²⁻¹⁴, Ar²⁻¹⁷, Ar²⁻¹⁸, Ar²⁻¹⁹ or Ar²⁻²⁰ 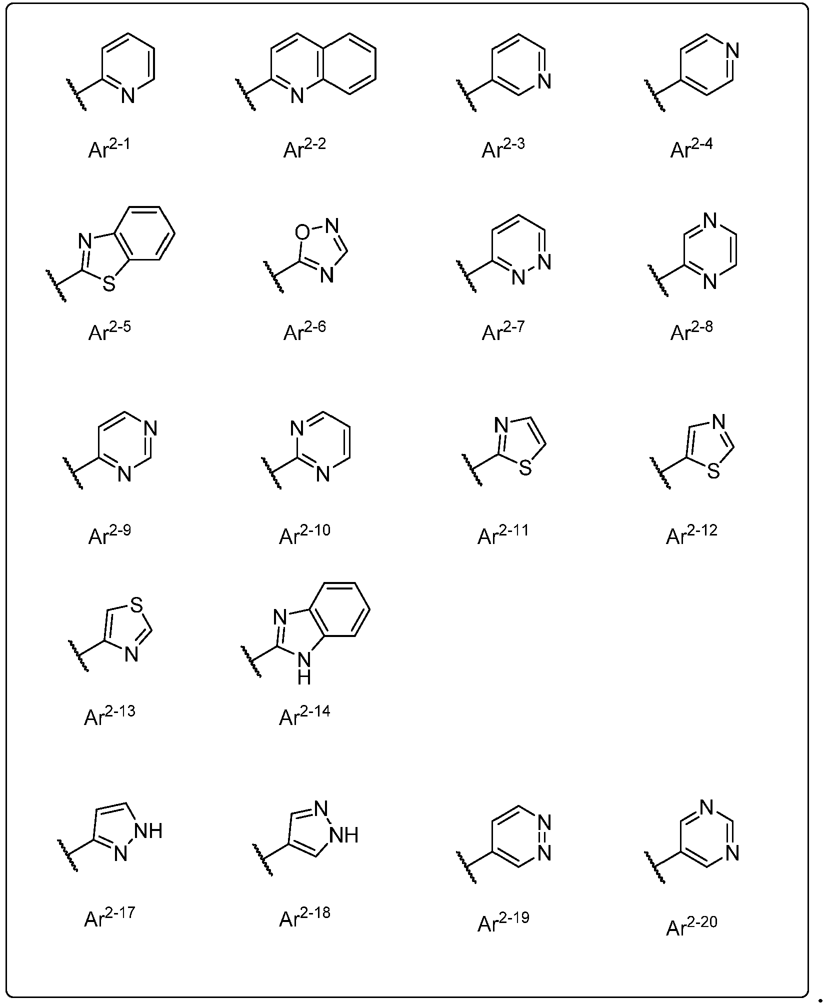
[3] The compound or salt thereof of [2], wherein
   R¹ and R⁵ may be the same or different and each is
      (a1) a hydrogen atom;
      (a2) a halogen atom;
      (a3) a cyano group;
      (a4) a nitro group;
      (a5) a hydroxy group;
      (a7) a (C1-C6) alkyl group;
      (a8) a halo (C1-C6) alkyl group;
      (a9) a (C2-C6) alkenyl group;
      (a11) a (C2-C6) alkynyl group;
      (a13) a (C3-C6) cycloalkyl group;
      (a14) a (C3-C6) cycloalkenyl group;
      (a22) a hydroxy (C1-C6) alkyl group;
      (a36) a (C1-C6) alkoxy (C2-C6) alkenyl group;
      (a38) a (C1-C10) alkoxy group;
      (a39) a halo (C1-C10) alkoxy group;
      (a40) a (C2-C6) alkenyloxy group;
      (a44) a (C3-C7) cycloalkoxy group;
      (a45) a (C3-C6) cycloalkyl (C1-C6) alkoxy group;
      (a46) a (C1-C6) alkoxy (C1-C6) alkoxy group;
      (a47) a (C1-C6) alkylcarbonyloxy group;
      (a48) a (C3-C6) cycloalkylcarbonyloxy group;
      (a50) a (C1-C6) alkylsulfanyl group;
      (a51) a halo (C1-C6) alkylsulfanyl group;
      (a52) a (C1-C6) alkylsulfinyl group;
      (a54) a (C1-C6) alkylsulfonyl group;
      (a56) a Z group;
      (a57) a Z (C1-C6) alkyl group;
      (a58) a Z (C1-C6) alkoxy group;
      (a62) a Z (C2-C6) alkynyl group;
      (a66) a Z carbonyloxy group;
      (a67) a Z (C1-C6) alkylsulfanyl group;
      (a68) an N-mono-Z (C1-C6) alkylamino group;
      (a69) an N-(C1-C6) alkyl-N-Z (C1-C6) alkylamino group;
      (a70) an N-Z (C1-C6) alkyl-N-Z (C1-C6) alkylamino group;
      (a71) a Z oxy group;
      (a72) a Z sulfonylamino group;
      (a76) a Z carbonylamino group;
      (a77) an N-((C1-C6) alkyl)-N-(Z carbonyl)amino group;
      (a78) an N-mono-(C1-C6) alkoxycarbonylamino group;
      (a79) an N-((C1-C6) alkoxycarbonyl) -N-((C1-C6) alkyl) amino group;
      (a81) a Z carbonyl group;
      (a82) a Z (C1-C6) alkylcarbonylamino group;
      (a83) a Z amino group;
      (a84) an amino group;
      (a85) an N-mono-(C1-C6) alkylamino group;
      (a86) an N-(C1-C6) alkyl-N-(C1-C6) alkylamino group;
      (a87) an N-mono-(C2-C6) alkenylamino group;
      (a91) a (C1-C6) alkylcarbonylamino group;
      (a92) a (C3-C6) cycloalkylcarbonylamino group;
      (a95) an aminosulfonyl group;
      (a100) an N-mono-(C1-C6) alkylaminosulfonyl group;
      (a101) an N-(C1-C6) alkyl-N-(C1-C6) alkylaminosulfonyl group;
      (a102) an aminocarbonyl group;
      (a103) an N-mono-(C1-C6) alkylaminocarbonyl group;
      (a104) an N-(C1-C6) alkyl-N-(C1-C6) alkylaminocarbonyl group;
      (a105) a (C1-C6) alkylcarbonyl group;
      (a106) a (C3-C6) cycloalkylcarbonyl group;
      (a107) a (C1-C6) alkoxycarbonyl group;
      (a111) a formyl group;
      (a112) a carboxy group;
      (a113) an oxido group;
      (a114) a (C1-C12) alkylsilyl group;
      (a120) an N-((C1-C6) alkoxycarbonyl)-N-Z amino group;
      (a121) a (C3-C6) cycloalkoxy group;
      (a122) a halo (C1-C6) alkylcarbonylamino group;
      (a123) a (C3-C6) cycloalkylamino group;
      (a124) a Z aminocarbonyl group;
      (a125) a (C3-C6) cycloalkylaminocarbonyl group; or
      (a126) a group represented by the following formula A1, A2, A3, A4 or A5 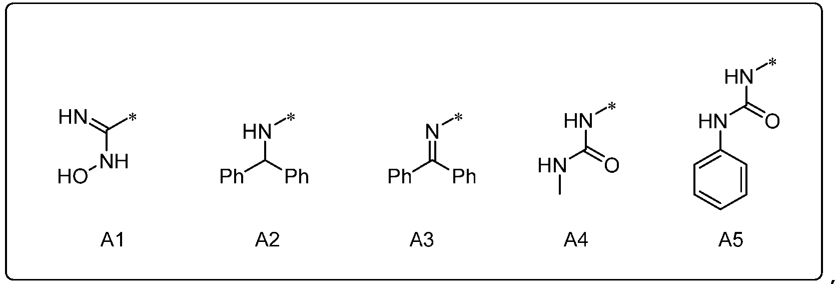
   R⁴ is
      (d1) a hydrogen atom;
      (d2) a (C1-C6) alkyl group;
      (d4) a (C1-C6) alkoxy group;
      (d6) a (C2-C6) alkenyl group;
      (d10) a (C1-C6) alkoxy (C1-C6) alkyl group;
      (d11) a (C1-C6) alkylcarbonyl group;
      (d12) a (C1-C6) alkoxycarbonyl group;
      (d14) a (C1-C6) alkoxy (C1-C6) alkylcarbonyl group;
      (d16) a (C1-C6) alkoxycarbonyl (C1-C6) alkyl group;
      (d22) a (C1-C6) alkylsulfinyl group;
      (d24) a (C1-C6) alkylsulfonyl group;
      (d28) a (C3-C6) cycloalkylsulfonyl group;
      (d31) an N,N-di-(C1-C6) alkylamino (C1-C6) alkyl group;
      (d32) a Z (C1-C6) alkyl group;
      (d36) a (C2-C6) alkenyloxycarbonyl group;
      (d38) a (C2-C6) alkenylcarbonyl group;
      (d39) an amino (C1-C6) alkylcarbonyl group; or
      (d41) an N,N-di-(C1-C6) alkylamino (C1-C6) alkylcarbonyl group, and
   X is an oxygen atom.
[4] An agricultural and horticultural fungicide comprising the compound or salt thereof of the above [1] as an active ingredient,
[5] An agricultural and horticultural fungicide comprising the compound or salt thereof of the above [2] as an active ingredient,
[6] An agricultural and horticultural fungicide comprising the compound or salt thereof of the above [3] as an active ingredient,
[7] A method for controlling an agricultural and horticultural disease, comprising treating a plant or soil with an effective amount of the agricultural and horticultural fungicide of the above [4],
[8] A method for controlling an agricultural and horticultural disease, comprising treating a plant or soil with an effective amount of the agricultural and horticultural fungicide of the above [5],
[9] A method for controlling an agricultural and horticultural disease, comprising treating a plant or soil with an effective amount of the agricultural and horticultural fungicide of the above [6],
[10] Use of the compound or salt thereof of the above [1] as a fungicide,
[11] Use of the compound or salt thereof of the above [2] as a fungicide, and
[12] Use of the compound or salt thereof of the above [3] as a fungicide.

### [Advantageous Effects of Invention]

The amide compound of the present invention or salts thereof has an excellent control effect as an agricultural and horticultural fungicide. In particular, the amide compound of the present invention or salts thereof is highly effective against Chinese cabbage alternaria leaf spot, tomato late blight, cucumber gray mold, cucumber anthracnose, rice blast, apple scab, barley powdery mildew, wheat leaf blotch, cucumber sclerotinia rot, ring rot, grape ripe rot, soybean sudden death syndrome, fusarium ear blight of barley, wheat **etc.,** Brassicaceae sclerotinia rot, and rice brown spot.

### [Description of Embodiments]

In the definitions of the general formula [I] representing the amide compound of the present invention or salts thereof, "halo" refers to a "halogen atom" and represents a chlorine atom, a bromine atom, an iodine atom or a fluorine atom.

The "(C1-C6) alkyl group" refers to a linear or branched alkyl group having 1 to 6 carbon atoms, for example, a methyl group, an ethyl group, a normal propyl group, an isopropyl group, a normal butyl group, an isobutyl group, a secondary butyl group, a tertiary butyl group, a normal pentyl group, an isopentyl group, a tertiary pentyl group, a neopentyl group, a 2,3-dimethylpropyl group, a 1-ethylpropyl group, a 1-methylbutyl group, a 2-methylbutyl group, a normal hexyl group, an isohexyl group, a 2-hexyl group, a 3-hexyl group, a 2-methylpentyl group, a 3-methylpentyl group, a 1,1,2-trimethylpropyl group, a 3,3-dimethylbutyl group, or the like. The "(C2-C6) alkenyl group" refers to a linear or branched alkenyl group having 2 to 6 carbon atoms, for example, a vinyl group, an allyl group, an isopropenyl group, a 1-butenyl group, a 2-butenyl group, a 2-methyl-2-propenyl group, a 1-methyl-2-propenyl group, a 2-methyl-1-propenyl group, a pentenyl group, a 1-hexenyl group, a 3,3-dimethyl-1-butenyl group, or the like. The "(C2-C6) alkynyl group" refers to a linear or branched alkynyl group having 2 to 6 carbon atoms, for example, an ethynyl group, a 1-propynyl group, a 2-propynyl group, a 1-butynyl group, a 2-butynyl group, a 3-butynyl group, a 3-methyl-1-propynyl group, a 2-methyl-3-propynyl group, a pentynyl group, a 1-hexynyl group, a 3-methyl-1-butynyl group, a 3,3-dimethyl-1-butynyl group, or the like.

The "(C3-C6) cycloalkyl group" refers to a cyclic alkyl group having 3 to 6 carbon atoms, for example, a cyclopropyl group, a cyclobutyl group, a cyclopentyl group, a cyclohexyl group, or the like. The "(C3-C6) cycloalkenyl group" refers to a cyclic alkenyl group having 3 to 6 atoms, for example, a cyclopropenyl group, a cyclobutenyl group, a cyclopentenyl group, a cyclohexyl group, or the like. The "(C1-C6) alkoxy group" refers to a linear or branched alkoxy group having 1 to 6 carbon atoms, for example, a methoxy group, an ethoxy group, a normal propoxy group, an isopropoxy group, a normal butoxy group, a secondary butoxy group, a tertiary butoxy group, a normal pentyloxy group, an isopentyloxy group, a tertiary pentyloxy group, a neopentyloxy group, a 2,3-dimethylpropyloxy group, a 1-ethylpropyloxy group, a 1-methylbutyloxy group, a normal hexyloxy group, an isohexyloxy group, a 1,1,2-trimethylpropyloxy group, or the like. The "(C3-C6) cycloalkoxy group" refers to a cyclic alkoxy group having 1 to 6 carbon atoms, for example, a cyclopropyloxy group, a cyclobutyloxy group, a cyclopropyloxy group, a cyclobutyloxy group, or the like.

The "(C2-C6) alkenyloxy group" refers to a linear or branched alkenyloxy group having 2 to 6 carbon atoms, for example, a propenyloxy group, a butenyloxy group, a pentenyloxy group, a hexenyloxy group, or the like. The "(C2-C6) alkynyloxy group" refers to a linear or branched alkynyloxy group having 2 to 6 carbon atoms, for example, a propynyloxy group, a butynyloxy group, a pentynyloxy group, a hexynyloxy group, or the like.

The "(C1-C6) alkylsulfanyl group" refers to a linear or branched alkylsulfanyl group having 1 to 6 carbon atoms, for example, a methylsulfanyl group, an ethylsulfanyl group, a normal a propylsulfanyl group, an isopropylsulfanyl group, a normal butylsulfanyl group, a secondary butylsulfanyl group, a tertiary butylsulfanyl group, a normal pentylsulfanyl group, an isopentylsulfanyl group, a tertiary pentylsulfanyl group, a neopentylsulfanyl group, a 2,3-dimethylpropylsulfanyl group, a 1-ethylpropylsulfanyl group, a 1-methylbutylsulfanyl group, a normal hexylsulfanyl group, an isohexylsulfanyl group, a 1,1,2-trimethylpropylsulfanyl group, or the like. The "(C1-C6) alkylsulfinyl group" refers to a linear or branched alkylsulfinyl group having 1 to 6 carbon atoms, for example, a methylsulfinyl group, an ethylsulfinyl group, a normal propylsulfinyl group, an isopropylsulfinyl group, a normal butylsulfinyl group, a secondary butylsulfinyl group, a tertiary butylsulfinyl group, a normal pentylsulfinyl group, an isopentylsulfinyl group, a tertiary pentylsulfinyl group, a neopentylsulfinyl group, a 2,3-dimethylpropylsulfinyl group, a 1-ethylpropylsulfinyl group, a 1-methylbutylsulfinyl group, a normal hexylsulfinyl group, an isohexylsulfinyl group, a 1,1,2-trimethylpropylsulfinyl group, or the like. The "(C1-C6) alkylsulfonyl group" refers to a linear or branched alkylsulfonyl group having 1 to 6 carbon atoms, for example, a methylsulfonyl group, an ethylsulfonyl group, a normal propylsulfonyl group, an isopropylsulfonyl group, a normal butylsulfonyl group, a secondary butylsulfonyl group, a tertiary butylsulfonyl group, a normal pentylsulfonyl group, an isopentylsulfonyl group, a tertiary pentylsulfonyl group, a neopentylsulfonyl group, a 2,3-dimethylpropylsulfonyl group, a 1-ethylpropylsulfonyl group, a 1-methylbutylsulfonyl group, a normal hexylsulfonyl group, an isohexylsulfonyl group, a 1,1,2-trimethylpropylsulfonyl group, or the like.

The "hydroxy (C1-C6) alkyl group" refers to a linear or branched alkyl group having 1 to 6 carbon atoms, for example, a 2-hydroxypropyl group, a 2-hydroxy-2-methyl-propyl group, or the like.

The "(C1-C6) alkylcarbonyl group" refers to an alkylcarbonyl group having 2 to 7 carbon atoms, such as an alkylcarbonyl group having the above-mentioned (C1-C6) alkyl group, for example, an acetyl group, a propanoyl group, a butanoyl group, a 2-methylpropanoyl group, a pentanoyl group, a 2-methylbutanoyl group, a 3-methylbutanoyl group, a pivaloyl group, a hexanoyl group, or the like.

The "(C1-C6) alkoxycarbonyl group" refers to an alkoxycarbonyl group having 2 to 7 carbon atoms, such as an alkoxycarbonyl group having the above-mentioned (C1-C6) alkoxy group, for example, a methoxycarbonyl group, an ethoxycarbonyl group, a normal propoxycarbonyl group, an isopropoxycarbonyl group, a normal butoxycarbonyl group, an isobutoxycarbonyl group, a secondary butoxycarbonyl group, a tertiary butoxycarbonyl group, a pentyloxycarbonyl group, or the like.

The "(C1-C6) alkylcarbonyloxy group" refers to a linear or branched alkylcarbonyloxy group having 2 to 7 carbon atoms, for example, a 1-oxoethoxy group, a 1-oxopropoxy group, or the like. The "(C3-C6) cycloalkylcarbonyloxy group" refers to a linear or branched alkylcarbonyloxy group having 4 to 7 carbon atoms, for example, a 2-cyclopropyl-1-oxoethoxy group, a 2-cyclobutyl-1-oxoethoxy group, or the like.

The above-mentioned "(C1-C6) alkyl group", "(C3-C6) cycloalkyl group", "(C1-C6) alkylsulfanyl group", "(C1-C6) alkylsulfinyl group", "(C1-C6) alkylsulfonyl group", "(C2-C6) alkenyl group", "(C2-C6) alkynyl group", "(C3-C6) cycloalkyl group", "(C1-C10) alkoxy group", "(C2-C6) alkenyloxy group" and "(C2-C6) alkynyloxy group" may be substituted with one or more halogen atoms at a substitutable position(s), and in the case where any of the above-mentioned groups is substituted with two or more halogen atoms, the halogen atoms may be the same or different.

The above-mentioned "groups substituted with one or more halogen atoms at a substitutable position (s)" are expressed as "halo (C1-C6) alkyl group", "halo (C3-C6) cycloalkyl group", "halo (C1-C6) alkylsulfanyl group", "halo (C1-C6) alkylsulfinyl group", "halo (C1-C6) alkylsulfonyl group", "halo (C2-C6) alkenyl group", "halo (C2-C6) alkynyl group", "halo (C3-C6) cycloalkyl group", "halo (C1-C10) alkoxy group", "halo (C2-C6) alkenyloxy group" and "halo (C2-C6) alkynyloxy group", respectively.

Examples of the "halo (C1-C6) alkyl group" include a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2,2-difluoroethyl group, a 2,2,2-trifluoroethyl group, a pentafluoroethyl group, a heptafluoropropyl group, and a heptafluoroisopropyl group.

Examples of the "halo (C3-C6) cycloalkyl group" include a 2-fluorocyclopropyl group, a 2-chlorocyclopropyl group, a 2-iodocyclopropyl group, a 1,2-difluorocyclopropyl group, a 2,2-difluorocyclopropyl group, a 2-chloro-3-fluorocyclopropyl group, and a 1,2,2,3,3-pentachlorocyclopropyl group.

Examples of the "halo (C1-C6) alkoxy group" include a difluoromethoxy group, a trifluoromethoxy group, a 2,2,2-trifluoroethoxy group, a 2,2,2-trichloroethoxy group, a 2,2,3,3-tetrafluoropropoxy group, and a 2,2,3,4,4,4-hexafluorobutoxy group.

Examples of the "halo (C1-C6) alkylsulfanyl" include a fluoromethylsulfanyl group, a 2-fluoroethylsulfanyl group, a 3-fluoro normal propylsulfanyl group, a (2,2-difluoro-1-methylethyl)sulfanyl group, and a (2,2,2-trichloro-1-methylethyl)sulfanyl group.

Examples of the "halo (C1-C6) alkylsulfinyl" include a fluoromethylsulfinyl group, a 2-fluoroethylsulfinyl group, a 3-fluoro normal propylsulfinyl group, a (2,2-difluoro-1-methylethyl)sulfinyl group, and a (2,2,2-trichloro-1-methylethyl)sulfinyl group.

Examples of the "halo (C1-C6) alkylsulfonyl" include a fluoromethylsulfonyl group, a 2-fluoroethylsulfonyl group, a 3-fluoro normal propylsulfonyl group, a (2,2-difluoro-1-methylethyl)sulfonyl group, and a (2,2,2-trichloro-1-methylethyl)sulfonyl group.

Examples of the "halo (C2-C6) alkenyl group" include a 2-fluorovinyl group, a 3-chloroallyl group, a 2,2-dichloro-1-methylethenyl group, and a 2,3,3-trichloro-1-methyl-2-propenyl group.

The "halo (C2-C6) alkynyl group" refers to a linear or branched alkynyl group having 2 to 6 carbon atoms, for example, a 3-chloro-2-propynyl group, a 3-chloro-1,1-dimethyl-2-propynyl group, or the like.

The "cyano (C1-C6) alkyl group" refers to a linear or branched alkyl group having 1 to 6 carbon atoms, for example, a cyanomethyl group, a 1-cyanoethyl group, a 2-cyanoethyl group, a 3-cyanopropyl group, a 2-cyanopropyl group, a 1-cyanopropyl group, a 1-cyano-1-methylethyl group, a 2-cyanobutyl group, a 2-cyanohexyl group, or the like.

The "N-mono-(C1-C6) alkylamino group" refers to an alkylamino group having a linear or branched alkyl group having 1 to 6 carbon atoms, for example, an N-methylamino group, an N-ethylamino group, an N-isopropylamino group, or the like. The "N-mono-(C2-C6) alkenylamino group" refers to an alkenylamino group having a linear or branched alkenyl group having 2 to 6 carbon atoms, for example, an N-ethenylamino group, an N-propenylamino group, an N-(1-methyl-2-propen-1-yl)amino group, or the like. The "N-mono-(C2-C6) alkynylamino group" refers to an alkynylamino group having a linear or branched alkynyl group having 2 to 6 carbon atoms, for example, an N-ethynylamino group, an N-2-propynylamino group, or the like.

The "N-(C1-C6) alkyl-N-(C1-C6) alkylamino group" refers to a dialkylamino group having 2 to 12 carbon atoms, which has a linear or branched alkyl group and having 1 to 6 carbon atoms, for example, an N,N-dimethylamino group, an N-ethyl-N-methylamino group, or the like. The "N-(C2-C6) alkenyl-N-(C2-C6) alkenylamino group" refers to a dialkenylamino group having 4 to 12 carbon atoms, which has a linear or branched alkenyl group having 2 to 6 carbon atoms, for example, an **N,N-**diethenylamino group, an N-ethenyl-N-2-propenylamino group, an N-(2-methyl-1-propenyl)-N-2-propenylamino group, or the like. The "N-(C2-C6) alkynyl-N-(C2-C6) alkynylamino group" refers to a dialkynylamino group having 5 to 13 carbon atoms, which has a linear or branched alkynyl group having 2 to 6 carbon atoms, for example, an N,N-diethynylamino group, an N-ethynyl-N-2-propynylamino group, or the like.

The "N-mono-(C1-C6) alkylaminocarbonyl group" refers to an alkylaminocarbonyl group having 2 to 7 carbon atoms, which has a linear or branched alkyl group having 1 to 6 carbon atoms, for example, an N-methylaminocarbonyl group, an **N-**ethylaminocarbonyl group, an N-normal propylaminocarbonyl group, an N-isopropylaminocarbonyl group, an N-normal butylaminocarbonyl group, an N-isobutylaminocarbonyl group, an N-secondary butylaminocarbonyl group, an N-tertiary butylaminocarbonyl group, an N-normal pentylaminocarbonyl group, an N-isopentylaminocarbonyl group, an N-tertiary pentylaminocarbonyl group, an N-neopentylaminocarbonyl group, an N-normal hexylaminocarbonyl group, an **N-**isohexylaminocarbonyl group, or the like.

"N-(C1-C6) alkyl-N-(C1-C6) alkylaminocarbonyl group" refers to a dialkylaminocarbonyl group having 3 to 13 carbon atoms, which has a linear or branched alkyl group having 1 to 6 carbon atoms, for example, an N,N-dimethylaminocarbonyl group, an N,N-diethylaminocarbonyl group, an N,N-dinormal propylaminocarbonyl group, an N,N-diisopropylaminocarbonyl group, an N,N-dinormal butylaminocarbonyl group, an N,N-disecondary butylaminocarbonyl group, an N,N-ditertiary butylaminocarbonyl group, an N-methyl-N-ethylaminocarbonyl group, an N-methyl-N-normal propylaminocarbonyl group, an N-methyl-N-isopropylaminocarbonyl group, an N-methyl-N-normal butylaminocarbonyl group, an N-methyl-N-secondary butylaminocarbonyl group, an N-methyl-N-tertiary butylaminocarbonyl group, an N-methyl-N-normal pentylaminocarbonyl group, an N-methyl-N-isopentylaminocarbonyl group, an N-methyl-N-tertiary pentylaminocarbonyl group, an N-methyl-N-neopentylaminocarbonyl group, an N-methyl-N-(2,3-dimethylpropyl)aminocarbonyl group, an N-methyl-N-(1-ethylpropyl)aminocarbonyl group, an N-methyl-N-(1-methylbutyl)aminocarbonyl group, an N-methyl-N-normal hexylaminocarbonyl group, an N-methyl-N-isohexylaminocarbonyl group, an N-methyl-N-(1,1,2-trimethylpropyl)aminocarbonyl group or the like.

The "(C1-C6) alkylcarbonylamino group" refers to an alkylcarbonylamino group having 2 to 7 carbon atoms, such as an alkylcarbonylamino group having the above-mentioned (C1-C6) alkyl group, for example, an acetylamino group, an N-propanoylamino group, an N-butanoylamino group, an N-(2-methylpropanoyl)amino group, an N-pentanoylamino group, an N-(2-methylbutanoyl)amino group, an N-(3-methylbutanoyl)amino group, an N-pivaloylamino group, an N-hexanoylamino group, or the like. The "(C3-C6) cycloalkylcarbonylamino group" refers to a cycloalkylcarbonylamino group having 4 to 7 carbon atoms, for example, a cyclopropylcarbonylamino group, or the like.

The expressions "(C1-C6)", "(C1-C10)", "(C2-C6)", "(C3-C6)", "(C3-C7)" and the like each refer to the range of the number of carbon atoms in each group. The above definitions also apply to groups in which two or more of the above-mentioned groups are linked together, and for example, the "(C1-C6) alkoxy (C1-C6) alkyl group" means that a linear or branched alkoxy group having 1 to 6 carbon atoms is bonded to a linear or branched alkyl group having 1 to 6 carbon atoms.

The "heterocyclic group" and "heterocycle" refer to a 5- or 6-membered monocyclic aromatic heterocyclic group, a 4- to 6-membered monocyclic non-aromatic heterocyclic group, or the like, each containing, as ring constituting atoms, carbon atoms and 1 to 4 heteroatoms selected from an oxygen atom, a sulfur atom and a nitrogen atom.

Examples of the "aromatic heterocyclic group" include a monocyclic aromatic heterocyclic group or a fused polycyclic heterocyclic group, such as furyl, thienyl, pyridyl, pyrimidinyl, pyridazinyl, pyrazinyl, pyrrolyl, imidazolyl, pyrazolyl, thiazolyl, isothiazolyl, oxazolyl, isoxazolyl, oxadiazolyl, thiadiazolyl, triazolyl, tetrazolyl, triazinyl, indolyl, indazolyl, benzimidazolyl, azaindolyl, azaindazolyl, benzofuranyl, benzothiophenyl, benzisoxazolyl, benzisothiazolyl, benzoxazolyl, benzothiazolyl, benzothiadiazolyl, quinolyl, quinolizinyl, quinoxalinyl, phthalazinyl, quinazolinyl, cinnolinyl, naphthyridinyl, pyridopyrimidinyl, pyridopyrazinyl, benzpyranyl, benzoxazinyl, carbazolyl, dibenzofuranyl, acridinyl, phenazinyl, phenothiazinyl, and phenoxathiinyl.

Examples of the "non-aromatic heterocyclic group" include a monocyclic non-aromatic heterocyclic group or a fused polycyclic heterocyclic group, such as oxetanyl, thietanyl, azetidinyl, pyrrolidinyl, piperidyl, morpholinyl, thiomorpholinyl, piperazinyl, hexamethyleneiminyl, oxazolidinyl, thiazolidinyl, imidazolidinyl, oxazolinyl, thiazolinyl, isoxazolinyl, imidazolinyl, dioxolyl, dioxolanyl, dihydrooxadiazolyl, 2-oxo-pyrrolidin-1-yl, 2-oxo-1,3-oxazolidin-5-yl, 5-oxo-1,2,4-oxadiazolin-3-yl, 1,3-dioxolan-2-yl, 1,3-dioxan-2-yl, 1,3-dioxepan-2-yl, pyranyl, tetrahydropyranyl, thiopyranyl, tetrahydrothiopyranyl, 1-oxidetetrahydrothiopyranyl, 1,1-dioxidotetrahydrothiopyranyl, tetrahydrofuranyl, dioxanyl, pyrazolidinyl, pyrazolinyl, tetrahydropyrimidinyl, dihydrotriazolyl, tetrahydrotriazolyl, decahydroisoquinoline, quinuclidine, azaadamantane, and 8-oxa-2-azaspiro[4.5]decane.

Examples of the "(C1-C12) alkylsilyl group" include a TMS (trimethylsilyl) group, a TES (triethylsilyl) group, a TBS (tert-butyldimethylsilyl) group, a TIPS (triisopropylsilyl) group, and a BIBS (di-tert-butylisobutylsilyl) group.

Examples of the "Z,Z (C1-C6) alkoxy group" include a diphenylmethoxy group, a 1,1-diphenylethoxy group, a 1,2-diphenylethoxy group, or the like.

Examples of the "(C1-C6) alkoxy (C1-C6) alkoxy (C1-C6) alkoxy group" include a 2-methoxy-1-(methoxymethyl)ethoxy group, and an ethoxymethoxymethoxy group.

Examples of the salt of the amide compound represented by the general formula [I] of the present invention include inorganic acid salts, such as hydrochlorides, sulfates, nitrates and phosphates; organic acid salts, such as acetates, fumarates, maleates, oxalates, methanesulfonates, benzenesulfonates and p-toluenesulfonates; and salts with an inorganic or organic base such as a sodium ion, a potassium ion, a calcium ion and a trimethylammonium ion.

The amide compound represented by the general formula [I] of the present invention and salts thereof can have one or more chiral centers in the structural formula and can exist as two or more kinds of optical isomers or diastereomers. All the optical isomers and mixtures of the isomers at any ratio are also included in the present invention. Further, the amide compound represented by the general formula [I] of the present invention and salts thereof can exist as two kinds of geometric isomers due to a carbon-carbon double bond in the structural formula. All the geometric isomers and mixtures of the isomers at any ratio are also included in the present invention.

The amide compound represented by the general formula [I] of the present invention or salts thereof can be produced according to, for example, the production methods described below, which are non-limiting examples.

### Production Method 1.

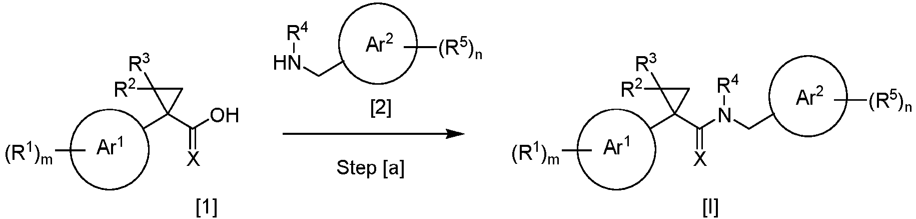

wherein each symbol is as defined above.

### Production method in Step [a]

In this reaction, the amide compound represented by the general formula [I] can be produced by reacting a carboxylic acid represented by the general formula [1] with an amine compound represented by the general formula [2] in the presence of a condensing agent, a base and an inert solvent. Since this reaction is an equimolar reaction of the compounds, they are basically used in equimolar amounts, but either of them may be used in an excess amount.

The condensing agent used in this reaction may be those commonly used in the production of amides, and examples thereof include trifluoroacetic anhydride, a chlorocarbonate ester, Mukaiyama reagent (2-chloro-N-methylpyridinium iodide), DCC (1,3-dicyclohexylcarbodiimide), CDI (carbonyldiimidazole), and DEPC (diethyl cyanophosphate). The amount of the condensing agent used may be appropriately selected from the range of equimolar to excess molar amount relative to the carboxylic acid represented by the general formula [1].

The base used in this reaction may be an include inorganic or organic base, and examples of the inorganic base include alkali metal hydroxides such as sodium hydroxide, and potassium hydroxide; and carbonates such as sodium carbonate, potassium carbonate, and sodium hydrogencarbonate; examples of the organic base include triethylamine, pyridine, DMAP, and DBU. The amount of the base used may be selected from the range of catalytic amount to excess molar amount relative to the carboxylic acid represented by the general formula [1].

The inert solvent used in this reaction may be any solvent that does not markedly inhibit the progress of this reaction, and examples thereof include aromatic hydrocarbons such as benzene, toluene, and xylene; halogenated hydrocarbons such as methylene chloride, chloroform, and carbon tetrachloride; halogenation aromatic hydrocarbons such as chlorobenzene, and dichlorobenzene; chain or cyclic ethers such as diethyl ether, dioxane, and tetrahydrofuran; amides such as dimethylformamide, and dimethylacetamide; dimethyl sulfoxide, 1,3-dimethyl-2-imidazolidinone, acetone, methyl ethyl ketone and the like. These inert solvents may be used alone or in combination of two or more.

This reaction can also be carried out under an atmosphere of an inert gas such as nitrogen gas or argon gas. This reaction can be carried out in the range from room temperature to the boiling point of the inert solvent used. The reaction time varies depending on the reaction scale and reaction temperature, but can range from several minutes to 48 hours. After completion of the reaction, the target product can be isolated from the post-reaction mixture by a conventional method. As needed, recrystallization, column chromatography or the like can be employed for the purification of the target product.

### Production Method 2.

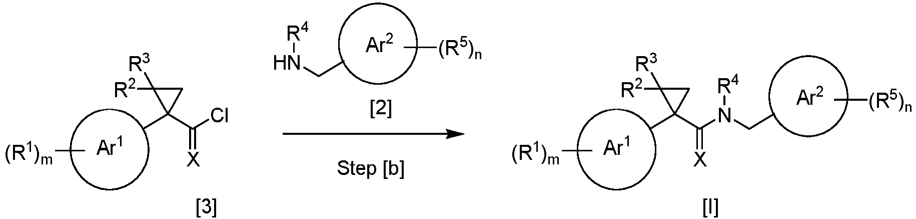

wherein each symbol is as defined above.

### Production method in Step [b]

In this reaction, the amide compound represented by the general formula [I] can be produced by reacting a carboxylic acid chloride represented by the general formula [3] with a compound represented by the general formula [2] in the presence of a base and an inert solvent.

Examples of the base that can be used in this reaction include inorganic bases such as sodium hydroxide, potassium hydroxide, sodium carbonate, potassium carbonate, sodium hydrogencarbonate, and potassium hydrogencarbonate, alkali metal hydrides such as sodium hydride, and potassium hydride, acetates such as potassium acetate, alkali metal alkoxides such as potassium t-butoxide, sodium methoxide, and sodium ethoxide, tertiary amines such as triethylamine, diisopropylethylamine, and 1,8-diazabicyclo[5.4.0]undec-7-ene, nitrogen-containing aromatic compounds such as pyridine, and dimethylaminopyridine, and the like. The amount of the base used is usually in the range of 1-fold molar amount to 10-fold molar amount relative to the compound represented by the general formula [2].

The inert solvent that can be used in this reaction may be any solvent that does not markedly inhibit the progress of this reaction, and examples thereof include alcohols such as methanol, ethanol, propanol, butanol, and 2-propanol; chain or cyclic ethers such as diethyl ether, tetrahydrofuran, and dioxane; aromatic hydrocarbons such as benzene, toluene, and xylene; halogenation aliphatic hydrocarbons such as methylene chloride, chloroform, and carbon tetrachloride, halogenation aromatic hydrocarbons such as chlorobenzene, and dichlorobenzene; nitriles such as acetonitrile; esters such as ethyl acetate; polar solvents such as N,N-dimethylformamide, N,N-dimethylacetamide, dimethyl sulfoxide, and 1,3-dimethyl-2-imidazolidinone. These inert solvents may be used alone or in combination of two or more.

This reaction can also be carried out under an atmosphere of an inert gas such as nitrogen gas or argon gas. This reaction may be carried out usually in the range from about 0°C to the boiling point of the solvent used. The reaction time varies depending on the reaction scale, the reaction temperature and the like, but can be appropriately selected from the range of several minutes to 48 hours. After completion of the reaction, the target product can be isolated from the post-reaction mixture by a conventional method. As needed, recrystallization, column chromatography or the like can be employed for the purification of the target product. Alternatively, the isolated product may be subjected to the next step without purification.

### Production Method 3.

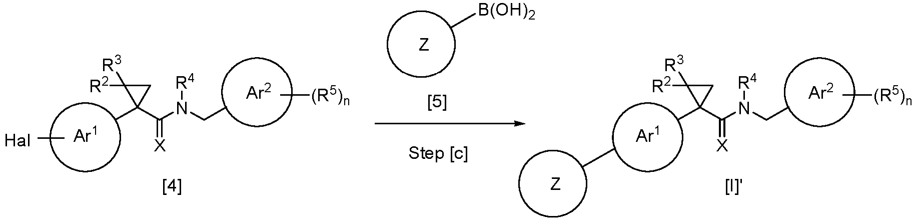

wherein Hal represents a halogen atom, and the other each symbol is as defined above.

### Production method in Step [c]

In this reaction, a biaryl compound represented by the general formula [I]' can be produced by reacting a halobenzene derivative represented by the general formula [4] with a boronic acid represented by the general formula [5] in the presence of a palladium catalyst.

This reaction can be carried out, for example, in the presence of a base (sodium ethylate, sodium hydroxide, potassium hydroxide, triethylamine, sodium carbonate, sodium hydrogencarbonate, potassium carbonate, cesium carbonate, tripotassium phosphate, tetrabutylammonium fluoride, etc.)) or an aqueous solution thereof, or a mixture thereof, and a catalyst (tetrakis(triphenylphosphine)palladium (Pd(PPh₃)₄), bis(triphenylphosphine)palladium dichloride (PdCl₂(PPh₃)₂), palladium acetate (Pd(OAc)₂), palladium black, 1,1'-bis(diphenylphosphinoferrocene)dichloropalladium (PdCl₂(dppf)₂), 1,1'-bis(diphenylphosphinoferrocene)dichloropalladium (acetonitrile adduct) (PdCl₂(dppf)₂·MeCN), diallylpalladium dichloride (PdCl₂(allyl)₂), phenylbis(triphenylphosphine)palladium iodide (PhPdI(PPh₃)₂), etc.), in an organic solvent (benzene, toluene, dimethylformamide, dioxane, tetrahydrofuran, acetonitrile, dimethoxyethane, acetone, etc.).

This reaction can also be carried out under an atmosphere of an inert gas such as nitrogen gas or argon gas. This reaction can be carried out in the range from room temperature to the boiling point of the inert solvent used. The reaction time varies depending on the reaction scale and reaction temperature, but can range from several minutes to 48 hours. After completion of the reaction, the target product can be isolated from the post-reaction mixture by a conventional method. As needed, recrystallization, column chromatography or the like can be employed for the purification of the target product.

Specific examples of the compound of the present invention are shown below. In the following tables (Table 1-1, Table 1-2, Table 2-1, Table 3-1, Table 4-1, Table 5-1, Table 6-1, Table 7-1, Table 8-1, Table 9-1, Table 10-1, Table 11-1 and Table 12-1), H represents a hydrogen atom, F represents fluorine, Cl represents a chlorine atom, and Br represents bromine. In the tables, the melting point (°C), refractive index _{nD} (measurement temperature; °C) or NMR data are given as physical properties. The NMR data are shown in Appended Table 1.

Examples of specific compounds represented by the following general formula [I-A] are shown in the tables below. In the tables, Py. represents pyridyl, Bn represents benzyl, MOM represents methoxymethyl, TMS represents trimethylsilyl, and Ac represents acetyl.

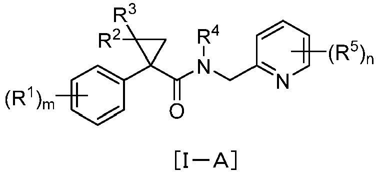

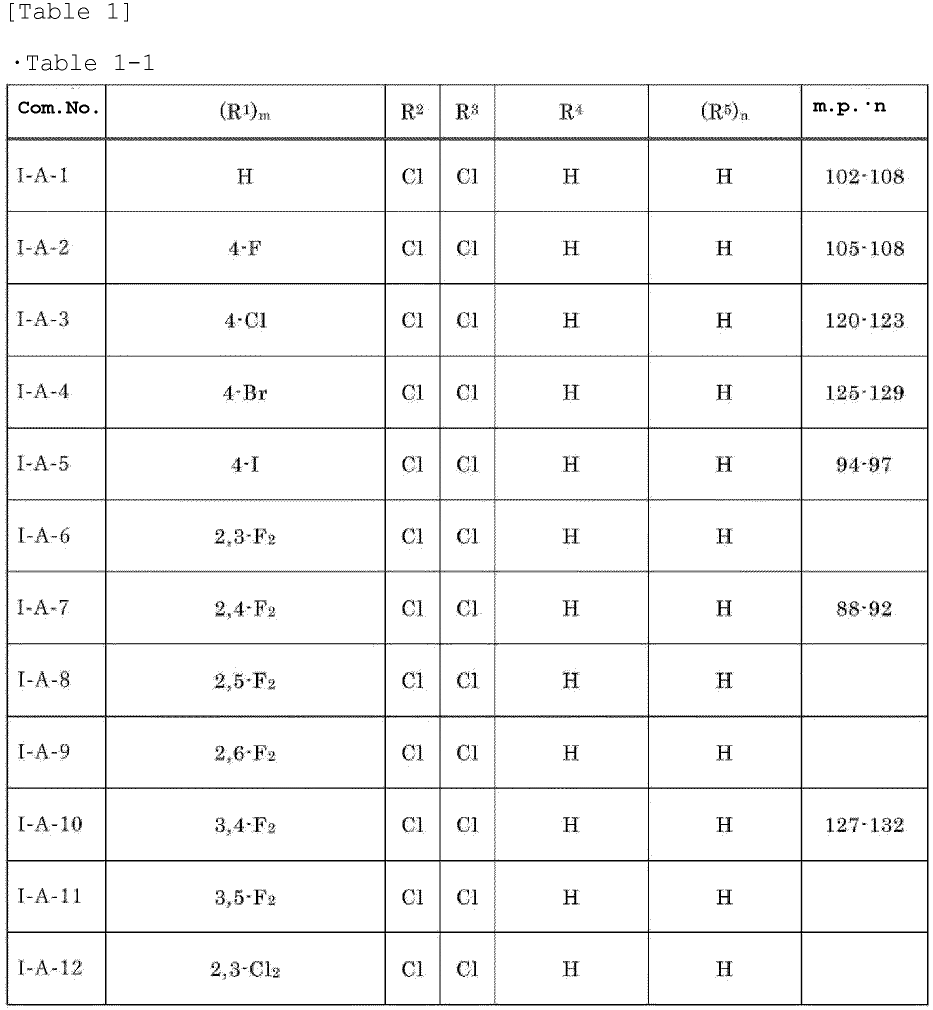

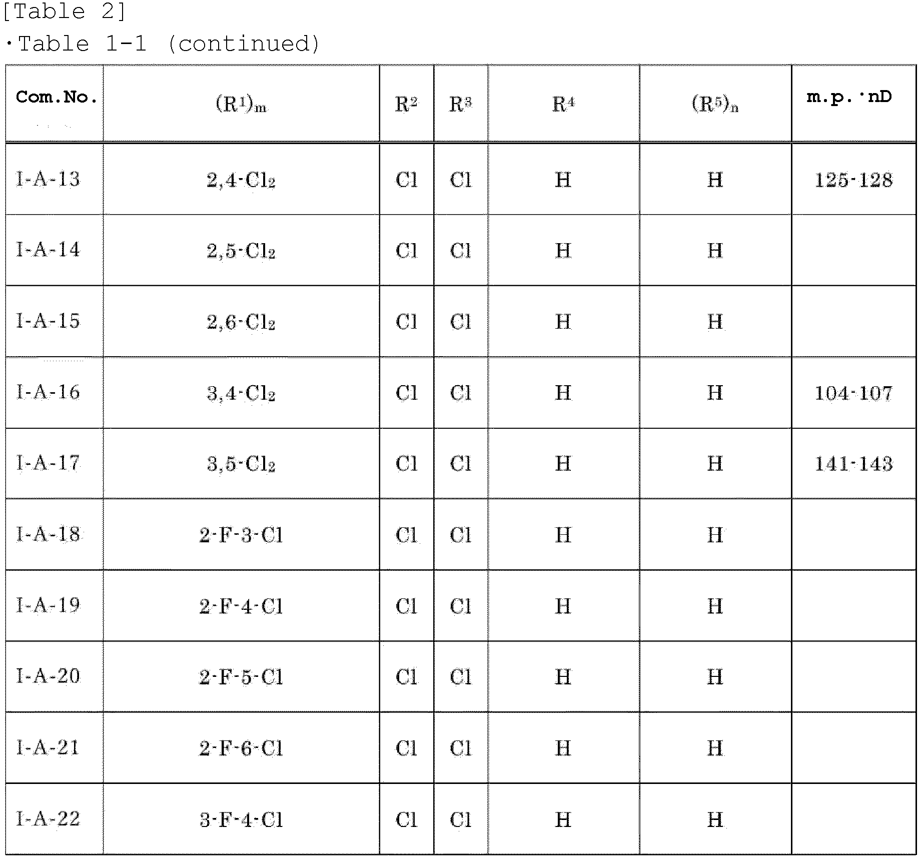

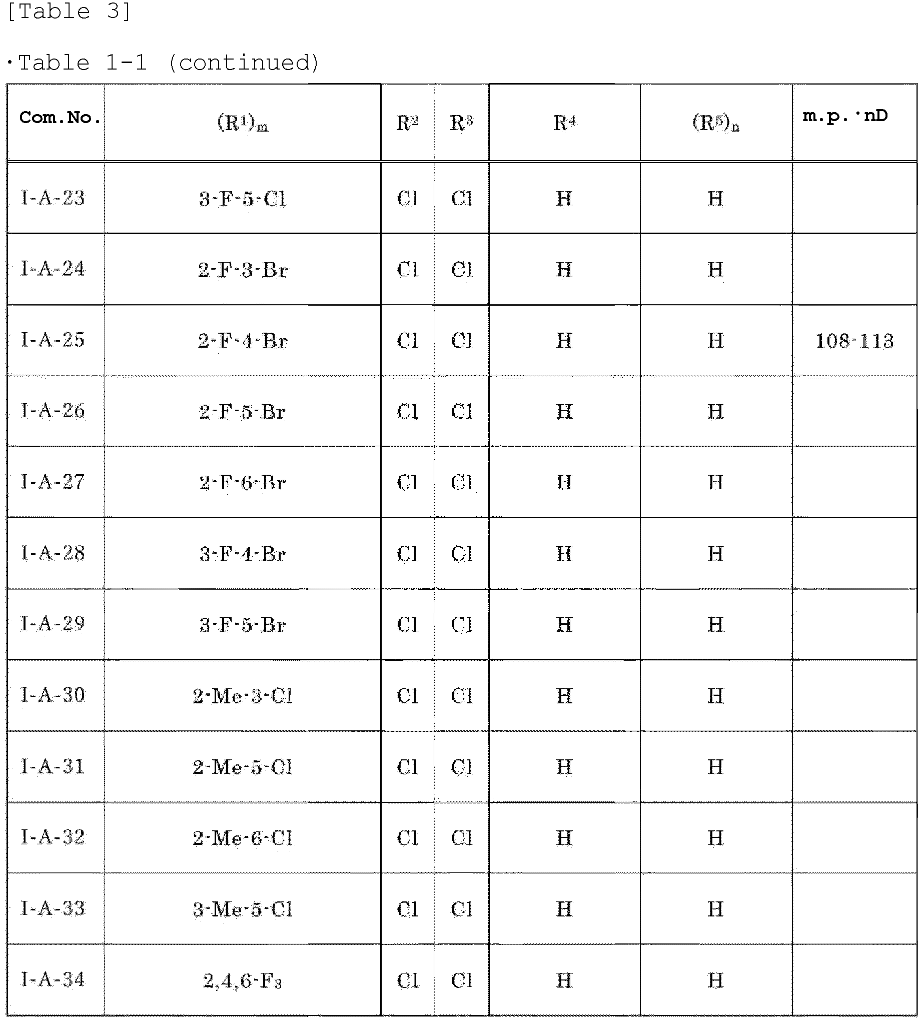

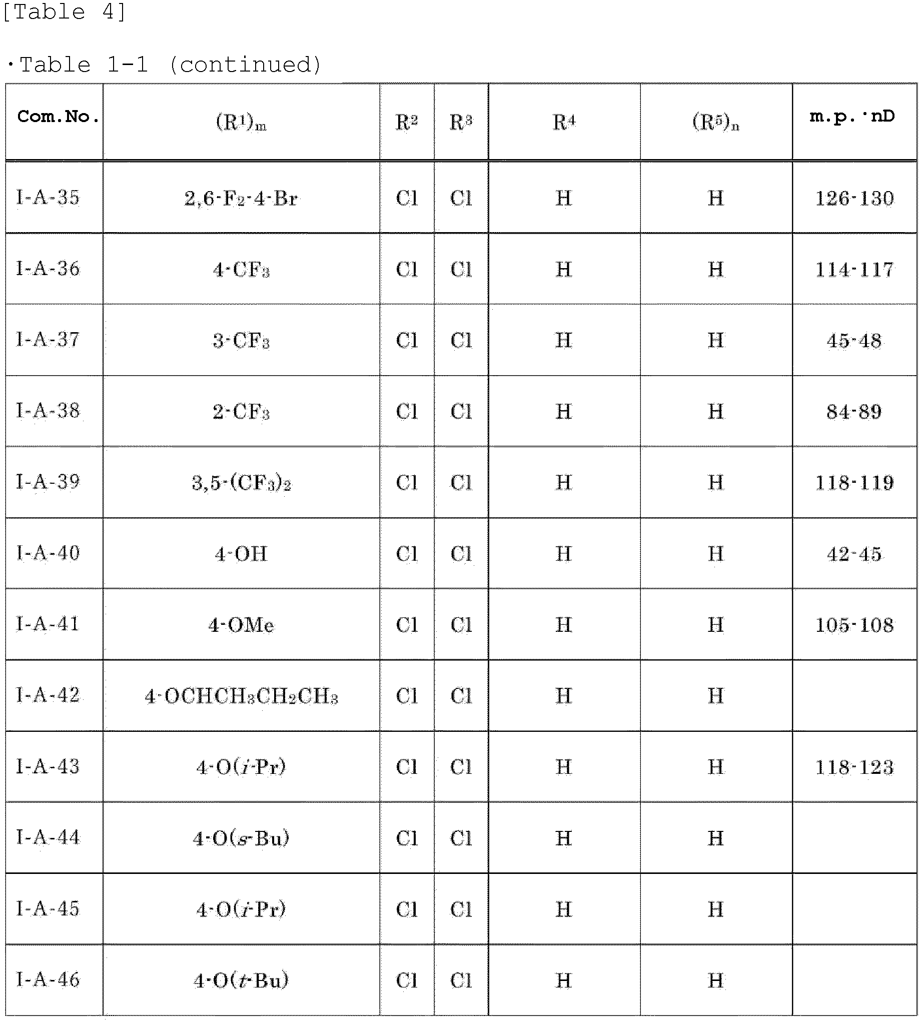

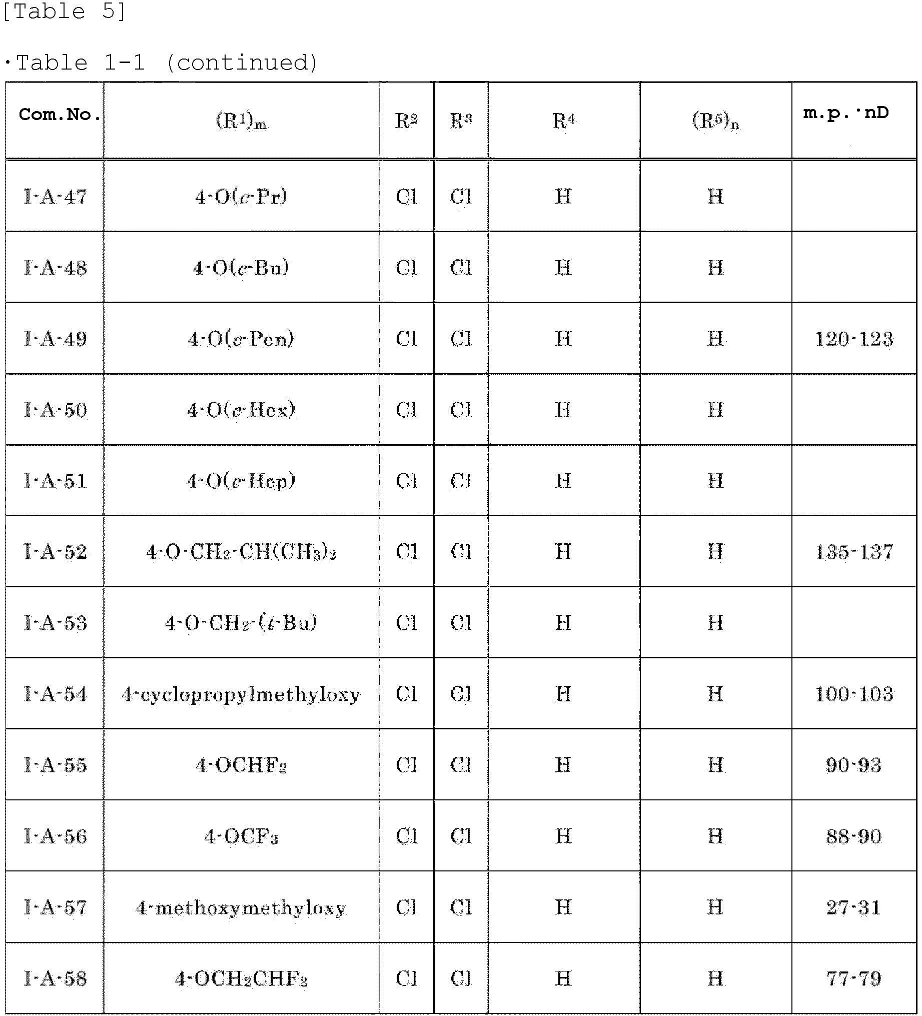

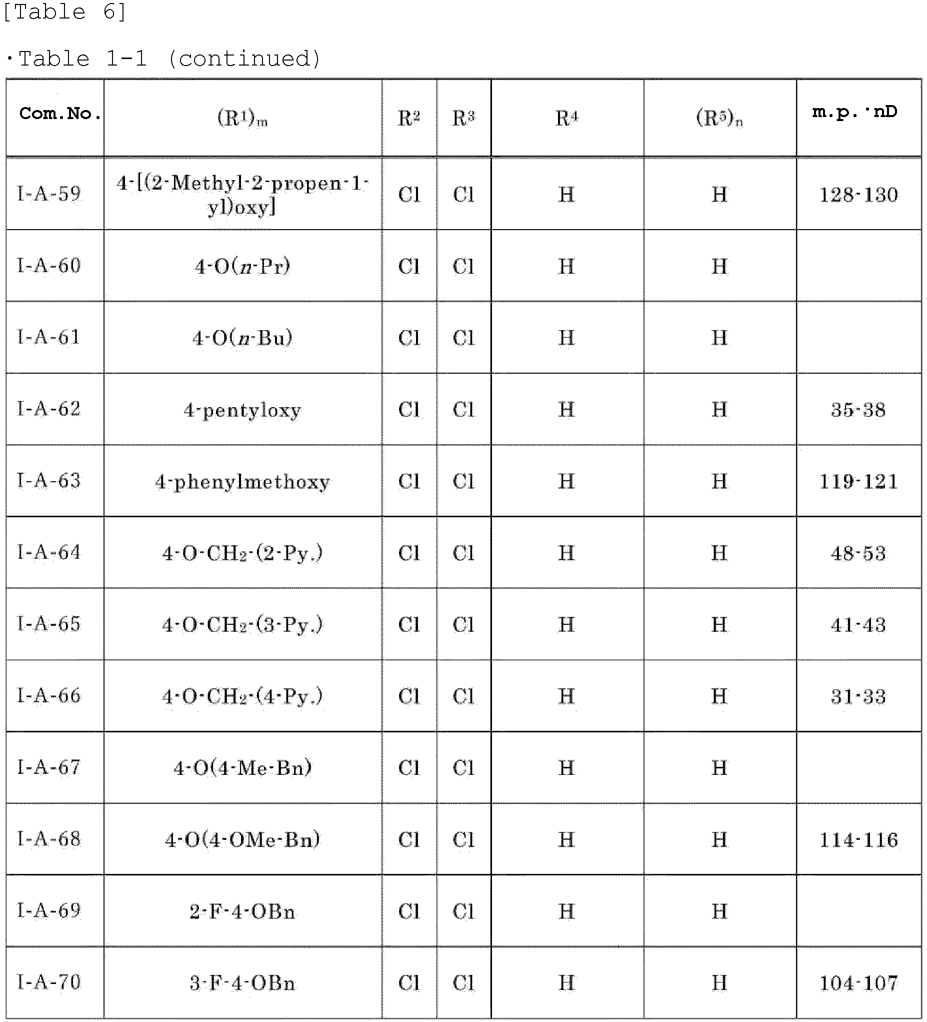

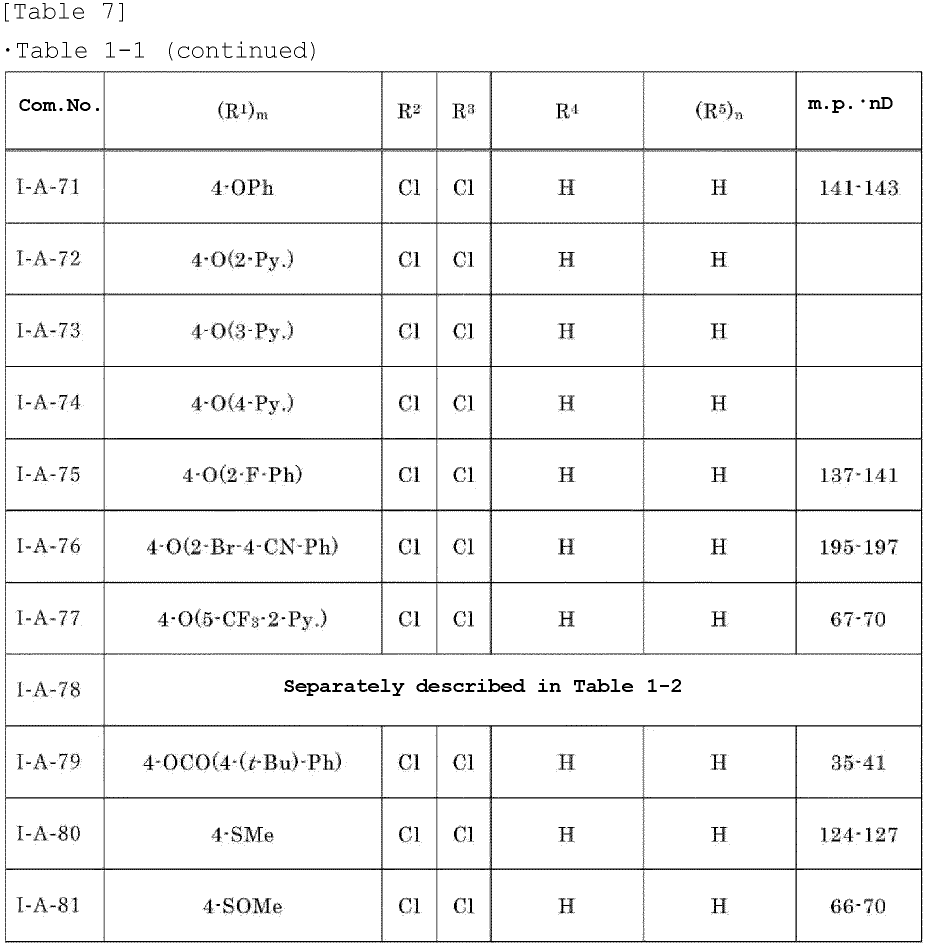

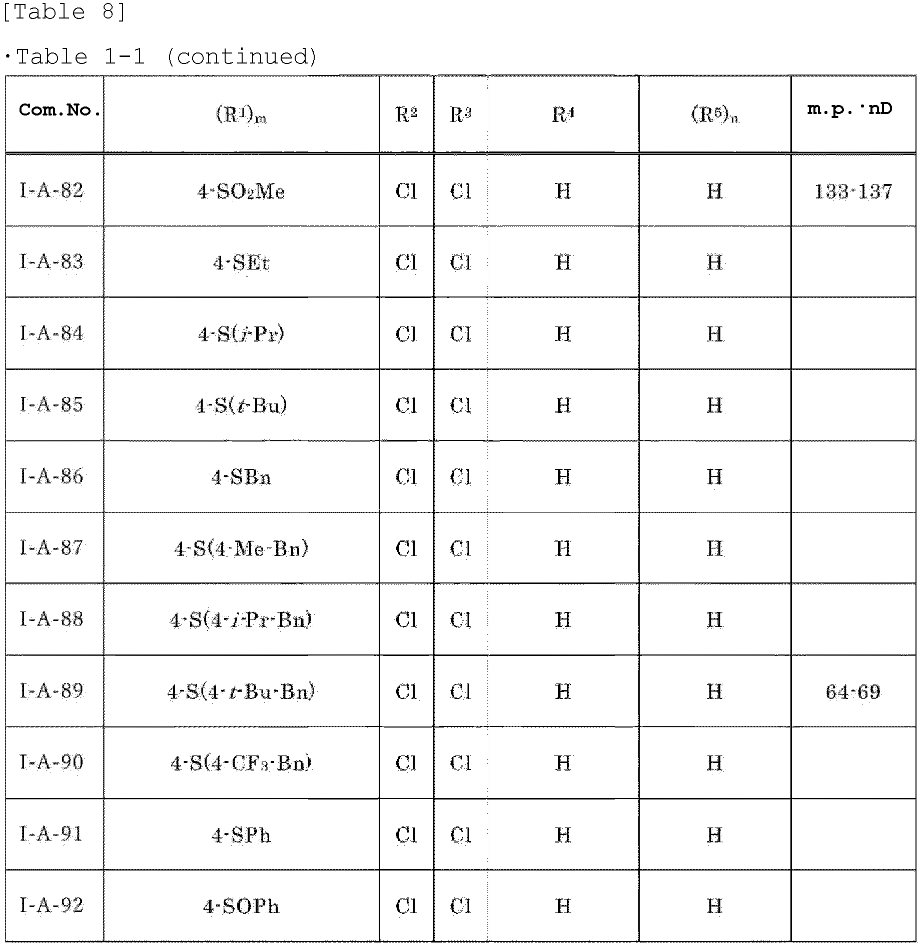

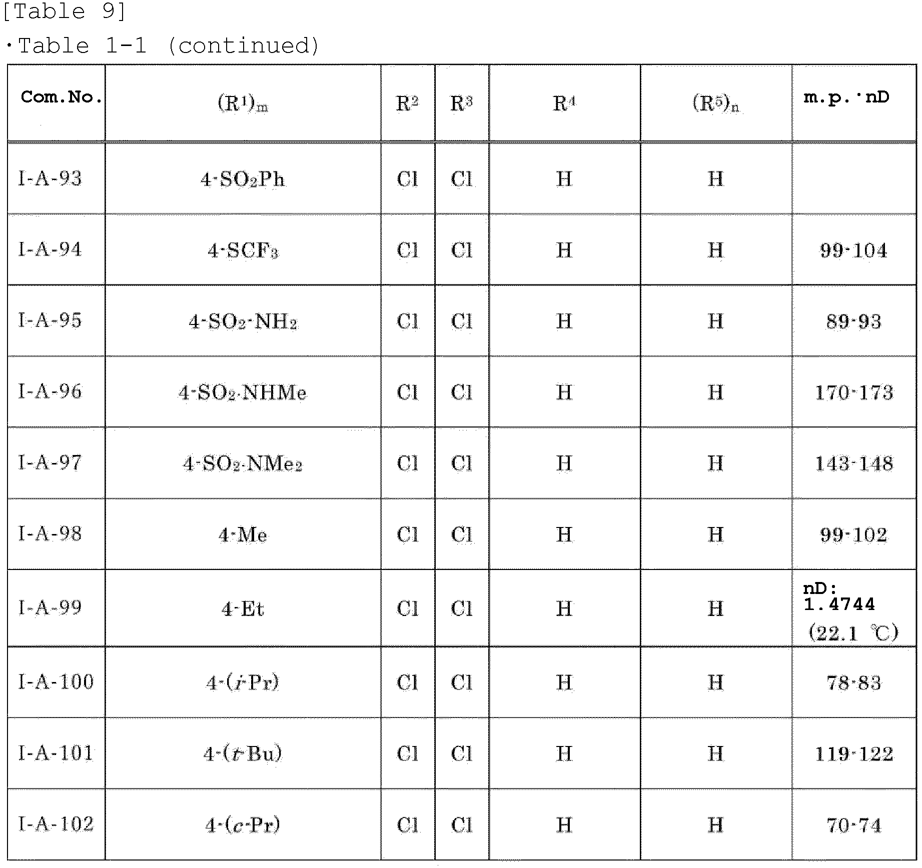

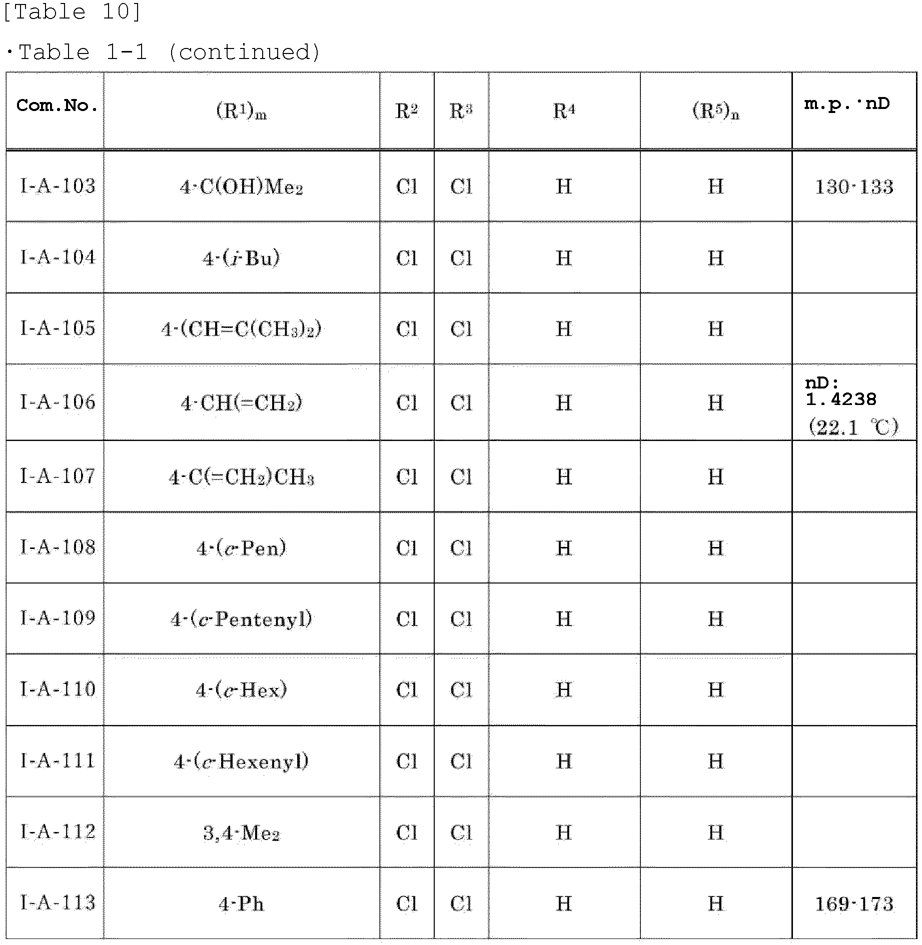

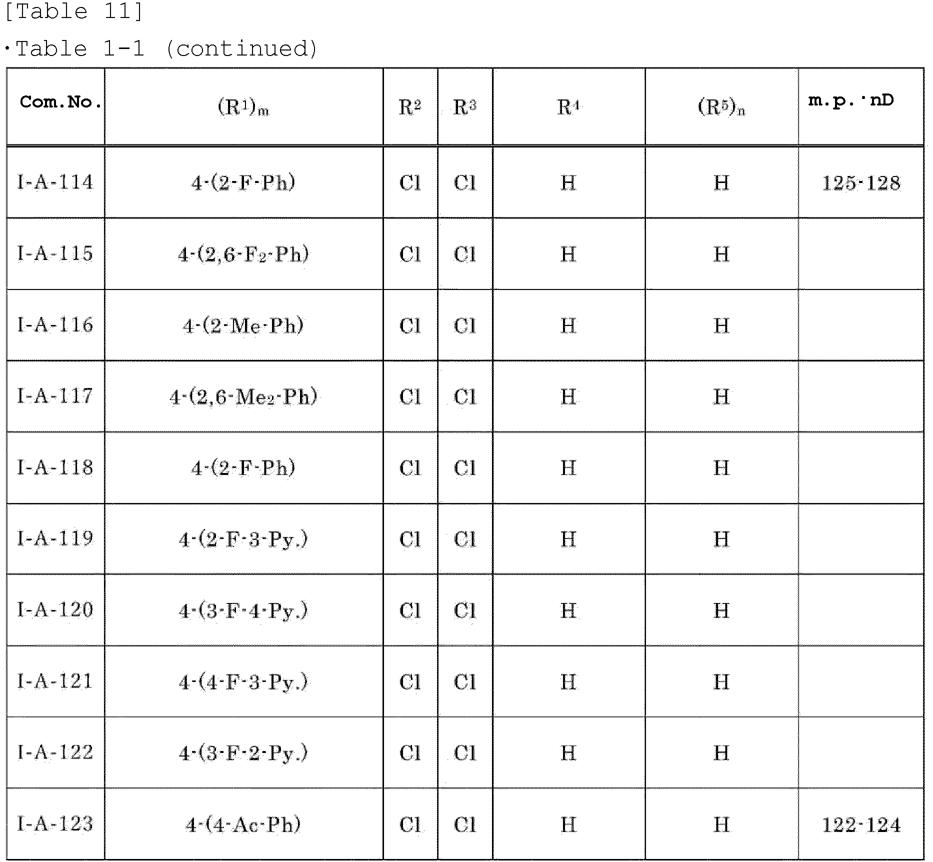

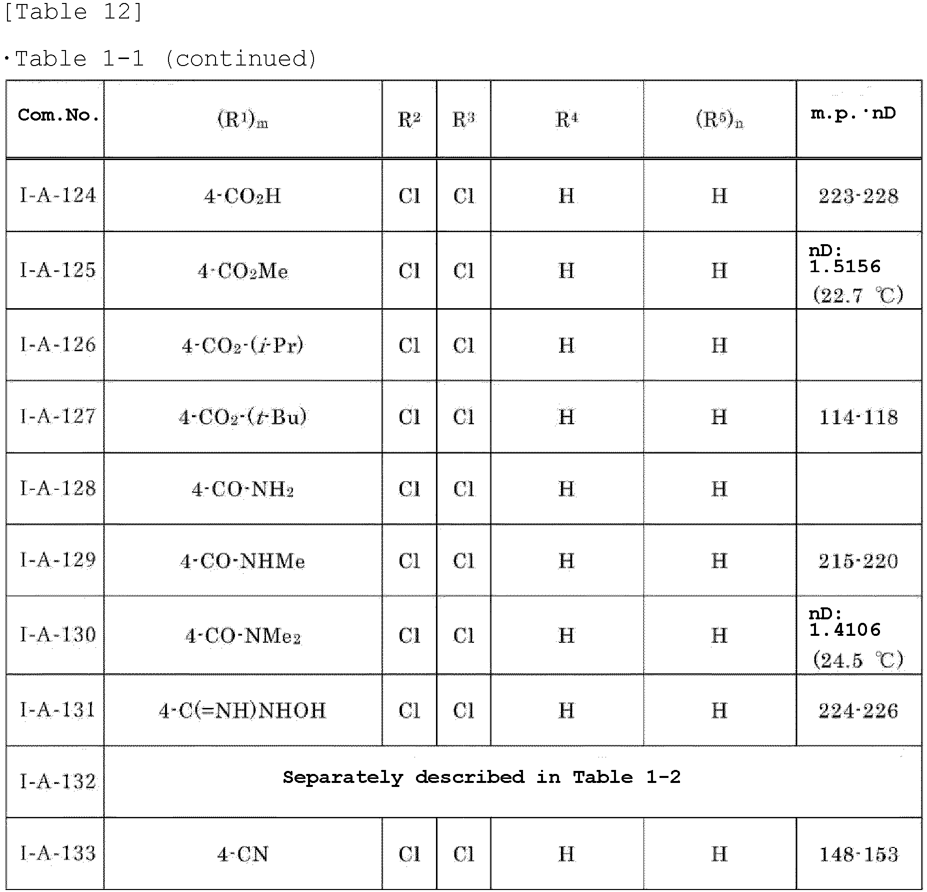

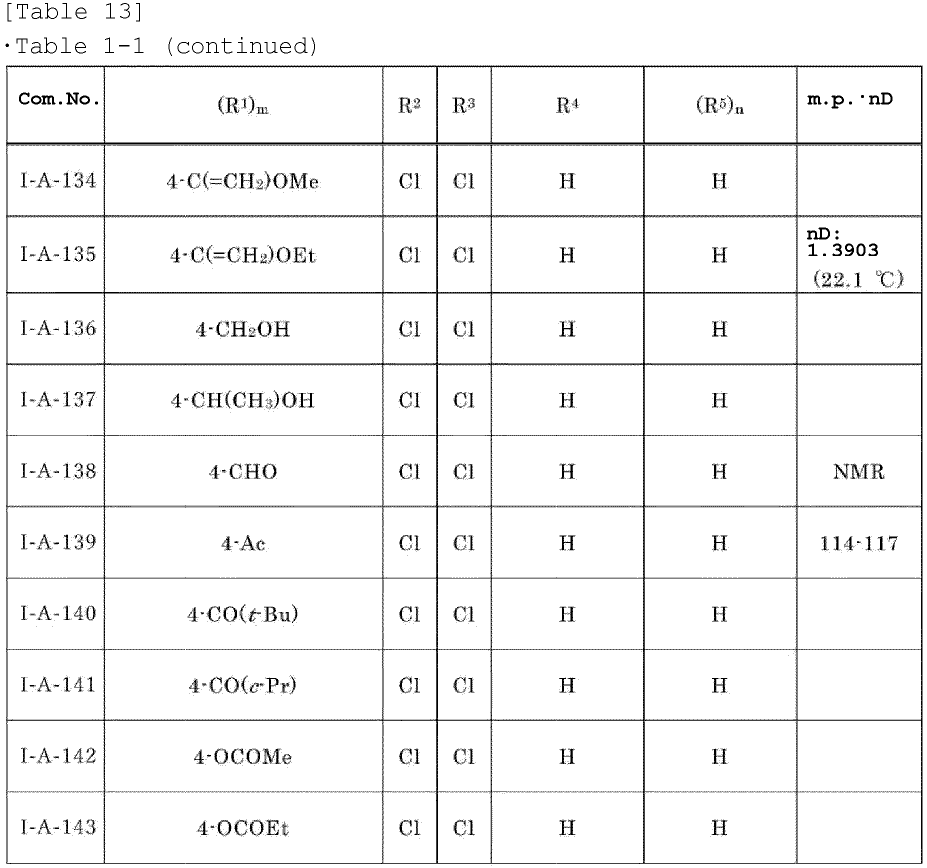

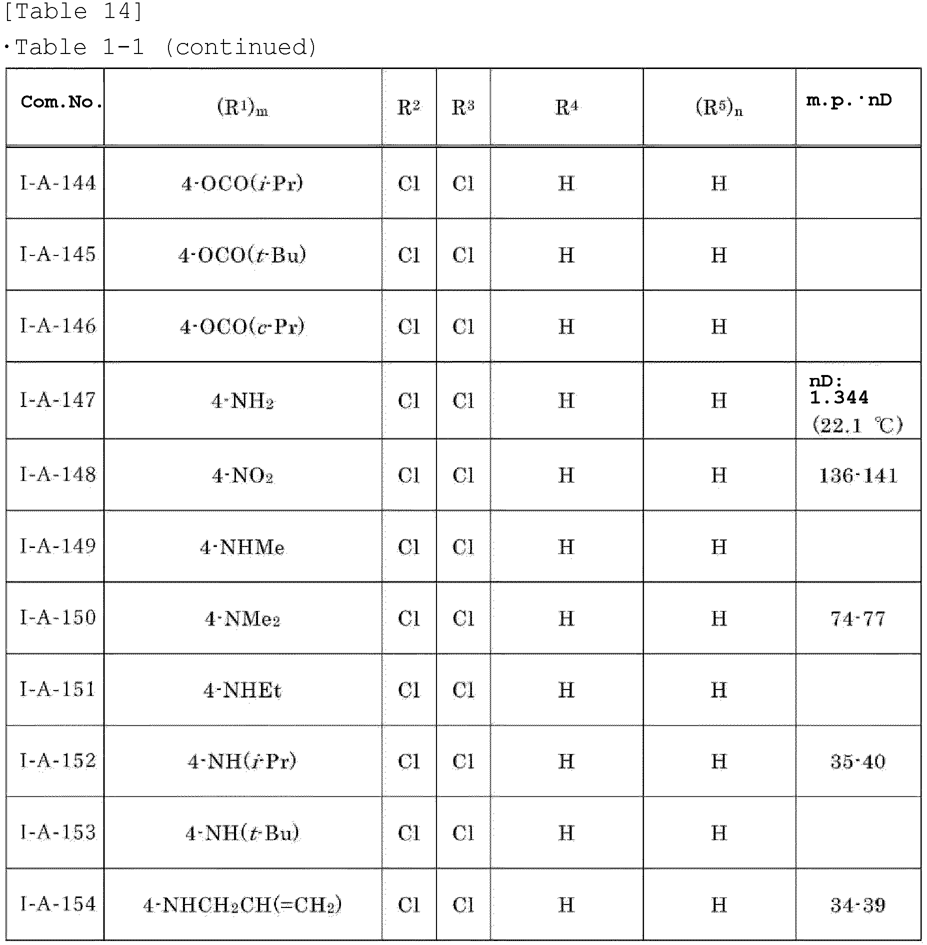

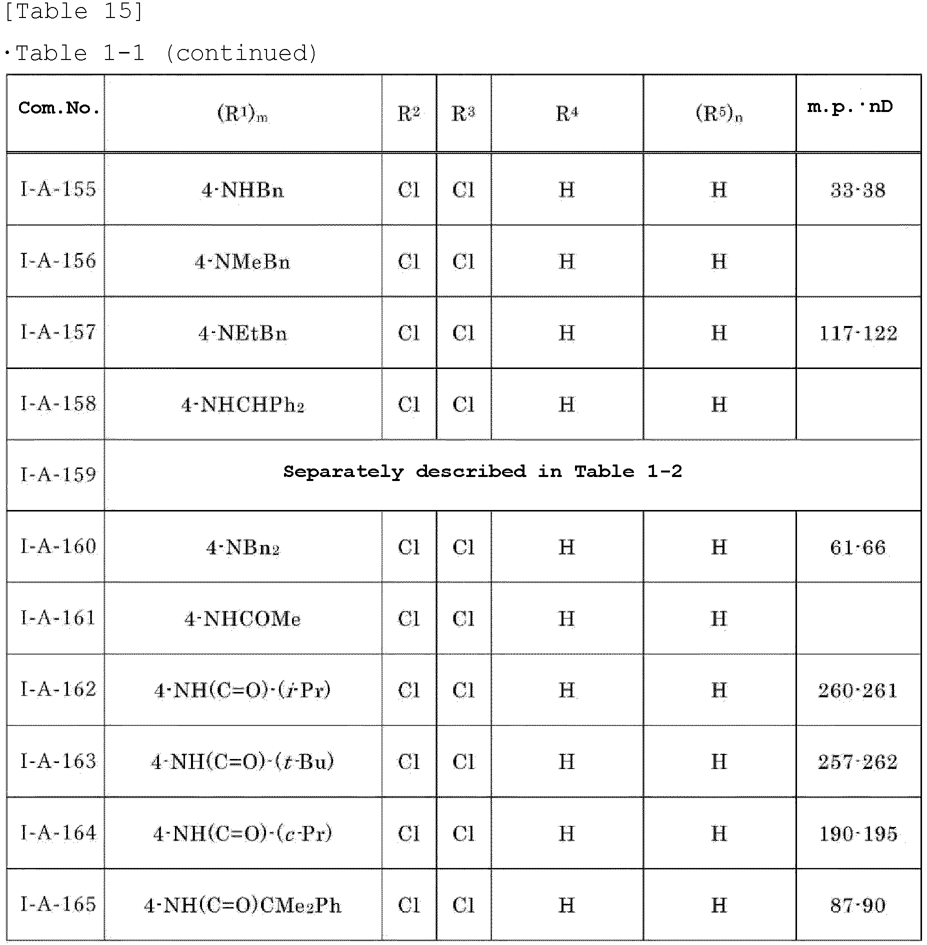

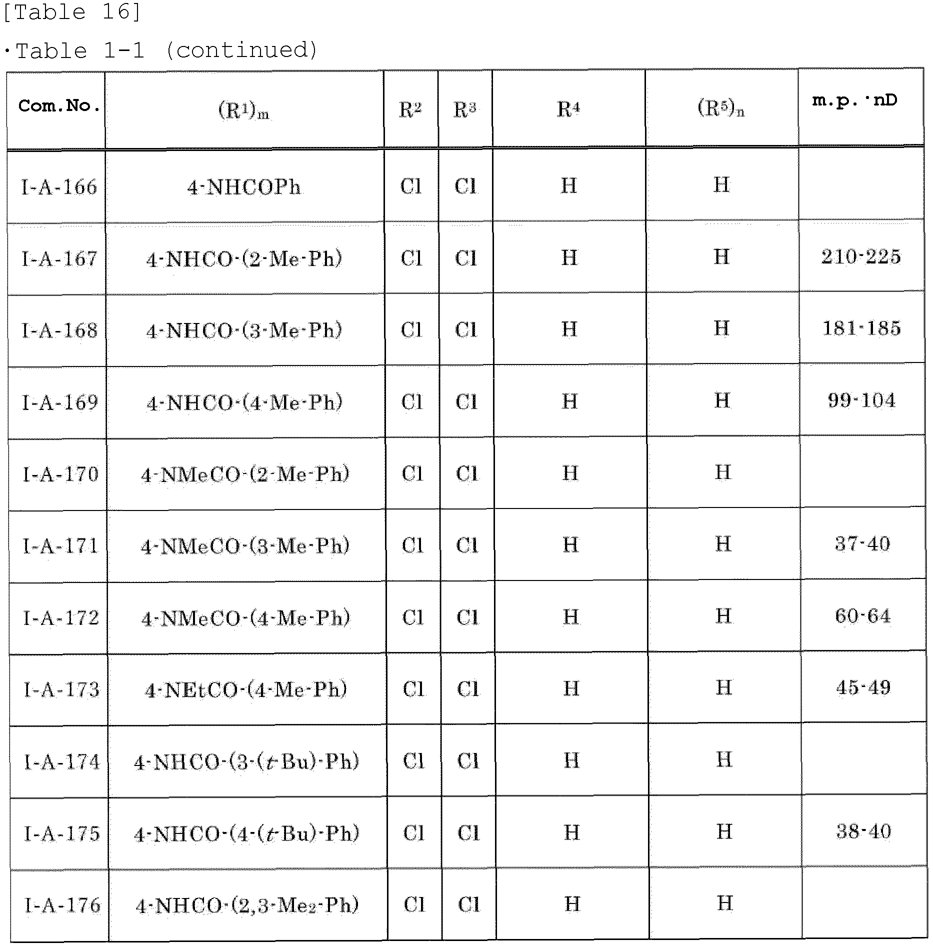

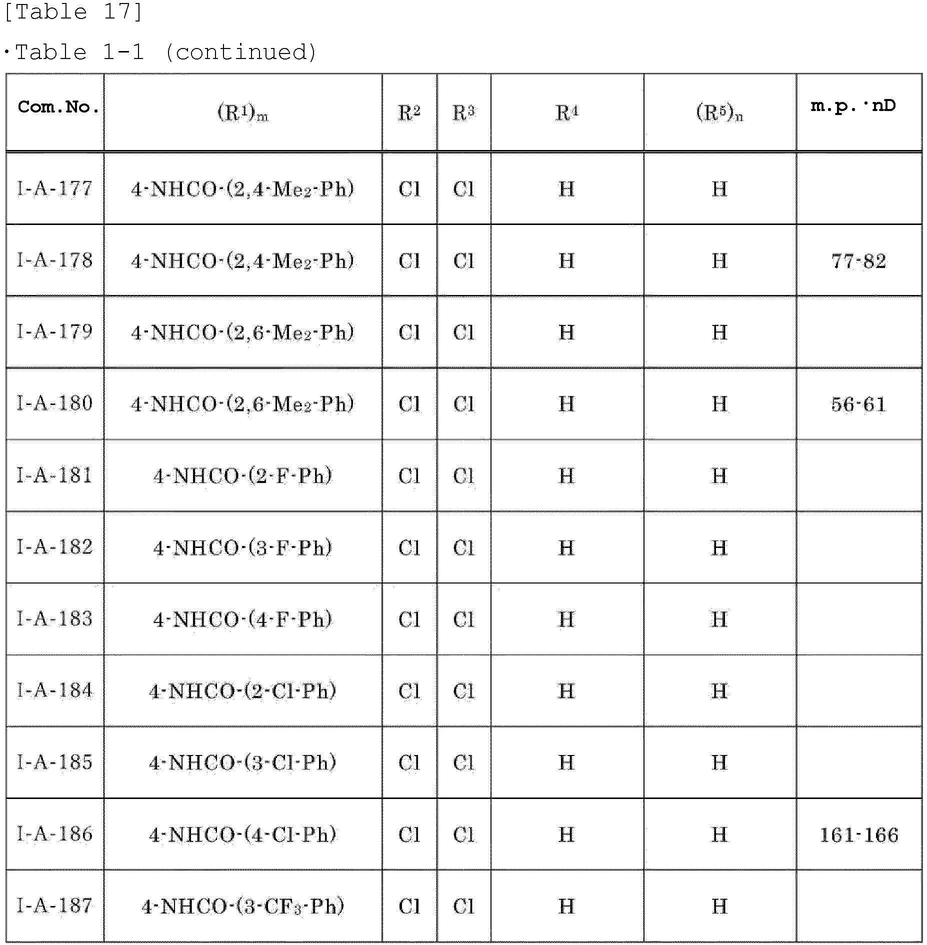

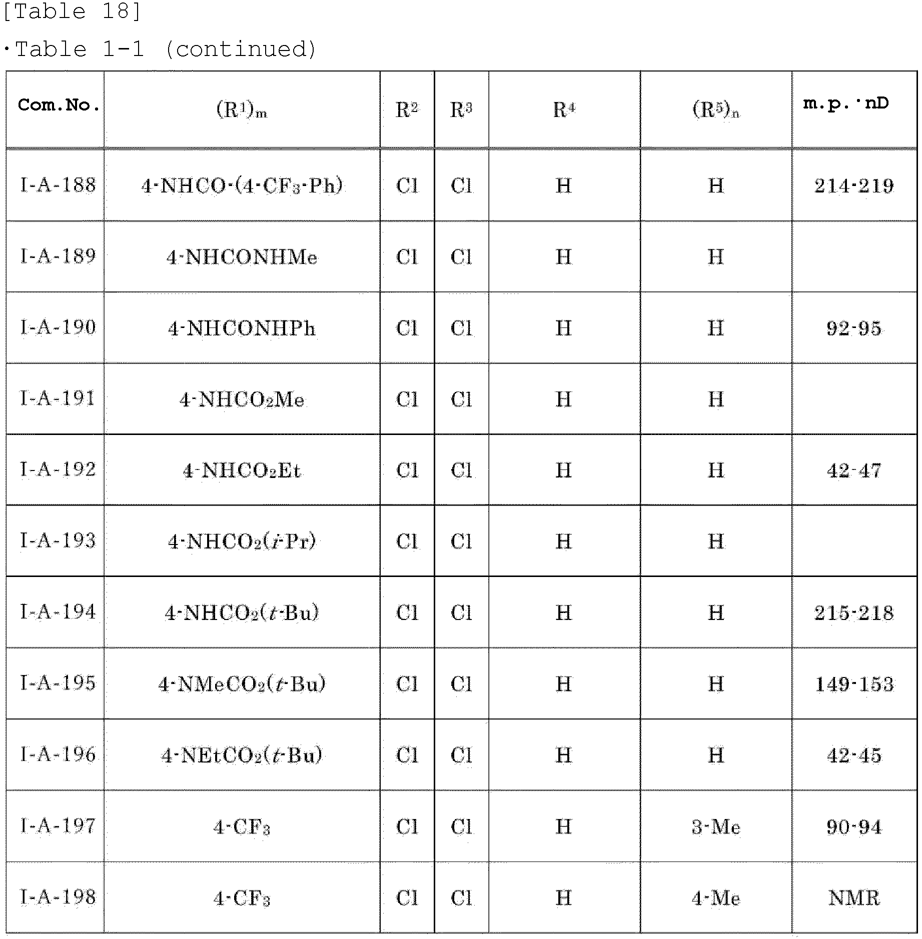

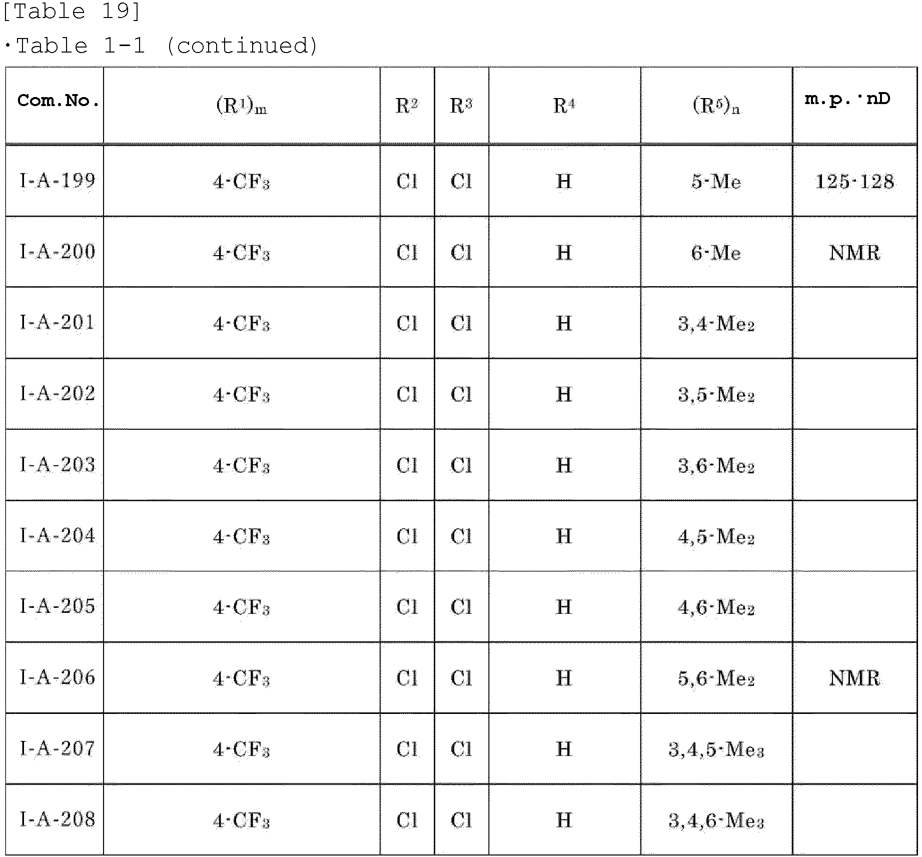

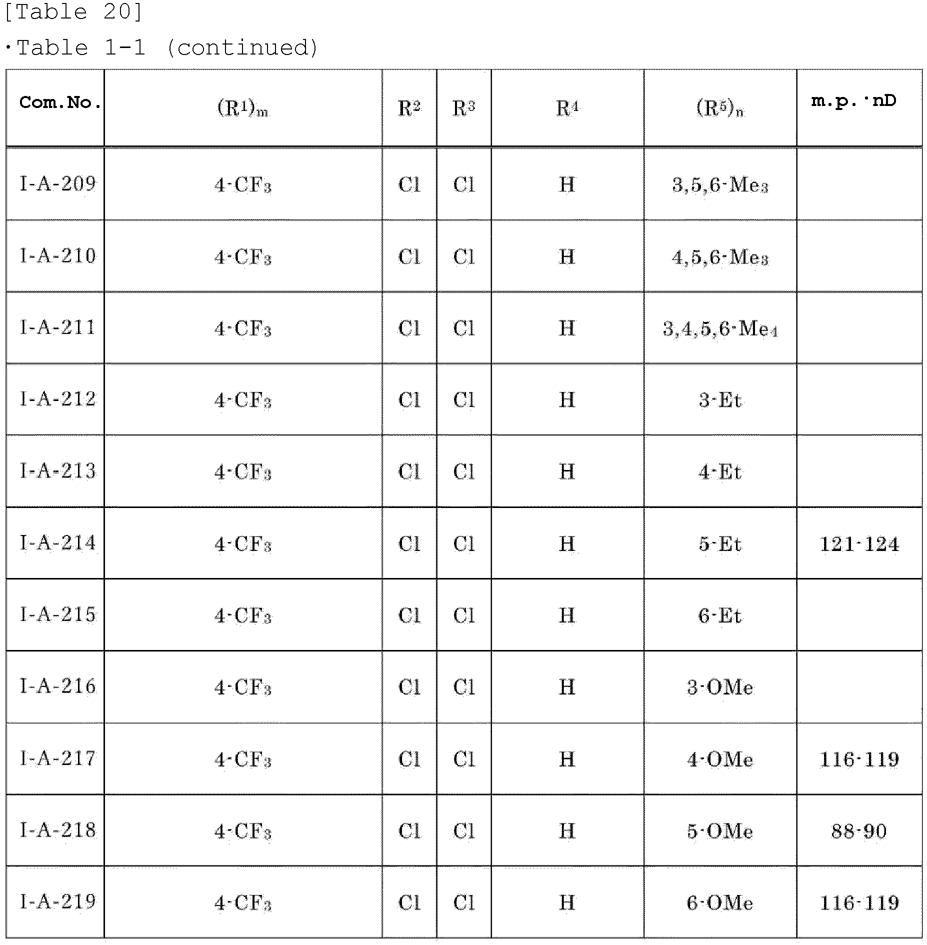

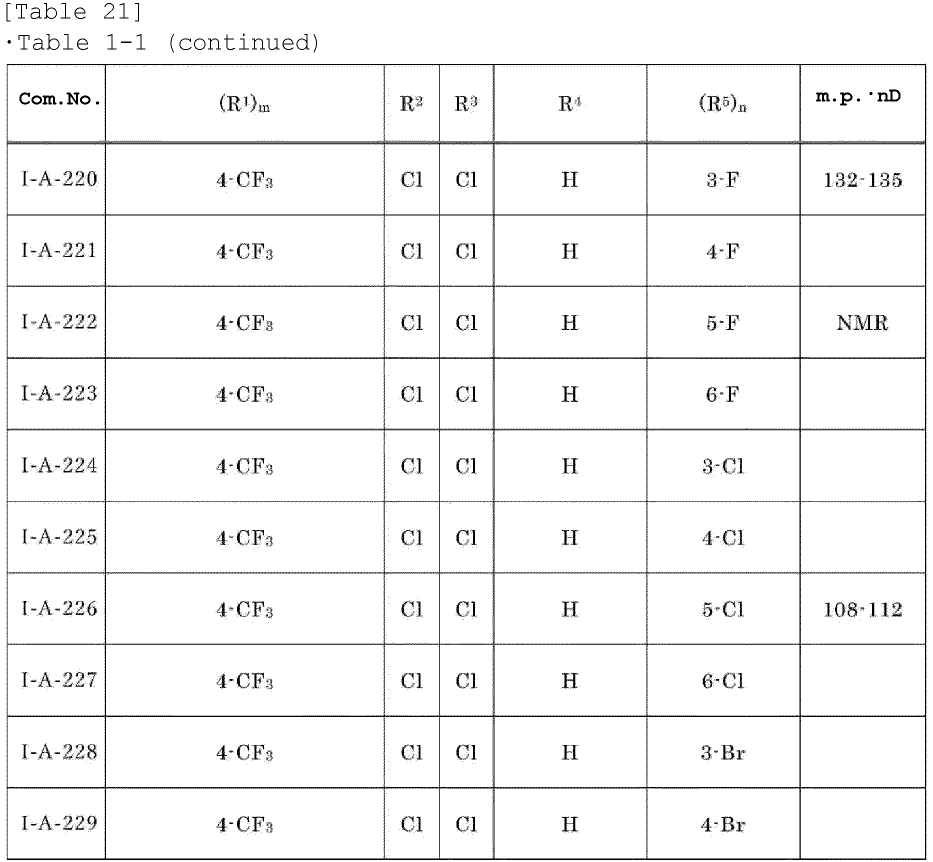

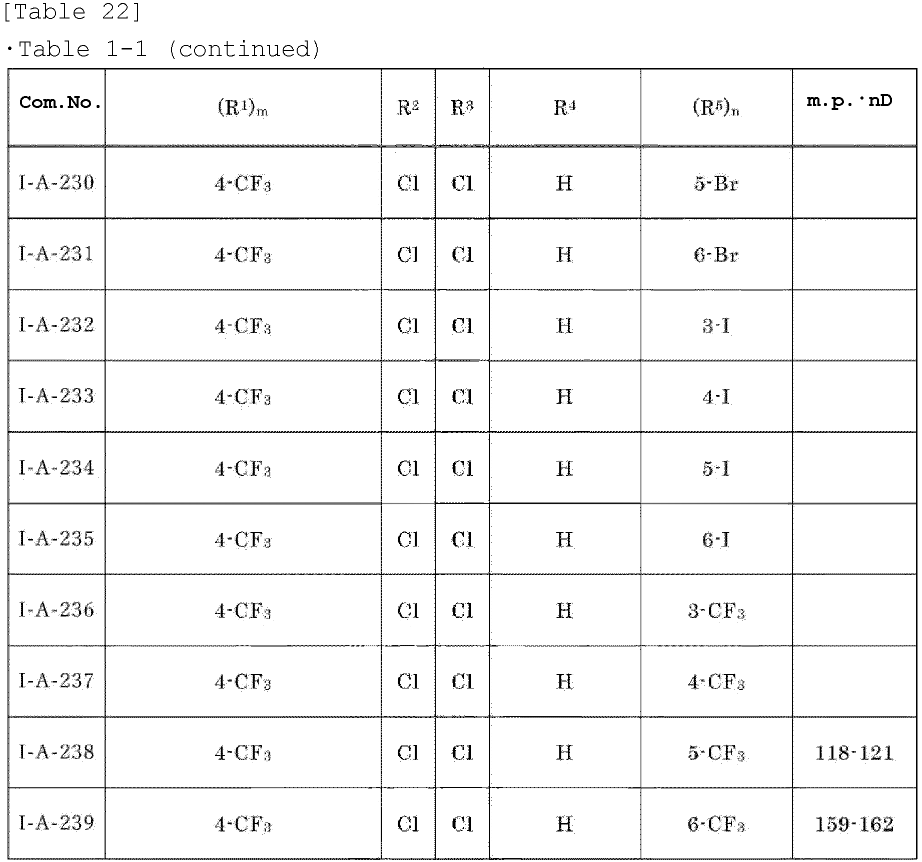

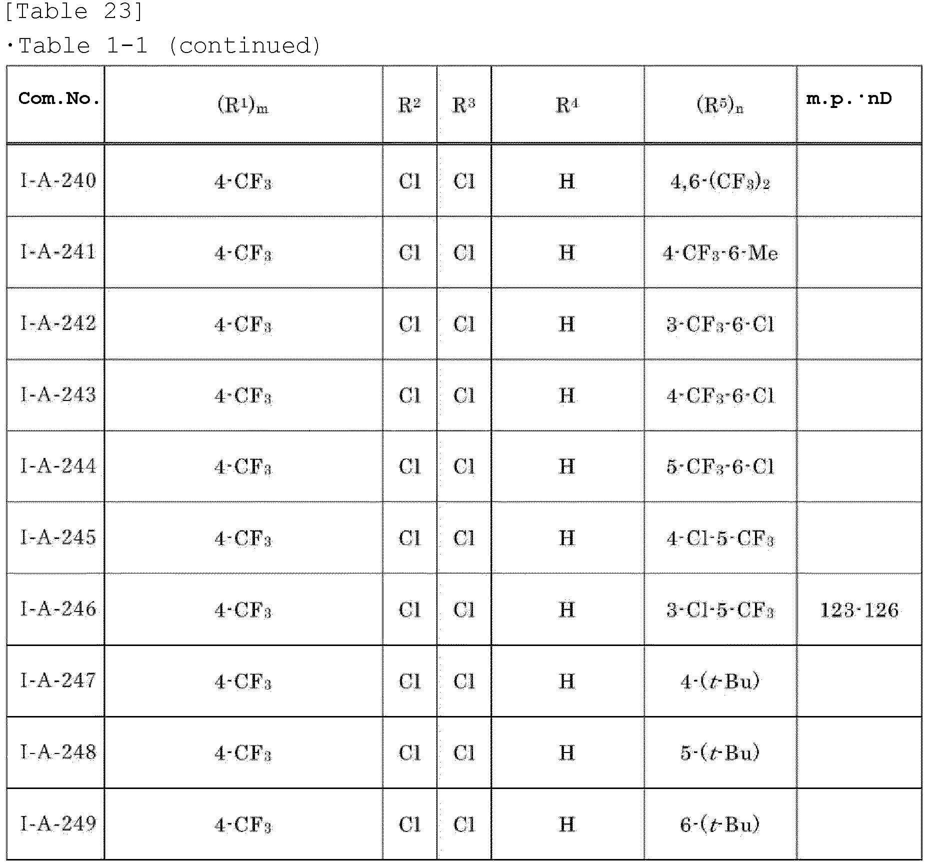

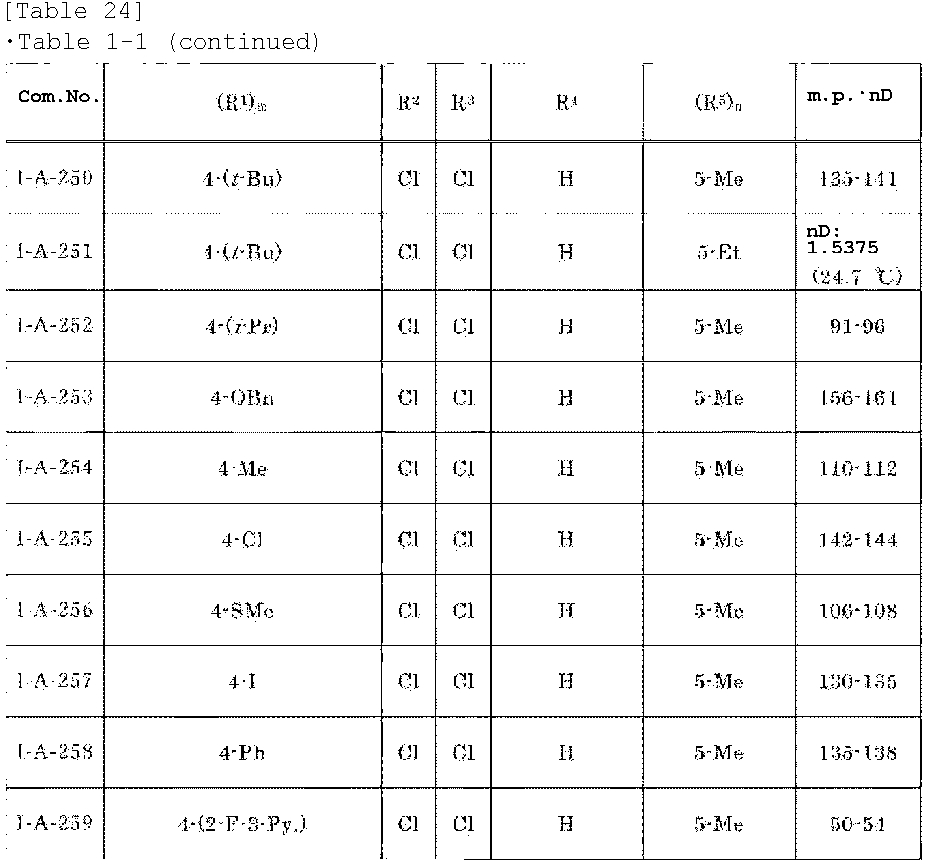

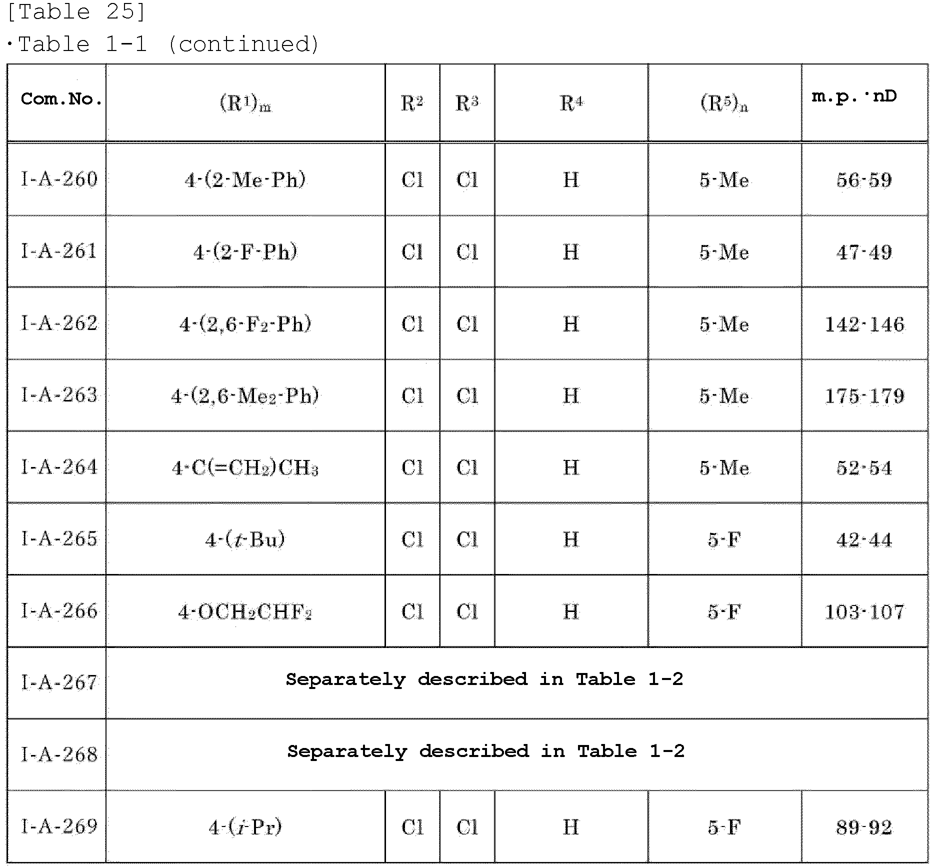

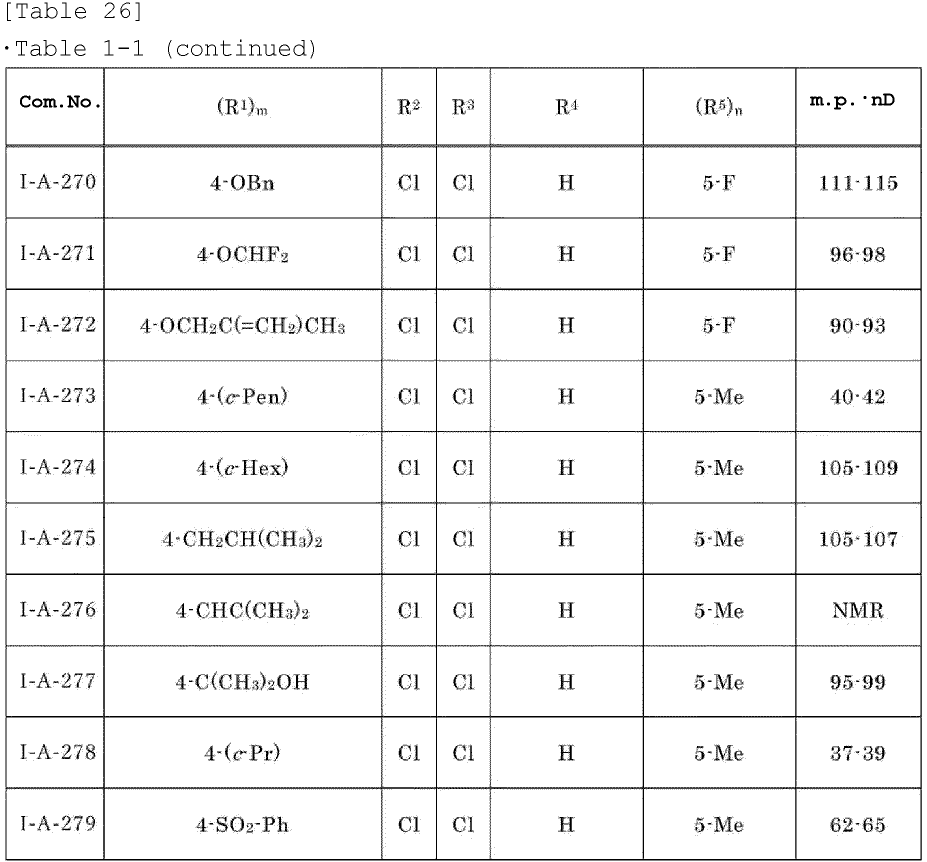

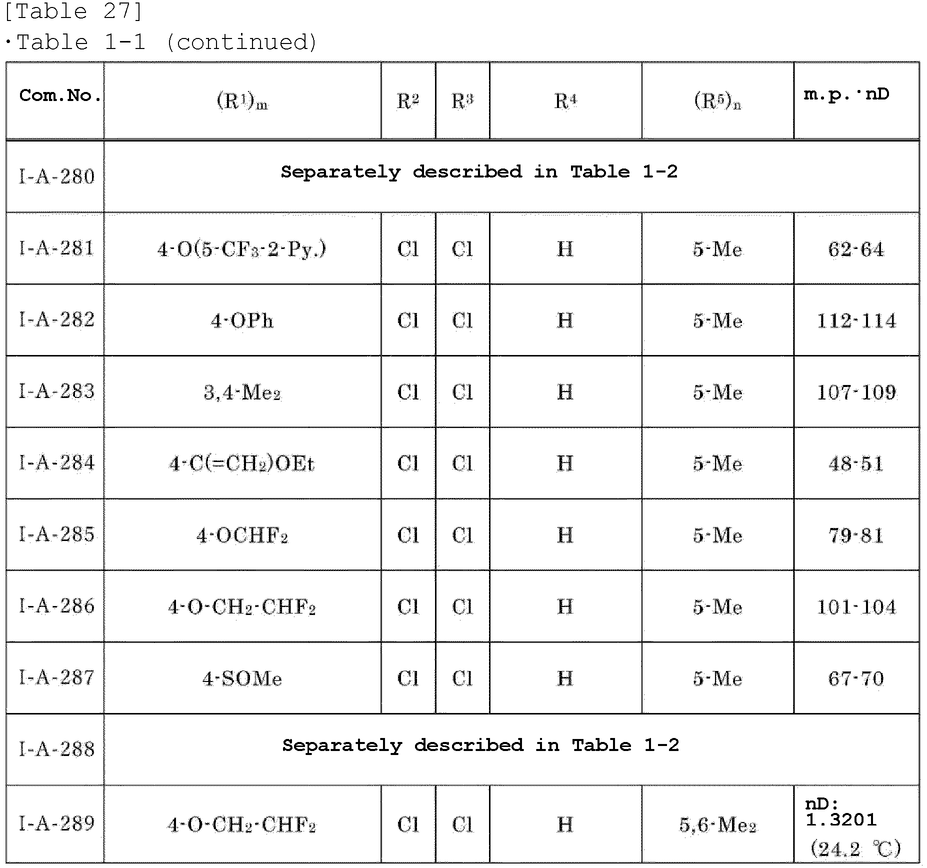

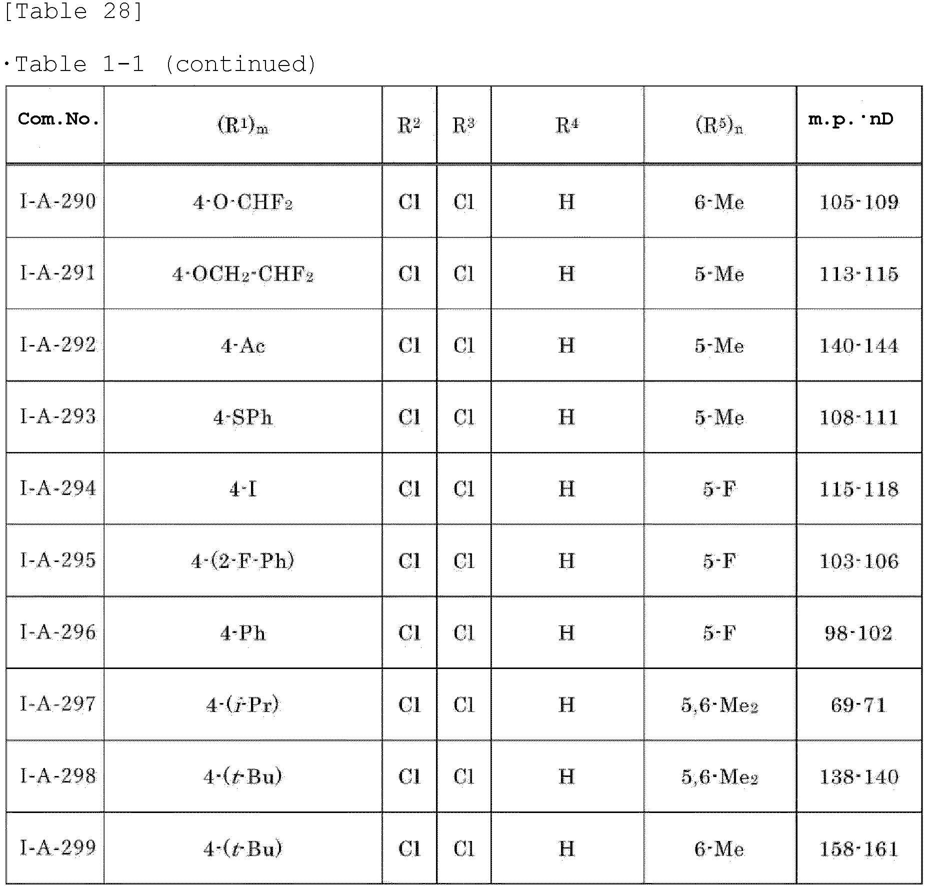

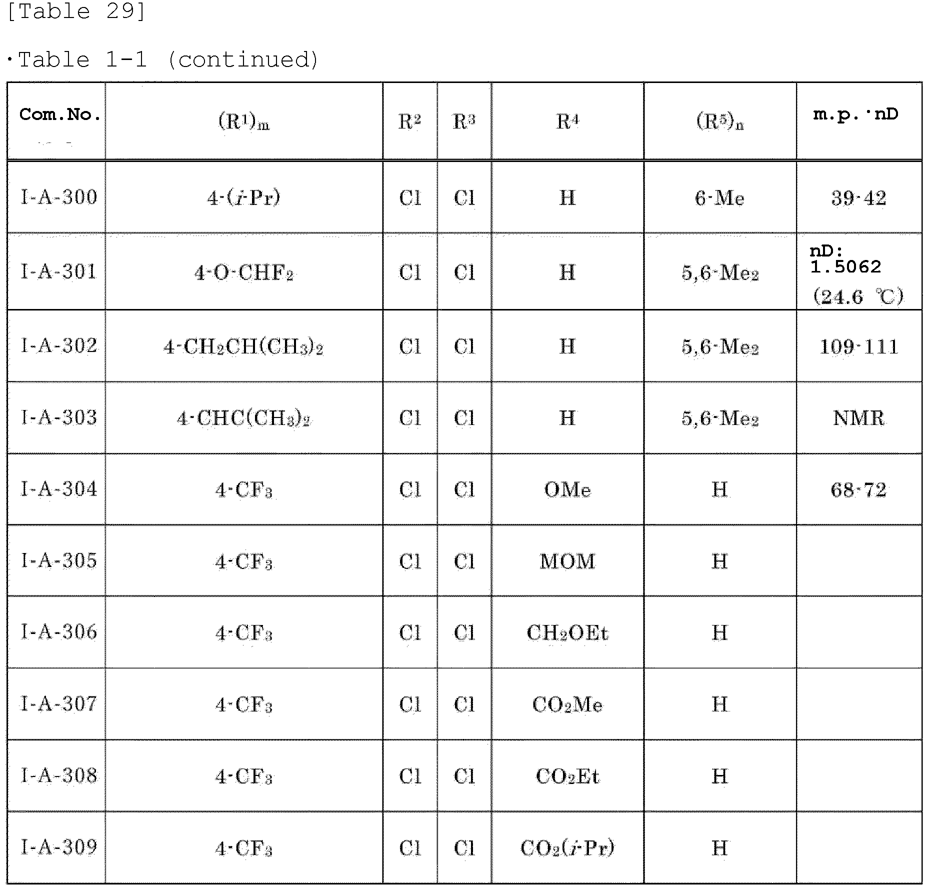

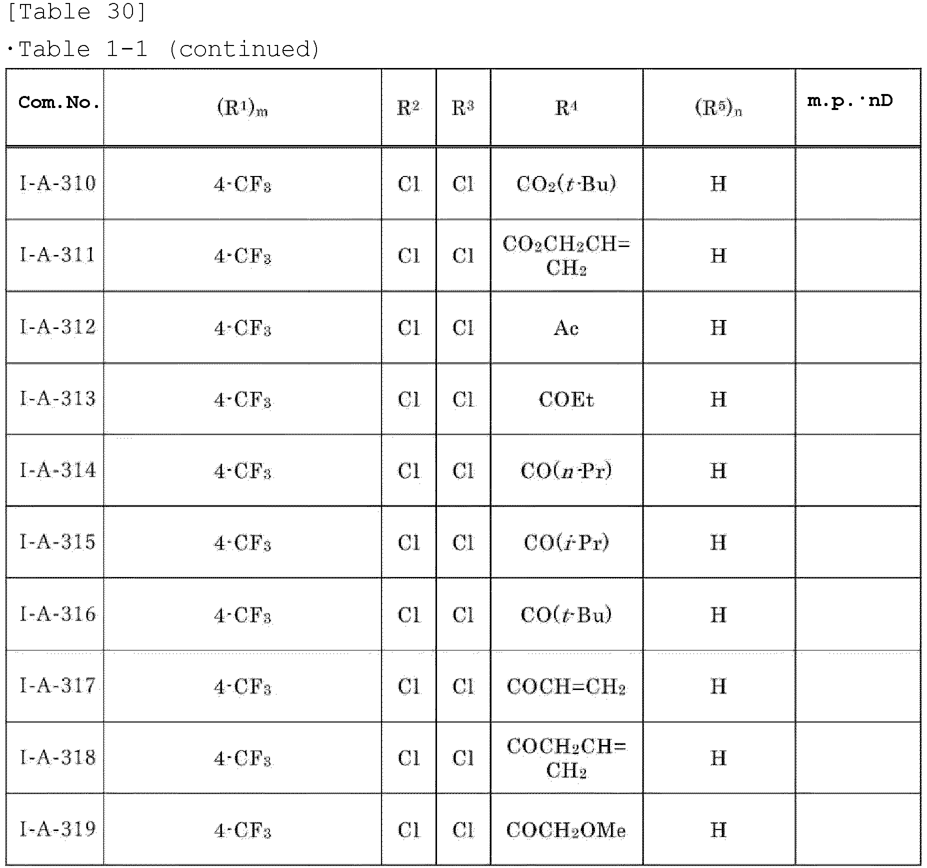

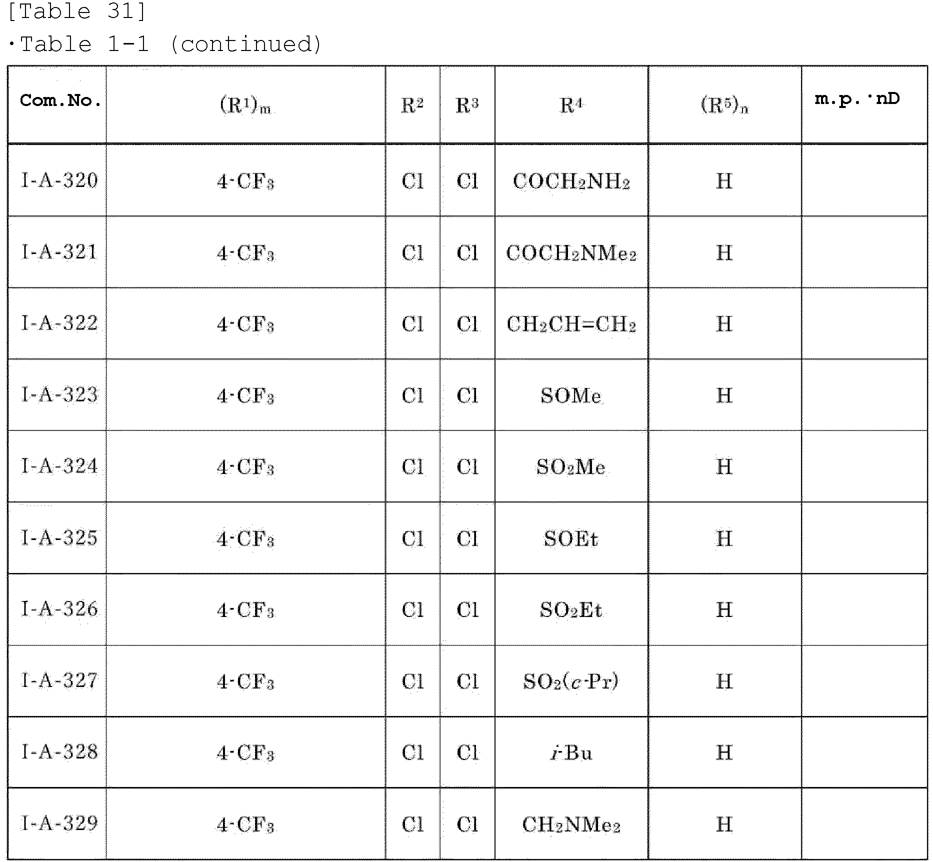

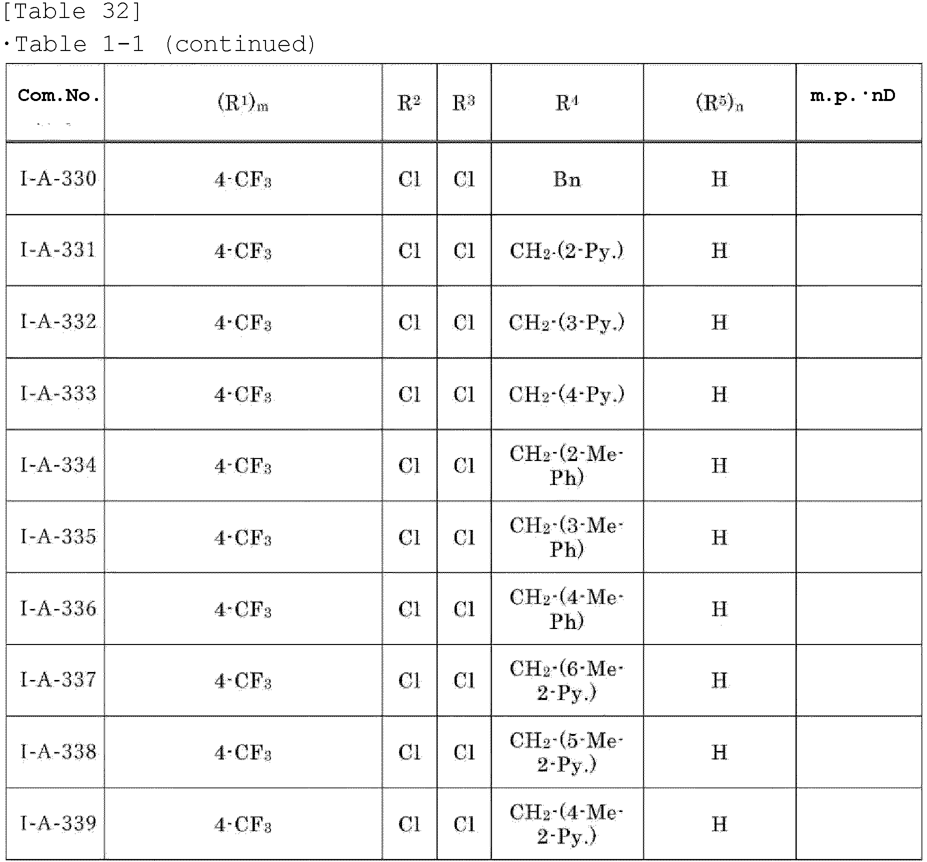

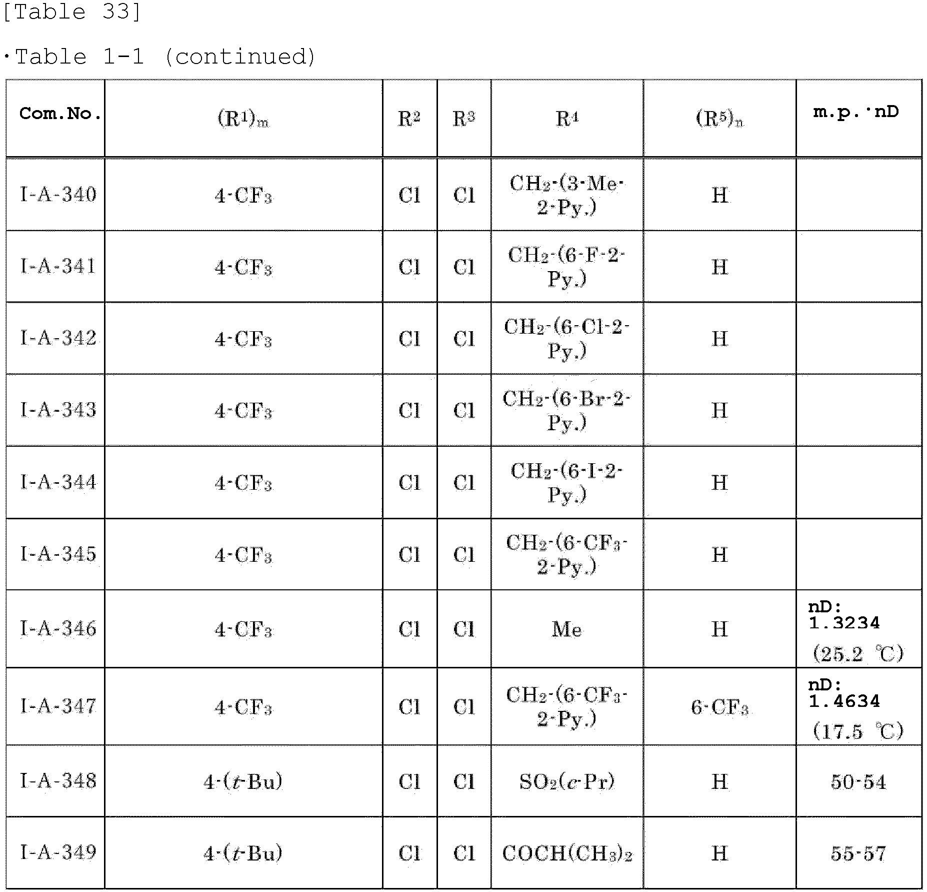

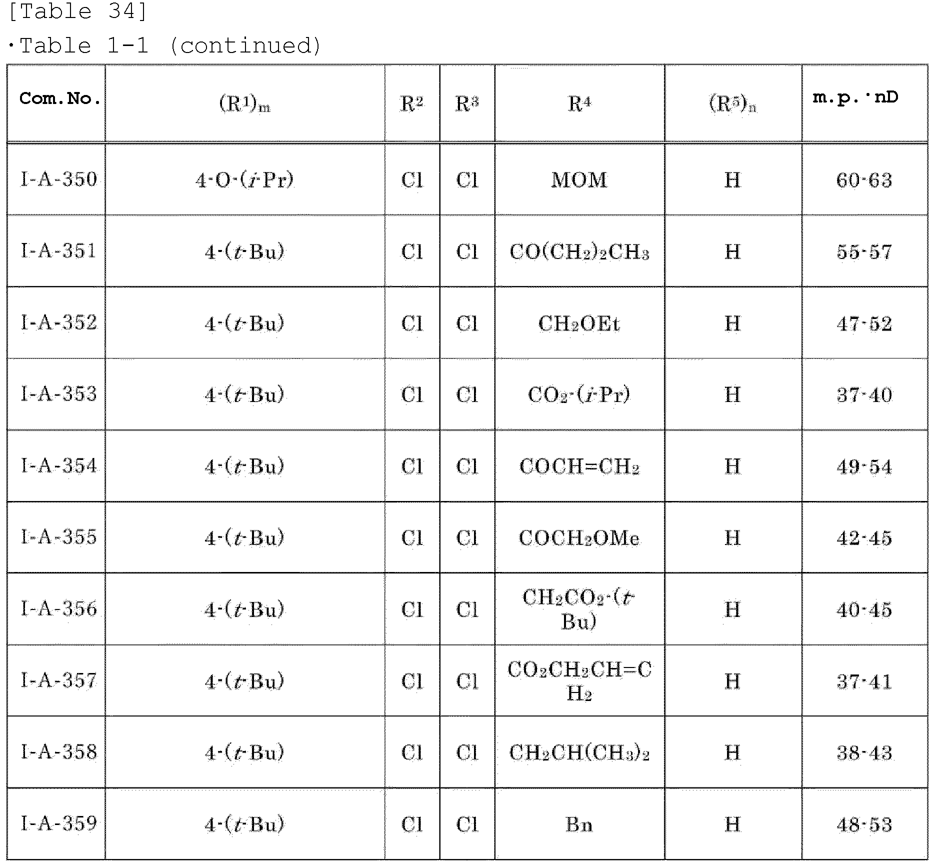

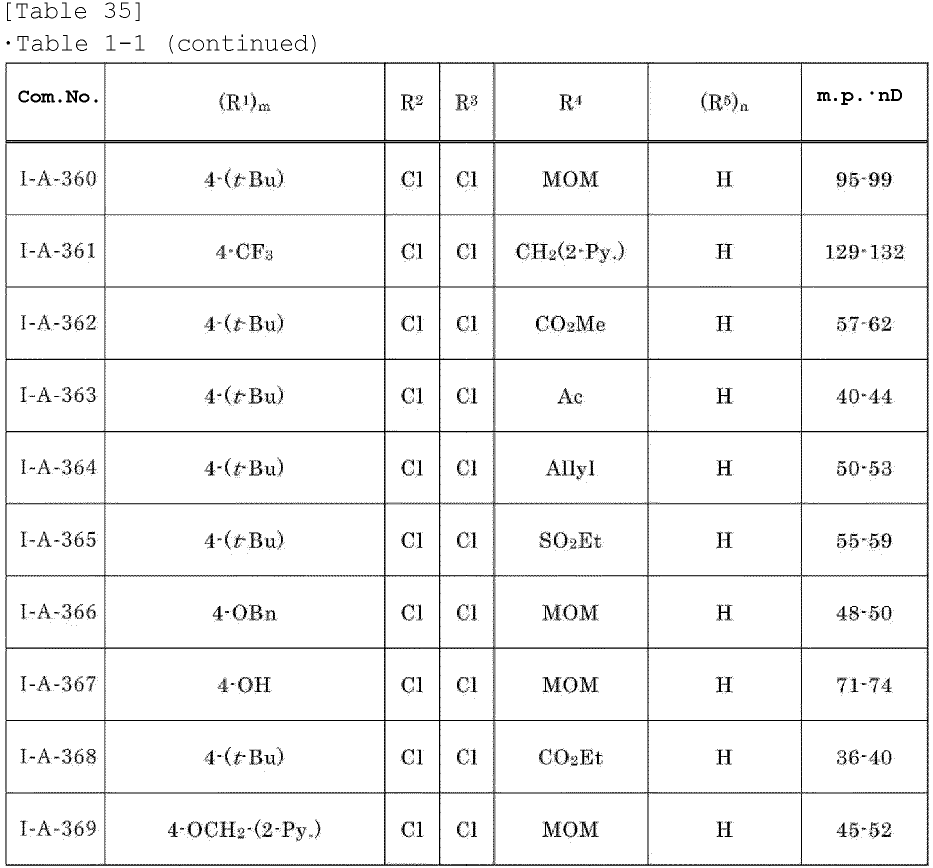

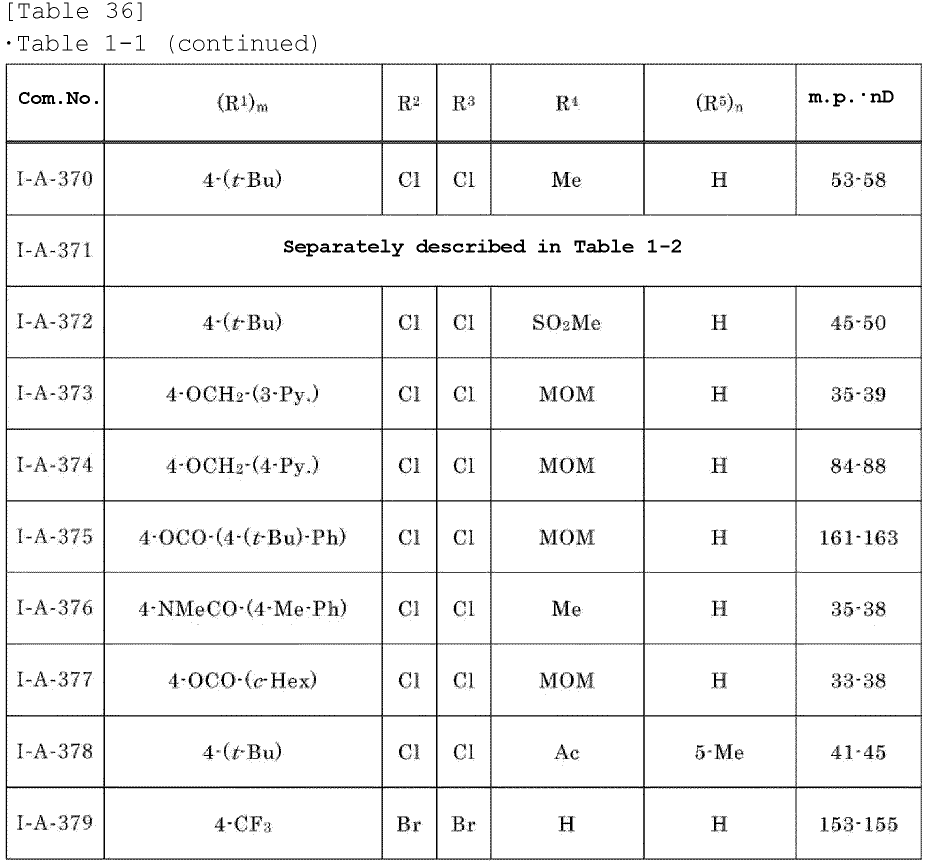

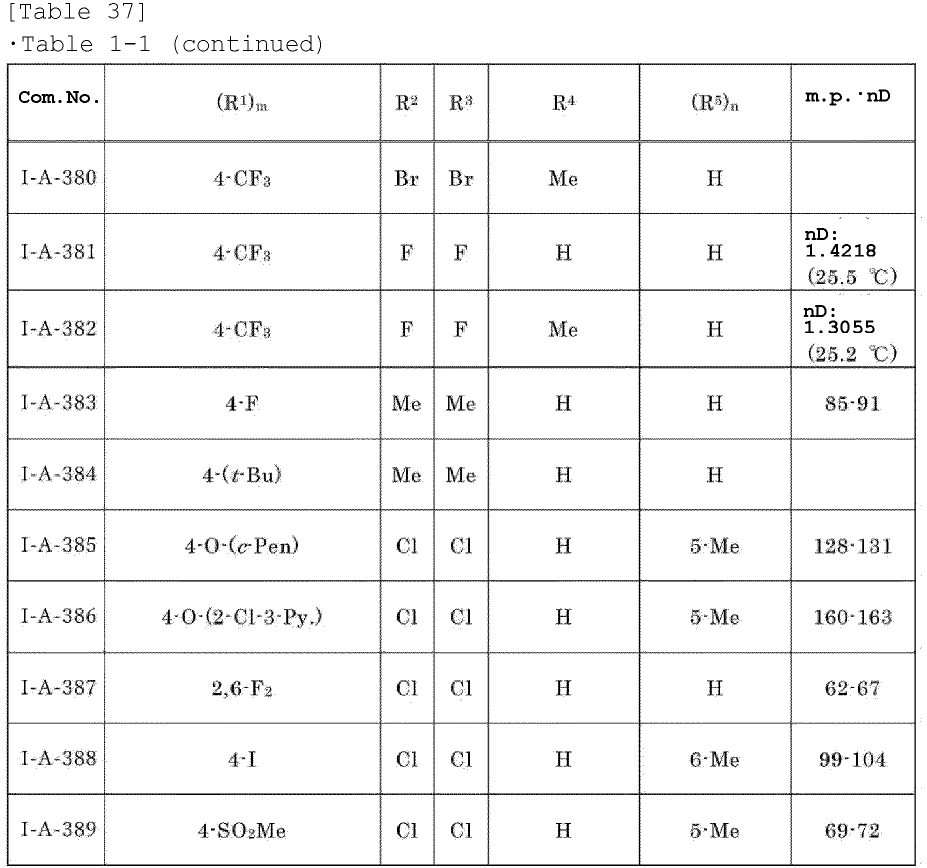

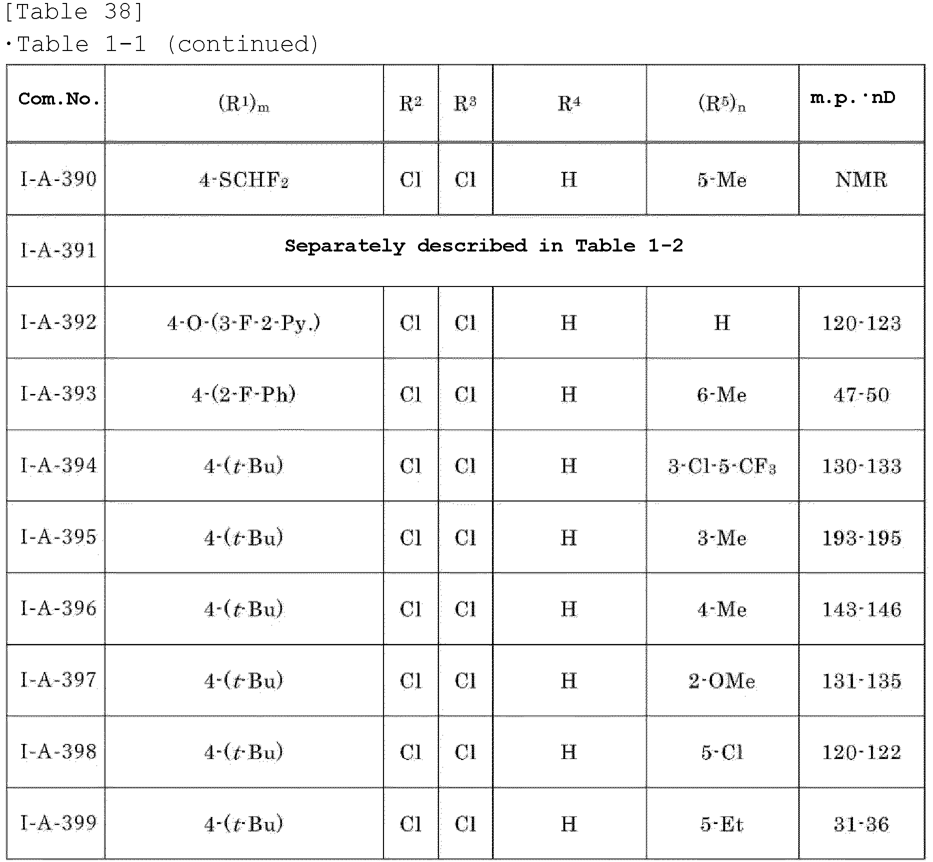

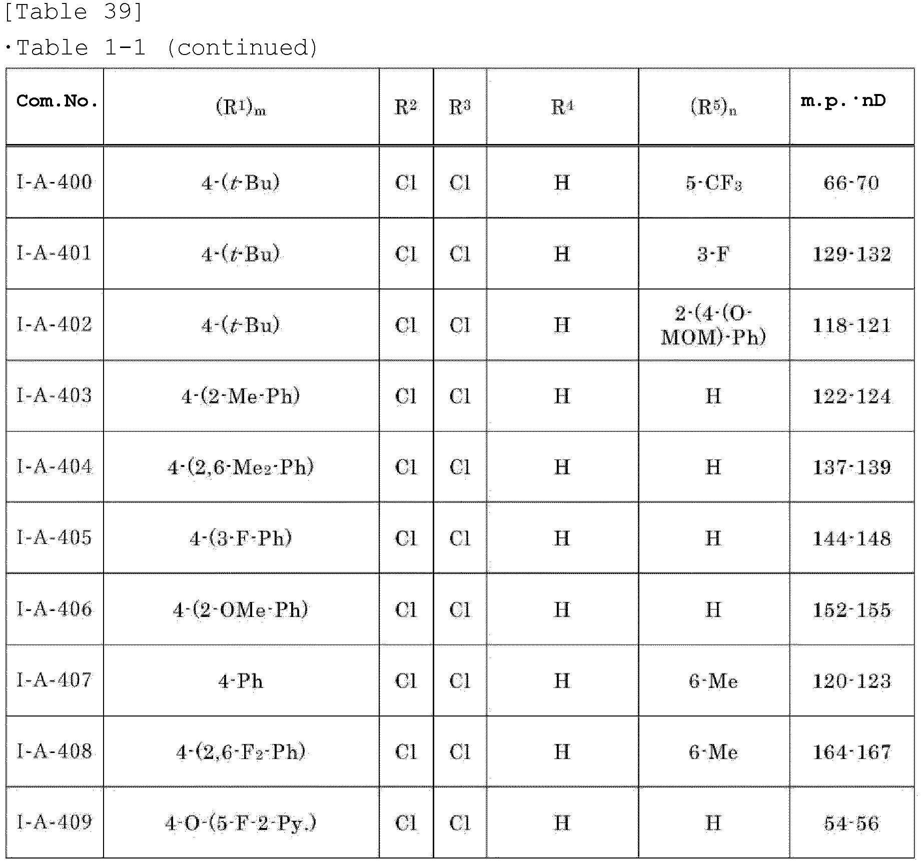

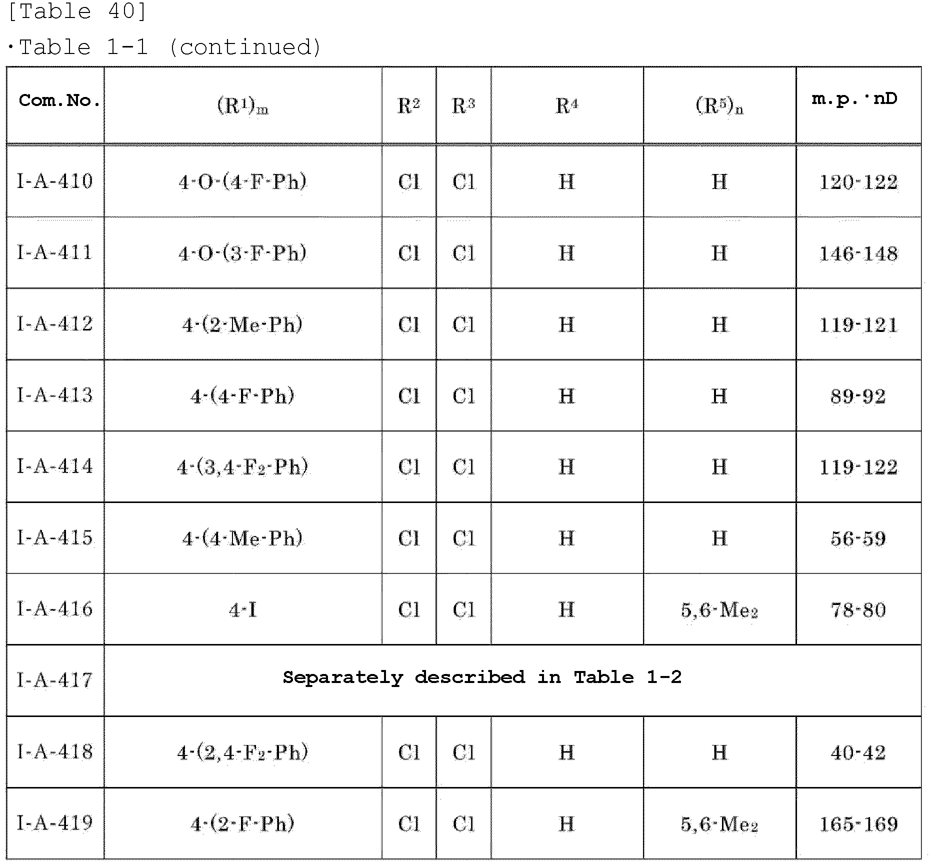

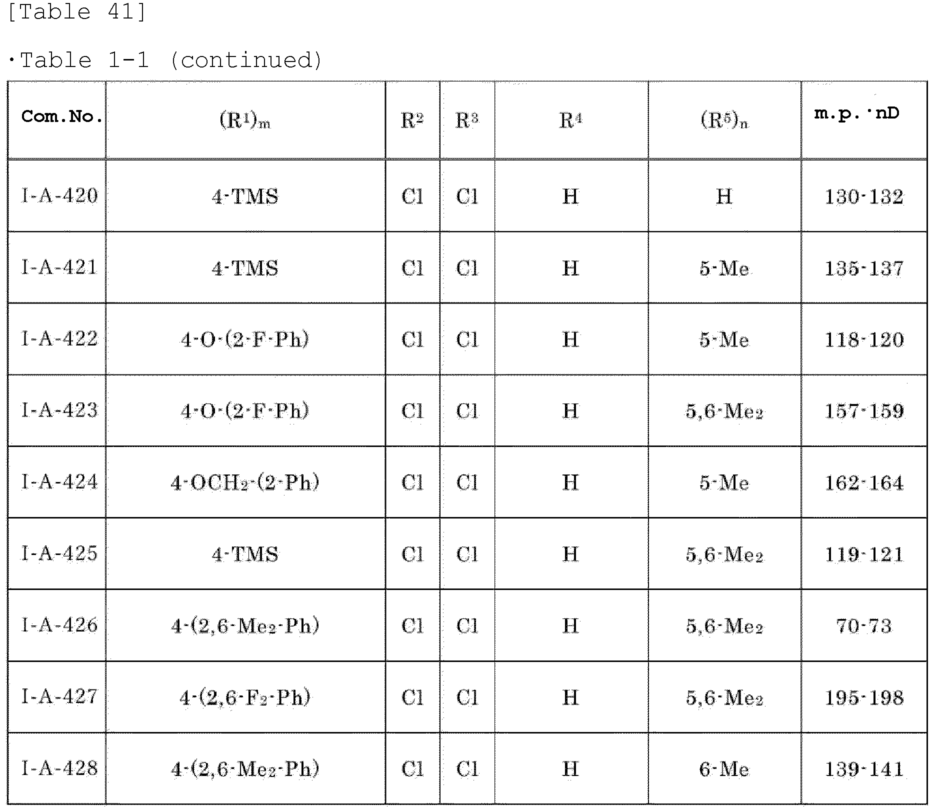

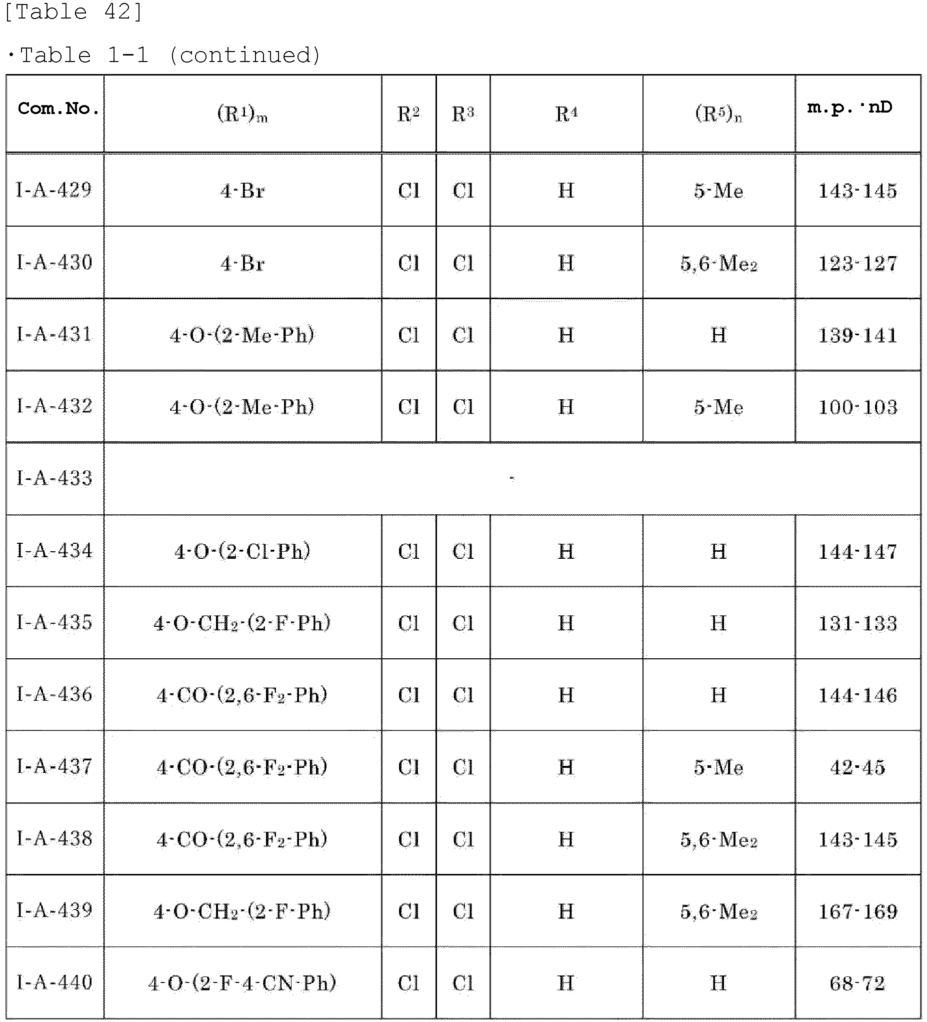

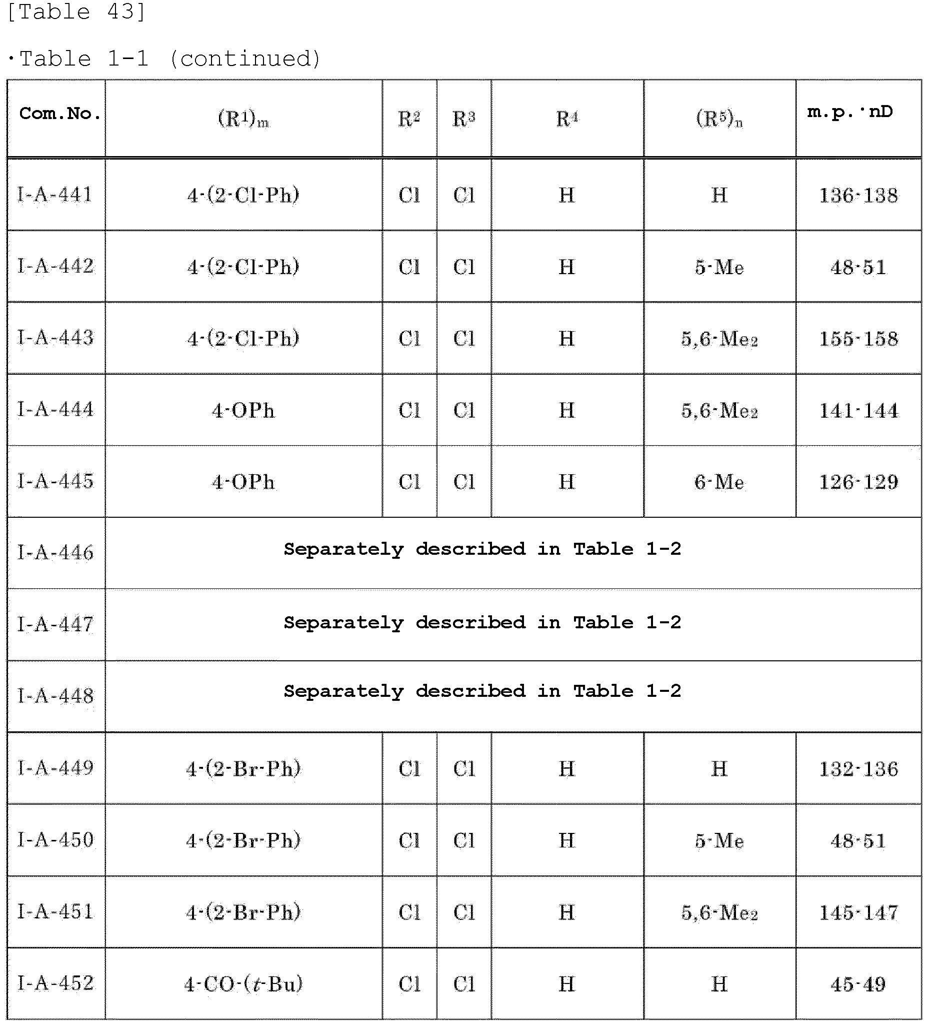

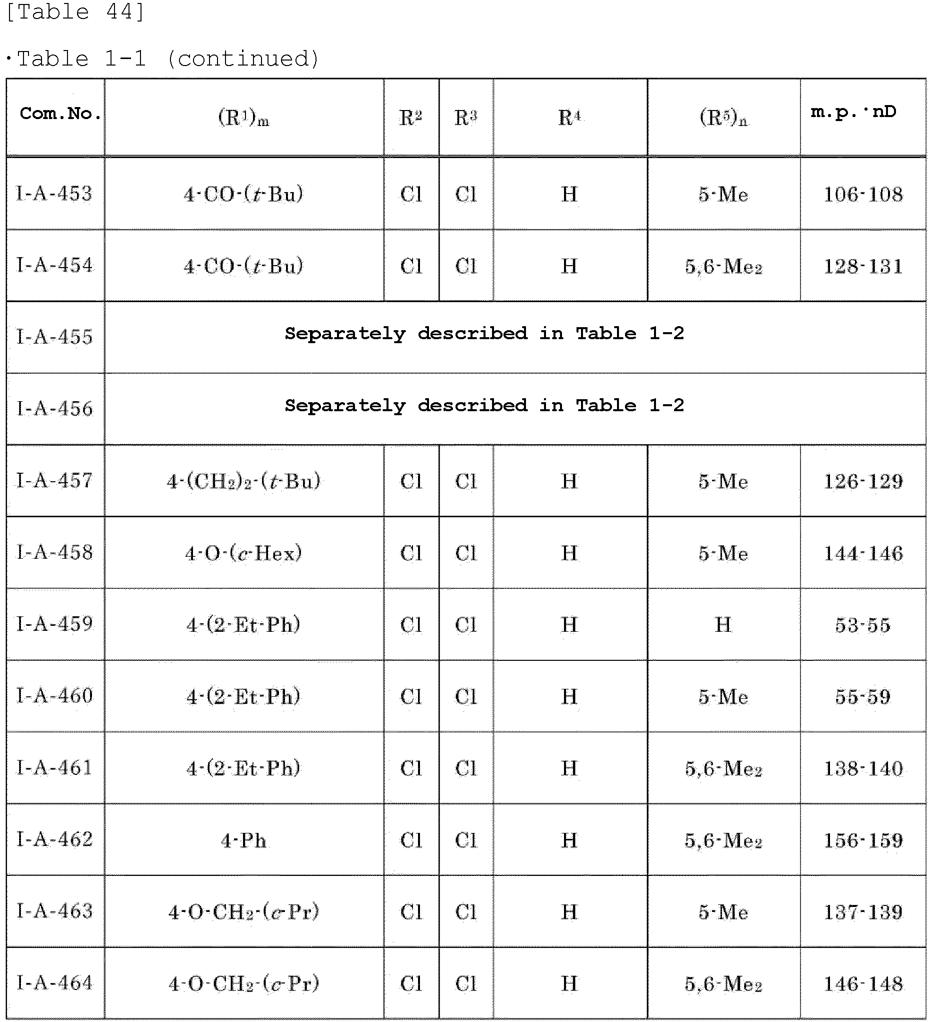

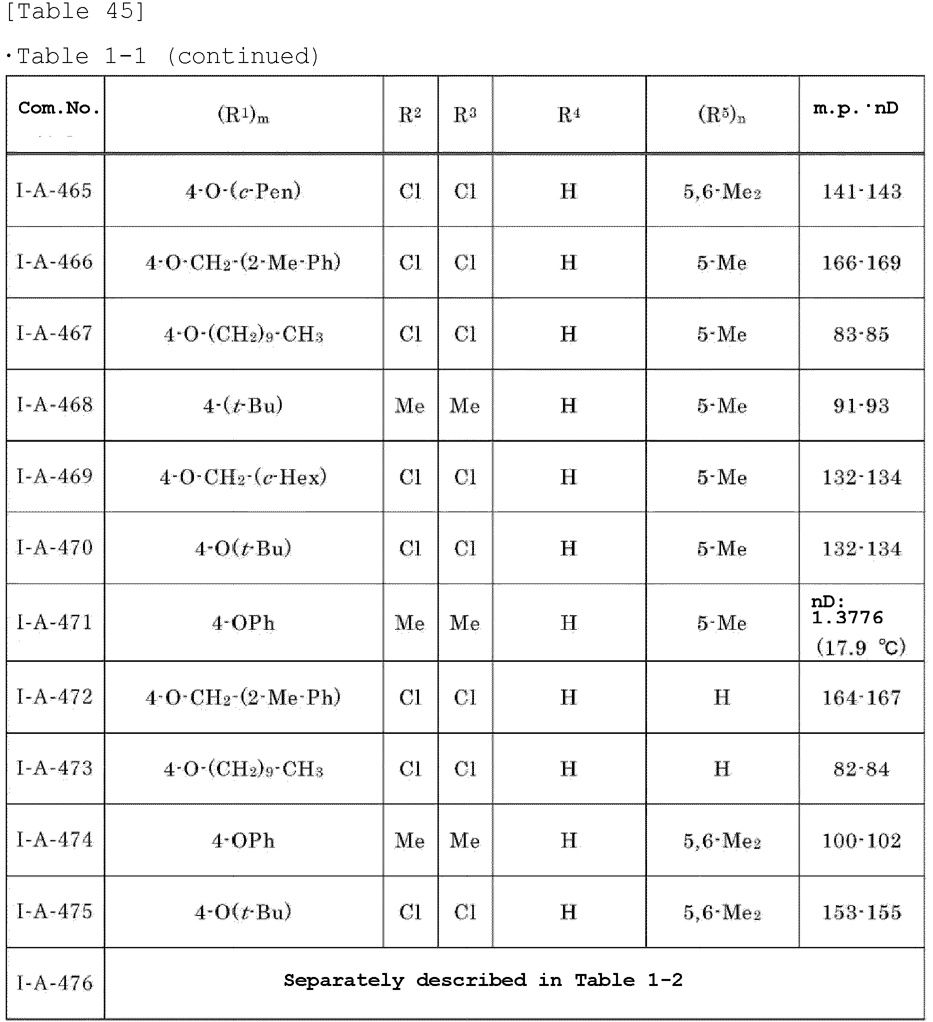

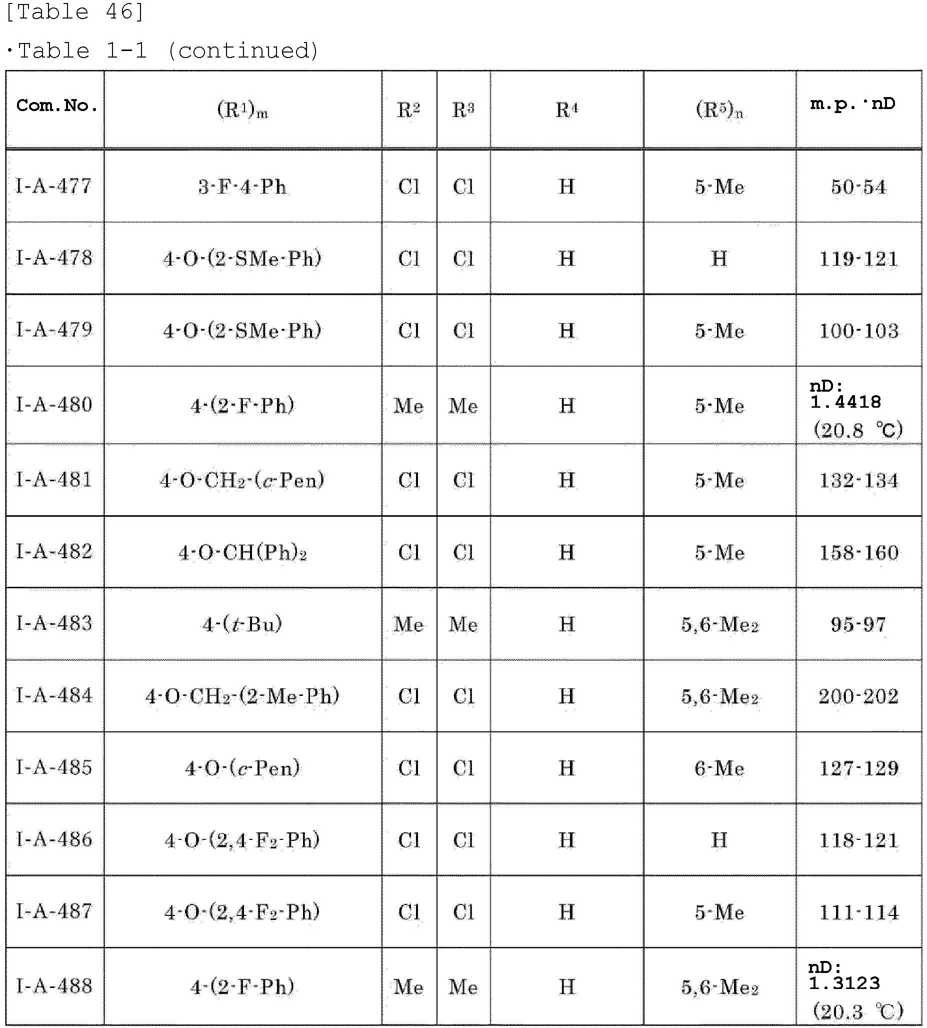

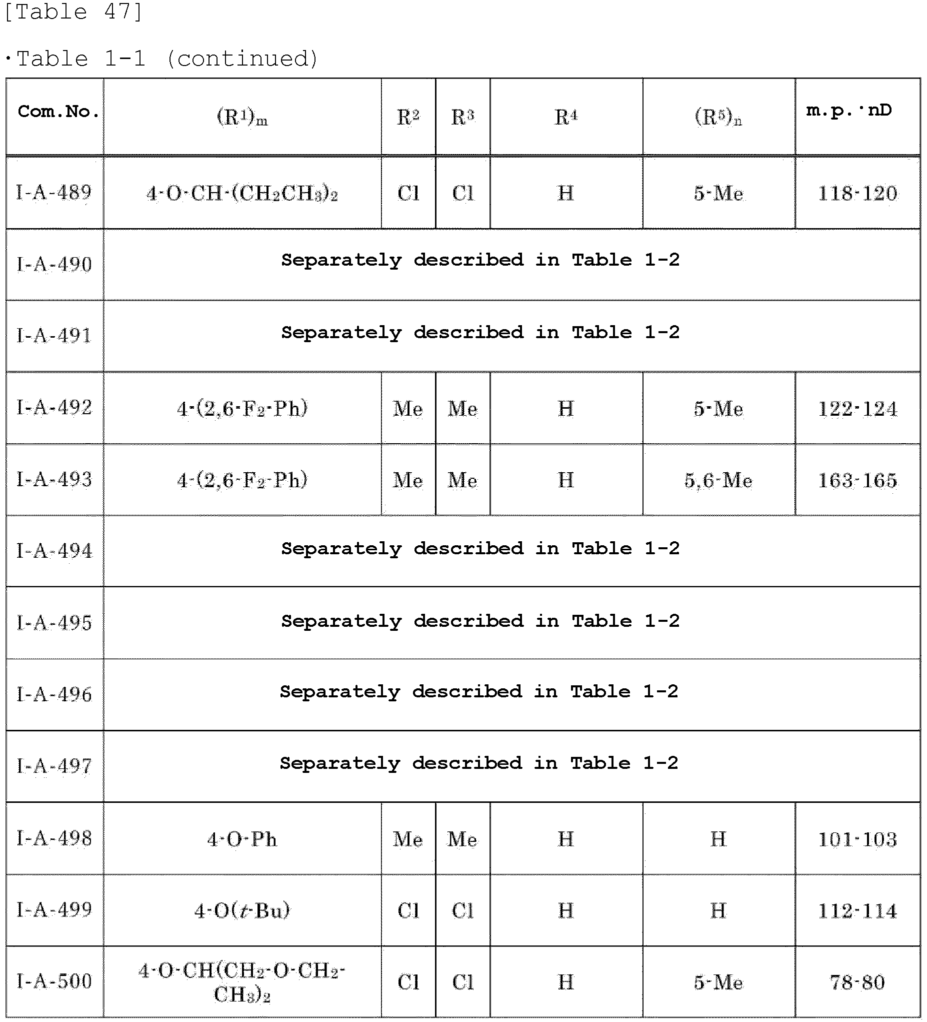

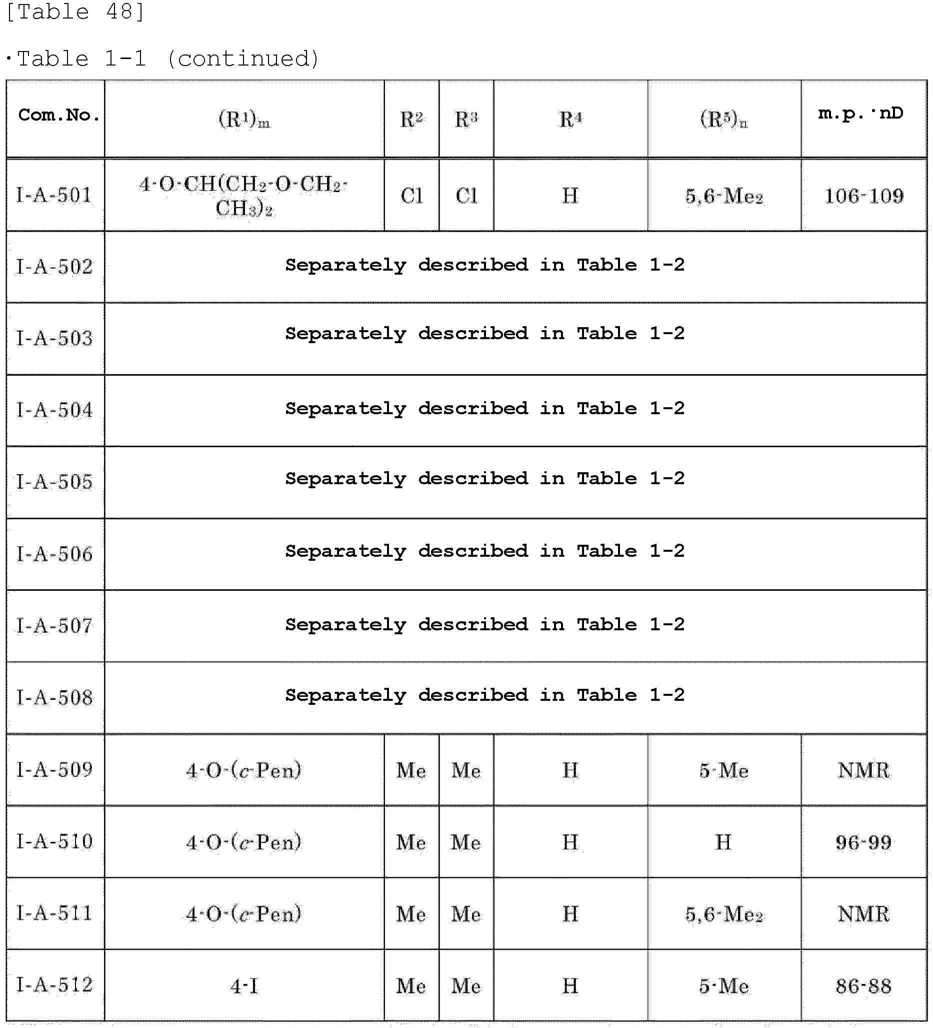

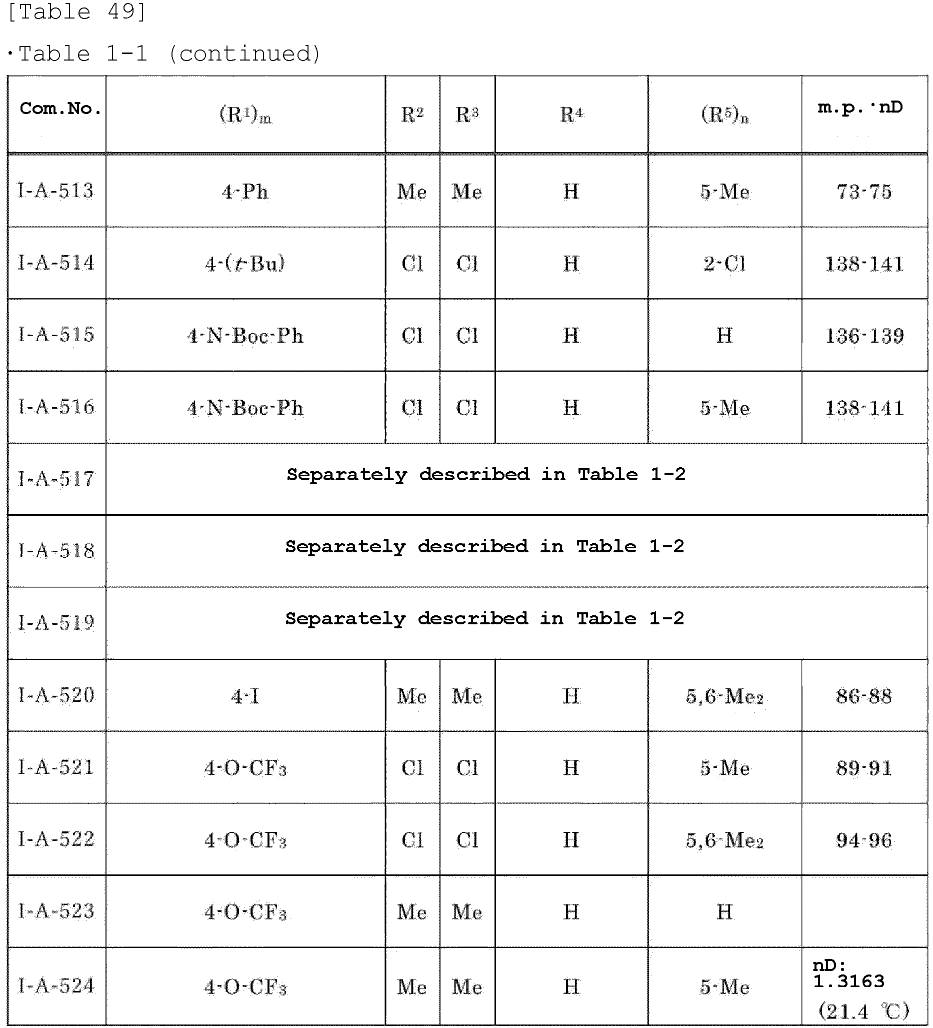

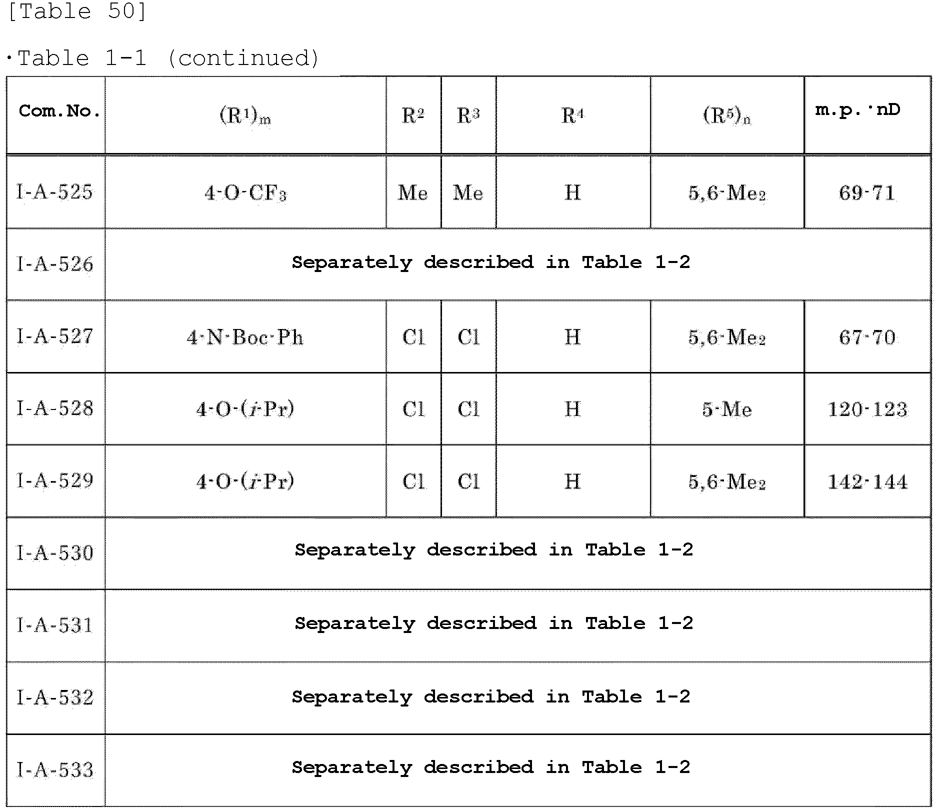

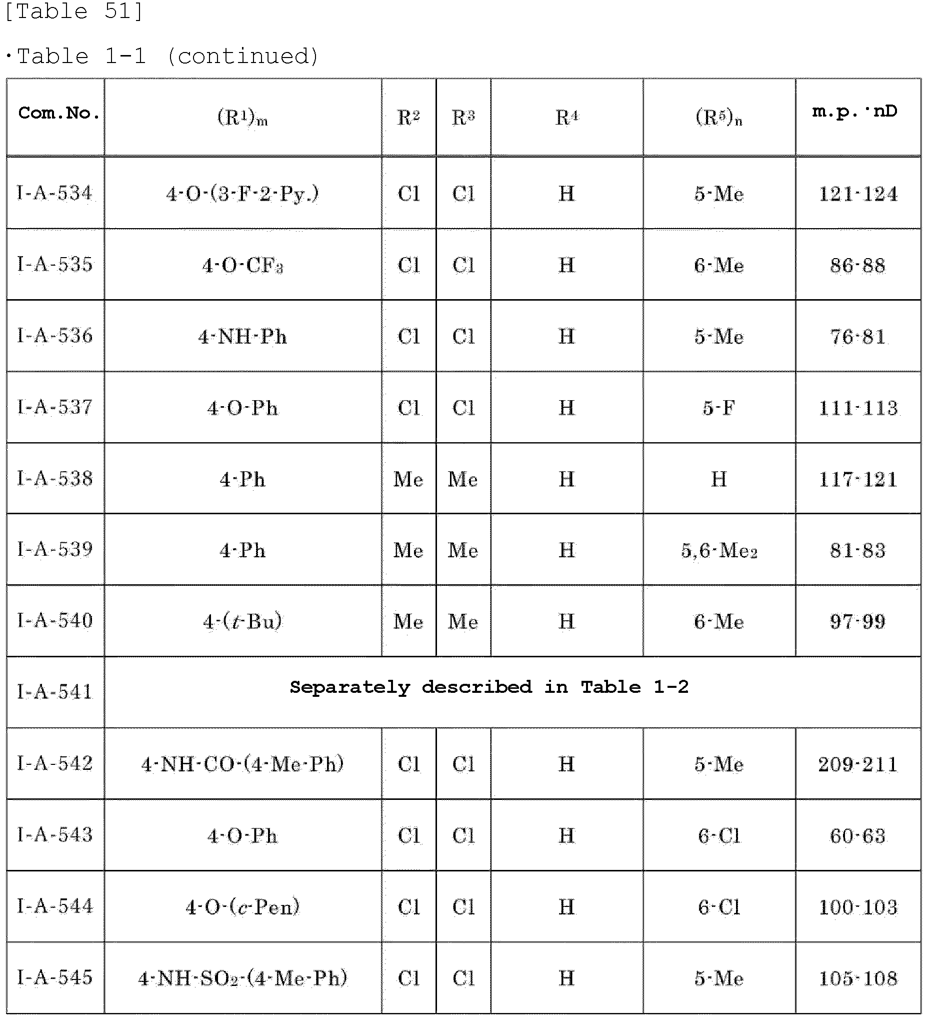

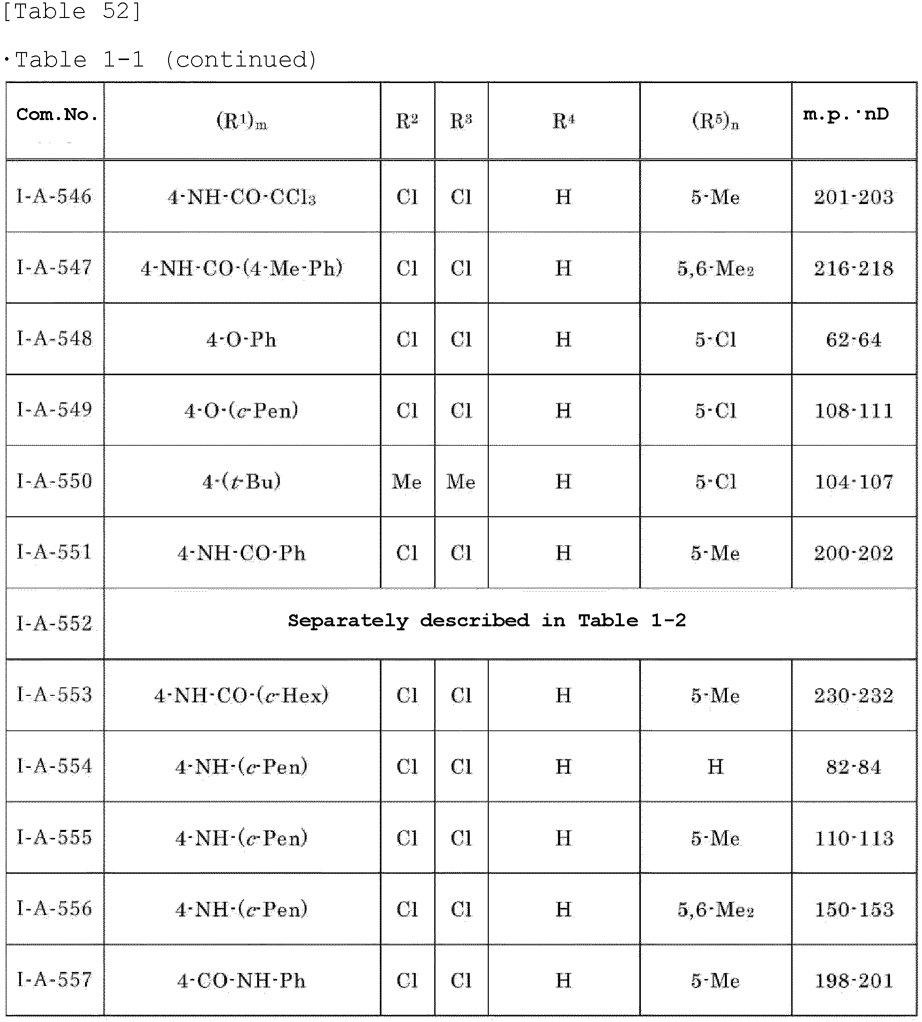

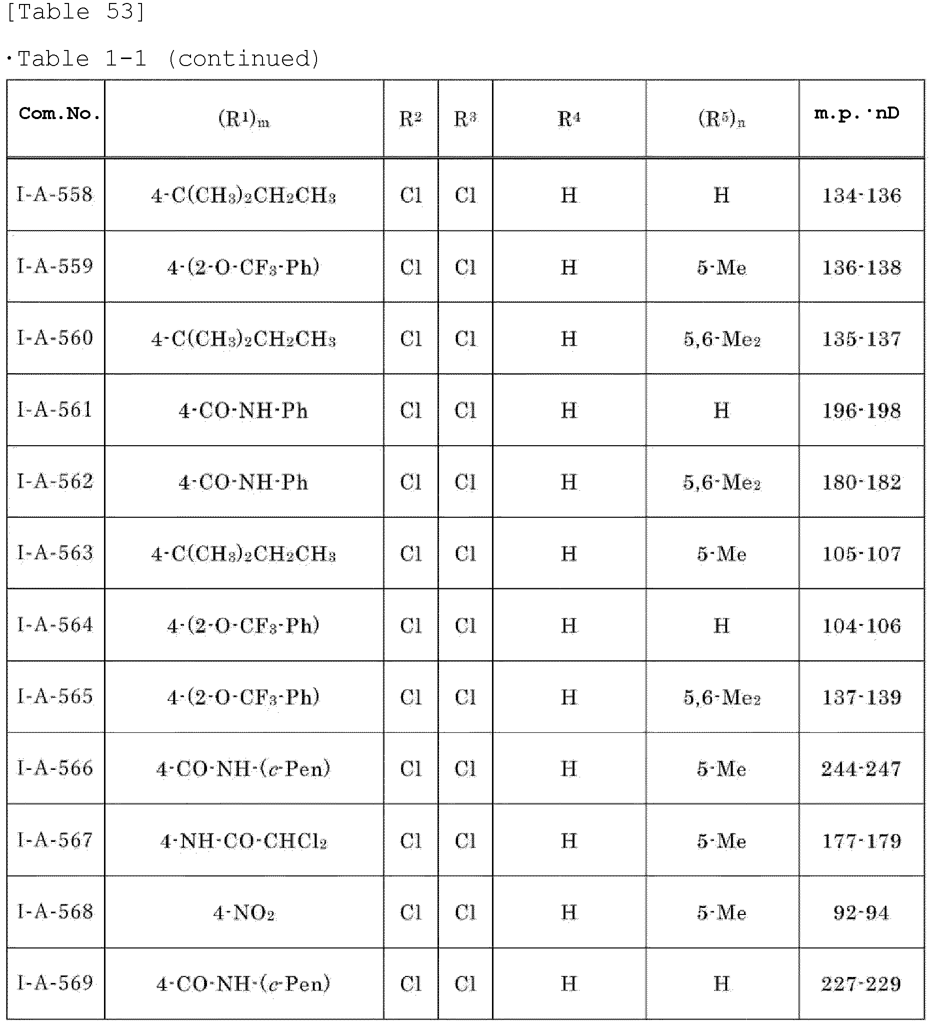

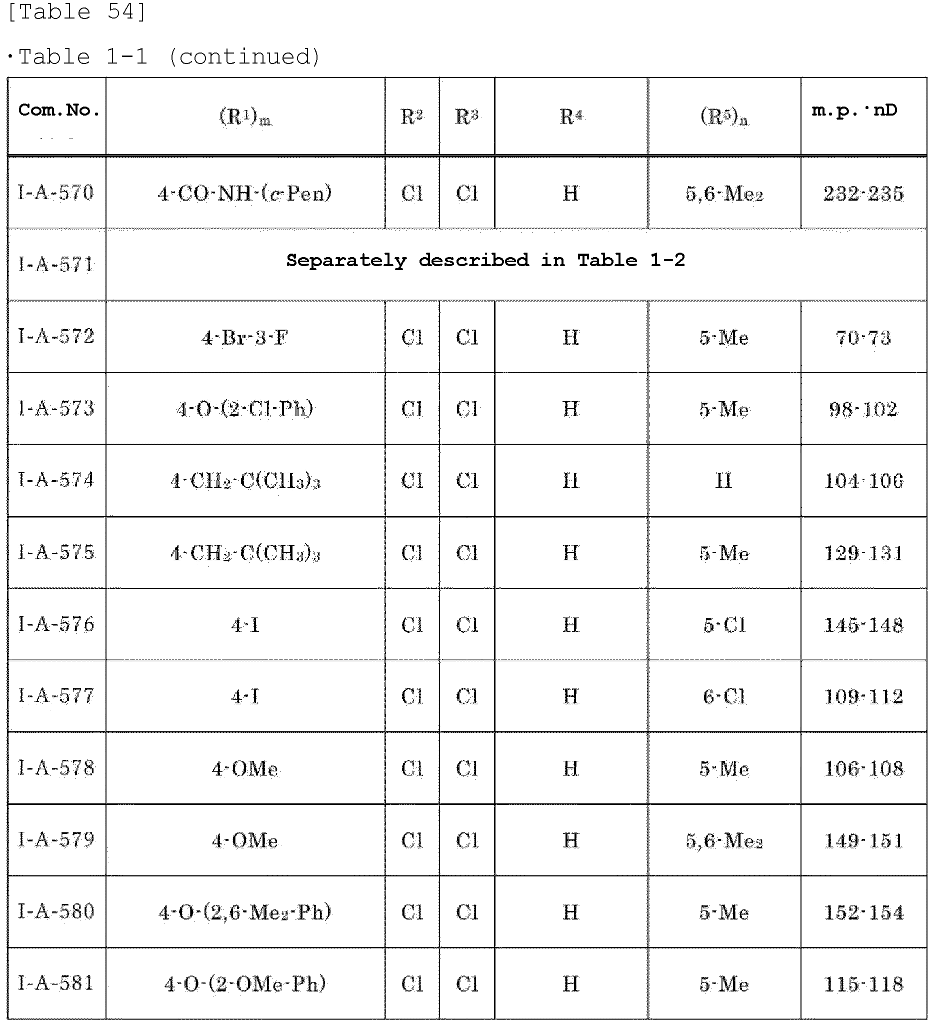

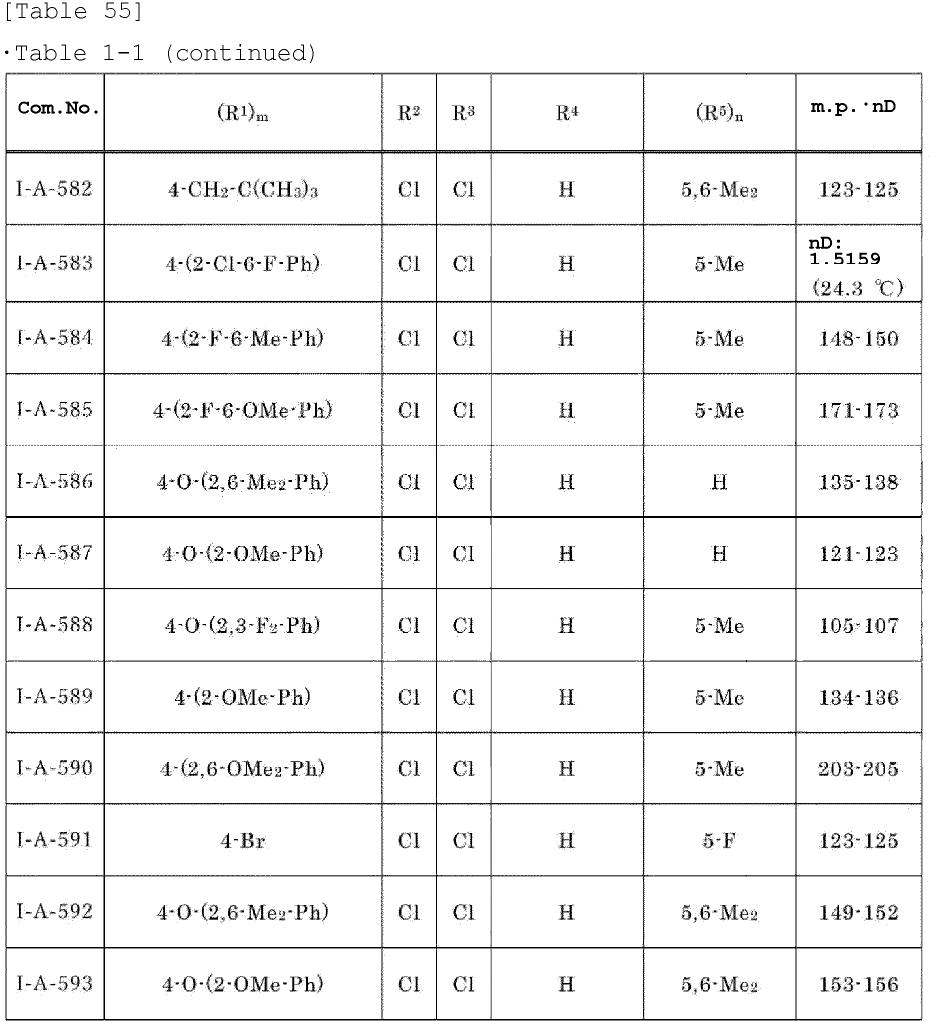

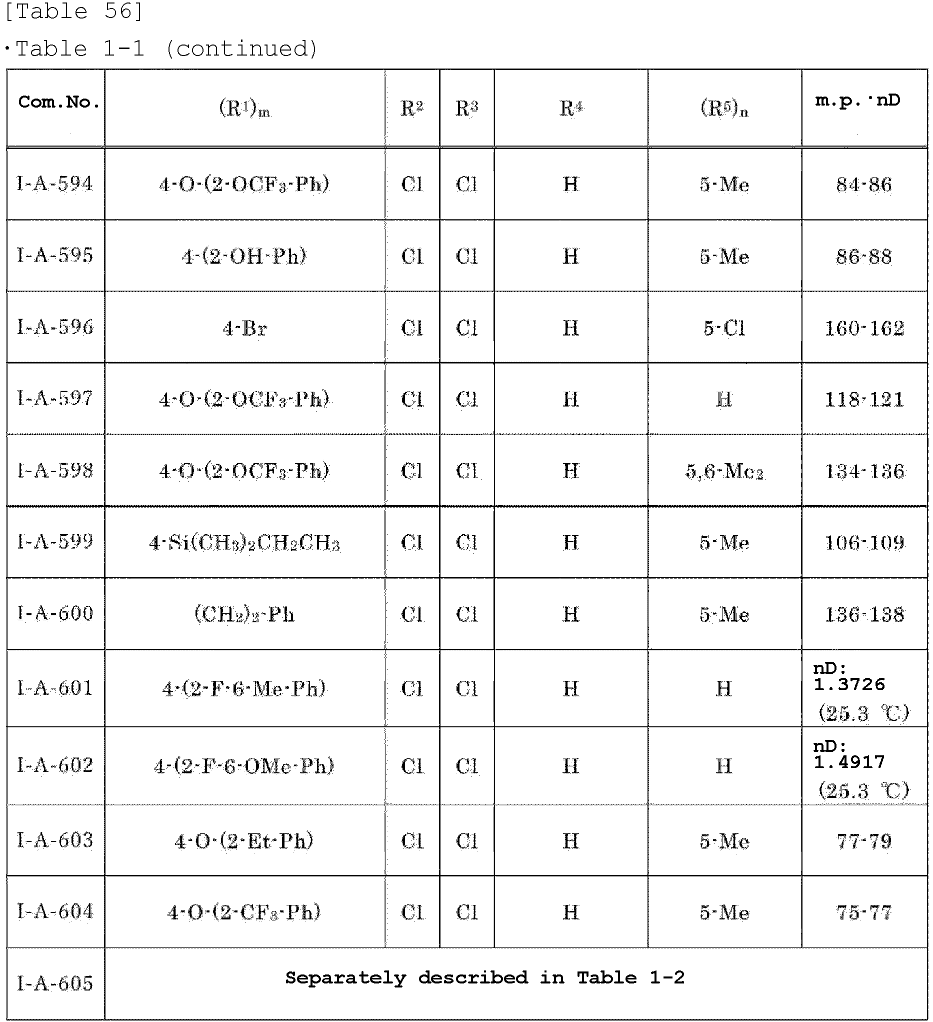

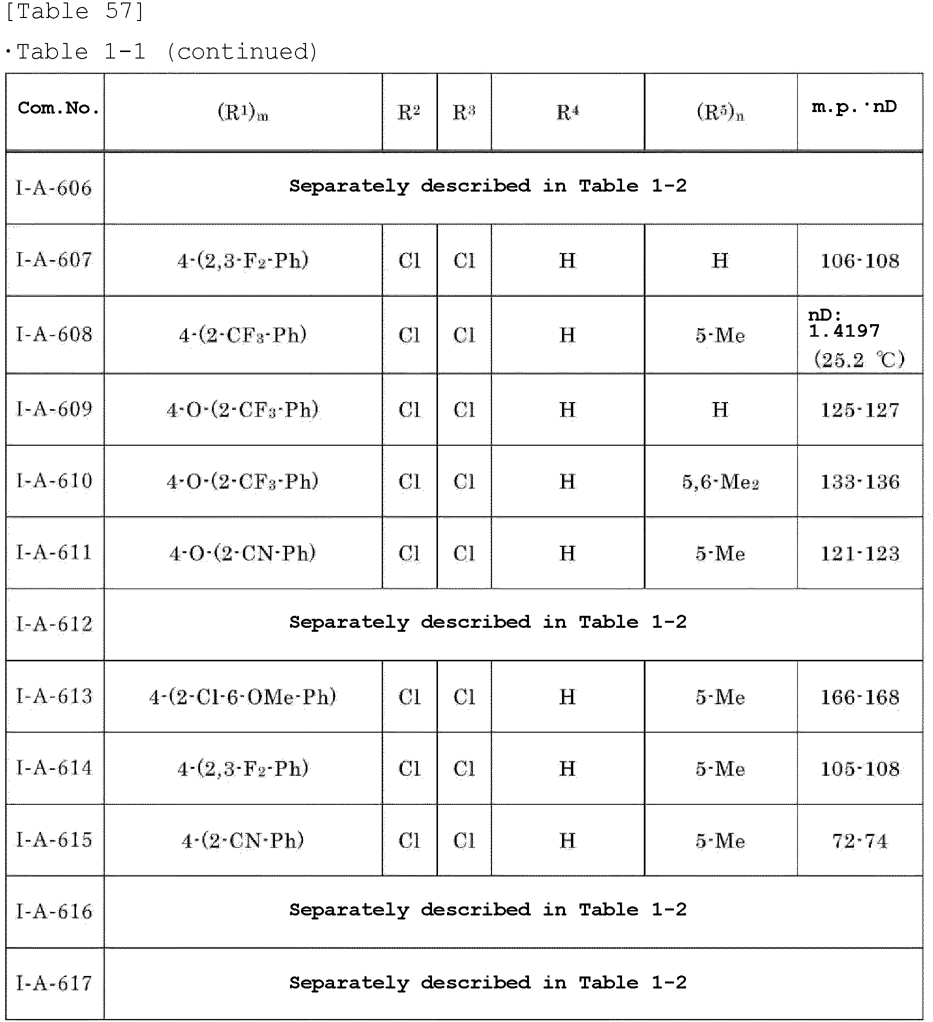

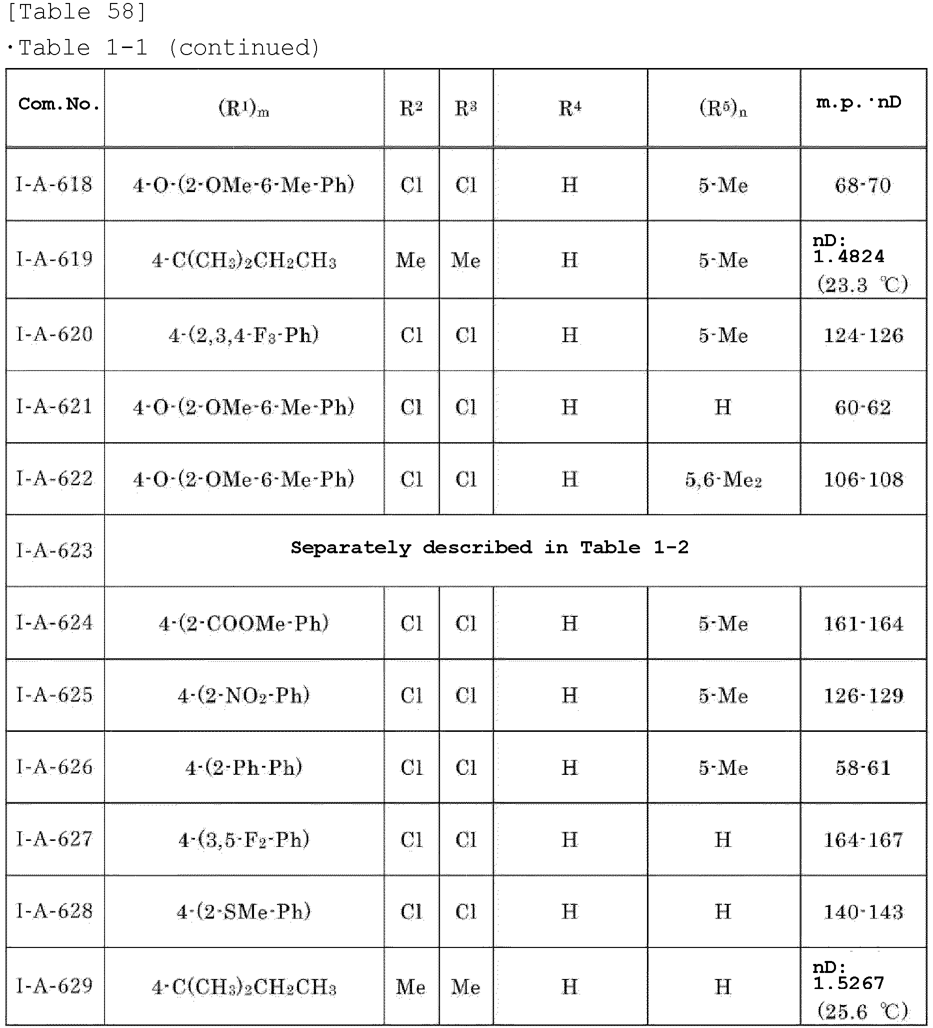

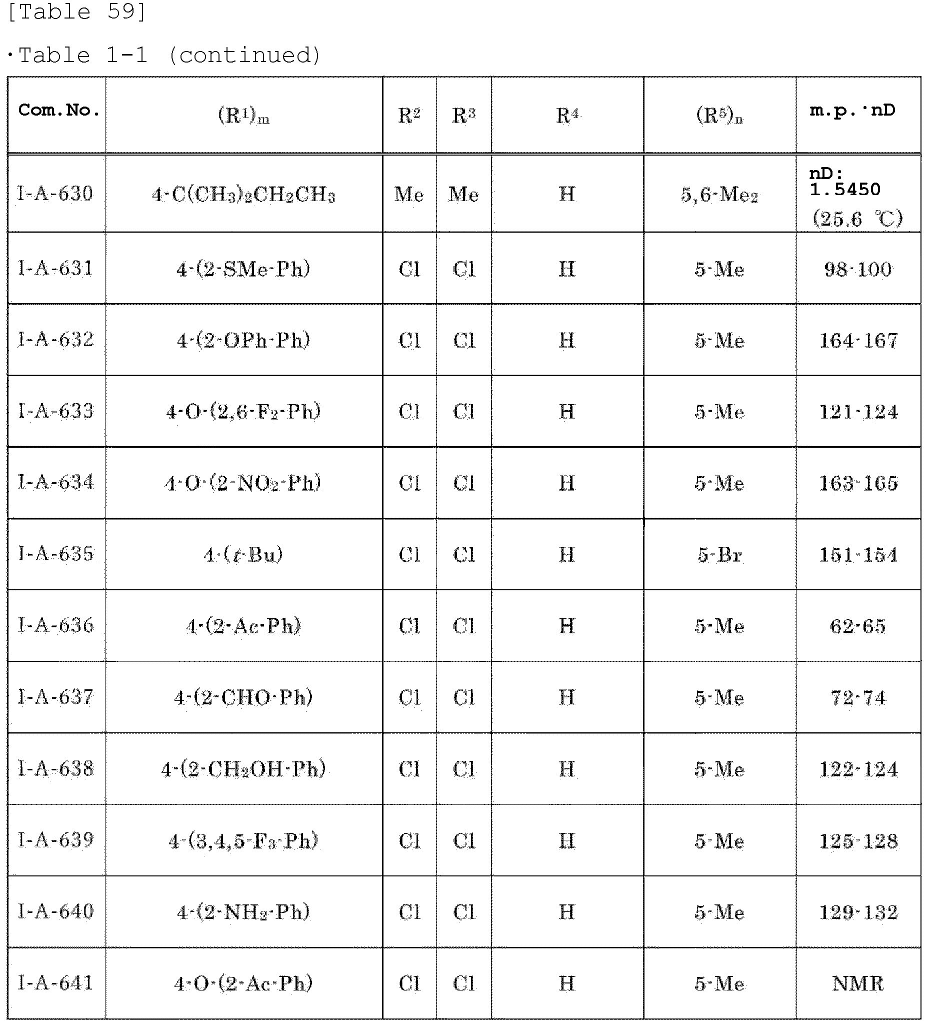

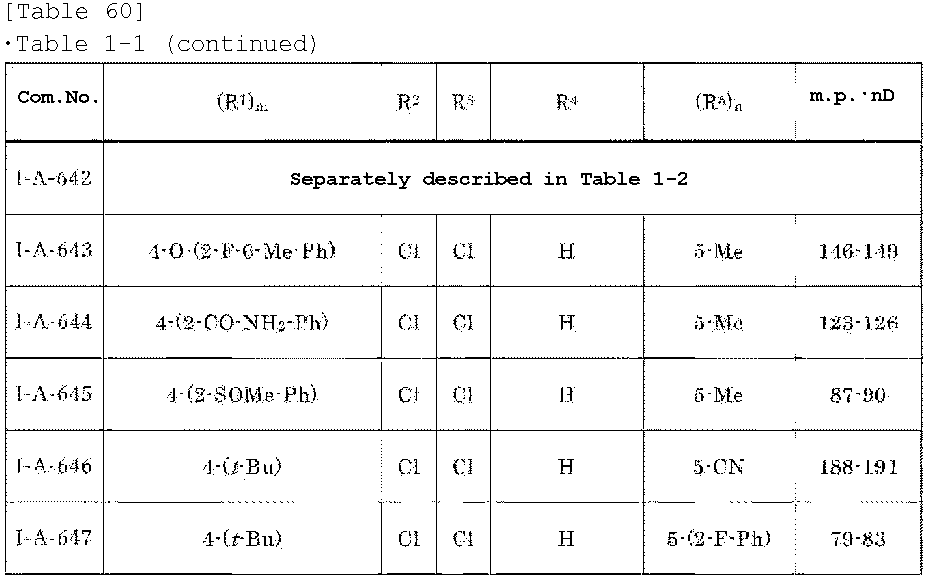

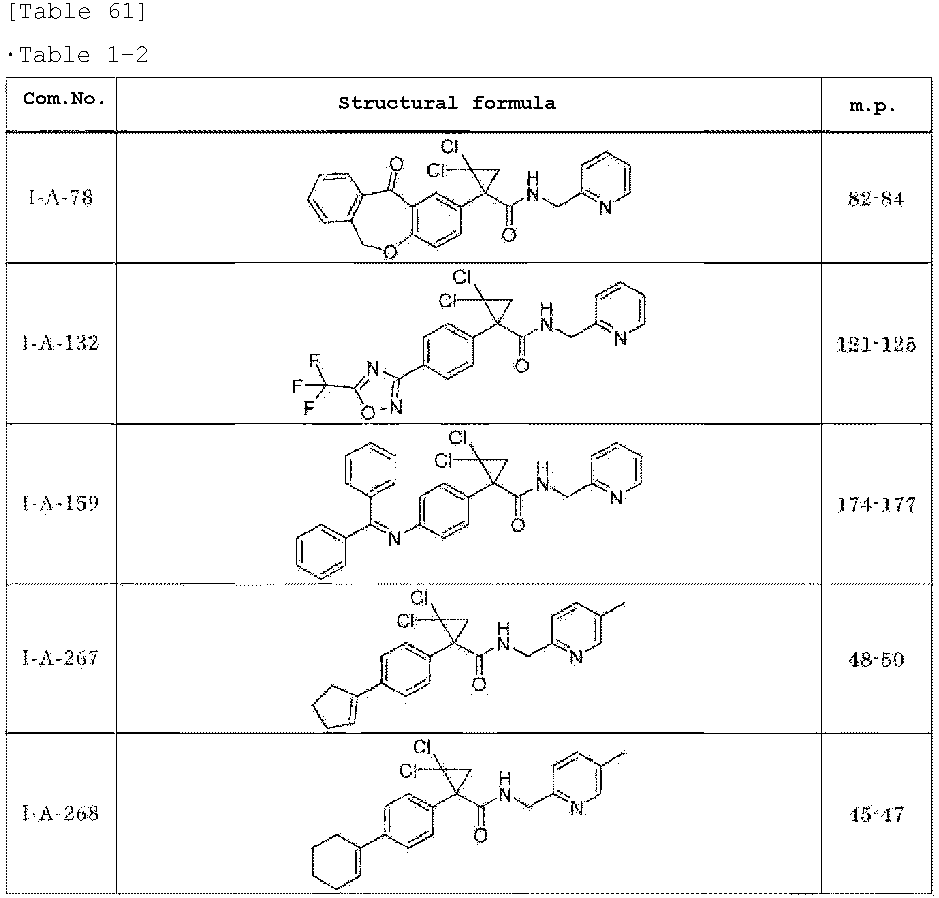

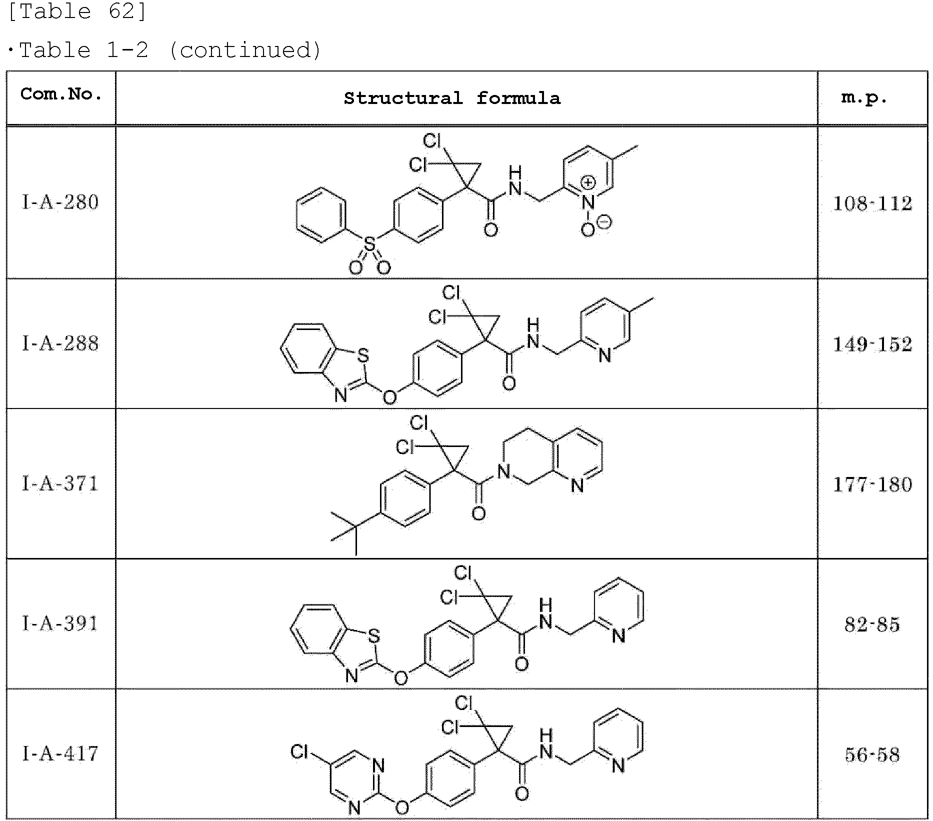

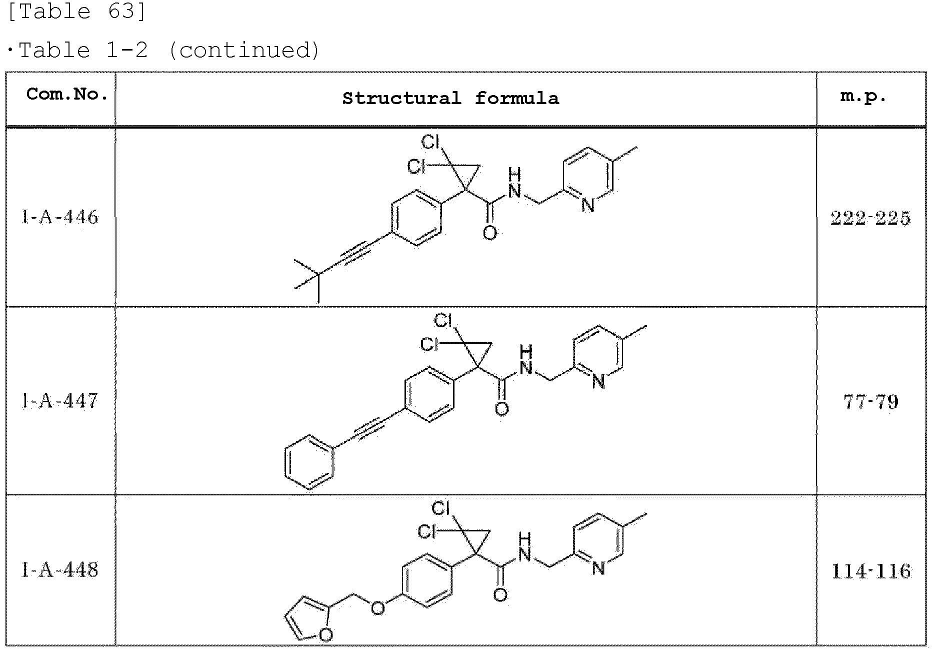

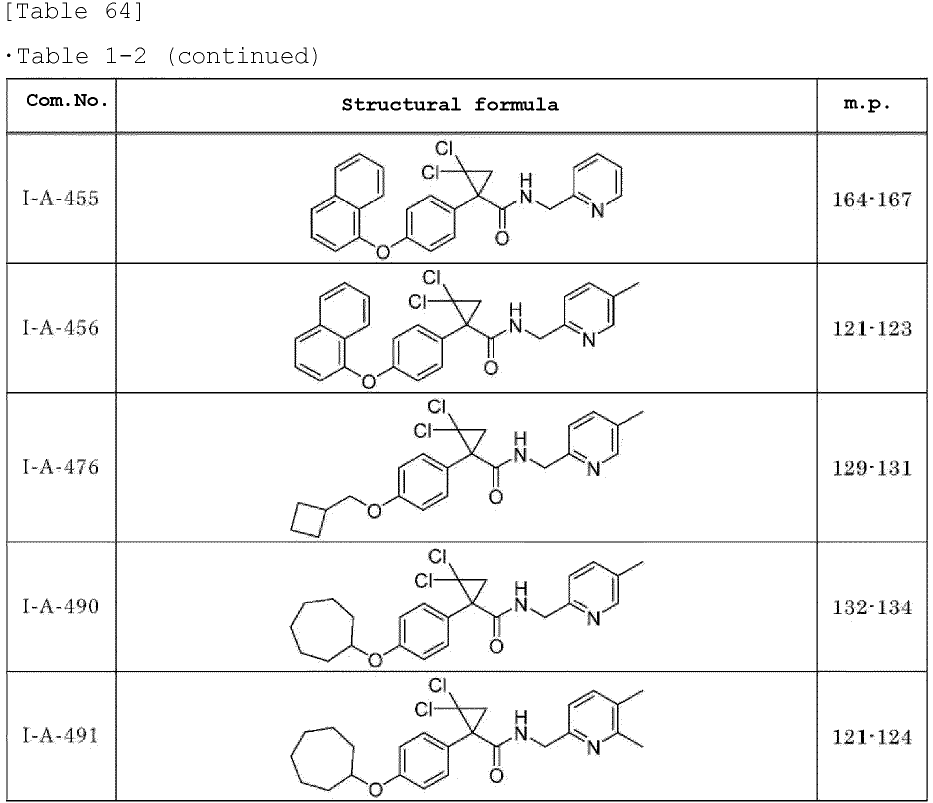

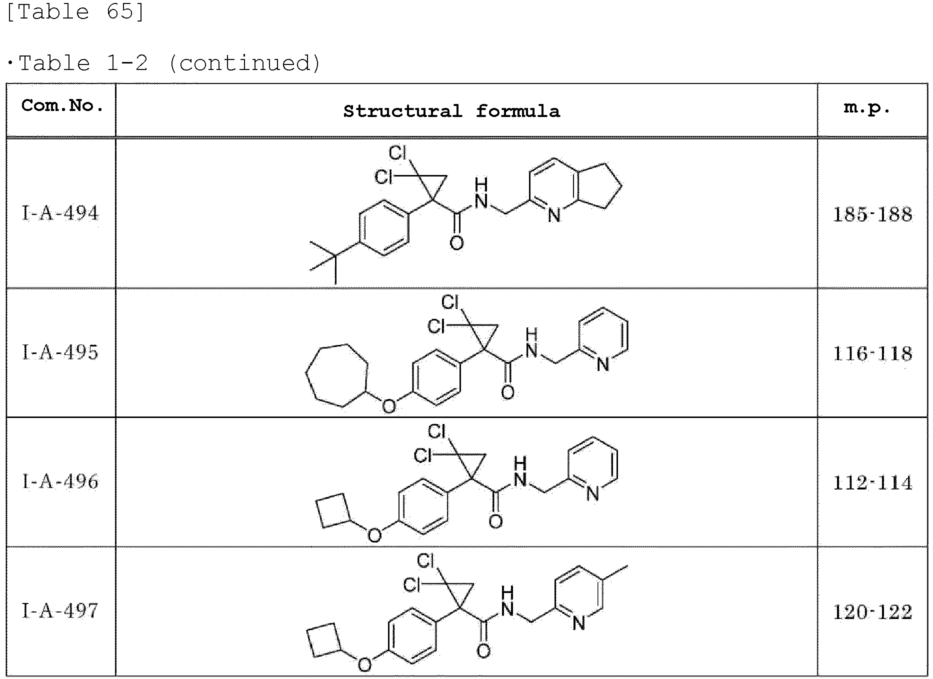

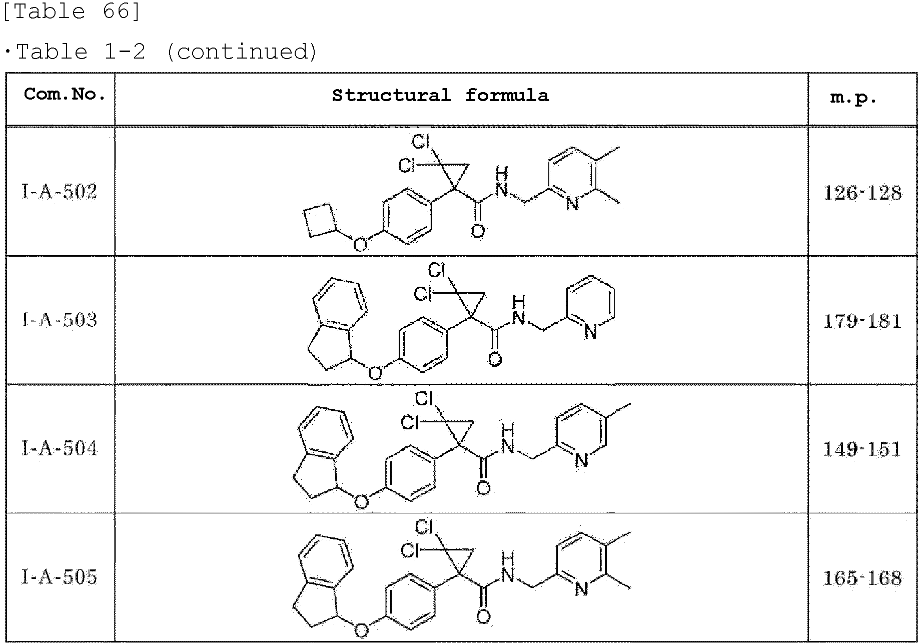

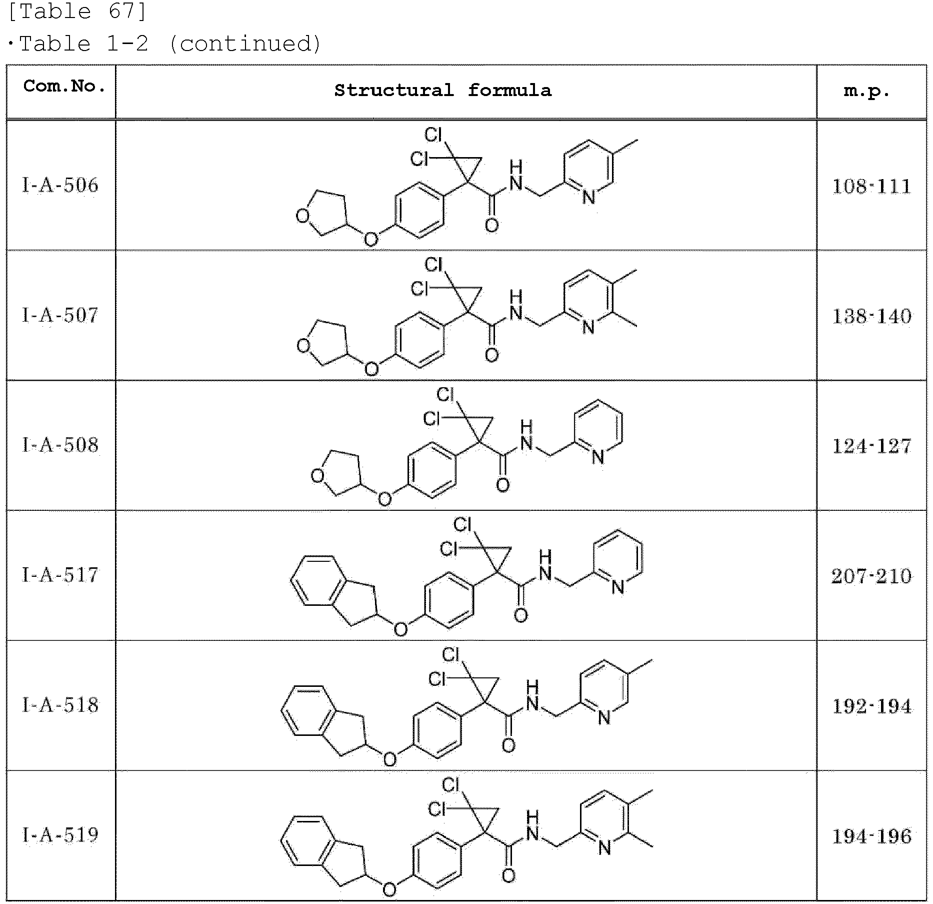

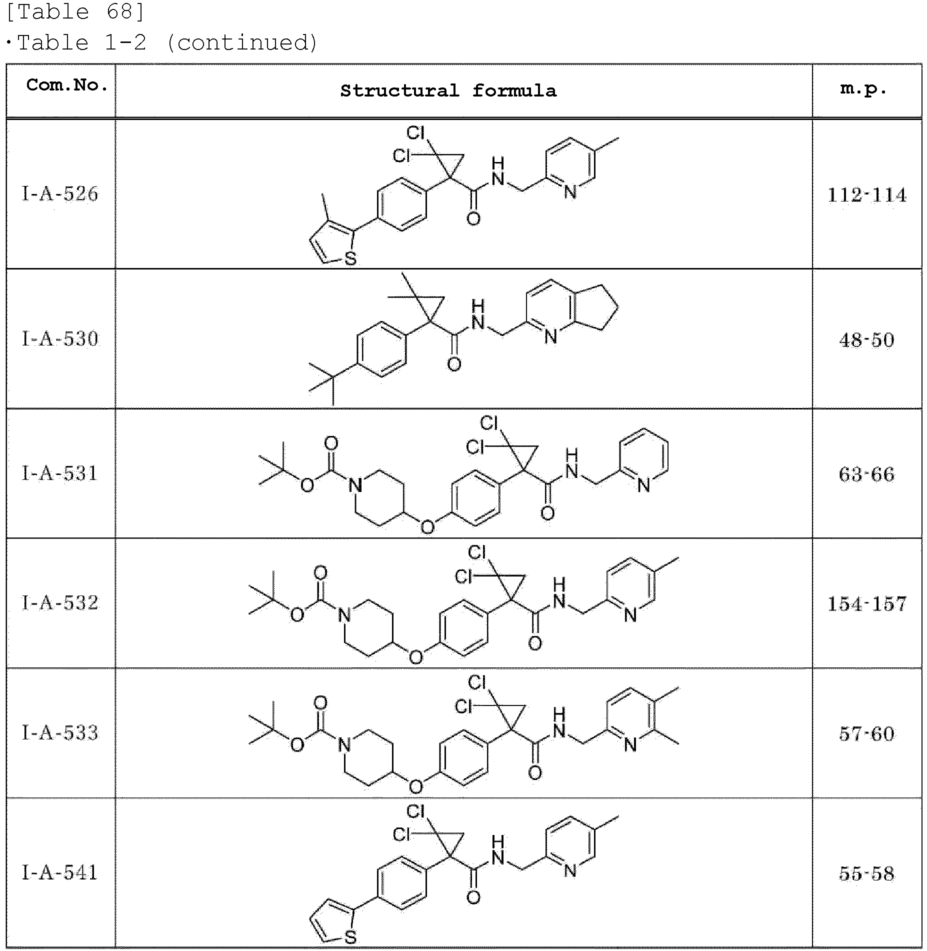

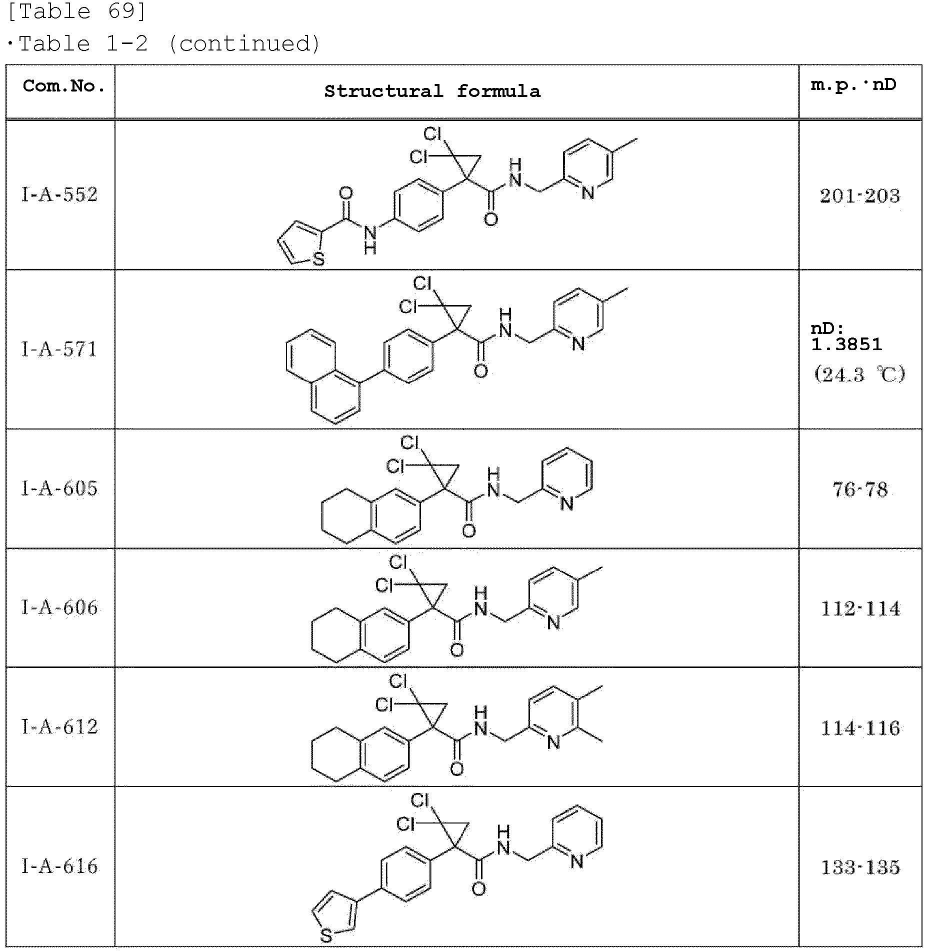

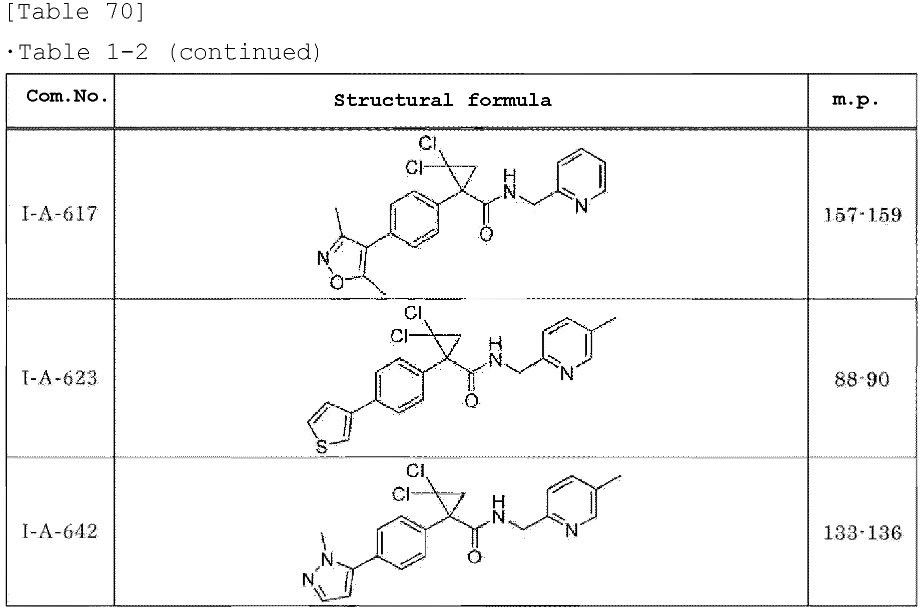

Examples of specific compounds represented by the following general formula [I-B] are shown in the table below.

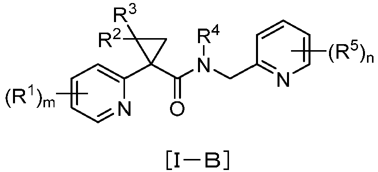

In the above formula [I-B], R² and R³ each represent a chlorine atom.

**[Table 71]**

| ·Table 2-1 | | | | |
|---|---|---|---|---|
| **Com. No.** | (R¹)ₘ | R⁴ | (R⁵)ₙ | **m.p.** |
| I-B-1 | H | H | H | |
| I-B-2 | 5-CF₃ | H | H | 59-62 |
| I-B-3 | 3-CF₃ | H | H | |
| I-B-4 | 4-CF₃ | H | H | |
| I-B-5 | 6-CF₃ | H | H | |
| I-B-6 | 5-F | H | H | |
| I-B-7 | 5-Cl | H | H | |
| I-B-8 | 5-Br | H | H | |
| I-B-9 | 5-I | H | H | |
| I-B-10 | 5-Me | H | H | |
| I-B-11 | 5-Et | H | H | |
| I-B-12 | 5-(*i*-Pr) | H | H | |
| I-B-13 | 5-(*t*-Bu) | H | H | |
| I-B-14 | 5-(*c*-Pr) | H | H | |
| I-B-15 | 5-Ph | H | H | |
| I-B-16 | 5-Br | H | 5-Me | 165-169 |
| I-B-17 | 5-Ph | H | 5-Me | 57-60 |
| I-B-18 | H | H | 5-Me | 54-59 |

Examples of specific compounds represented by the following general formula [I-C] are shown in the tables below.

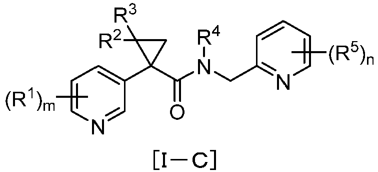

In the above formula [I-C], R² and R³ each represent a chlorine atom.

**[Table 72]**

| ·Table 3-1 | | | | |
|---|---|---|---|---|
| **Com. No.** | (R¹)ₘ | R⁴ | (R⁵)ₙ | **m.p.** |
| I-C-1 | H | H | H | |
| I-C-2 | 6-CF₃ | H | H | 48-50 |
| I-C-3 | 2-CF₃ | H | H | |
| I-C-4 | 6-F | H | H | |
| I-C-5 | 6-Cl | H | H | 78-82 |
| I-C-6 | 6-Br | H | H | |
| I-C-7 | 6-I | H | H | |
| I-C-8 | 6-Me | H | H | |
| I-C-9 | 6-Et | H | H | |
| I-C-10 | 5-(*i*-Pr) | H | H | |
| I-C-11 | 6-(*t*-Bu) | H | H | |
| I-C-12 | 6-(*c*-Pr) | H | H | |

**[Table 73]**

| ·Table 3-1 (continued) | | | | |
|---|---|---|---|---|
| **Com. No.** | (R¹)ₘ | R⁴ | (R⁵)ₙ | **m.p.** |
| I-C-13 | 6-Ph | H | H | 146-149 |
| I-C-14 | 6-Cl | H | 5-Me | 117-119 |
| I-C-15 | 6-Ph | H | 5-Me | 58-62 |
| I-C-16 | 6-Ph | H | 6-Me | 84-90 |
| I-C-17 | 6-(2-F-Ph) | H | 5-Me | 42-46 |
| I-C-18 | 6-C(=CH₂)-CH₃ | H | 5-Me | 77-82 |
| I-C-19 | 6-(2,6-F₂-Ph) | H | 5-Me | 52-55 |
| I-C-20 | 6-(2,6-F₂-Ph) | H | H | 43-46 |
| I-C-21 | 6-(2-F-Ph) | H | H | 46-48 |
| I-C-22 | 6-C(=CH₂)-CH₃ | H | H | 47-50 |
| I-C-23 | 6-(2-F-Ph) | H | 6-Me | 50-52 |
| I-C-24 | 6-(2,6-F₂-Ph) | H | 6-Me | 47-49 |
| I-C-25 | 6-C(=CH₂)-CH₃ | H | 6-Me | 45-47 |
| I-C-26 | 6-(*i*-Pr) | H | 6-Me | 102-105 |
| I-C-27 | 6-(*i*-Pr) | H | 5-Me | 38-40 |
| I-C-28 | 6-(*i-*Pr) | H | H | 130-133 |
| I-C-29 | 6-OPh | H | H | 113-115 |
| I-C-30 | 6-OPh | H | 5-Me | 105-107 |
| I-C-31 | 6-OPh | H | 5,6-Me₂ | 152-154 |

Examples of specific compounds represented by the following general formula [I-D] are shown in the table below.

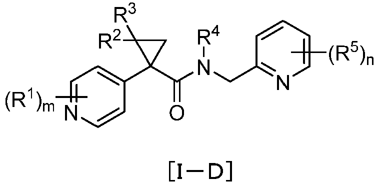

In the above formula [I-D], R² and R³ each represent a chlorine atom.

**[Table 74]**

| ·Table 4-1 | | | | |
|---|---|---|---|---|
| **Com. No.** | (R¹)ₘ | R⁴ | (R⁵)ₙ | **m.p.** |
| I-D-1 | H | H | H | |
| I-D-2 | 2-CF₃ | H | H | |
| I-D-3 | 2-F | H | H | |
| I-D-4 | 2-Cl | H | H | |
| I-D-5 | 2-Br | H | H | |
| I-D-6 | 2-I | H | H | |
| I-D-7 | 2-Me | H | H | |
| I-D-8 | 2-Et | H | H | |
| I-D-9 | 2-(*i*-Pr) | H | H | |
| I-D-10 | 2-(*t*-Bu) | H | H | |
| I-D-11 | 2-(*c*-Pr) | H | H | |
| I-D-12 | 2-Ph | H | H | |

Examples of specific compounds represented by the following general formula [I-E] are shown in the table below.

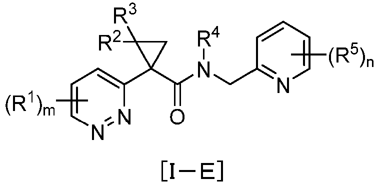

In the above formula [I-E], R² and R³ each represent a chlorine atom.

**[Table 75]**

| ·Table 5-1 | | | | |
|---|---|---|---|---|
| **Com. No.** | (R¹)ₘ | R⁴ | (R⁵)ₙ | **m.p.** |
| I-E-1 | H | H | H | |
| I-E-2 | 6-CF₃ | H | H | |
| I-E-3 | 6-F | H | H | |
| I-E-4 | 6-Cl | H | H | |
| I-E-5 | 6-Br | H | H | |
| I-E-6 | 6-I | H | H | |
| I-E-7 | 6-Me | H | H | |
| I-E-8 | 6-Et | H | H | |
| I-E-9 | 6-(*i*-Pr) | H | H | |
| I-E-10 | 6-(*t*-Bu) | H | H | |
| I-E-11 | 6-(*c*-Pr) | H | H | |
| I-E-12 | 6-Ph | H | H | |

Examples of specific compounds represented by the following general formula [I-F] are shown in the table below.

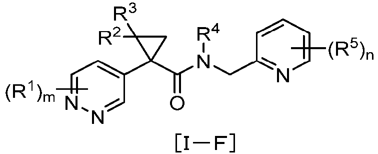

In the above formula [I-F], R² and R³ each represent a chlorine atom.

**[Table 76]**

| ·Table 6-1 | | | | |
|---|---|---|---|---|
| **Com. No.** | (R¹)ₘ | R⁴ | (R⁵)ₙ | **m.p.** |
| I-F-1 | H | H | H | |
| I-F-2 | 6-F | H | H | |
| I-F-3 | 6-Cl | H | H | |
| I-F-4 | 6-Br | H | H | |
| I-F-5 | 6-I | H | H | |
| I-F-6 | 3-Me | H | H | |
| I-F-7 | 5-Me | H | H | |
| I-F-8 | 6-Me | H | H | |

Examples of specific compounds represented by the following general formula [I-G] are shown in the table below.

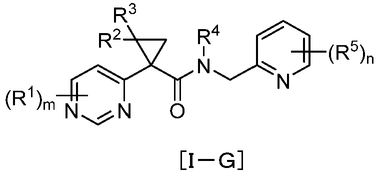

In the above formula [I-G], R² and R³ each represent a chlorine atom.

**[Table 77]**

| ·Table 7-1 | | | | |
|---|---|---|---|---|
| **Com. No.** | (R¹)ₘ | R⁴ | (R⁵)ₙ | **m.p.** |
| I-G-1 | H | H | H | |
| I-G-2 | 2-Me | H | H | |
| I-G-3 | 6-Me | H | H | |
| I-G-4 | 5-Me | H | H | |

Examples of specific compounds represented by the following general formula [I-H] are shown in the table below.

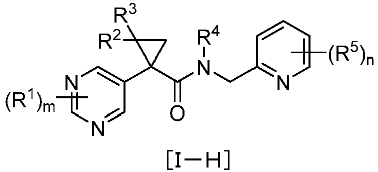

In the above formula [I-H], R² and R³ each represent a chlorine atom.

**[Table 78]**

| ·Table 8-1 | | | | |
|---|---|---|---|---|
| **Com. No.** | (R¹)ₘ | R⁴ | (R⁵)ₙ | **m.p.** |
| I-H-1 | H | H | H | |
| I-H-2 | 2-Me | H | H | |
| I-H-3 | 4-Me | H | H | |
| I-H-4 | 2-Et | H | H | |
| I-H-5 | 2-(*i*- Pr) | H | H | |
| I-H-6 | 2-(*t*-Bu) | H | H | |
| I-H-7 | *2-(c-Pr)* | H | H | |
| I-H-8 | 2-F | H | H | |
| I-H-9 | 2-Cl | H | H | |
| I-H-10 | 2-Br | H | H | |
| I-H-11 | 2-I | H | H | |
| I-H-12 | 2-CF₃ | H | H | |
| I-H-13 | 2-Ph | H | H | |

Examples of specific compounds represented by the following general formula [I-I] are shown in the table below.

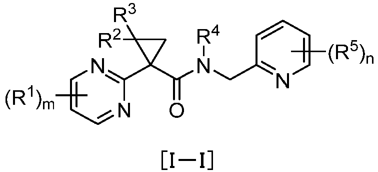

In the above formula [I-I], R² and R³ each represent a chlorine atom.

**[Table 79]**

| ·Table 9-1 | | | | |
|---|---|---|---|---|
| **Com. No.** | (R¹)ₘ | R⁴ | (R⁵)ₙ | **m.p.** |
| I-I-1 | H | H | H | |
| I-I-2 | 5-Me | H | H | |
| I-I-3 | 4-Me | H | H | |
| I-I-4 | 5-F | H | H | |
| I-I-5 | 5-Cl | H | H | |
| I-I-6 | 5-Br | H | H | |
| I-I-7 | 5-I | H | H | |
| I-I-8 | 5-Et | H | H | |
| I-I-9 | 5-(*i*-Pr) | H | H | |
| I-I-10 | 5-(*t*-Bu) | H | H | |
| I-I-11 | 5-(*c*-Pr) | H | H | |
| I-I-12 | 5-Ph | H | H | |

Examples of specific compounds represented by the following general formula [I-J] are shown in the table below.

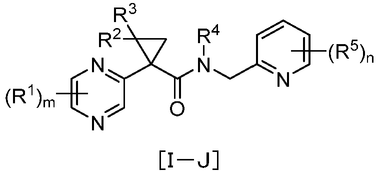

In the above formula [I-J], R² and R³ each represent a chlorine atom.

**[Table 80]**

| ·Table 10-1 | | | | |
|---|---|---|---|---|
| **Com. No.** | (R¹)ₘ | R⁴ | (R⁵)ₙ | **m.p.** |
| I-J-1 | H | H | H | |
| I-J-2 | 3-Me | H | H | |
| I-J-3 | 5-Me | H | H | |
| I-J-4 | 6-Me | H | H | |
| I-J-5 | 5-F | H | H | |
| I-J-6 | 5-Cl | H | H | |
| I-J-7 | 5-Br | H | H | |
| I-J-8 | 5-I | H | H | |
| I-J-9 | 5-Et | H | H | |
| I-J-10 | 5-(*i*-Pr) | H | H | |
| I-J-11 | 5-(*t*-Bu) | H | H | |
| I-J-12 | 5-(*c*-Pr) | H | H | |
| I-J-13 | 5-Ph | H | H | |

Examples of specific compounds represented by the following general formula [I-K] are shown in the tables below.

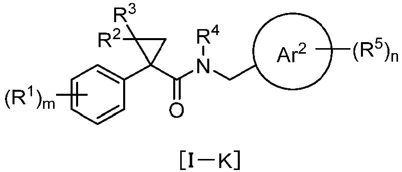

In the above formula [I-K], R² and R³ each represent a chlorine atom.

**[Table 81]**

| ·Table 11-1 | | | | |
|---|---|---|---|---|
| **Com. No.** | (R¹)ₘ | R⁴ | 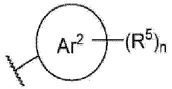 | **m.p.** |
| I-K-1 | 4-CF₃ | H | 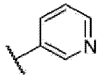 | 140-143 |
| I-K-2 | 4-CF₃ | H | 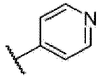 | NMR |
| I-K-3 | 4-CF₃ | H | 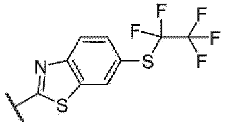 | 165-168 |
| I-K-4 | 4-CF₃ | H | 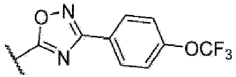 | 172-174 |
| I-K-5 | 4-CF₃ | H | 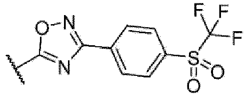 | 190-192 |
| I-K-6 | 4-CF₃ | H | 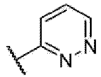 | NMR |

**[Table 82]**

| ·Table 11-1 (continued) | | | | |
|---|---|---|---|---|
| **Com. No.** | (R¹)ₘ | R⁴ | 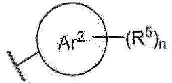 | **m.p.** |
| I-K-7 | 4-CF₃ | H | 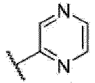 | 135-137 |
| I-K-8 | 4-CF₃ | H | 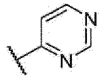 | NMR |
| I-K-9 | 4-CF₃ | H | 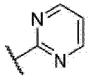 | NMR |
| I-K-10 | 4-CF₃ | H | 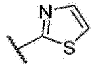 | 145-147 |
| I-K-11 | 4-CF₃ | H | 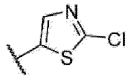 | 144-146 |
| I-K-12 | 4-CF₃ | H | 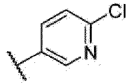 | 158-161 |
| I-K-13 | 4-CF₃ | H | 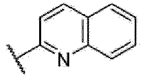 | NMR |
| I-K-14 | 4-(*t*-Bu) | H | 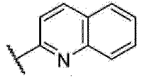 | 169-172 |
| I-K-15 | 4-1 | H | 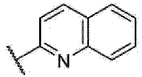 | 49-51 |
| I-K-16 | 4-CN | H | 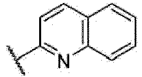 | 64-66 |

**[Table 83]**

| ·Table 11-1 (continued) | | | | |
|---|---|---|---|---|
| **Com. No.** | (R¹)ₘ | R⁴ | 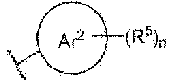 | **m.p.** |
| I-K-17 | 4-(*t*-Bu) | H | 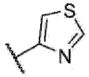 | 128-130 |
| I-K-18 | 4-(*t*-Bu) | H | 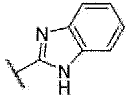 | 125-128 |
| I-K-19 | 4-(t-Bu) | H | 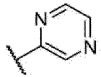 | 148-151 |
| I-K-20 | 4-(*t*-Bu) | H | 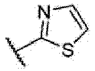 | 132-137 |
| I-K-21 | 4-(*t*-Bu) | H | 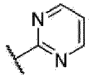 | 162-165 |
| I-K-22 | 4-(*t*-Bu) | H | 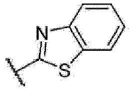 | 152-155 |
| I-K-23 | 4-(*t*-Bu) | H | 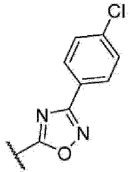 | 58-62 |
| I-K-24 | 4-(*t*-Bu) | H | 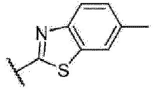 | 101-105 |
| I-K-25 | 4-(*t*-Bu) | H | 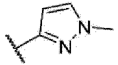 | 120-122 |
| 1-K-26 | 4-(*t*-Bu) | H | 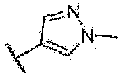 | 181-183 |

**[Table 84]**

| ·Table 11-1 (continued) | | | | |
|---|---|---|---|---|
| **Com. No.** | (R¹)ₘ | R⁴ | 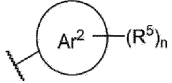 | **m.p.·nD** |
| I-K-27 | 4-Br | H | 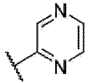 | **nD: 1.3898** (25.5 °C) |
| I-K-28 | 4-Br | H | 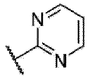 | 122-124 |

Examples of specific compounds represented by the following general formula [I-L] are shown in the table below.

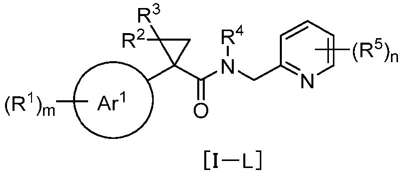

In the above formula [I-L], R² and R³ each represent a chlorine atom.

**[Table 85]**

| ·Table 12-1 | | | | |
|---|---|---|---|---|
| **Com. No.** | 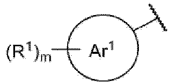 | R⁴ | (R⁵)ₙ | **m.p.** |
| I-L-1 | 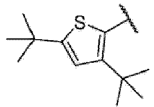 | H | 5-Me | 118-120 |
| I-L-2 | 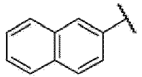 | H | H | 49-53 |
| I-L-3 | 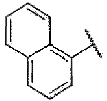 | H | H | 41-43 |

**[Table 86]**

| ·Appended Table 1 | |
|---|---|
| **Com. No.** | **¹H-NMR data (CDCl₃)** |
| I-A-138 | 10.06 - 10.05 (1H, m), 8.46 (1H, d), 7.97 (2H, d), 7.74 (2H, d), 7.65 - 7.60 (1H, m), 7. 37(1H, br.s), 7.21 - 7.15 (2H, m), 4.62 - 4.52 (1H, m), 4.50 - 4. 42 (1H, m), 2.83 (1H, d, J=7.3 Hz), 2.05 (1H, d) |
| I-A-198 | 8.31 - 8.28 (1H, m), 7.70 - 7.68 (4H, m), 7.40 - 7.36 (1H, m), 7.02 - 6.99 (2H, m), 4.54 - 4.37 (2H, m), 2.80 (1H, d), 2.31 (3H, s), 2.02 (1H, d) |
| I-A-200 | 7.85 - 7.82 (1H, m), 7.69 (4H, q), 7.59 - 7.54 (1H, m), 7.07 - 7.04 (2H, m), 4.46 (2H, ddd), 2.86 - 2.83 (1H, m), 2.45 - 2.44 (3H, m), 2.00 (1H, d) |
| I-A-206 | 7.76 - 7.66 (4H, m), 7.56 - 7.51 (1H, m), 7.35 - 7. 31 (1H, m), 6.91 - 6.88 (1H, m), 4.42 (2H, ddd), 2.89 (1H, d), 2.36 - 2.35 (3H, m), 2.23 (3H, s), 2.01 (1H, d) |
| 1-A-222 | 8.32 (1H, d), 7. 73 - 7.64 (4H, m), 7.39 - 7.34 (1H, m), 7.23 - 7.19 (1H, m), 7.10 - 7.03 (1H, m), 4.56 (1H, dd), 4.44 (1H, dd), 2.82 (1H, d), 2.02 (1H, d) |
| I-A-276 | 8.28 (s, 1H), 7.40 - 7.50 (m, 3H), 7.27 (s, 2H), 7.20 (br. s, 1H), 7.10 (d, 1H), 6.28 (s, 1H), 4.55 (dd, 1H), 4.40 (dd, 1H), 2.80 (d, 1H), 2.30 (s, 3H), 1.95 (d, 2H), 1.92 (s, 3H), 1.90 (s, 3H) |
| I-A-303 | 8.50 (br. s, 1H), 7.47 (d, 2H), 7.31 (d, 1H), 7.30 (d, 2H), 6.90 (d, 1H), 6.26 (s, 1H), 4.49 (dd, 1H), 4.37 (dd, 1H), 2.85 (d, 1H), 2.40 (s, 3H), 2.23 (s, 3H), 1.95 (d, 1H), 1.92 (s, 3H), 1. 90 (s, 3H) |
| 1-A-390 | 8. 28 - 8.27 (1H, m), 7.65 - 7.63 (2H, m), 7.57 - 7.55 (2H, m), 7.44-7.42 (1H, m), 7.08 - 7.06 (1H, d, J = 8 Hz), 7.01 - 6.99 (1H, m), 6.86 (1H, t, J = 56 Hz), 4.54 - 4.38 (2H, m), 2.80 - 2.78 (1H, d, J = 8 Hz), 2.30 (3H, s), 2.00 - 1.98 (1H, d, J = 8 Hz) |
| I-A-509 | 8.27(s, 1H), 7.40(d, 1H), 7.26-7. 24 (m, 2H), 7.04 (d, 1H), 6.84 (d, 2H), 6.67 (br.s, 1H), 4. 74(quint, 1H), 4.42 (dd, 2H), 2.28 (s, 3H), 1.95-1.76(m, 6H), 1.74(d, 1H), 1.67-1.57 (m, 2H), 1.32(s, 3H), 1.29-1.24 (m, 1H), 0.96(d, 1H), 0.91 (s, 3H) |
| I-A-511 | 7.30-7. 26 (m, 3H), 6.97 (br.s, 1H), 6.88-6.85 (m, 3H), 4.75(quint, 1H), 4.38 (dd, 2H), 2.38 (s, 3H), 2.21 (s, 3H), 1.96-1.79 (m, 6H), 1.78 (d, 1H), 1.67-1.60 (m, 2H), 1.36 (s, 3H), 0.96 (d, 1H), 0.92 (s, 3H) |
| I-A-641 | 8.29(1H, s), 7.86(1H, dd), 7.52(2H, d), 7.48-7.44(2 H, m), 7.29(1H, t), 7.23-7.19(1H, m), 7.10(1H, d), 7.07(2H, d), 6.96(1H, d), 4.49(2H, dd), 2.78(1H, d), 2.63(3H, s), 2.32(3H, s), 1.98(1H, d) |

**[Table 87]**

| ·Appended Table 1 (continued) | |
|---|---|
| **Com. No.** | **¹H-NMR data (CDCl₃)** |
| I-K-2 | 8.52 (2H, dd), 7.69 (4H, dd), 7.08 (2H, d), 6.19 (1H, s), 4.44 (2H, ddd), 2.86 (1H, d), 2.02 (1H, d) |
| I-K-6 | 7.69 (4H, q), 7.51 (1H, t), 7.08 (1H, s), 6.74 (1H, d), 6.60 (1H, d), 4.47 (2H, d), 2.91 (1H, d), 2.00 (1H, d) |
| I-K-8 | 9.18 (1H, d), 7.80-7.67 (6H, m), 7.44 (1H, s), 4.78 (2H, ddd), 2. 79 (1H, d), 2.07 (1H, d) |
| I-K-9 | 8.55 (1H, d), 8.47 (2H, dd), 7.69 (4H, q), 6.94 (1H, s), 4.59 (2H, ddd), 2.83 (1H, d), 2.05 (1H, d) |
| I-K-13 | 8.10 (1H, d), 7.88 (1H, s), 7.84-7.69 (7H, m), 7.54 (1H, t), 7.24 (2H, d), 4.74-4.62 (2H, m), 2.93 (1H, d), 2.06 (1H, d) |

The agricultural and horticultural fungicide comprising the amide compound represented by the general formula [I] of the present invention or salts thereof as an active ingredient is suitable for controlling diseases which may infest cereals, fruit trees, vegetables, other crops, and ornamental flowering plants.

The target diseases include filamentous fungal diseases, bacterial diseases, and viral diseases.

Examples of the filamentous fungal diseases include diseases caused by fungi-imperfecti including the genera Botrytis, Helminthosporium, Fusarium, Septoria, Cercospora, Pseudocercosporella, Rhynchosporium, Pyricularia and Alternaria; diseases caused by basidiomycetes including the genera Hemilelia, Rhizoctonia, Ustilago, Typhula and Puccinia; diseases caused by ascomycota including the genera Venturia, Podosphaera, Leptosphaeria, Blumeria, Erysiphe, Microdochium, Sclerotinia, Gaeumannomyces, Monilinia and Unsinula; and diseases caused by other fungi including the genera Ascochyta, Phoma, Pythium, Corticium and Pyrenophora.

Examples of the bacterial diseases include diseases caused by the genera Pseudomonas, Xanthomonas, and Erwinia.

Examples of the viral diseases include diseases caused by Tabacco mosaic virus, or the like.

Specific examples of the filamentous fungal diseases include rice blast (Pyricularia oryzae), rice sheath blight (Rhizoctonia solani), rice brown spot (Cochiobolus miyabeanus), rice seedling blight (Rhizopus chinensis, Pythium graminicola, Fusarium graminicola, Fusarium roseum, Mucor sp., Phoma sp., Tricoderma sp.), rice bakanae disease (Gibberella fujikuroi), powdery mildew of barley, wheat, etc. (Blumeria graminis), powdery mildew of cucumbers etc. (Sphaerotheca fuliginea), powdery mildew of eggplants etc. (Erysiphe cichoracoarum), powdery mildew of other host plants, eyespot of barley, wheat, etc. (Pseudocercosporella herpotrichoides), smut of wheat etc. (Urocystis tritici), snow mold of barley, wheat, etc. (Microdochium nivalis, Pythium iwayamai, Typhla ishikariensis, Typhla incarnata, Sclerotinia borealis), fusarium ear blight of barley, wheat etc. (Fusarium graminearum, Fusarium avenaceum, Fusarium culmorum, Microdochium nivalis), rust of barley, wheat, etc. (Puccinia recondita, Puccinia striiformis, Puccinia graminis), take-all of barley, wheat, etc. (Gaeumannomyces graminis), oat crown rust (Puccinia coronata), rust of other plants, gray mold of cucumbers, strawberries, etc. (Botrytis cinerea), sclerotinia rot of cucumbers, tomatoes, etc. (Sclerotinia sclerotiorum), late blight of potatoes, tomatoes, etc. (Phytophthora infestans), late blight of other plants, downy mildew of various plants such as cucumber downy mildew (Pseudoperonospora cubensis) and grape downy mildew (Plasmopara viticola), apple scab (Venturia inaequalis), apple alternaria blotch (Alternaria mali), apple ring rot (Botryosphaeria berengeriana), grape ripe rot (Colletotrichum gloeosporioides, Colletotrichum acutatum), grape anthracnose (Elsinoe ampelina), pear black spot (Alternaria kikuchiana), citrus melanose (Diaporthe citri), citrus scab (Elsinoe fawcetti), cucumber anthracnose (Colletotrichum lagenarium), sugarbeet leaf spot (Cercospora beticola), peanut brown leaf spot (Cercospora arachidicola), peanut late leaf spot (Cercospora personata), wheat leaf blotch (Septoria tritici), wheat glume blotch (Leptosphaeria nodorum), barley net blotch (Pyrenophora teres), barley stripe (Pyrenophora graminea), barley scald (Rhynchosporium secalis), wheat loose smut (Ustilago nuda), wheat stinking smut (Tilletia caries), brown patch of turfgrass (Rhizoctonia solani), dollar spot of turfgrass (Sclerotinia homoeocarpa) Chinese cabbage alternaria leaf spot (Alternaria brassicola), tomato late blight (Phytophthora infestans), cucumber gray mold (Botrytis cinerea), barley powdery mildew (Blumeria graminis f.sp. Hordei), soybean sudden death syndrome (Fusarium virguliforme), and Brassicaceae sclerotinia rot (Sclerotinia sclerotirum).

Specific examples of the bacterial diseases include diseases caused by Pseudomonas spp. such as cucumber bacterial spot (Pseudomonas syringae pv. Lachrymans), tomato bacterial wilt disease (Pseudomonas syringae pv. Lachrymans) and bacterial grain rot of rice (Pseudomonas glumae); diseases caused by Xanthomonas spp. such as cabbage black rot (Xanthomonas campestris), rice bacterial leaf blight (Xanthomonas oryzae) and citrus canker (Xanthomonas citri); and diseases caused by Erwinia spp. such as cabbage soft rot (Erwinia carotovora).

Specific examples of the viral diseases include tobacco mosaic disease (tobacco mosaic virus).

In particular, the agricultural and horticultural fungicide comprising the amide compound represented by the general formula [I] of the present invention or salts thereof as an active ingredient is highly effective against filamentous fungal diseases, particularly, Chinese cabbage alternaria leaf spot (Alternaria brassicola), tomato late blight (Phytophthora infestans), cucumber gray mold (Botrytis cinerea), cucumber anthracnose (Colletotrichum lagenarium), rice blast (Magnaporthe oryzae), apple scab (Venturia inaequalis), barley powdery mildew (Blumeria graminis f.sp. Hordei), wheat leaf blotch (Septoria tritici), cucumber sclerotinia rot (Sclerotinia sclerotiorum), apple ring rot (Botryosphaeria berengeriana), grape ripe rot (Colletotrichum gloeosporioides, Colletotrichum acutatum), soybean sudden death syndrome (Fusarium virguliforme), fusarium ear blight of barley, wheat etc. (Fusarium graminearum), Brassicaceae sclerotinia rot (Sclerotinia sclerotirum), and rice brown spot (Cochliobolus miyabeanus).

The agricultural and horticultural fungicide comprising the amide compound represented by the general formula [I] of the present invention or salts thereof as an active ingredient can be used as an insecticide as well as a fungicide. The agricultural and horticultural fungicide can be used alone, or in combination with or as a mixture with other agrochemicals to control pests which may damage paddy rice, fruit trees, vegetables, other crops, and ornamental flowering plants, including agricultural and forest pests, horticultural pests, stored grain pests, sanitary pests, nematodes, etc. Specific examples of the pests, nematodes, etc. include the following: the species of the order Lepidoptera such as Parasa consocia, Anomis mesogona, Papilio xuthus, Matsumuraeses azukivora, Ostrinia scapulalis, Spodoptera exempta, Hyphantria cunea, Ostrinia furnacalis, Pseudaletia separata, Tinea translucens, Bactra furfurana, Parnara guttata, Marasmia exigua, Parnara guttata, Sesamia inferens, Brachmia triannulella, Monema flavescens, Trichoplusia ni, Pleuroptya ruralis, Cystidia couaggaria, Lampides boeticus, Cephonodes hylas, Helicoverpa armigera, Phalerodonta manleyi, Eumeta japonica, Malacosoma neustria testacea, Stathmopoda masinissa, Cuphodes diospyrosella, Archips xylosteanus, Agrotis segetum, Tetramoera schistaceana, Papilio machaon hippocrates, Endoclyta sinensis, Lyonetia prunifoliella, Phyllonorycter ringoneella, Cydia kurokoi, Eucoenogenes aestuosa, Lobesia botrana, Latoia sinica, Euzophera batangensis, Phalonidia mesotypa, Spilosoma imparilis, Glyphodes pyloalis, Olethreutes mori, Tineola bisselliella, Endoclyta excrescens, Nemapogon granellus, Synanthedon hector, Cydia pomonella, Plutella xylostella, Cnaphalocrocis medinalis, Sesamia calamistis, Scirpophaga incertulas, Pediasia teterrellus, Phthorimaea operculella, Stauropus fagi persimilis, Etiella zinckenella, Spodoptera exigua, Palpifer sexnotata, Spodoptera mauritia, Scirpophaga innotata, Xestia c-nigrum, Spodoptera depravata, Ephestia kuehniella, Angerona prunaria, Clostera anastomosis, Pseudoplusia includens, Matsumuraeses falcana, Helicoverpa assulta, Autographa nigrisigna, Agrotis ipsilon, Euproctis pseudoconspersa, Adoxophyes orana, Caloptilia theivora, Homona magnanima, Ephestia elutella, Eumeta minuscula, Clostera anachoreta, Heliothis maritima, Sparganothis pilleriana, Busseola fusca, Euproctis subflava, Biston robustum, Heliothis zea, Aedia leucomelas, Narosoideus flavidorsalis, Viminia rumicis, Bucculatrix pyrivorella, Grapholita molesta, Spulerina astaurota, Ectomyelois pyrivorella, Chilo suppressalis, Acrolepiopsis sapporensis, Plodia interpunctella, Hellula undalis, Sitotroga cerealella, Spodoptera litura, Eucosma aporema, Acleris comariana, Scopelodes contractus, Orgyia thyellina, Spodoptera frugiperda, Ostrinia zaguliaevi, Naranga aenescens, Andraca bipunctata, Paranthrene regalis, Acosmeryx castanea, Phyllocnistis toparcha, Endopiza viteana, Eupoecillia ambiguella, Anticarsia gemmatalis, Cnephasia cinereipalpana, Lymantria dispar, Dendrolimus spectabilis, Leguminivora glycinivorella, Maruca testulalis, Matsumuraeses phaseoli, Caloptilia soyella, Phyllocnistis citrella, Omiodes indicata, Archips fuscocupreanus, Acanthoplusia agnata, Bambalina sp., Carposina niponensis, Conogethes punctiferalis, Synanthedon sp., Lyonetia clerkella, Papilio helenus, Colias erate poliographus, Phalera flavescens, the species of the family Pieridae such as Pieris brassicae and Pieris rapae crucivora, Euproctis similis, Acrolepiopsis suzukiella, Ostrinia nubilalis, Mamestra brassicae, Ascotis selenaria, Phtheochroides clandestina, Hoshinoa adumbratana, Odonestis pruni japonensis, Triaena intermedia, Adoxophyes orana fasciata, Grapholita inopinata, Spilonota ocellana, Spilonota lechriaspis, Illiberis pruni, Argyresthia conjugella, Caloptilia zachrysa, Archips breviplicanus, Anomis flava, Pectinophora gossypiella, Notarcha derogata, Diaphania indica, Heliothis virescens and Earias cupreoviridis;
the species of the order Hemiptera such as Nezara antennata, Stenotus rubrovittatus, Graphosoma rubrolineatum, Trigonotylus coelestialium, Aeschynteles maculatus, Creontiades pallidifer, Dysdercus cingulatus, Chrysomphalus ficus, Aonidiella aurantii, Graptopsaltria nigrofuscata, Blissus leucopterus, Icerya purchasi, Piezodorus hybneri, Lagynotomus elongatus, Thaia subrufa, Scotinophara lurida, Sitobion ibarae, Stariodes iwasakii, Aspidiotus destructor, Taylorilygus pallidulus, Myzus mumecola, Pseudaulacaspis prunicola, Acyrthosiphon pisum, Anacanthocoris striicornis, Ectometopterus micantulus, Eysarcoris lewisi, Molipteryx fuliginosa, Cicadella viridis, Rhopalosophum rufiabdominalis, Saissetia oleae, Trialeurodes vaporariorum, Aguriahana quercus, Lygus spp., Euceraphis punctipennis, Andaspis kashicola, Coccus pseudomagnoliarum, Cavelerius saccharivorus, Galeatus spinifrons, Macrosiphoniella sanborni, Aonidiella citrina, Halyomorpha mista, Stephanitis fasciicarina, Trioza camphorae, Leptocorisa chinensis, Trioza quercicola, Uhlerites latius, Erythroneura comes, Paromius exiguus, Duplaspidiotus claviger, Nephotettix nigropictus, Halticiellus insularis, Perkinsiella saccharicida, Psylla malivorella, Anomomeura mori, Pseudococcus longispinis, Pseudaulacaspis pentagona, Pulvinaria kuwacola, Apolygus lucorum, Togo hemipterus, Toxoptera aurantii, Saccharicoccus sacchari, Geoica lucifuga, Numata muiri, Comstockaspis perniciosa, Unaspis citri, Aulacorthum solani, Eysarcoris ventralis, Bemisia argentifolii, Cicadella spectra, Aspidiotus hederae, Liorhyssus hyalinus, Calophya nigridorsalis, Sogatella furcifera, Megoura crassicauda, Brevicoryne brassicae, Aphis glycines, Leptocorisa oratorius, Nephotettix virescens, Uroeucon formosanum, Cyrtopeltis tennuis, Bemisia tabaci, Lecanium persicae, Parlatoria theae, Pseudaonidia paeoniae, Empoasca onukii, Plautia stali, Dysaphis tulipae, Macrosiphum euphorbiae, Stephanitis pyrioides, Ceroplastes ceriferus, Parlatoria camelliae, Apolygus spinolai, Nephotettix cincticeps, Glaucias subpunctatus, Orthotylus flavosparsus, Rhopalosiphum maidis, Peregrinus maidis, Eysarcoris parvus, Cimex lectularius, Psylla abieti, Nilaparvata lugens, Psylla tobirae, Eurydema rugosum, Schizaphis piricola, Psylla pyricola, Parlatoreopsis pyri, Stephanitis nashi, Dysmicoccus wistariae, Lepholeucaspis japonica, Sappaphis piri, Lipaphis erysimi, Neotoxoptera formosana, Rhopalosophum nymphaeae, Edwardsiana rosae, Pinnaspis aspidistrae, Psylla alni, Speusotettix subfusculus, Alnetoidia alneti, Sogatella panicicola, Adelphocoris lineolatus, Dysdercus poecilus, Parlatoria ziziphi, Uhlerites debile, Laodelphax striatella, Eurydema pulchrum, Cletus trigonus, Clovia punctata, Empoasca sp., Coccus hesperidum, Pachybrachius luridus, Planococcus kraunhiae, Stenotus binotatus, Arboridia apicalis, Macrosteles fascifrons, Dolycoris baccarum, Adelphocoris triannulatus, Viteus vitifolii, Acanthocoris sordidus, Leptocorisa acuta, Macropes obnubilus, Cletus punctiger, Riptortus clavatus, Paratrioza cockerelli, Aphrophora costalis, Lygus disponsi, Lygus saundersi, Crisicoccus pini, Empoasca abietis, Crisicoccus matsumotoi, Aphis craccivora, Megacopta punctatissimum, Eysarcoris guttiger, Lepidosaphes beckii, Diaphorina citri, Toxoptera citricidus, Planococcus citri, Dialeurodes citri, Aleurocanthus spiniferus, Pseudococcus citriculus, Zyginella citri, Pulvinaria citricola, Coccus discrepans, Pseudaonidia duplex, Pulvinaria aurantii, Lecanium corni, Nezara viridula, Stenodema calcaratum, Rhopalosiphum padi, Sitobion akebiae, Schizaphis graminum, Sorhoanus tritici, Brachycaudus helichrysi, Carpocoris purpureipennis, Myzus persicae, Hyalopterus pruni, Aphis farinose yanagicola, Metasalis populi, Unaspis yanonensis, Mesohomotoma camphorae, Aphis spiraecola, Aphis pomi, Lepidosaphes ulmi, Psylla mali, Heterocordylus flavipes, Myzus malisuctus, Aphidonuguis mali, Orientus ishidai, Ovatus malicolens, Eriosoma lanigerum, Ceroplastes rubens and Aphis gossypii;
the species of the order Coleoptera such as Xystrocera globosa, Paederus fuscipes, Eucetonia roelofsi, Callosobruchus chinensis, Cylas formicarius, Hypera postica, Echinocnemus squameus, Oulema oryzae, Donacia provosti, Lissorhoptrus oryzophilus, Colasposoma dauricum, Euscepes postfasciatus, Epilachna varivestis, Acanthoscelides obtectus, Diabrotica virgifera virgifera, Involvulus cupreus, Aulacophora femoralis, Bruchus pisorum, Epilachna vigintioctomaculata, Carpophilus dimidiatus, Cassida nebulosa, Luperomorpha tunebrosa, Phyllotreta striolata, Psacothea hilaris, Aeolesthes chrysothrix, Curculio sikkimensis, Carpophilus hemipterus, Oxycetonia jucunda, Diabrotica spp., Mimela splendens, Sitophilus zeamais, Tribolium castaneum, Sitophilus oryzae, Palorus subdepressus, Melolontha japonica, Anoplophora malasiaca, Neatus picipes, Leptinotarsa decemlineata, Diabrotica undecimpunctata howardi, Sphenophorus venatus, Crioceris quatuordecimpunctata, Conotrachelus nenuphar, Ceuthorhynchidius albosuturalis, Phaedon brassicae, Lasioderma serricorne, Sitona japonicus, Adoretus tenuimaculatus, Tenebrio molitor, Basilepta balyi, Hypera nigrirostris, Chaetocnema concinna, Anomala cuprea, Heptophylla picea, Epilachna vigintioctopunctata, Diabrotica longicornis, Eucetonia pilifera, Agriotes spp., Attagenus unicolor japonicus, Pagria signata, Anomala rufocuprea, Palorus ratzeburgii, Alphitobius laevigatus, Anthrenus verbasci, Lyctus brunneus, Tribolium confusum, Medythia nigrobilineata, Xylotrechus pyrrhoderus, Epitrix cucumeris, Tomicus piniperda, Monochamus alternatus, Popillia japonica, Epicauta gorhami, Sitophilus zeamais, Rhynchites heros, Listroderes costirostris, Callosobruchus maculatus, Phyllobius armatus, Anthonomus pomorum, Linaeidea aenea and Anthonomus grandis;
the species of the order Diptera such as Culex pipiens pallens, Pegomya hyoscyami, Liriomyza huidobrensis, Musca domestica, Chlorops oryzae, Hydrellia sasakii, Agromyza oryzae, Hydrellia griseola, Hydrellia griseola, Ophiomyia phaseoli, Dacus cucurbitae, Drosophila suzukii, Rhacochlaena japonica, Muscina stabulans, the species of the family Phoridae such as Megaselia spiracularis, Clogmia albipunctata, Tipula aino, Phormia regina, Culex tritaeniorhynchus, Anopheles sinensis, Hylemya brassicae, Asphondylia sp., Delia platura, Delia antiqua, Rhagoletis cerasi, Culex pipiens molestus Forskal, Ceratitis capitata, Bradysia agrestis, Pegomya cunicularia, Liriomyza sativae, Liriomyza bryoniae, Chromatomyia horticola, Liriomyza chinensis, Culex quinquefasciatus, Aedes aegypti, Aedes albopictus, Liriomyza trifolii, Liriomyza sativae, Dacus dorsalis, Dacus tsuneonis, Sitodiplosis mosellana, Meromuza nigriventris, Anastrepha ludens and Rhagoletis pomonella;
the species of the order Hymenoptera such as Pristomyrmex pungens, the species of the family Bethylidae, Monomorium pharaonis, Pheidole noda, Athalia rosae, Dryocosmus kuriphilus, Formica fusca japonica, Vespinae spp. (wasps), Athalia infumata infumata, Arge pagana, Athalia japonica, Acromyrmex spp., Solenopsis spp., Arge mali and Ochetellus glaber;
the species of the order Orthoptera such as Homorocoryphus lineosus, Gryllotalpa sp., Oxya hyla intricata, Oxya yezoensis, Locusta migratoria, Oxya japonica, Homorocoryphus jezoensis and Teleogryllus emma;
the species of the order Thysanoptera such as Selenothrips rubrocinctus, Stenchaetothrips biformis, Haplothrips aculeatus, Ponticulothrips diospyrosi, Thrips flavus, Anaphothrips obscurus, Liothrips floridensis, Thrips simplex, Thrips nigropilosus, Heliothrips haemorrhoidalis, Pseudodendrothrips mori, Microcephalothrips abdominalis, Leeuwenia pasanii, Litotetothrips pasaniae, Scirtothrips citri, Haplothrips chinensis, Mycterothrips glycines, Thrips setosus, Scirtothrips dorsalis, Dendrothrips minowai, Haplothrips niger, Thrips tabaci, Thrips alliorum, Thrips hawaiiensis, Haplothrips kurdjumovi, Chirothrips manicatus, Frankliniella intonsa, Thrips coloratus, Franklinella occidentalis, Thrips palmi, Frankliniella lilivora and Liothrips vaneeckei;
the species of the order Acari such as Tetranychus truncatus, Tetranychus viennensis, Tetranychus kanzawai, Acaphylla theavagrans, Polyphagotarsonemus latus, Aculops lycopersici, Tetranychus urticae, Eriophyes chibaensis, Brevipalpus spp., Tyrophagus similis, Panonychus citri, Brevipalpus phoenicis, Phyllocoptruta citri, Aculus schlechtendali, Panonychus ulmi, Rhyzoglyphus robini and Sancassania spp.;
the species of the order Isoptera such as Reticulitermes miyatakei, Incisitermes minor, Coptotermes formosanus, Hodotermopsis japonica, Reticulitermes sp., Reticulitermes flaviceps amamianus, Glyptotermes kushimensis, Coptotermes guangzhoensis, Neotermes koshunensis, Glyptotermes kodamai, Glyptotermes satsumensis, Cryptotermes domesticus, Odontotermes formosanus, Glyptotermes nakajimai, Pericapritermes nitobei and Reticulitermes speratus;
the species of the order Blattodea such as Periplaneta fuliginosa, Blattella germanica, Blatta orientalis, Periplaneta brunnea, Blattella lituricollis, Periplaneta japonica and Periplaneta americana;
the species of the phylum Nematoda such as Nothotylenchus acris, Aphelenchoides besseyi, Pratylenchus penetrans, Meloidogyne hapla, Meloidogyne incognita, Globodera rostochiensis, Meloidogyne javanica, Heterodera glycines, Pratylenchus coffeae, Pratylenchus neglectus and Tylenchus semipenetrans; and
the species of the phylum Mollusca such as Pomacea canaliculata, Achatina fulica, Meghimatium bilineatum, Lehmannina valentiana, Limax flavus and Acusta despecta sieboldiana.

In addition, the agricultural and horticultural fungicide of the present invention has a strong insecticidal effect on Tuta absoluta as well.

The agricultural and horticultural fungicide comprising the amide compound represented by the general formula [I] of the present invention or salts thereof as an active ingredient has a remarkable control effect on the above-described diseases which damage lowland crops, field crops, fruit trees, vegetables, other crops, ornamental flowering plants, etc. The desired effect can be obtained when the agricultural and horticultural fungicide is applied to nursery facilities for seedlings, paddy fields, fields, fruit trees, vegetables, other crops, ornamental flowering plants, etc. and their seeds, paddy water, foliage, cultivation media such as soil, or the like around the expected time of disease infestation, i.e., before the infestation or upon the confirmation of the infestation. In addition, the application of the agricultural and horticultural fungicide utilizes so-called penetration and translocation. That is, nursery soil, soil in transplanting holes, plant foot, irrigation water, cultivation water in hydroponics, or the like is treated with the agricultural and horticultural fungicide to allow crops, ornamental flowering plants, etc. to absorb the compound of the present invention through the roots via soil or otherwise.

The useful plants to which the agricultural and horticultural fungicide of the present invention can be applied include, but are not particularly limited to, for example, cereals (e.g., rice, barley, wheat, rye, oats, corn, etc.), legumes (e.g., soybeans, azuki beans, broad beans, green peas, kidney beans, peanuts, etc.), fruit trees and fruits (e.g., apples, citrus fruits, pears, grapes, peaches, plums, cherries, walnuts, chestnuts, almonds, bananas, etc.), leaf and fruit vegetables (e.g., cabbages, tomatoes, spinach, broccoli, lettuce, onions, green onions (chives and Welsh onions), green peppers, eggplants, strawberries, pepper crops, okra, Chinese chives, etc.), root vegetables (e.g., carrots, potatoes, sweet potatoes, taros, Japanese radishes, turnips, lotus roots, burdock roots, garlic, Chinese scallions, etc.), crops for processing (e.g., cotton, hemp, beet, hops, sugarcane, sugar beet, olives, rubber, coffee, tobacco, tea, etc.), gourds (e.g., Japanese pumpkins, cucumbers, watermelons, oriental sweet melons, melons, etc.), pasture grass (e.g., orchardgrass, sorghum, timothy, clover, alfalfa, etc.), lawn grass (e.g., Korean lawn grass, bent grass, etc.), spice and aromatic crops and ornamental crops (e.g., lavender, rosemary, thyme, parsley, pepper, ginger, etc.), ornamental flowering plants (e.g., chrysanthemum, rose, carnation, orchid, tulip, lily, etc.), garden trees (e.g., ginkgo trees, cherry trees, Japanese aucuba, etc.) and forest trees (e.g., Abies sachalinensis, Picea jezoensis, pine, yellow cedar, Japanese cedar, hinoki cypress, eucalyptus, etc.).

The above-mentioned "plants" also include plants provided with herbicide tolerance by a classical breeding technique or a gene recombination technique. Examples of such herbicide tolerance include tolerance to HPPD inhibitors, such as isoxaflutole; ALS inhibitors, such as imazethapyr and thifensulfuron-methyl; EPSP synthase inhibitors, such as glyphosate; glutamine synthetase inhibitors, such as glufosinate; acetyl-CoA carboxylase inhibitors, such as sethoxydim; or other herbicides, such as bromoxynil, dicamba and 2,4-D.

Examples of the plants provided with herbicide tolerance by a classical breeding technique include varieties of rapeseed, wheat, sunflower and rice tolerant to the imidazolinone family of ALS-inhibiting herbicides such as imazethapyr, and such rice is sold under the trade name of Clearfield (registered trademark). Also included is a variety of soybean provided with tolerance to the sulfonyl urea family of ALS-inhibiting herbicides such as thifensulfuron-methyl by a classical breeding technique, and this is sold under the trade name of STS soybean. Also included are plants provided with tolerance to acetyl-CoA carboxylase inhibitors such as trione oxime herbicides and aryloxy phenoxy propionic acid herbicides by a classical breeding technique, for example, SR corn and the like.

Plants provided with tolerance to acetyl-CoA carboxylase inhibitors are described in Proc. Natl. Acad. Sci. USA, 87, 7175-7179 (1990), and the like. Further, acetyl-CoA carboxylase mutants resistant to acetyl-CoA carboxylase inhibitors are reported in Weed Science, 53, 728-746 (2005), and the like, and by introducing the gene of such an acetyl-CoA carboxylase mutant into plants by a gene recombination technique, or introducing a resistance-conferring mutation into acetyl-CoA carboxylase of plants, plants tolerant to acetyl-CoA carboxylase inhibitors can be engineered. Alternatively, by introducing a nucleic acid causing base substitution mutation into plant cells (a typical example of this technique is chimeraplasty technique (Gura T. 1999. Repairing the Genome's Spelling Mistakes. Science 285: 316-318.)) to allow site-specific substitution mutation in the amino acids encoded by an acetyl-CoA carboxylase gene, an ALS gene or the like of plants, plants tolerant to acetyl-CoA carboxylase inhibitors, ALS inhibitors or the like can be engineered. The agricultural and horticultural fungicide of the present invention can be applied to these plants as well.

Further, exemplary toxins expressed in genetically modified plants include insecticidal proteins derived from Bacillus cereus or Bacillus popilliae; Bacillus thuringiensis-derived δ-endotoxins, such as Cry1Ab, Cry1Ac, Cry1F, Cry1Fa2, Cry2Ab, Cry3A, Cry3Bb1 and Cry9C, and other insecticidal proteins, such as VIP1, VIP2, VIP3 and VIP3A; nematode-derived insecticidal proteins; toxins produced by animals, such as scorpion toxins, spider toxins, bee toxins and insect-specific neurotoxins; toxins of filamentous fungi; plant lectins; agglutinin; protease inhibitors, such as trypsin inhibitors, serine protease inhibitors, patatin, cystatin and papain inhibitors; ribosome inactivating proteins (RIP), such as ricin, maize RIP, abrin, luffin, saporin and bryodin; steroid metabolizing enzymes, such as 3-hydroxy steroid oxidase, ecdysteroid-UDP-glucosyltransferase and cholesterol oxidase; ecdysone inhibitors; HMG-CoA reductase; ion channel inhibitors, such as sodium channel inhibitors and calcium channel inhibitors; juvenile hormone esterase; diuretic hormone receptors; stilbene synthase; bibenzyl synthase; chitinase; and glucanase.

Also included are hybrid toxins, partially deficient toxins and modified toxins derived from the following: δ-endotoxin proteins such as Cry1Ab, Cry1Ac, Cry1F, Cry1Fa2, Cry2Ab, Cry3A, Cry3Bb1, Cry9C, Cry34Ab and Cry35Ab, and other insecticidal proteins such as VIP1, VIP2, VIP3 and VIP3A. The hybrid toxin can be produced by combining domains derived from these proteins differently from the original combination in nature with the use of a recombination technique. As the partially deficient toxin, a CrylAb toxin in which a part of the amino acid sequence is deleted is known. In the modified toxin, one or more amino acids of a naturally occurring toxin are substituted.

Examples of the foregoing toxins and genetically modified plants capable of synthesizing these toxins are described in EP-A-0 374 753, WO 93/07278, WO 95/34656, EP-A-0 427 529, EP-A-451 878, WO 03/052073, etc.

Due to the toxins contained in such genetically modified plants, the plants exhibit resistance to pests, in particular, Coleopteran insect pests, Hemipteran insect pests, Dipteran insect pests, Lepidopteran insect pests and nematodes. The above-described technologies and the agricultural and horticultural fungicide of the present invention can be used in combination or used systematically.

In order to control various diseases, the agricultural and horticultural fungicide of the present invention, with or without appropriate dilution or suspension in water etc., is applied to plants potentially infested with diseases in an amount effective for the control of the diseases. For example, in order to control diseases that may infest crop plants such as fruit trees, cereals and vegetables, foliar application and seed treatment such as dipping, dust coating and calcium peroxide coating can be performed. Further, treatment of soil or the like may also be performed to allow plants to absorb agrochemicals through their roots. Examples of such treatment include whole soil incorporation, planting row treatment, bed soil incorporation, plug seedling treatment, planting hole treatment, plant foot treatment, top-dressing, treatment of nursery boxes for paddy rice, and submerged application. In addition, application to culture media in hydroponics, smoking treatment, trunk injection and the like can also be performed.

Further, the agricultural and horticultural fungicide of the present invention, with or without appropriate dilution or suspension in water **etc.,** can be applied to sites potentially infested with plant diseases in an amount effective for the control of the diseases. For example, it can be directly applied to stored grain pests, house pests, sanitary pests, forest pests, etc., and also be used for coating of residential building materials, for smoking treatment, or as a bait formulation.

Further, the agricultural and horticultural fungicide of the present invention, with or without appropriate dilution or suspension in water etc., can be applied to sites potentially infested with diseases in an amount effective for the control of the diseases.

Exemplary methods of seed treatment include dipping of seeds in a diluted or undiluted fluid of a liquid or solid formulation for the permeation of agrochemicals into the seeds; mixing or dust coating of seeds with a solid or liquid formulation for the adherence of the formulation onto the surfaces of the seeds; coating of seeds with a mixture of a solid or liquid formulation and an adhesive carrier such as resins and polymers; and application of a solid or liquid formulation to the vicinity of seeds at the same time as seeding.

The term "seed" in the above-mentioned seed treatment refers to a plant body which is in the early stages of cultivation and used for plant propagation. The examples include, in addition to a so-called seed, a plant body for vegetative propagation, such as a bulb, a tuber, a seed potato, a bulbil, a propagule, a discoid stem and a stem used for cuttage.

The term "soil" or "cultivation medium" in the method of the present invention for using an agricultural and horticultural fungicide refers to a support medium for crop cultivation, in particular a support medium which allows crop plants to spread their roots therein, and the materials are not particularly limited as long as they allow plants to grow. Examples of the support medium include what is called soils, seedling mats and water, and specific examples of the materials include sand, pumice, vermiculite, diatomite, agar, gelatinous substances, high-molecular-weight substances, rock wool, glass wool, wood chip and bark.

Exemplary methods of the application to crop foliage include application of a liquid formulation, such as an emulsifiable concentrate and a flowable, or a solid formulation, such as a wettable powder and a water-dispersible granule, after appropriate dilution in water; dust application; and smoking.

Exemplary methods of soil application include application of a water-diluted or undiluted liquid formulation to the foot of plants, nursery beds for seedlings, or the like; application of a granule to the foot of plants, nursery beds for seedlings, or the like; application of a dust, a wettable powder, a water-dispersible granule, a granule or the like onto soil and subsequent incorporation of the formulation into the whole soil before seeding or transplanting; application of a dust, a wettable powder, a water-dispersible granule, a granule or the like to planting holes before seeding or planting; and application of a dust, a wettable powder, a water-dispersible granule, a granule or the like to planting rows or the like.

To nursery boxes for paddy rice, for example, a dust, a water-dispersible granule, a granule or the like can be applied, although the suitable formulation may vary depending on the application time, in other words, depending on the cultivation stage such as seeding time, greening period and planting time. A formulation such as a dust, a water-dispersible granule, a granule or the like may be mixed with nursery soil. For example, such a formulation is incorporated into bed soil, covering soil or the whole soil. Simply, nursery soil and such a formulation may be alternately layered.

In the application to paddy fields, a solid formulation, such as a jumbo, a pack, a granule and a water-dispersible granule, or a liquid formulation, such as a flowable and an emulsifiable concentrate, is applied usually to flooded paddy fields. In a rice planting period, a suitable formulation, as it is or after mixed with a fertilizer or the like, may be applied onto soil or injected into soil. In addition, a solution of an emulsifiable concentrate, a flowable or the like may be applied to the source of water supply for paddy fields, such as a water inlet and an irrigation device. In this case, treatment can be accomplished with the supply of water and thus achieved in a labor-saving manner.

In the case of field crops, their seeds, cultivation media in the vicinity of their plants, or the like may be treated in the period of seeding to seedling culture. In the case of plants of which the seeds are directly sown in the field, in addition to direct seed treatment, plant foot treatment during cultivation is preferable. Specifically, the treatment can be performed by, for example, applying a granule onto soil, or drenching soil with a formulation in a water-diluted or undiluted liquid form. Another preferable treatment is incorporation of a granule into cultivation media before seeding.

In the case of culture plants to be transplanted, preferable examples of the treatment in the period of seeding to seedling culture include, in addition to direct seed treatment, drench treatment of nursery beds for seedlings with a formulation in a liquid form; and granule application to nursery beds for seedlings. Also included are treatment of planting holes with a granule; and incorporation of a granule into cultivation media in the vicinity of planting points at the time of fix planting.

The agricultural and horticultural fungicide of the present invention is commonly used as a formulation convenient for application, which is prepared in the usual method for preparing agrochemical formulations.

That is, the amide compound represented by the general formula [I] of the present invention or salts thereof and an appropriate carrier, and if needed an adjuvant, are blended in an appropriate ratio, and through the step of dissolution, separation, suspension, mixing, impregnation, adsorption and/or adhesion, can be formulated into an appropriate form for application, such as a suspension concentrate, an emulsifiable concentrate, a soluble concentrate, a wettable powder, a water-dispersible granule, a granule, a dust, a tablet and a pack.

The agricultural and horticultural fungicidal composition of the present invention can optionally contain an additive usually used for agrochemical formulations in addition to the active ingredient. Examples of the additive include carriers such as solid or liquid carriers, surfactants, dispersants, wetting agents, binders, tackifiers, thickeners, colorants, spreaders, sticking/spreading agents, antifreezing agents, anti-caking agents, disintegrants and stabilizing agents. If needed, preservatives, plant fragments, etc. may also be used as the additive. These additives may be used alone or in a combination of two or more kinds.

Examples of the solid carriers include natural minerals, such as quartz, clay, kaolinite, pyrophyllite, sericite, talc, bentonite, acid clay, attapulgite, zeolite and diatomite; inorganic salts, such as calcium carbonate, ammonium sulfate, sodium sulfate and potassium chloride; organic solid carriers, such as synthetic silicic acid, synthetic high-dispersion silicic acid, synthetic silicates, starch, cellulose and plant powders (for example, sawdust, coconut shell, corn cob, tobacco stalk, etc.); plastics carriers, such as polyethylene, polypropylene and polyvinylidene chloride; urea; hollow inorganic materials; hollow plastic materials; and fumed silica (white carbon). These solid carriers may be used alone or in a combination of two or more kinds.

Examples of the liquid carriers include alcohols including monohydric alcohols, such as methanol, ethanol, propanol, isopropanol and butanol, and polyhydric alcohols, such as ethylene glycol, diethylene glycol, propylene glycol, hexylene glycol, polyethylene glycol, polypropylene glycol and glycerin; polyol compounds, such as propylene glycol ether; ketones, such as acetone, methyl ethyl ketone, methyl isobutyl ketone, diisobutyl ketone and cyclohexanone; straight-chain or cyclic ethers, such as ethyl ether, dioxane, ethylene glycol monoethyl ether, dipropyl ether and tetrahydrofuran; aliphatic hydrocarbons, such as normal paraffin, naphthene, isoparaffin, kerosene and mineral oil; aromatic hydrocarbons, such as benzene, toluene, xylene, solvent naphtha and alkyl naphthalene; halogenated hydrocarbons, such as dichloromethane, chloroform and carbon tetrachloride; esters, such as ethyl acetate, diisopropyl phthalate, dibutyl phthalate, dioctyl phthalate and dimethyl adipate; lactones, such as γ-butyrolactone; amides, such as dimethylformamide, diethylformamide, dimethylacetamide, N-alkyl pyrrolidinone and N-methylpyrrolidone; nitriles, such as acetonitrile; sulfur compounds, such as dimethyl sulfoxide; vegetable oils, such as soybean oil, rapeseed oil, cotton seed oil and castor oil; and water. These liquid carriers may be used alone or in a combination of two or more kinds.

Exemplary surfactants used as the dispersant or the wetting/spreading agent include nonionic surfactants, such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl aryl ether, polyoxyethylene alkyl phenyl ether (e.g., polyoxyethylene nonylphenyl ether), polyoxyethylene dialkyl phenyl ether, polyoxyethylene alkyl phenyl ether-formaldehyde condensates, polyoxyethylene-polyoxypropylene block copolymers, polystyrene-polyoxyethylene block polymers, alkyl polyoxyethylene-polypropylene block copolymer ether, polyoxyethylene alkylamine, polyoxyethylene fatty acid amide, polyoxyethylene fatty acid bis(phenyl ether), polyalkylene benzyl phenyl ether, polyoxyalkylene styryl phenyl ether, acetylene diol, polyoxyalkylene-added acetylene diol, polyoxyethylene ether-type silicone, ester-type silicone, fluorosurfactants, polyoxyethylene castor oil and polyoxyethylene hydrogenated castor oil; anionic surfactants, such as alkyl sulfates, polyoxyethylene alkyl ether sulfates, polyoxyethylene alkyl phenyl ether sulfates, polyoxyethylene styryl phenyl ether sulfates, alkylbenzene sulfonates, alkylaryl sulfonates, lignosulfonates, alkyl sulfosuccinates, naphthalene sulfonates, alkylnaphthalene sulfonates, salts of naphthalenesulfonic acid-formaldehyde condensates, salts of alkylnaphthalenesulfonic acid-formaldehyde condensates, fatty acid salts, polycarboxylic acid salts, polyacrylates, N-methyl-fatty acid sarcosinates, resinates, polyoxyethylene alkyl ether phosphates and polyoxyethylene alkyl phenyl ether phosphates; cationic surfactants including alkyl amine salts, such as lauryl amine hydrochloride, stearyl amine hydrochloride, oleyl amine hydrochloride, stearyl amine acetate, stearyl aminopropyl amine acetate, alkyl trimethyl ammonium chloride and alkyl dimethyl benzalkonium chloride; and amphoteric surfactants, such as amino acid-type or betaine-type amphoteric surfactants. These surfactants may be used alone or in a combination of two or more kinds.

Examples of the binders or the tackifiers include carboxymethyl cellulose or salts thereof, dextrin, soluble starch, xanthan gum, guar gum, sucrose, polyvinyl pyrrolidone, gum arabic, polyvinyl alcohol, polyvinyl acetate, sodium polyacrylate, polyethylene glycols with an average molecular weight of 6,000 to 20,000, polyethylene oxides with an average molecular weight of 100,000 to 5,000,000, phospholipids (for example, cephalin, lecithin, etc.), cellulose powder, dextrin, modified starch, polyaminocarboxylic acid chelating compounds, cross-linked polyvinyl pyrrolidone, maleic acid-styrene copolymers, (meth)acrylic acid copolymers, half esters of polyhydric alcohol polymer and dicarboxylic anhydride, water soluble polystyrene sulfonates, paraffin, terpene, polyamide resins, polyacrylates, polyoxyethylene, waxes, polyvinyl alkyl ether, alkylphenol-formaldehyde condensates and synthetic resin emulsions.

Examples of the thickeners include water soluble polymers, such as xanthan gum, guar gum, diutan gum, carboxymethyl cellulose, polyvinyl pyrrolidone, carboxyvinyl polymers, acrylic polymers, starch compounds and polysaccharides; and inorganic fine powders, such as high-grade bentonite and fumed silica (white carbon).

Examples of the colorants include inorganic pigments, such as iron oxide, titanium oxide and prussian blue; and organic dyes, such as alizarin dyes, azo dyes and metal phthalocyanine dyes.

Examples of the antifreezing agents include polyhydric alcohols, such as ethylene glycol, diethylene glycol, propylene glycol and glycerin.

Examples of the adjuvants serving to prevent caking or facilitate disintegration include polysaccharides (starch, alginic acid, mannose, galactose, etc.), polyvinyl pyrrolidone, fumed silica (white carbon) , ester gum, petroleum resin, sodium tripolyphosphate, sodium hexametaphosphate, metal stearates, cellulose powder, dextrin, methacrylate copolymers, polyvinyl pyrrolidone, polyaminocarboxylic acid chelating compounds, sulfonated styrene-isobutylene-maleic anhydride copolymers and starch-polyacrylonitrile graft copolymers.

Examples of the stabilizing agents include desiccants, such as zeolite, quicklime and magnesium oxide; antioxidants, such as phenolic compounds, amine compounds, sulfur compounds and phosphoric acid compounds; and ultraviolet absorbers, such as salicylic acid compounds and benzophenone compounds.

Examples of the preservatives include potassium sorbate and 1,2-benzothiazolin-3-one.

Further, other adjuvants including functional spreading agents, activity enhancers such as metabolic inhibitors (piperonyl butoxide etc.), antifreezing agents (propylene glycol etc.), antioxidants (BHT etc.) and ultraviolet absorbers can also be used if needed.

The content of the active ingredient compound in the agricultural and horticultural fungicide of the present invention can be adjusted as needed, and for example, is appropriately selected from the range of 0.01 to 90 parts by weight in 100 parts by weight of the agricultural and horticultural fungicide. For example, in the case where the agricultural and horticultural fungicide is a dust, a granule, an emulsifiable concentrate or a wettable powder, it is suitable that the content of the active ingredient compound is 0.01 to 50 parts by weight (0.01 to 50% by weight relative to the total weight of the agricultural and horticultural fungicide).

The application rate of the agricultural and horticultural fungicide of the present invention may vary with various factors, for example, the purpose, the status of the target disease, the growing conditions of crops, the tendency of disease infestation, the weather, the environmental conditions, the dosage form, the application method, the application site, the application time, etc., but for example, the application amount of the active ingredient compound per 10 ares is appropriately selected from the range of 0.001 g to 10 kg, and preferably 0.01 g to 1 kg depending on the purpose.

Furthermore, for the expansion of the range of target pests and diseases and the appropriate application time for control of pests and diseases, or for dose reduction, the agricultural and horticultural fungicide of the present invention can be used after mixed with other agricultural or horticultural insecticides, acaricides, nematicides, fungicides, biopesticides and/or the like. Further, the agricultural and horticultural fungicide can be used after mixed with herbicides, plant growth regulators, fertilizers and/or the like depending on its application.

Examples of such additional agricultural and horticultural insecticides, acaricides and nematicides used for the above-mentioned purposes include 3,5-xylyl methylcarbamate (XMC), crystalline protein toxins produced by Bacillus thuringiensis such as Bacillus thuringiensis aizawai, Bacillus thuringiensis israelensis, Bacillus thuringiensis japonensis, Bacillus thuringiensis kurstaki and Bacillus thuringiensis tenebrionis, BPMC (2-(1-methylpropyl)phenyl N-methylcarbamate), Bt toxin-derived insecticidal compounds, chlorfenson (CPCBS), dichlorodiisopropyl ether (DCIP), 1,3-dichloropropene (D-D), DDT, NAC, O-4-dimethylsulfamoylphenyl O,O-diethyl phosphorothioate (DSP), O-ethyl O-4-nitrophenyl phenylphosphonothioate (EPN), tripropylisocyanurate (TPIC), acrinathrin, azadirachtin, azinphos-methyl, acequinocyl, acetamiprid, acetoprole, acephate, abamectin, avermectin-B, amidoflumet, amitraz, alanycarb, aldicarb, aldoxycarb, aldrin, alpha-endosulfan, alpha-cypermethrin, albendazole, allethrin, isazofos, isamidofos, isoamidofos, isoxathion, isofenphos, isoprocarb (MIPC), ivermectin, imicyafos, imidacloprid, imiprothrin, indoxacarb, esfenvalerate, ethiofencarb, ethion, ethiprole, etoxazole, ethofenprox, ethoprophos, etrimfos, emamectin, emamectin-benzoate, endosulfan, empenthrin, oxamyl, oxydemeton-methyl, oxydeprofos (ESP), oxibendazole, oxfendazole, potassium oleate, sodium oleate, cadusafos, cartap, carbaryl, carbosulfan, carbofuran, gamma-cyhalothrin, xylylcarb, quinalphos, kinoprene, chinomethionat, cloethocarb, clothianidin, clofentezine, chromafenozide, chlorantraniliprole, chlorethoxyfos, chlordimeform, chlordane, chlorpyrifos, chlorpyrifos-methyl, chlorphenapyr, chlorfenson, chlorfenvinphos, chlorfluazuron, chlorobenzilate, chlorobenzoate, kelthane (dicofol), salithion, cyanophos (CYAP), diafenthiuron, diamidafos, cyantraniliprole, theta-cypermethrin, dienochlor, cyenopyrafen, dioxabenzofos, diofenolan, sigma-cypermethrin, dichlofenthion (ECP), cycloprothrin, dichlorvos (DDVP), disulfoton, dinotefuran, cyhalothrin, cyphenothrin, cyfluthrin, diflubenzuron, cyflumetofen, diflovidazin, cyhexatin, cypermethrin, dimethylvinphos, dimethoate, dimefluthrin, silafluofen, cyromazine, spinetoram, spinosad, spirodiclofen, spirotetramat, spiropidion, spirobudifen, spiromesifen, sulfluramid, sulprofos, sulfoxaflor, zeta-cypermethrin, diazinon, tau-fluvalinate, dazomet, thiacloprid, thiamethoxam, thiodicarb, thiocyclam, thiosultap, thiosultap-sodium, thionazin, thiometon, tiorantraniliprole, deet, dieldrin, tetrachlorvinphos, tetradifon, tetramethylfluthrin, tetramethrin, tebupirimfos, tebufenozide, tebufenpyrad, tefluthrin, teflubenzuron, demeton-S-methyl, temephos, deltamethrin, terbufos, tralopyril, tralomethrin, transfluthrin, triazamate, triazuron, trichlamide, trichlorphon (DEP), triflumuron, tolfenpyrad, naled (BRP), nithiazine, nitenpyram, novaluron, noviflumuron, hydroprene, vaniliprole, vamidothion, parathion, parathion-methyl, halfenprox, halofenozide, bistrifluron, bisultap, hydramethylnon, hydroxy propyl starch, binapacryl, bifenazate, bifenthrin, pymetrozine, pyraclofos, pyrafluprole, pyridafenthion, pyridaben, pyridalyl, pyrifluquinazon, pyriprole, pyriproxyfen, pirimicarb, pyrimidifen, pirimiphos-methyl, pyrethrins, fipronil, fenazaquin, fenamiphos, phenisobromolate, fenitrothion (MEP), fenoxycarb, fenothiocarb, phenothrin, fenobucarb, fensulfothion, fenthion (MPP), phenthoate (PAP), fenvalerate, fenpyroximate, fenpropathrin, fenbendazole, fosthiazate, formetanate, butathiofos, buprofezin, furathiocarb, prallethrin, fluacrypyrim, fluazinam, fluazuron, fluensulfone, flucycloxuron, flucythrinate, fluvalinate, flupyrazofos, flufenerim, flufenoxuron, flufenzine, flufenprox, fluproxyfen, flubrocythrinate, flubendiamide, flupentiofenox, flumethrin, flurimfen, prothiofos, protrifenbute, flonicamid, propaphos, propargite (BPPS), profenofos, profluthrin, propoxur (PHC), bromopropylate, beta-cyfluthrin, hexaflumuron, hexythiazox, heptenophos, permethrin, benclothiaz, bendiocarb, bensultap, benzoximate, benfuracarb, phoxim, phosalone, fosthiazate, fosthietan, phosphamidon, phosphocarb, phosmet (PMP), polynactins, formetanate, formothion, phorate, machine oil, malathion, milbemycin, milbemycin-A, milbemectin, mecarbam, mesulfenfos, methomyl, metaldehyde, metaflumizone, methamidophos, metam-ammonium, metam-sodium, methiocarb, methidathion (DMTP), methylisothiocyanate, methylneodecanamide, methylparathion, metoxadiazone, methoxychlor, methoxyfenozide, metofluthrin, methoprene, metolcarb, meperfluthrin, mevinphos, monocrotophos, monosultap, lambda-cyhalothrin, ryanodine, lufenuron, resmethrin, lepimectin, rotenone, levamisole hydrochloride, fenbutatin oxide, morantel tartarate, methyl bromide, tricyclohexyltin hydroxide (cyhexatin), calcium cyanamide, calcium polysulfide, sulfur, nicotine-sulfate, isocycloseram, dimpropyridaz, and flupyrimin.

Examples of the agricultural and horticultural fungicides used for the same purposes as above include aureofungin, azaconazole, azithiram, acypetacs, acibenzolar, acibenzolar-S-methyl, azoxystrobin, anilazine, amisulbrom, ampropylfos, ametoctradin, allyl alcohol, aldimorph, amobam, isotianil, isovaledione, isopyrazam, isoprothiolane, ipconazole, iprodione, iprovalicarb, iprobenfos, imazalil, iminoctadine, iminoctadine-albesilate, iminoctadine-triacetate, imibenconazole, uniconazole, uniconazole-P, echlomezole, edifenphos, etaconazole, ethaboxam, ethirimol, etem, ethoxyquin, etridiazole, enestroburin, epoxiconazole, oxadixyl, oxycarboxin, copper-8-quinolinolate, oxytetracycline, copper-oxinate, oxpoconazole, oxpoconazole-fumarate, oxolinic acid, octhilinone, ofurace, orysastrobin, metam-sodium, kasugamycin, carbamorph, carpropamid, carbendazim, carboxin, carvone, quinazamid, quinacetol, quinoxyfen, quinomethionate, captafol, captan, kiralaxyl, quinconazole, quintozene, guazatine, cufraneb, cuprobam, glyodin, griseofulvin, climbazole, cresol, kresoxim-methyl, chlozolinate, clotrimazole, chlobenthiazone, chloraniformethan, chloranil, chlorquinox, chloropicrin, chlorfenazole, chloroinconazide, chlorodinitronaphthalene, chlorothalonil, chloroneb, zarilamid, salicylanilide, cyazofamid, diethyl pyrocarbonate, diethofencarb, cyclafuramid, diclocymet, dichlozoline, diclobutrazol, dichlofluanid, cycloheximide, diclomezine, dicloran, dichlorophen, dichlone, disulfiram, ditalimfos, dithianon, diniconazole, diniconazole-M, zineb, dinocap, dinocton, dinosulfon, dinoterbon, dinobuton, dinopenton, dipyrithione, diphenylamine, difenoconazole, cyflufenamid, diflumetorim, cyproconazole, cyprodinil, cyprofuram, cypendazole, simeconazole, dimethirimol, dimethomorph, cymoxanil, dimoxystrobin, methyl bromide, ziram, silthiofam, streptomycin, spiroxamine, sultropen, sedaxane, seboctylamine, zoxamide, dazomet, thiadiazin, tiadinil, thiadifluor, thiabendazole, tioxymid, thiochlorfenphim, thiophanate, thiophanate-methyl, thicyofen, thioquinox, chinomethionat, thifluzamide, thiram, decafentin, tecnazene, tecloftalam, tecoram, tetraconazole, debacarb, dehydroacetic acid, tebuconazole, tebufloquin, dodicin, dodine, dodecyl benzensulfonate bis-ethylene diamine copper(II) (DBEDC), dodemorph, drazoxolon, triadimenol, triadimefon, triazbutil, triazoxide, triamiphos, triarimol, trichlamide, tricyclazole, triticonazole, tridemorph, tributyltin oxide, triflumizole, trifloxystrobin, triforine, tolylfluanid, tolclofos-methyl, natamycin, nabam, nitrothal-isopropyl, nitrostyrene, nuarimol, copper nonylphenol sulfonate, halacrinate, validamycin, valifenalate, harpin protein, bixafen, picoxystrobin, picobenzamide, bithionol, bitertanol, hydroxyisoxazole, hydroxyisoxazole-potassium, binapacryl, biphenyl, piperalin, hymexazol, pyraoxystrobin, pyracarbolid, pyraclostrobin, pyrazophos, pyrametostrobin, pyriofenone, pyridinitril, pyrifenox, pyribencarb, pyrimethanil, pyroxychlor, pyroxyfur, pyroquilon, vinclozolin, famoxadone, fenapanil, fenamidone, fenaminosulf, fenarimol, fenitropan, fenoxanil, ferimzone, ferbam, fentin, fenpiclonil, fenpyrazamine, fenbuconazole, fenfuram, fenpropidin, fenpropimorph, fenhexamid, phthalide, buthiobate, butylamine, bupirimate, fuberidazole, blasticidin-S, furametpyr, furalaxyl, fluacrypyrim, fluazinam, fluoxastrobin, fluotrimazole, fluopicolide, fluopyram, fluoroimide, furcarbanil, fluxapyroxad, fluquinconazole, furconazole, furconazole-cis, fludioxonil, flusilazole, flusulfamide, flutianil, flutolanil, flutriafol, flufenoxadiazam, flufenoxystrobin, furfural, furmecyclox, flumetylsulforim, flumetover, flumorph, proquinazid, prochloraz, procymidone, prothiocarb, prothioconazole, propamocarb, propiconazole, propineb, furophanate, probenazole, bromuconazole, hexachlorobutadiene, hexaconazole, hexylthiofos, bethoxazin, benalaxyl, benalaxyl-M, benodanil, benomyl, pefurazoate, benquinox, penconazole, benzamorf, pencycuron, benzohydroxamic acid, bentaluron, benthiazole, benthiavalicarb-isopropyl, penthiopyrad, penflufen, boscalid, phosdiphen, fosetyl, fosetyl-aluminum, polyoxins, polyoxorim, polycarbamate, folpet, formaldehyde, machine oil, maneb, mancozeb, mandipropamid, myclozolin, myclobutanil, mildiomycin, milneb, mecarbinzid, methasulfocarb, metazoxolon, metam, metam-sodium, metalaxyl, metalaxyl-M, metarylpicoxamid, metiram, methyl isothiocyanate, meptyldinocap, metconazole, metsulfovax, methfuroxam, metominostrobin, metrafenone, mepanipyrim, mefenoxam, meptyldinocap, mepronil, mebenil, iodomethane, rabenzazole, benzalkonium chloride, inorganic fungicides such as basic copper chloride, basic copper sulfate and silver, sodium hypochlorite, cupric hydroxide, wettable sulfur, calcium polysulfide, potassium hydrogen carbonate, sodium hydrogen carbonate, sulfur, copper sulfate anhydride, nickel dimethyldithiocarbamate, copper compounds such as copper-8-quinolinolate (oxine copper), zinc sulfate, copper sulfate pentahydrate, ipflufenoquin, pyridachlometyl, fluoxapiprolin, fluoxytioconazole, pronitridine, florylpicoxamid, and metyltetraprole.

Further, examples of the herbicides include 1-naphthylacetamide, 2,4-PA, 2,3,6-TBA, 2,4,5-T, 2,4,5-TB, 2, 4-D, 2, 4-DB, 2, 4-DEB, 2, 4-DEP, 3, 4-DA, 3,4-DB, 3,4-DP, 4-CPA, 4-CPB, 4-CPP, MCP, MCPA, MCPA-thioethyl, MCPB, ioxynil, aclonifen, azafenidin, acifluorfen, aziprotryne, azimsulfuron, asulam, acetochlor, atrazine, atraton, anisuron, anilofos, aviglycine, abscisic acid, amicarbazone, amidosulfuron, amitrole, aminocyclopyrachlor, aminopyralid, amibuzin, amiprophos-methyl, ametridione, ametryn, alachlor, allidochlor, alloxydim, alorac, isouron, isocarbamid, isoxachlortole, isoxapyrifop, isoxaflutole, isoxaben, isocil, isonoruron, isoproturon, isopropalin, isopolinate, isomethiozin, inabenfide, ipazine, ipfencarbazone, iprymidam, imazaquin, imazapic, imazapyr, imazamethapyr, imazamethabenz, imazamethabenz-methyl, imazamox, imazethapyr, imazosulfuron, indaziflam, indanofan, indolebutyric acid, uniconazole-P, eglinazine, esprocarb, ethametsulfuron, ethametsulfuron-methyl, ethalfluralin, ethiolate, ethychlozate-ethyl, ethidimuron, etinofen, ethephon, ethoxysulfuron, ethoxyfen, etnipromid, ethofumesate, etobenzanid, epronaz, erbon, endothal, oxadiazon, oxadiargyl, oxaziclomefone, oxasulfuron, oxapyrazon, oxyfluorfen, oryzalin, orthosulfamuron, orbencarb, cafenstrole, cambendichlor, carbasulam, carfentrazone, carfentrazone-ethyl, karbutilate, carbetamide, carboxazole, quizalofop, quizalofop-P, quizalofop-ethyl, xylachlor, quinoclamine, quinonamid, quinclorac, quinmerac, cumyluron, cliodinate, glyphosate, glufosinate, glufosinate-P, credazine, clethodim, cloxyfonac, clodinafop, clodinafop-propargyl, chlorotoluron, clopyralid, cloproxydim, cloprop, chlorbromuron, clofop, clomazone, chlomethoxynil, chlomethoxyfen, clomeprop, chlorazifop, chlorazine, chloranocryl, chloramben, cloransulam, cloransulam-methyl, chloridazon, chlorimuron, chlorimuron-ethyl, chlorsulfuron, chlorthal, chlorthiamid, chlortoluron, chlornitrofen, chlorfenac, chlorfenprop, chlorbufam, chlorflurazole, chlorflurenol, chlorprocarb, chlorpropham, chlormequat, chloreturon, chloroxynil, chloroxuron, chloropon, saflufenacil, cyanazine, cyanatryn, di-allate, diuron, diethamquat, dicamba, cycluron, cycloate, cycloxydim, diclosulam, cyclosulfamuron, dichlorprop, dichlorprop-P, dichlobenil, diclofop, diclofop-methyl, dichlormate, dichloralurea, diquat, cisanilide, disul, siduron, dithiopyr, dinitramine, cinidon-ethyl, dinosam, cinosulfuron, dinoseb, dinoterb, dinofenate, dinoprop, cyhalofop-butyl, diphenamid, difenoxuron, difenopenten, difenzoquat, cybutryne, cyprazine, cyprazole, diflufenican, diflufenzopyr, dipropetryn, cypromid, cyperquat, gibberellin, simazine, dimexano, dimethachlor, dimidazon, dimethametryn, dimethenamid, simetryn, simeton, dimepiperate, dimefuron, cinmethylin, swep, sulglycapin, sulcotrione, sulfallate, sulfentrazone, sulfosulfuron, sulfometuron, sulfometuron-methyl, secbumeton, sethoxydim, sebuthylazine, terbacil, daimuron, dazomet, dalapon, thiazafluron, thiazopyr, thiencarbazone, thiencarbazone-methyl, tiocarbazil, tioclorim, thiobencarb, thidiazimin, thidiazuron, thifensulfuron, thifensulfuron-methyl, desmedipham, desmetryn, tetrafluron, thenylchlor, tebutam, tebuthiuron, terbumeton, tepraloxydim, tefuryltrione, tembotrione, delachlor, terbacil, terbucarb, terbuchlor, terbuthylazine, terbutryn, topramezone, tralkoxydim, triaziflam, triasulfuron, tri-allate, trietazine, tricamba, triclopyr, tridiphane, tritac, tritosulfuron, triflusulfuron, triflusulfuron-methyl, trifluralin, trifloxysulfuron, tripropindan, tribenuron-methyl, tribenuron, trifop, trifopsime, trimeturon, naptalam, naproanilide, napropamide, nicosulfuron, nitralin, nitrofen, nitrofluorfen, nipyraclofen, neburon, norflurazon, noruron, barban, paclobutrazol, paraquat, parafluron, haloxydine, haloxyfop, haloxyfop-P, haloxyfop-methyl, halosafen, halosulfuron, halosulfuron-methyl, picloram, picolinafen, bicyclopyrone, bispyribac, bispyribac-sodium, pydanon, pinoxaden, bifenox, piperophos, hymexazol, pyraclonil, pyrasulfotole, pyrazoxyfen, pyrazosulfuron, pyrazosulfuron-ethyl, pyrazolate, bilanafos, pyraflufen-ethyl, pyriclor, pyridafol, pyrithiobac, pyrithiobac-sodium, pyridate, pyriftalid, pyributicarb, pyribenzoxim, pyrimisulfan, primisulfuron, pyriminobac-methyl, pyroxasulfone, pyroxsulam, fenasulam, phenisopham, fenuron, fenoxasulfone, fenoxaprop, fenoxaprop-P, fenoxaprop-ethyl, phenothiol, fenoprop, phenobenzuron, fenthiaprop, fenteracol, fentrazamide, phenmedipham, phenmedipham-ethyl, butachlor, butafenacil, butamifos, buthiuron, buthidazole, butylate, buturon, butenachlor, butroxydim, butralin, flazasulfuron, flamprop, furyloxyfen, prynachlor, primisulfuron-methyl, fluazifop, fluazifop-P, fluazifop-butyl, fluazolate, fluchloraminopyr, fluroxypyr, fluothiuron, fluometuron, fluoroglycofen, flurochloridone, fluorodifen, fluoronitrofen, fluoromidine, flucarbazone, flucarbazone-sodium, fluchloralin, flucetosulfuron, fluthiacet, fluthiacet-methyl, flupyrsulfuron, flufenacet, flufenican, flufenpyr, flupropacil, flupropanate, flupoxam, flumioxazin, flumiclorac, flumiclorac-pentyl, flumipropyn, flumezin, fluometuron, flumetsulam, fluridone, flurtamone, fluroxypyr, pretilachlor, proxan, proglinazine, procyazine, prodiamine, prosulfalin, prosulfuron, prosulfocarb, propaquizafop, propachlor, propazine, propanil, propyzamide, propisochlor, prohydrojasmon, propyrisulfuron, propham, profluazol, profluralin, prohexadione-calcium, propoxycarbazone, propoxycarbazone-sodium, profoxydim, bromacil, brompyrazon, prometryn, prometon, bromoxynil, bromofenoxim, bromobutide, bromobonil, florasulam, florpyrauxifen, (C-461) hexachloroacetone, hexazinone, pethoxamid, benazolin, penoxsulam, pebulate, beflubutamid, vernolate, perfluidone, bencarbazone, benzadox, benzipram, benzylaminopurine, benzthiazuron, benzfendizone, bensulide, bensulfuron-methyl, benzoylprop, benzobicyclon, benzofenap, benzofluor, bentazone, pentanochlor, benthiocarb, pendimethalin, pentoxazone, benfluralin, benfuresate, fosamine, fomesafen, foramsulfuron, forchlorfenuron, maleic hydrazide, mecoprop, mecoprop-P, medinoterb, mesosulfuron, mesosulfuron-methyl, mesotrione, mesoprazine, methoprotryne, metazachlor, methazole, metazosulfuron, methabenzthiazuron, metamitron, metamifop, metam, methalpropalin, methiuron, methiozolin, methiobencarb, methyldymron, metoxuron, metosulam, metsulfuron, metsulfuron-methyl, metflurazon, metobromuron, metobenzuron, methometon, metolachlor, metribuzin, mepiquat-chloride, mefenacet, mefluidide, monalide, monisouron, monuron, monochloroacetic acid, monolinuron, molinate, morfamquat, iodosulfuron, iodosulfuron-methyl-sodium, iodobonil, iodomethane, lactofen, linuron, rimsulfuron, lenacil, rhodethanil, calcium peroxide, methyl bromide, cyclopyranil, and tetflupyrolimet.

Examples of the biopesticides include viral formulations such as nuclear polyhedrosis viruses (NPV), granulosis viruses (GV), cytoplasmic polyhedrosis viruses (CPV) and entomopox viruses (EPV) ; microbial pesticides used as an insecticide or a nematicide, such as Monacrosporium phymatophagum, Steinernema carpocapsae, Steinernema kushidai and Pasteuria penetrans; microbial pesticides used as a fungicide, such as Trichoderma lignorum, Agrobacterium radiobactor, avirulent Erwinia carotovora and Bacillus subtilis; and biopesticides used as a herbicide, such as Xanthomonas campestris. A combined use of the agricultural and horticultural fungicide of the present invention with the foregoing biopesticide as a mixture can be expected to provide the same effect as above.

Other examples of the biopesticide used in combination with the agricultural or horticultural fungicide of the present invention include natural predators such as Encarsia formosa, Aphidius colemani, Aphidoletes aphidimyza, Diglyphus isaea, Dacnusa sibirica, Phytoseiulus persimilis, Amblyseius cucumeris, and Orius sauteri; microbial pesticides such as Beauveria brongniartii; and pheromones such as (Z)-10-tetradecenyl acetate, (E,Z)-4,10-tetradecadienyl acetate, (Z)-8-dodecenyl acetate, (Z)-11-tetradecenyl acetate, (Z)-13-icosen-10-one, and 14-methyl-1-octadecene.

Hereinafter, the production examples of representative compounds of the present invention and their intermediates will be described in more detail, but the present invention is not limited only to these examples. THF, DMF, TEA, DMAP and EtOH represent tetrahydrofuran, dimethylformamide, triethylamine, 4-dimethylaminopyridine and ethanol, respectively, and mmol represents millimoles. The values (%) in parentheses following the amounts of the products obtained in the following examples and reference examples indicate the yields.

### [Examples]

### Example 1. Production of Compound No. I-A-101

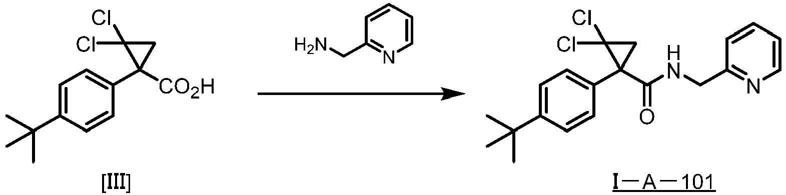

To a mixed solution of carboxylic acid [III] (11 g, 41 mmol) produced by the method of Reference Example 2 were added 2-picolylamine (5.3 g, 49 mmol), tetrahydrofuran (0.41 L), 2-chloro-1-methylpyridinium iodide (21 g, 82 mmol), triethylamine (17 mL, 0.12 mol), and DMAP (0.50 g, 4.1 mmol), and the mixture was stirred under heating and refluxing conditions for 6 hr. After the completion of the reaction, an aqueous ammonium chloride solution was added, and the mixture was subjected to extraction with ethyl acetate. The obtained organic phase was dried over sodium sulfate, the solvent was evaporated under reduced pressure, and the obtained residue was purified by silica gel column chromatography to give 12 g (yield: 78%) of Compound I-A-101.

### Example 2. Production of Compound No. I-A-261

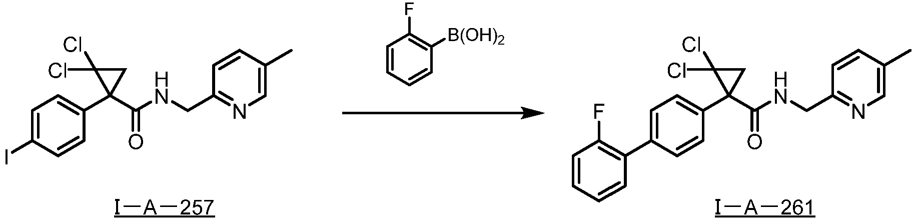

To a mixed solution of Compound I-A-257 (0.10 g, 0.22 mmol), 2-fluoroboronic acid (33 mg, 0.24 mmol), Pd(dppf)Cl₂· MeCN (18 mg, 0.022 mmol), and toluene (1.8 mL) was added 1M K₃PO₄ aqueous solution (0.43 mL, 0.43 mmol), and the mixture was stirred under argon atmosphere at 90°C for 3 hr. After the completion of the reaction, an aqueous ammonium chloride solution was added, and the mixture was subjected to extraction with ethyl acetate. The obtained organic phase was dried over sodium sulfate, the solvent was evaporated under reduced pressure, and the obtained residue was purified by silica gel column chromatography to give 86 mg (yield: 92%) of Compound I-A-261.

The raw materials or intermediates of the present invention can be produced, for example, by the following methods. These compounds can be produced by known methods or methods known per se.

### Reference Example 1.

### Production method for Intermediate [II]

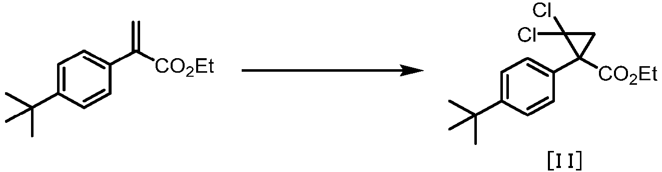

A mixed solution of ethyl 4-(1,1-dimethylethyl)-α-methylenebenzeneacetate (16 g, 70 mmol), triethylbenzylammonium chloride (TEBAC 1.6 g, 7.0 mmol), and chloroform (210 mL) was added dropwise to 25 wt% aqueous sodium hydroxide solution (42 g, 260 mmol) with stirring at room temperature, and the mixture was vigorously stirred overnight at the same temperature. The reaction mixture was separated into an organic phase and an aqueous phase, and the organic phase was washed with saturated brine. The organic phase was dried over anhydrous sodium sulfate, the solvent was evaporated under reduced pressure, and the obtained residue was isolated and purified by silica gel column chromatography to give 21 g (yield: 95%) of ethyl 2,2-dichloro-1-[4-(1,1-dimethylethyl)phenyl]cyclopropanecarboxylate [II].

### Reference Example 2.

### Production method for Intermediate [III]

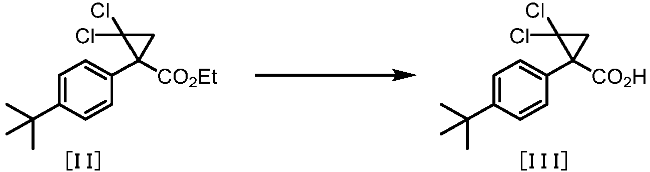

To a mixed solution of ethyl 2,2-dichloro-1-[4-(1,1-dimethylethyl)phenyl]cyclopropanecarboxylate [II] (21 g, 67 mmol), EtOH (135 mL), and water (13 mL) was added lithium hydroxide monohydrate (14 g, 0.33 mol), and the mixture was stirred at 60°C for 4 hr. After the completion of the reaction, the solvent was evaporated under reduced pressure, and the residue was acidified with 1M hydrochloric acid. Then, the mixture was subjected to extraction twice with ethyl acetate. The obtained organic phase was dried over sodium sulfate, and the solvent was evaporated under reduced pressure to give 20 g (yield: quant.) of 2,2-dichloro-1-[4-(1,1-dimethylethyl)phenyl]cyclopropanecarboxylic acid [III].

**[Table 88]**

| ·Table III-A: Carboxylic acids used for the production of the general formula [I-A] | | |
|---|---|---|
| No. | Structure | IUPAC name |
| III-A-1 | 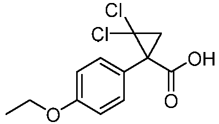 | 2,2-dichloro-1-(4-e thoxyphenyl)cyclopr opane-1-carboxylic acid |
| III-A-2 | 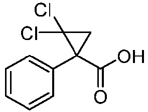 | 2,2-dichloro-1-phen ylcyclopropane-1-ca rboxylic acid |
| III-A-3 | 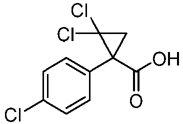 | 2,2-dichloro-1-(4-c hlorophenyl)cyclopr opane-1-carboxylic acid |
| III-A-4 | 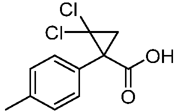 | 2,2-dichloro-1-(p-t olyl)cyclopropane-1 -carboxylic acid |
| III-A-5 | 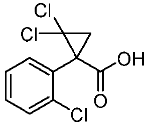 | 2,2-dichloro-1-(2-c hlorophenyl)cyclopr opane-1-carboxylic acid |
| III-A-6 | 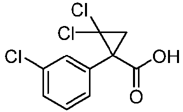 | 2,2-dichloro-1-(3-c hlorophenyl)cyclopr opane-1-carboxylic acid |
| III-A-7 | 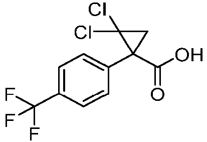 | 2,2-dichloro-1-(4-( trifluoromethyl)phe nyl)cyclopropane-1-carboxylic acid |

**[Table 89]**

| ·Table III-A: Carboxylic acids used for the production of the general formula [I-A] (continued) | | |
|---|---|---|
| No. | Structure | IUPAC name |
| III-A-8 | 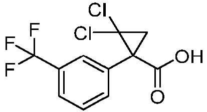 | 2,2-dichloro-1-(3-( trifluoromethyl)phe nyl)cyclopropane-1-carboxylic acid |
| III-A-9 | 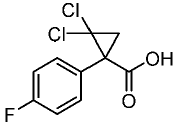 | 2,2-dichloro-1-(4-f luorophenyl)cyclopr opane-1-carboxylic acid |
| III-A-10 | 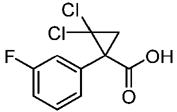 | 2,2-dichloro-1-(3-f luorophenyl)cyclopr opane-1-carboxylic acid |
| III-A-11 | 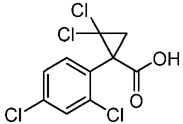 | 2,2-dichloro-1-(2,4 -dichlorophenyl)cyc lopropane-1-carboxy lic acid |
| III-A-12 | 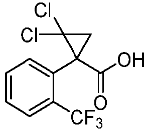 | 2,2-dichloro-1-(2-( trifluoromethyl)phe nyl)cyclopropane-1-carboxylic acid |
| III-A-13 | 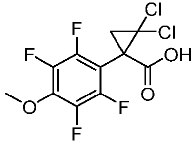 | 2,2-dichloro-1-(2,3 ,5,6-tetrafluoro-4-methoxyphenyl)cyclo propane-1-carboxyli c acid |
| III-A-14 | 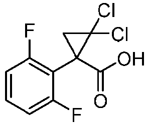 | 2,2-dichloro-1-(2,6 -difluorophenyl)cyc lopropane-1-carboxy lic acid |

**[Table 90]**

| ·Table III-A: Carboxylic acids used for the production of the general formula [I-A] (continued) | | |
|---|---|---|
| No. | Structure | IUPAC name |
| III-A-15 | 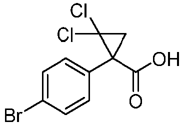 | 1-(4-bromophenyl)-2 ,2-dichlorocyclopro pane-1-carboxylic acid |
| III-A-16 | 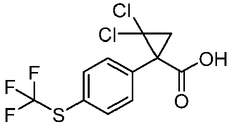 | 2,2-dichloro-1-(4-( (trifluoromethyl)th io)phenyl)cycloprop ane-1-carboxylic aci |
| III-A-17 | 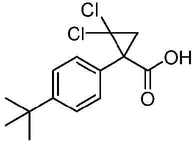 | 1-(4-(tert-butyl)ph enyl)-2,2-dichloroc yclopropane-1-carbo xylic acid |
| III-A-18 | 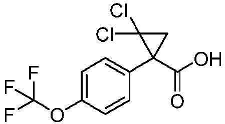 | 2,2-dichloro-1-(4-( trifluoromethoxy)ph enyl)cyclopropane-1 -carboxylic acid |
| III-A-19 | 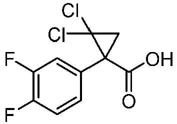 | 2,2-dichloro-1-(3,4 -difluorophenyl)cyc lopropane-1-carboxy lic acid |
| III-A-20 | 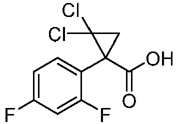 | 2,2-dichloro-1-(2,4 -difluorophenyl)cyc lopropane-1-carboxy lic acid |
| III-A-21 | 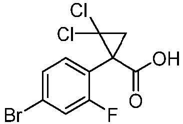 | 1-(4-bromo-2-fluoro phenyl)-2,2-dichlor ocyclopropane-1-car boxylic acid |

**[Table 91]**

| ·Table III-A: Carboxylic acids used for the production of the general formula [I-A] (continued) | | |
|---|---|---|
| No. | Structure | IUPAC name |
| III-A-22 | 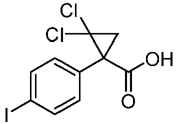 | 2,2-dichloro-1-(4-i odophenyl)cycloprop ane-1-carboxylic ac id |
| III-A-23 | 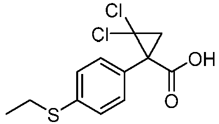 | 2,2-dichloro-1-(4-( ethylthio)phenyl)cy clopropane-1-carbox ylic acid |
| III-A-24 | 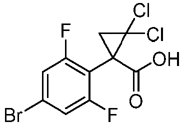 | 1-(4-bromo-2,6-difl uorophenyl)-2,2-dic hlorocyclopropane-1 -carboxylic acid |
| III-A-25 | 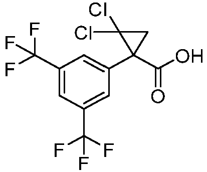 | 1-(3,5-bis(trifluor omethyl)phenyl)-2,2 -dichlorocyclopropa ne-1-carboxylic aci d |
| III-A-26 | 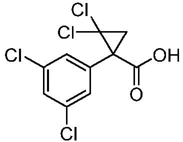 | 2,2-dichloro-1-(3,5 -dichlorophenyl)cyc lopropane-1-carboxy lic acid |
| III-A-27 | 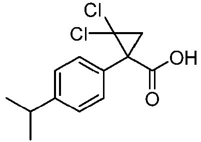 | 2,2-dichloro-1-(4-i sopropylphenyl)cycl opropane-1-carboxyl ic acid |
| III-A-28 | 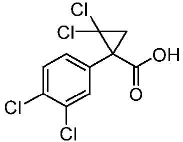 | 2,2-dichloro-1-(3,4 -dichlorophenyl)cyc lopropane-1-carboxy lic acid |

**[Table 92]**

| ·Table III-A: Carboxylic acids used for the production of the general formula [I-A] (continued) | | |
|---|---|---|
| No. | Structure | IUPAC name |
| III-A-29 | 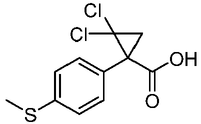 | 2,2-dichloro-1-(4-( methylthio)phenyl)c yclopropane-1-carbo xylic acid |
| III-A-30 | 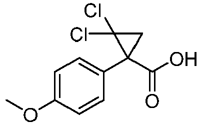 | 2,2-dichloro-1-(4-m ethoxyphenyl)cyclop ropane-1-carboxylic acid |
| III-A-31 | 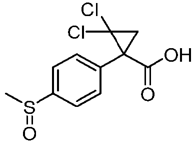 | 2,2-dichloro-1-(4-( methylsulfinyl)phen yl)cyclopropane-1-c arboxylic acid |
| III-A-32 | 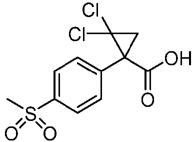 | 2,2-dichloro-1-(4-( methylsulfonyl)phen yl)cyclopropane-1-c arboxylic acid |
| III-A-33 | 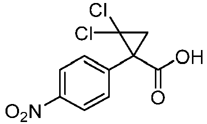 | 2,2-dichloro-1-(4-n itrophenyl)cyclopro pane-1-carboxylic a cid |
| III-A-34 | 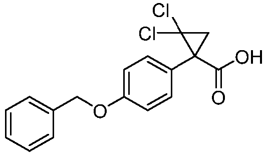 | 1-(4-(benzyloxy)phe nyl)-2,2-dichlorocy clopropane-1-carbox ylic acid |
| III-A-35 | 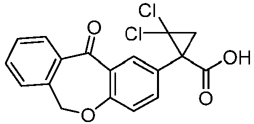 | 2,2-dichloro-1-(11-oxo-6,11-dihydrodib enzo[*b*,*e*]oxepin-2-y l)cyclopropane-1-ca rboxylic acid |

**[Table 93]**

| ·Table III-A: Carboxylic acids used for the production of the general formula [I-A] (continued) | | |
|---|---|---|
| No. | Structure | IUPAC name |
| III-A-36 | 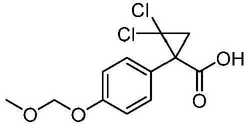 | 2,2-dichloro-1-(4-( methoxymethoxy)phen yl)cyclopropane-1-c arboxylic acid |
| III-A-37 | 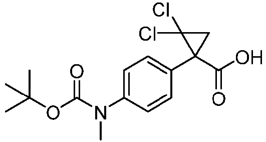 | 1-(4-((tert-butoxyc arbonyl) (methyl)ami no)phenyl)-2,2-dich lorocyclopropane-1-carboxylic acid |
| III-A-38 | 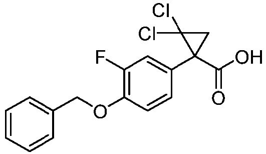 | 1-(4-(benzyloxy)-3-fluorophenyl)-2,2-d ichlorocyclopropane -1-carboxylic acid |
| III-A-39 | 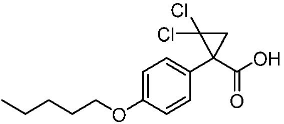 | 2,2-dichloro-1-(4-( pentyloxy)phenyl)cy clopropane-1-carbox ylic acid |
| III-A-40 | 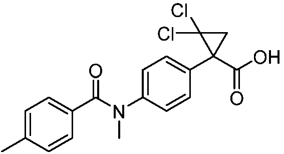 | 2,2-dichloro-1-(4-( N,4-dimethylbenzami do)phenyl)cycloprop ane-1-carboxylic ac id |
| III-A-41 | 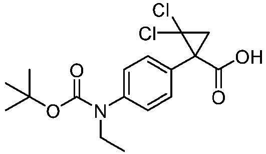 | 1-(4-((tert-butoxyc arbonyl) (ethyl)amin o)phenyl)-2,2-dichl orocyclopropane-1-c arboxylic acid |
| III-A-42 | 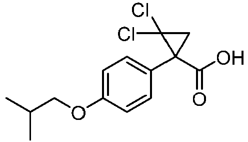 | 2,2-dichloro-1-(4-i sobutoxyphenyl)cycl opropane-1-carboxyl ic acid |

**[Table 94]**

| ·Table III-A: Carboxylic acids used for the production of the general formula [I-A] (continued) | | |
|---|---|---|
| No. | Structure | IUPAC name |
| III-A-43 | 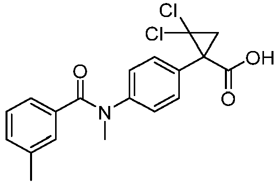 | 2,2-dichloro-1-(4-(N,3-dimethylbenza mido)phenyl)cyclop ropane-1-carboxyli c acid |
| III-A-44 | 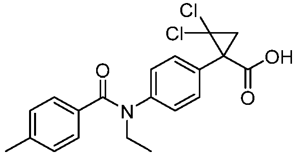 | 2,2-dichloro-1-(4-(*N*-ethyl-4-methylb enzamido)phenyl)cy clopropane-1-carbo xylic acid |
| III-A-45 | 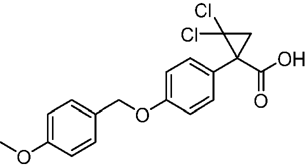 | 2,2-dichloro-1-(4-((4-methoxybenzyl) oxy)phenyl)cyclopr opane-1-carboxylic acid |
| III-A-46 | 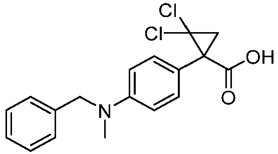 | 1-(4-(benzyl(methy l)amino)phenyl)-2, 2-dichlorocyclopro pane-1-carboxylic acid |
| III-A-47 | 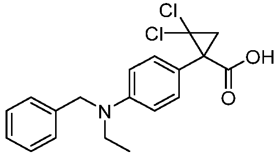 | 1-(4-(benzyl(ethyl )amino)phenyl)-2,2 -dichlorocycloprop ane-1-carboxylic a cid |
| III-A-48 | 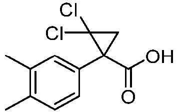 | 2,2-dichloro-1-(3, 4-dimethylphenyl)c yclopropane-1-carb oxylic acid |
| III-A-49 | 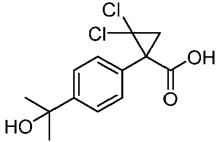 | 2,2-dichloro-1-(4-(2-hydroxypropan-2 -yl)phenyl)cyclopr opane-1-carboxylic acid |

**[Table 95]**

| ·Table III-A: Carboxylic acids used for the production of the general formula [I-A] (continued) | | |
|---|---|---|
| No. | Structure | IUPAC name |
| III-A-50 | 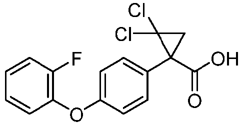 | 2,2-dichloro-1-(4-( 2-fluorophenoxy)phe nyl)cyclopropane-1-carboxylic acid |
| III-A-51 | 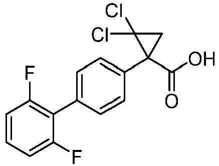 | 2,2-dichloro-1-(2', 6'-difluoro-[1,1'-b iphenyl]-4-yl)cyclo propane-1-carboxyli c acid |
| III-A-52 | 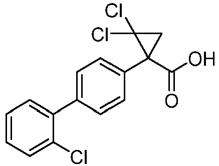 | 2,2-dichloro-1-(2'-chloro-[1,1'-biphen yl]-4-yl)cyclopropa ne-1-carboxylic aci d |
| III-A-53 | 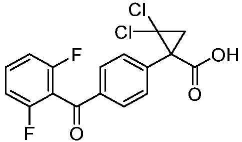 | 2,2-dichloro-1-(4-( 2,6-difluorobenzoyl )phenyl)cyclopropan e-1-carboxylic acid |
| III-A-54 | 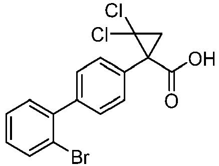 | 1-(2'-bromo-[1,1'-b iphenyl]-4-yl)-2,2-dichlorocyclopropan e-1-carboxylic acid |
| III-A-55 | 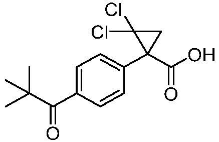 | 2,2-dichloro-1-(4-p ivaloylphenyl)cyclo propane-1-carboxyli c acid |
| III-A-56 | 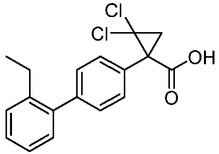 | 2,2-dichloro-1-(2'-ethyl-[1,1'-bipheny l]-4-yl)cyclopropan e-1-carboxylic acid |

**[Table 96]**

| ·Table III-A: Carboxylic acids used for the production of the general formula [I-A] (continued) | | |
|---|---|---|
| No. | Structure | IUPAC name |
| III-A-57 | 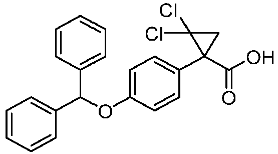 | 1-(4-(benzhydryloxy )phenyl)-2,2-dichlo rocyclopropane-1-ca rboxylic acid |
| III-A-58 | 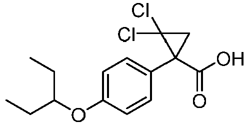 | 2,2-dichloro-1-(4-( pentan-3-yloxy)phen yl)cyclopropane-1-c arboxylic acid |
| III-A-59 | 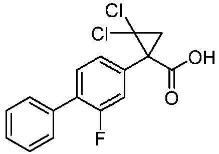 | 2,2-dichloro-1-(2-f luoro-[1,1'-bipheny l]-4-yl)cyclopropan e-1-carboxylic acid |
| III-A-60 | 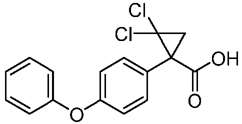 | 2,2-dichloro-1-(4-p henoxyphenyl)cyclop ropane-1-carboxylic acid |
| III-A-61 | 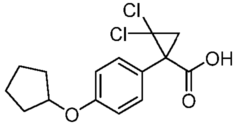 | 2,2-dichloro-1-(4-( cyclopentyloxy)phen yl)cyclopropane-1-c arboxylic acid |
| III-A-62 | 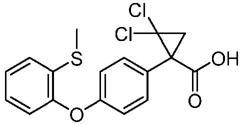 | 2,2-dichloro-1-(4-( 2-(methylthio)pheno xy)phenyl)cycloprop ane-1-carboxylic ac id |
| III-A-63 | 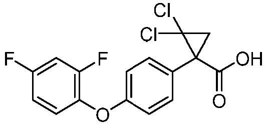 | 2,2-dichloro-1-(4-( 2,4-difluorophenoxy )phenyl)cyclopropan e-1-carboxylic acid |

**[Table 97]**

| ·Table III-A: Carboxylic acids used for the production of the general formula [I-A] (continued) | | |
|---|---|---|
| No. | Structure | IUPAC name |
| III-A-64 | 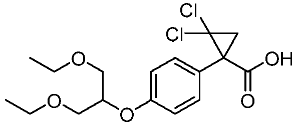 | 2,2-dichloro-1-(4-( (1,3-diethoxypropan -2-yl)oxy)phenyl)cy clopropane-1-carbox ylic acid |
| III-A-65 | 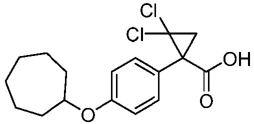 | 2,2-dichloro-1-(4-( cycloheptyloxy)phen yl)cyclopropane-1-c arboxylic acid |
| III-A-66 | 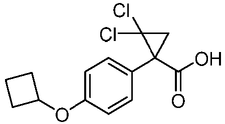 | 2,2-dichloro-1-(4-c yclobutoxyphenyl)cy clopropane-1-carbox ylic acid |
| III-A-67 | 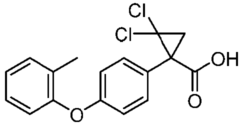 | 2,2-dichloro-1-(4-( o-tolyloxy)phenyl)c yclopropane-1-carbo xylic acid |
| III-A-68 | 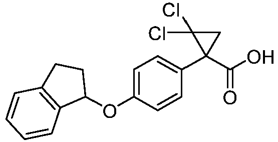 | 2,2-dichloro-1-(4-( (2,3-dihydro-1H-ind en-1-yl)oxy)phenyl) cyclopropane-1-carb oxylic acid |
| III-A-69 | 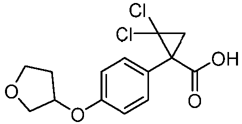 | 2,2-dichloro-1-(4-( (tetrahydrofuran-3-yl)oxy)phenyl)cyclo propane-1-carboxyli c acid |
| III-A-70 | 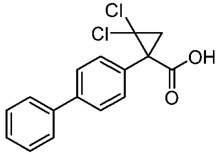 | 1-([1,1'-biphenyl]-4-yl)-2,2-dichloroc yclopropane-1-carbo xylic acid |

**[Table 98]**

| ·Table III-A: Carboxylic acids used for the production of the general formula [I-A] (continued) | | |
|---|---|---|
| No. | Structure | IUPAC name |
| III-A-71 | 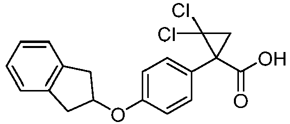 | 2,2-dichloro-1-(4-( (2,3-dihydro-1H-ind en-2-yl)oxy)phenyl) cyclopropane-1-carb oxylic acid |
| III-A-72 | 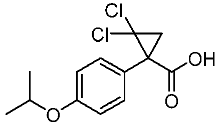 | 2,2-dichloro-1-(4-i sopropoxyphenyl)cyc lopropane-1-carboxy lic acid |
| III-A-73 | 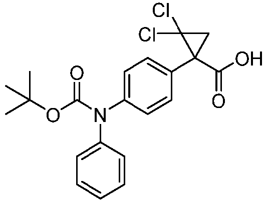 | 1-(4-((tert-butoxyc arbonyl) (phenyl)ami no)phenyl)-2,2-dich lorocyclopropane-1-carboxylic acid |
| III-A-74 | 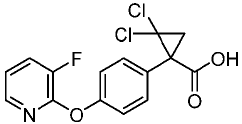 | 2,2-dichloro-1-(4-( (3-fluoropyridin-2-yl)oxy)phenyl)cyclo propane-1-carboxyli c acid |
| III-A-75 | 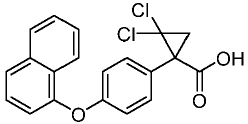 | 2,2-dichloro-1-(4-( naphthalen-1-yloxy) phenyl)cyclopropane -1-carboxylic acid |
| III-A-76 | 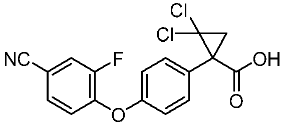 | 2,2-dichloro-1-(4-( 4-cyano-2-fluorophe noxy)phenyl)cyclopr opane-1-carboxylic acid |
| III-A-77 | 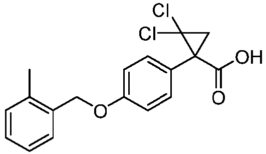 | 2,2-dichloro-1-(4-( (2-methylbenzyl)oxy )phenyl)cyclopropan e-1-carboxylic acid |

**[Table 99] ·**

| Table III-A: Carboxylic acids used for the production of the general formula [I-A] (continued) | | |
|---|---|---|
| No. | Structure | IUPAC name |
| III-A-78 | 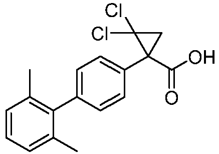 | 2,2-dichloro-1-(2',6'-dimethyl -[1,1'-biphenyl ]-4-yl)cyclopro pane-1-carboxyl ic acid |
| III-A-79 | 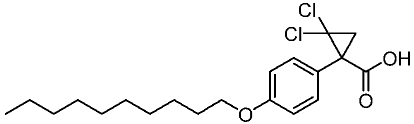 | 2,2-dichloro-1-(4- (decyloxy) ph enyl)cyclopropa ne-1-carboxylic acid |
| III-A-80 | 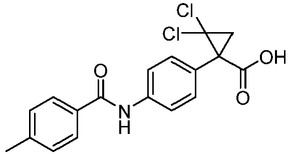 | 2,2-dichloro-1-(4-(4-methylben zamido)phenyl)c yclopropane-1-c arboxylic acid |
| III-A-81 | 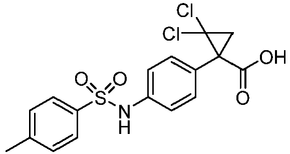 | 2,2-dichloro-1-(4-((4-methylph enyl)sulfonamid o)phenyl)cyclop ropane-1-carbox ylic acid |
| III-A-82 | 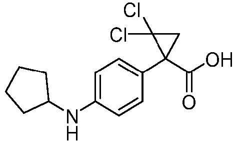 | 2,2-dichloro-1-(4-(cyclopentyl amino) phenyl) cy clopropane-1-ca rboxylic acid |
| III-A-83 | 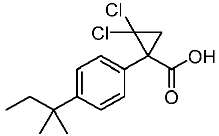 | 2,2-dichloro-1-(4-(*tert*-pentyl )phenyl)cyclopr opane-1-carboxy lic acid |
| III-A-84 | 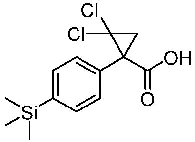 | 2,2-dichloro-1-(4-(trimethylsi lyl)phenyl)cycl opropane-1-carb oxylic acid |

**[Table 100]**

| ·Table III-A: Carboxylic acids used for the production of the general formula [I-A] (continued) | | |
|---|---|---|
| No. | Structure | IUPAC name |
| III-A-85 | 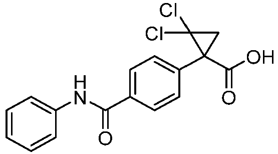 | 2,2-dichloro-1-(4-( phenylcarbamoyl)phe nyl)cyclopropane-1-carboxylic acid |
| III-A-86 | 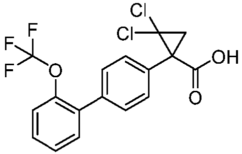 | 2,2-dichloro-1-(2'-(trifluoromethoxy)-[1,1'-biphenyl]-4-y l)cyclopropane-1-ca rboxylic acid |
| III-A-87 | 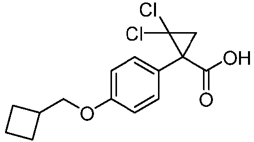 | 2,2-dichloro-1-(4-( cyclobutylmethoxy)p henyl)cyclopropane-1-carboxylic acid |
| III-A-88 | 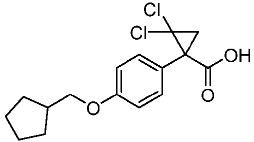 | 2,2-dichloro-1-(4-( cyclopentylmethoxy) phenyl)cyclopropane -1-carboxylic acid |
| III-A-89 | 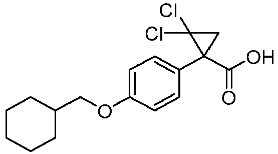 | 2,2-dichloro-1-(4-( cyclohexylmethoxy)p henyl)cyclopropane-1-carboxylic acid |
| III-A-90 | 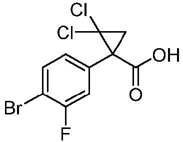 | 1-(4-bromo-3-fluoro phenyl)-2,2-dichlor ocyclopropane-1-car boxylic acid |
| III-A-91 | 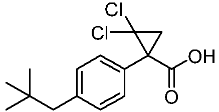 | 2,2-dichloro-1-(4-n eopentylphenyl)cycl opropane-1-carboxyl ic acid |

**[Table 101]**

| ·Table III-A: Carboxylic acids used for the production of the general formula [I-A] (continued) | | |
|---|---|---|
| No. | Structure | IUPAC name |
| III-A-92 | 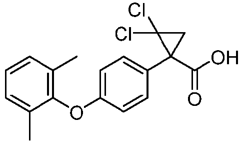 | 2,2-dichloro-1-(4-( 2,6-dimethylphenoxy )phenyl)cyclopropan e-1-carboxylic acid |
| III-A-93 | 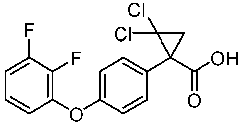 | 2,2-dichloro-1-(4-( 2,3-difluorophenoxy )phenyl)cyclopropan e-1-carboxylic acid |
| III-A-94 | 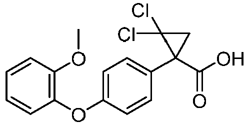 | 2,2-dichloro-1-(4-( 2-methoxyphenoxy)ph enyl)cyclopropane-1 -carboxylic acid |
| III-A-95 | 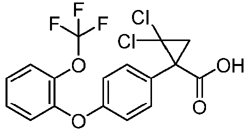 | 2,2-dichloro-1-(4-( 2-(trifluoromethoxy )phenoxy)phenyl)cyc lopropane-1-carboxy lic acid |
| III-A-96 | 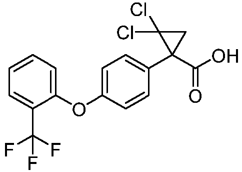 | 2,2-dichloro-1-(4-( 2-(trifluoromethyl) phenoxy)phenyl)cycl opropane-1-carboxyl ic acid |
| III-A-97 | 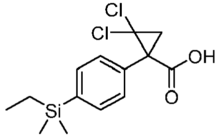 | 2,2-dichloro-1-(4-( ethyldimethylsilyl) phenyl)cyclopropane -1-carboxylic acid |
| III-A-98 | 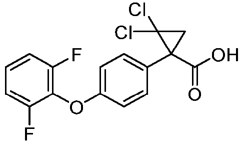 | 2,2-dichloro-1-(4-( 2,6-difluorophenoxy )phenyl)cyclopropan e-1-carboxylic acid |

**[Table 102]**

| ·Table III-A: Carboxylic acids used for the production of the general formula [I-A] (continued) | | |
|---|---|---|
| No. | Structure | IUPAC name |
| III-A-99 | 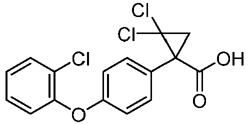 | 2,2-dichloro-1-(4-( 2-chlorophenoxy)phe nyl)cyclopropane-1-carboxylic acid |
| III-A-100 | 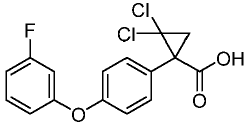 | 2,2-dichloro-1-(4-( 3-fluorophenoxy)phe nyl)cyclopropane-1-carboxylic acid |
| III-A-101 | 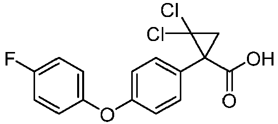 | 2,2-dichloro-1-(4-( 4-fluorophenoxy)phe nyl)cyclopropane-1-carboxylic acid |
| III-A-102 | 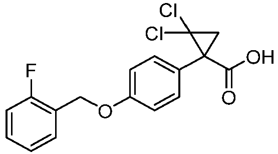 | 2,2-dichloro-1-(4-( (2-fluorobenzyl)oxy )phenyl)cyclopropan e-1-carboxylic acid |
| III-A-103 | 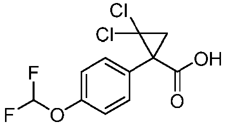 | 2,2-dichloro-1-(4-( difluoromethoxy)phe nyl)cyclopropane-1-carboxylic acid |
| III-A-104 | 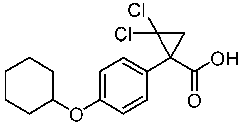 | 2,2-dichloro-1-(4-( cyclohexyloxy)pheny l)cyclopropane-1-ca rboxylic acid |
| III-A-105 | 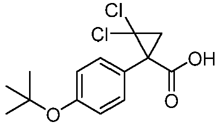 | 1-(4-(*tert*-butoxy)p henyl)-2,2-dichloro cyclopropane-1-carb oxylic acid |

**[Table 103]**

| ·Table III-A: Carboxylic acids used for the production of the general formula [I-A] (continued) | | |
|---|---|---|
| No. | Structure | IUPAC name |
| III-A-106 | 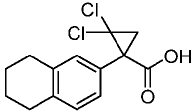 | 2,2-dichloro-1-(5 ,6,7,8-tetrahydro naphthalen-2-yl)c yclopropane-1-car boxylic acid |
| III-A-107 | 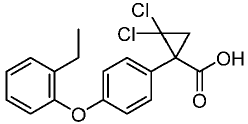 | 2,2-dichloro-1-(4 -(2-ethylphenoxy) phenyl)cyclopropa ne-1-carboxylic a cid |
| III-A-108 | 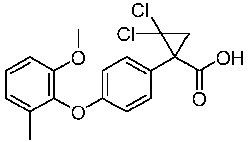 | 2,2-dichloro-1-(4 -(2-methoxy-6-met hylphenoxy)phenyl )cyclopropane-1-c arboxylic acid |
| III-A-109 | 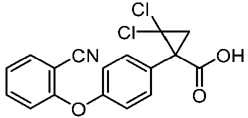 | 2,2-dichloro-1-(4 -(2-cyanophenoxy) phenyl)cyclopropa ne-1-carboxylic acid |
| III-A-110 | 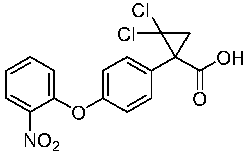 | 2,2-dichloro-1-(4 -(2-nitrophenoxy) phenyl)cyclopropa ne-1-carboxylic acid |
| III-A-111 | 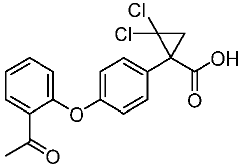 | 1-(4-(2-acetylphe noxy)phenyl)-2,2-dichlorocycloprop ane-1-carboxylic acid |
| III-A-112 | 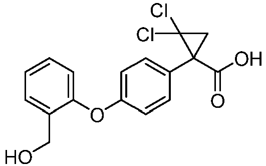 | 2,2-dichloro-1-(4 -(2-(hydroxymethy l)phenoxy)phenyl) cyclopropane-1-ca rboxylic acid |
| III-A-113 | 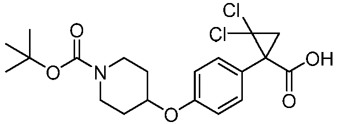 | 1-(4-((1-(tert-bu toxycarbonyl)pipe ridin-4-yl)oxy)ph enyl)-2,2-dichlor ocyclopropane-1-carboxylic acid |

**[Table 104]**

| ·Table III-A: Carboxylic acids used for the production of the general formula [I-A] (continued) | | |
|---|---|---|
| No. | Structure | IUPAC name |
| III-A-114 | 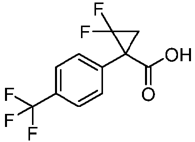 | 2,2-difluoro-1-(4-( trifluoromethyl)phe nyl)cyclopropane-1-carboxylic acid |
| III-A-115 | 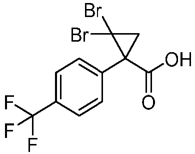 | 2,2-dibromo-1-(4-( trifluoromethyl)phen yl)cyclopropane-1-carboxylic acid |
| III-A-116 | 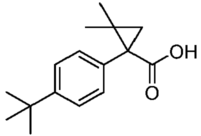 | 1-(4-(tert-butyl)ph enyl)-2,2-dimethylc yclopropane-1-carbo xylic acid |
| III-A-117 | 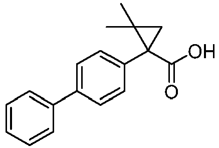 | 1-([1,1'-biphenyl]-4-yl)-2,2-dimethylc yclopropane-1-carbo xylic acid |
| III-A-118 | 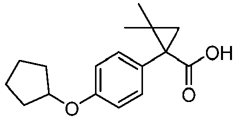 | 1-(4-(cyclopentylox y)phenyl)-2,2-dimet hylcyclopropane-1-carboxylic acid |
| III-A-119 | 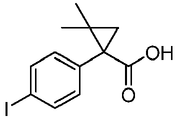 | 1-(4-iodophenyl)-2, 2-dimethylcycloprop ane-1-carboxylic ac id |
| III-A-120 | 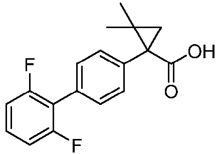 | 1-(2',6'-difluoro-[ 1,1'-biphenyl]-4-yl )-2,2-dimethylcyclo propane-1-carboxyli c acid |
| III-A-121 | 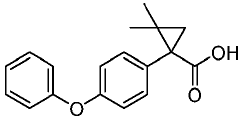 | 2,2-dimethyl-1-(4-p henoxyphenyl)cyclop ropane-1-carboxylic acid |

**[Table 105]**

| ·Table III-A: Carboxylic acids used for the production of the general formula [I-A] (continued) | | |
|---|---|---|
| No. | Structure | IUPAC name |
| III-A-122 | 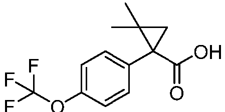 | 2,2-dimethyl-1-(4-( trifluoromethoxy)ph enyl)cyclopropane-1 -carboxylic acid |
| III-A-123 | 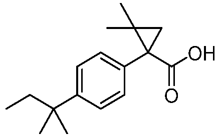 | 2,2-dimethyl-1-(4-( *tert-*pentyl)phenyl) cyclopropane-1-carb oxylic acid |

**[Table 106]**

| ·Table III-B: Carboxylic acids used for the production of the general formula [I-B] | | |
|---|---|---|
| No. | Structure | IUPAC name |
| III-B-1 | 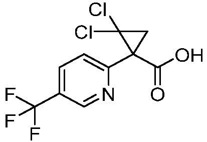 | 2,2-dichloro-1-(5-( trifluoromethyl)pyr idin-2-yl)cycloprop ane-1-carboxylic ac id |

**[Table 107]**

| ·Table III-C: Carboxylic acids used for the production of the general formula [I-C] | | |
|---|---|---|
| No. | Structure | IUPAC name |
| III-C-1 | 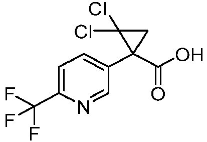 | 2,2-dichloro-1-(6-( trifluoromethyl)pyr idin-3-yl)cycloprop ane-1-carboxylic ac id |
| III-C-2 | 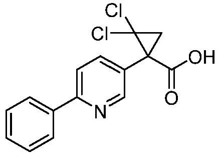 | 2,2-dichloro-1-(6-p henylpyridin-3-yl)c yclopropane-1-carbo xylic acid |
| III-C-3 | 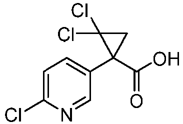 | 2,2-dichloro-1-(6-c hloropyridin-3-yl)c yclopropane-1-carbo xylic acid |
| III-C-4 | 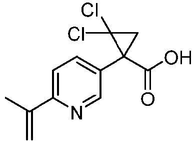 | 2,2-dichloro-1-(6-( prop-1-en-2-yl)pyri din-3-yl)cyclopropa ne-1-carboxylic aci d |
| III-C-5 | 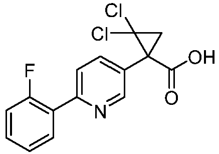 | 2,2-dichloro-1-(6-( 2-fluorophenyl)pyri din-3-yl)cyclopropa ne-1-carboxylic aci d |
| III-C-6 | 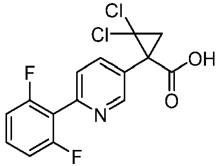 | 2,2-dichloro-1-(6-( 2,6-difluorophenyl) pyridin-3-yl)cyclop ropane-1-carboxylic acid |
| III-C-7 | 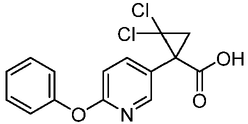 | 2,2-dichloro-1-(6-p henoxypyridin-3-yl) cyclopropane-1-carb oxylic acid |

**[Table 108]**

| ·Table III-L Carboxylic acids used for the production of the general formula [I-L] | | |
|---|---|---|
| No. | Structure | IUPAC name |
| III-L-1 | 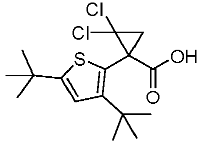 | 2,2-dichloro-1-(3,5 -di-*tert*-butylthiop hen-2-yl)cyclopropa ne-1-carboxylic aci d |
| III-L-2 | 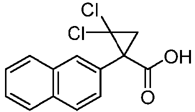 | 2,2-dichloro-1-(nap hthalen-2-yl)cyclop ropane-1-carboxylic acid |
| III-L-3 | 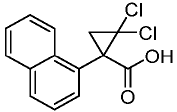 | 2,2-dichloro-1-(nap hthalen-1-yl)cyclop ropane-1-carboxylic acid |

Hereinafter, formulation examples are shown, but the present invention is not limited thereto. In the formulation examples, "part(s)" means part(s) by weight.

### Formulation Example 1.

| | |
|---|---|
| Compound [I] of the present invention | 10 parts |
| Xylene | 70 parts |
| N-methyl pyrrolidone | 10 parts |
| Mixture of polyoxyethylene nonylphenyl ether and calcium alkylbenzene sulfonate | 10 parts |

The above ingredients are uniformly mixed for dissolution to give an emulsifiable concentrate formulation.

### Formulation Example 2.

| | |
|---|---|
| Compound [I] of the present invention | 3 parts |
| Clay powder | 82 parts |
| Diatomite powder | 15 parts |

The above ingredients are uniformly mixed and then pulverized to give a dust formulation.

### Formulation Example 3.

| | |
|---|---|
| Compound [I] of the present invention | 5 parts |
| Mixture of bentonite powder and clay powder | 90 parts |
| Calcium lignosulfonate | 5 parts |

The above ingredients are uniformly mixed. After addition of an appropriate volume of water, the mixture is kneaded, granulated and dried to give a granule formulation.

### Formulation Example 4.

| | |
|---|---|
| Compound [I] of the present invention | 20 parts |
| Kaolinite and synthetic high-dispersion silicic acid | 75 parts |
| Mixture of polyoxyethylene nonylphenyl ether and calcium alkylbenzene sulfonate | 5 parts |

The above ingredients are uniformly mixed and then pulverized to give a wettable powder formulation.

### Formulation Example 5.

| | |
|---|---|
| Compound [I] of the present invention | 10 parts |
| Mixture of nonionic and anionic surfactants(Sorpol 3105 (manufactured by Toho Chemical Industry Co.,Ltd.)) | 5 parts |
| Propylene glycol | 2 parts |
| Xanthan gum | 1 part |
| Water | 82 parts |

Preparation procedure: The above ingredients other than water are uniformly mixed, and the mixture is dispersed in water to give a flowable formulation.

Hereinafter, test examples in connection with the present invention are shown, but the present invention is not limited thereto.

### Test Example 1.

### Test for control efficacy on Chinese cabbage alternaria leaf spot

A formulation prepared from the compound of the present invention according to Formulation Example 1 was diluted with water to a predetermined concentration. 10 ml of the diluted formulation was applied to the foliage of Chinese cabbage plants (variety: Musou) at the 3-5 leaf stage grown in a pot 5 cm in diameter. After the formulation was air-dried, the Chinese cabbage plants were inoculated by spraying a spore suspension of Chinese cabbage alternaria leaf spot fungus. The Chinese cabbage plants were kept at 20°C under humid condition for one day, and then transferred to a greenhouse to allow to develop the disease. At 5 days after the inoculation, the control efficacy was evaluated according to the criteria shown below. Control rate % = 100 × (Average percent lesion area in a non-treatment plot - Average percent lesion area in a treatment plot) / Average percent lesion area in a non-treatment plot

### Criteria

0: the control rate is 9% or less.
1: the control rate is 10 to 19%.
2: the control rate is 20 to 29%.
3: the control rate is 30 to 39%.
4: the control rate is 40 to 49%.
5: the control rate is 50 to 59%.
6: the control rate is 60 to 69%.
7: the control rate is 70 to 79%.
8: the control rate is 80 to 89%.
9: the control rate is 90 to 99%.
10: the control rate is 100%.

The results of the test revealed that the compounds I-A-3, I-A-4, I-A-5, I-A-25, I-A-36, I-A-38, I-A-39, I-A-43, I-A-52, I-A-54, I-A-56, I-A-58, I-A-68, I-A-81, I-A-94, I-A-98, I-A-99, I-A-100, I-A-101, I-A-102, I-A-103, I-A-106, I-A-114, I-A-123, I-A-132, I-A-135, I-A-160, I-A-162, I-A-168, I-A-175, I-A-186, I-A-198, I-A-200, I-A-206, I-A-214, I-A-218, I-A-219, I-A-238, I-A-239, I-A-250, I-A-251, I-A-252, I-A-253, I-A-254, I-A-256, I-A-257, I-A-258, I-A-260, I-A-261, I-A-262, I-A-263, I-A-264, I-A-265, I-A-266, I-A-267, I-A-268, I-A-269, I-A-271, I-A-272, I-A-273, I-A-274, I-A-275, I-A-278, I-A-279, I-A-281, I-A-282, I-A-284, I-A-285, I-A-286, I-A-287, I-A-288, I-A-289, I-A-290, I-A-291, I-A-293, I-A-295, I-A-297, I-A-298, I-A-299, I-A-300, I-A-302, I-A-348, I-A-349, I-A-351, I-A-352, I-A-353, I-A-355, I-A-357, I-A-360, I-A-364, I-A-365, I-A-383, I-A-385, I-A-386, I-A-387, I-A-388, I-A-390, I-A-392, I-A-393, I-A-394, I-A-398, I-A-400, I-A-408, I-A-410, I-A-411, I-A-412, I-A-414, I-A-416, I-A-422, I-A-423, I-A-425, I-A-426, I-A-427, I-A-428, I-A-429, I-A-468, I-A-474, I-A-476, I-A-483, I-A-488, I-A-489, I-A-494, I-A-529, I-A-535, I-A-543, I-A-544, I-A-550, I-A-558, I-A-559, I-A-560, I-A-562, I-A-563, I-A-568, I-A-572, I-A-573, I-A-574, I-A-575,I-A-576, I-A-577, I-A-578, I-A-579, I-A-580, I-A-581, I-A-582, I-A-583, I-A-584, I-A-585I-A-586, I-A-588, I-A-591, I-A-592, I-A-593, I-A-596, I-A-598, I-A-601, I-A-608, I-A-611, I-A-612, I-A-614, I-A-618, I-A-619, I-A-620, I-A-621, I-A-622, I-A-626, I-A-627, I-A-629, I-A-630, I-A-635, I-A-636, I-A-637, I-A-638, I-A-639, I-A-643, I-B-2, I-B-17, I-C-15, I-C-16, I-C-17, I-C-18, I-C-19, I-C-23, I-C-24, I-C-25, I-C-26, I-C-28, I-K-9, I-K-13, I-K-14, I-K-15, I-K-16, I-K-17, I-K-18, I-K-19, I-K-20, I-K-21, I-K-23, I-K-24, I-K-25 and I-K-26 at a concentration of 200 ppm showed the activity of level 1 or higher.

### Test Example 2.

### Test for control efficacy on tomato late blight

A formulation prepared from the compound of the present invention according to Formulation Example 1 was diluted with water to a predetermined concentration. 10 ml of the diluted formulation was applied to the foliage of tomato plants (variety: Momotaro) at the 3-4 leaf stage grown in a pot 9 cm in diameter. After the formulation was air-dried, the tomato plants were inoculated by spraying a zoosporangium suspension of tomato late blight fungus. The tomato plants were kept at 20°C under humid condition for one day, and then transferred to a greenhouse to allow to develop the disease. At 5 or 6 days after the inoculation, the control efficacy was evaluated according to the criteria of Test Example 1.

The results of the test revealed that the compound I-A-17 at a concentration of 200 ppm showed the activity of level 1 or higher.

### Test Example 3.

### Test for control efficacy on cucumber gray mold

A formulation prepared from the compound of the present invention according to Formulation Example 1 was diluted with water to a predetermined concentration. 10 ml of the diluted formulation was applied to the foliage of cucumber plants (variety: YOTUBA) at the one-leaf stage grown in a pot 9 cm in diameter. After the formulation was air-dried, the cucumber plants were inoculated by moistening paper disks placed in the center of the leaves with a spore suspension of cucumber gray mold fungus. The cucumber plants were kept at 15°C under constant humidity condition to allow to develop the disease. At 5 or 6 days after the inoculation, the control efficacy was evaluated according to the criteria of Test Example 1.

The results of the test revealed that the compounds I-A-1, I-A-3, I-A-4, I-A-5, I-A-13, I-A-17, I-A-25, I-A-35, I-A-36, I-A-38, I-A-39, I-A-40, I-A-49, I-A-52, I-A-54, I-A-55, I-A-56, I-A-58, I-A-59, I-A-62, I-A-63, I-A-65, I-A-68, I-A-71, I-A-75, I-A-76, I-A-77, I-A-78, I-A-79, I-A-81, I-A-89, I-A-94, I-A-99, I-A-100, I-A-101, I-A-102, I-A-113, I-A-114, I-A-123, I-A-124, I-A-129, I-A-132, I-A-133, I-A-147, I-A-150, I-A-152, I-A-155, I-A-157, I-A-159, I-A-162, I-A-163, I-A-165, I-A-167, I-A-169, I-A-171, I-A-172, I-A-173, I-A-180, I-A-186, I-A-188, I-A-196, I-A-197, I-A-198, I-A-199, I-A-200, I-A-206, I-A-214, I-A-217, I-A-218, I-A-220, I-A-222, I-A-226, I-A-238, I-A-250, I-A-251, I-A-252, I-A-253, I-A-254, I-A-255, I-A-256, I-A-257, I-A-258, I-A-259, I-A-260, I-A-261, I-A-264, I-A-265, I-A-266, I-A-267, I-A-268, I-A-269, I-A-270, I-A-271, I-A-272, I-A-273, I-A-274, I-A-275, I-A-276, I-A-278, I-A-279, I-A-280, I-A-281, I-A-282, I-A-283, I-A-284, I-A-285, I-A-286, I-A-287, I-A-288, I-A-289, I-A-290, I-A-291, I-A-292, I-A-293, I-A-294, I-A-295, I-A-296, I-A-297, I-A-298, I-A-299, I-A-300, I-A-301, I-A-302, I-A-303, I-A-348, I-A-349, I-A-351, I-A-352, I-A-355, I-A-356, I-A-357, I-A-360, I-A-361, I-A-362, I-A-364, I-A-365, I-A-366, I-A-367, I-A-369, I-A-370, I-A-376, I-A-378, I-A-379, I-A-381, I-A-383, I-A-385, I-A-386, I-A-387, I-A-388, I-A-389, I-A-390, I-A-391, I-A-392, I-A-393, I-A-398, I-A-399, I-A-400, I-A-401, I-A-403, I-A-404, I-A-405, I-A-406, I-A-407, I-A-408, I-A-409, I-A-410, I-A-411, I-A-412, I-A-413, I-A-414, I-A-415, I-A-416, I-A-417, I-A-419, I-A-420, I-A-421, I-A-422, I-A-423, I-A-424, I-A-425, I-A-426, I-A-427, I-A-428, I-A-429, I-A-430, I-A-431, I-A-432, I-A-434, I-A-435, I-A-436, I-A-437, I-A-438, I-A-441, I-A-442, I-A-443, I-A-444, I-A-445, I-A-447, I-A-448, I-A-449, I-A-450, I-A-451, I-A-452, I-A-453, I-A-454, I-A-456, I-A-457, I-A-458, I-A-459, I-A-460, I-A-461, I-A-462, I-A-463, I-A-464, I-A-465, I-A-466, I-A-467, I-A-468, I-A-469, I-A-470, I-A-471, I-A-472, I-A-474, I-A-475, I-A-476, I-A-477, I-A-478, I-A-479, I-A-480, I-A-481, I-A-483, I-A-485, I-A-486, I-A-487, I-A-488, I-A-489, I-A-492, I-A-493, I-A-494, I-A-495, I-A-496, I-A-497, I-A-498, I-A-499, I-A-500, I-A-502, I-A-503, I-A-504, I-A-506, I-A-507, I-A-509, I-A-510, I-A-511, I-A-512, I-A-513, I-A-514, I-A-516, I-A-518, I-A-519, I-A-520, I-A-521, I-A-522, I-A-524, I-A-525, I-A-526, I-A-527, I-A-528, I-A-529, I-A-530, I-A-531, I-A-532, I-A-534, I-A-535, I-A-536, I-A-537, I-A-538, I-A-539, I-A-540, I-A-541, I-A-543, I-A-544, I-A-550, I-A-552, I-A-555, I-A-558, I-A-559, I-A-560, I-A-561, I-A-562, I-A-563, I-A-564, I-A-565, I-A-566, I-A-567, I-A-568, I-A-569, I-A-571, I-A-572, I-A-573, I-A-574, I-A-575, I-A-576, I-A-577, I-A-578, I-A-579, I-A-580, I-A-581, I-A-582, I-A-583, I-A-584, I-A-585, I-A-586, I-A-587, I-A-588, I-A-589, I-A-590, I-A-591, I-A-592, I-A-593, I-A-594, I-A-595, I-A-596, I-A-601, I-A-602, I-A-603, I-A-604, I-A-605, I-A-606, I-A-607, I-A-608, I-A-609, I-A-610, I-A-611, I-A-612, I-A-613, I-A-614, I-A-615, I-A-617, I-A-618, I-A-619, I-A-620, I-A-621, I-A-622, I-A-623, I-A-624, I-A-625, I-A-626, I-A-627, I-A-628, I-A-629, I-A-630, I-A-631, I-A-633, I-A-634, I-A-635, I-A-636, I-A-637, I-A-638, I-A-640, I-A-641, I-A-642, I-A-643, I-A-645, I-A-646, I-A-647, I-B-2, I-B-16, I-B-17, I-C-2, I-C-13, I-C-14, I-C-15, I-C-16, I-C-17, I-C-18, I-C-19, I-C-20, I-C-21, I-C-23, I-C-24, I-C-25, I-C-26, I-C-28, I-C-29, I-C-30, I-C-31, I-K-7, I-K-8, I-K-9, I-K-10, I-K-13, I-K-15, I-K-16, I-K-19, I-K-20, I-K-21, I-K-22, I-K-25, I-K-28 and I-L-3 at a concentration of 200 ppm showed the activity of level 1 or higher.

### Test Example 4.

### Test for control efficacy on cucumber anthracnose

A formulation prepared from the compound of the present invention according to Formulation Example 1 was diluted with water to a predetermined concentration. 10 ml of the diluted formulation was applied to the foliage of cucumber plants (variety: YOTUBA) at the two-leaf stage grown in a pot 9 cm in diameter. After the formulation was air-dried, the cucumber plants were inoculated by spraying a spore suspension of cucumber anthracnose fungus. The cucumber plants were kept at 20°C under humid condition for one day, and then transferred to a greenhouse to allow to develop the disease. At 6 days after the inoculation, the control efficacy was evaluated according to the criteria of Test Example 1.

The results of the test revealed that the compounds I-A-1, I-A-2, I-A-3, I-A-4, I-A-5, I-A-7, I-A-10, I-A-16, I-A-17, I-A-25, I-A-36, I-A-37, I-A-38, I-A-39, I-A-41, I-A-43, I-A-49, I-A-52, I-A-54, I-A-55, I-A-56, I-A-57, I-A-58, I-A-59, I-A-62, I-A-63, I-A-64, I-A-66, I-A-68, I-A-70, I-A-71, I-A-75, I-A-76, I-A-77, I-A-78, I-A-80, I-A-81, I-A-89, I-A-94, I-A-98, I-A-99, I-A-100, I-A-101, I-A-102, I-A-103, I-A-106, I-A-113, I-A-114, I-A-123, I-A-127, I-A-130, I-A-132, I-A-133, I-A-135, I-A-139, I-A-147, I-A-148, I-A-150, I-A-152, I-A-154, I-A-155, I-A-157, I-A-159, I-A-160, I-A-162, I-A-164, I-A-165, I-A-167, I-A-168, I-A-169, I-A-171, I-A-172, I-A-173, I-A-175, I-A-178, I-A-180, I-A-186, I-A-188, I-A-190, I-A-192, I-A-194, I-A-195, I-A-196, I-A-197, I-A-198, I-A-199, I-A-200, I-A-206, I-A-214, I-A-218, I-A-219, I-A-220, I-A-226, I-A-238, I-A-239, I-A-250, I-A-251, I-A-252, I-A-253, I-A-254, I-A-255, I-A-256, I-A-257, I-A-258, I-A-259, I-A-260, I-A-261, I-A-262, I-A-263, I-A-264, I-A-265, I-A-266, I-A-267, I-A-268, I-A-269, I-A-270, I-A-271, I-A-272, I-A-273, I-A-274, I-A-275, I-A-276, I-A-277, I-A-278, I-A-279, I-A-281, I-A-282, I-A-284, I-A-285, I-A-286, I-A-287, I-A-288, I-A-289, I-A-290, I-A-291, I-A-293, I-A-295, I-A-296, I-A-297, I-A-298, I-A-299, I-A-300, I-A-301, I-A-302, I-A-303, I-A-346, I-A-348, I-A-349, I-A-350, I-A-351, I-A-353, I-A-354, I-A-355, I-A-357, I-A-359, I-A-360, I-A-361, I-A-362, I-A-363, I-A-364, I-A-365, I-A-366, I-A-368, I-A-369, I-A-370, I-A-372, I-A-375, I-A-378, I-A-379, I-A-381, I-A-383, I-A-384, I-A-385, I-A-386, I-A-387, I-A-388, I-A-389, I-A-390, I-A-391, I-A-392, I-A-393, I-A-394, I-A-398, I-A-399, I-A-400, I-A-401, I-A-403, I-A-404, I-A-405, I-A-406, I-A-407, I-A-408, I-A-409, I-A-410, I-A-411, I-A-412, I-A-413, I-A-414, I-A-415, I-A-416, I-A-417, I-A-418, I-A-419, I-A-420, I-A-421, I-A-422, I-A-423, I-A-424, I-A-425, I-A-426, I-A-427, I-A-428, I-A-429, I-A-430, I-A-431, I-A-432, I-A-433, I-A-434, I-A-435, I-A-436, I-A-437, I-A-438, I-A-439, I-A-440, I-A-441, I-A-442, I-A-443, I-A-444, I-A-445, I-A-446, I-A-447, I-A-448, I-A-449, I-A-450, I-A-451, I-A-452, I-A-453, I-A-454, I-A-455, I-A-456, I-A-457, I-A-458, I-A-459, I-A-460, I-A-461, I-A-462, I-A-463, I-A-464, I-A-465, I-A-466, I-A-467, I-A-469, I-A-470, I-A-471, I-A-473, I-A-474, I-A-475, I-A-476, I-A-477, I-A-478, I-A-479, I-A-480, I-A-481, I-A-482, I-A-483, I-A-484, I-A-485, I-A-486, I-A-487, I-A-488, I-A-489, I-A-490, I-A-491, I-A-492, I-A-493, I-A-494, I-A-495, I-A-496, I-A-497, I-A-498, I-A-499, I-A-500, I-A-501, I-A-502, I-A-503, I-A-504, I-A-505, I-A-506, I-A-507, I-A-508, I-A-509, I-A-510, I-A-511, I-A-512, I-A-513, I-A-514, I-A-515, I-A-516, I-A-517, I-A-518, I-A-519, I-A-520, I-A-521, I-A-522, I-A-524, I-A-525, I-A-526, I-A-527, I-A-528, I-A-529, I-A-530, I-A-531, I-A-532, I-A-533, I-A-534, I-A-535, I-A-536, I-A-537, I-A-538, I-A-539, I-A-540, I-A-541, I-A-542, I-A-543, I-A-544, I-A-547, I-A-548, I-A-550, I-A-551, I-A-552, I-A-553, I-A-554, I-A-555, I-A-556, I-A-557, I-A-558, I-A-559, I-A-560, I-A-561, I-A-562, I-A-563, I-A-564, I-A-565, I-A-566, I-A-567, I-A-568, I-A-570, I-A-571, I-A-572, I-A-573, I-A-574, I-A-575, I-A-576, I-A-577, I-A-578, I-A-579, I-A-580, I-A-581, I-A-582, I-A-583, I-A-584, I-A-585, I-A-586, I-A-587, I-A-588, I-A-589, I-A-590, I-A-591, I-A-592, I-A-593, I-A-594, I-A-595, I-A-596, I-A-597, I-A-598, I-A-599, I-A-600, I-A-601, I-A-602, I-A-603, I-A-604, I-A-605, I-A-606, I-A-607, I-A-608, I-A-609, I-A-610, I-A-611, I-A-612, I-A-613, I-A-614, I-A-615, I-A-616, I-A-617, I-A-618, I-A-619, I-A-620, I-A-621, I-A-622, I-A-623, I-A-624, I-A-625, I-A-626, I-A-627, I-A-628, I-A-629, I-A-630, I-A-631, I-A-632, I-A-633, I-A-634, I-A-635, I-A-636, I-A-637, I-A-638, I-A-639, I-A-640, I-A-641, I-A-642, I-A-643, I-A-644, I-A-645, I-A-646, I-A-647, I-B-2, I-B-16, I-B-17, I-B-18, I-C-2, I-C-5, I-C-13, I-C-14, I-C-15, I-C-16, I-C-17, I-C-18, I-C-19, I-C-20, I-C-21, I-C-22, I-C-23, I-C-24, I-C-25, I-C-26, I-C-27, I-C-28, I-C-29, I-C-30, I-C-31, I-K-8, I-K-9, I-K-10, I-K-13, I-K-17, I-K-21, I-K-22, I-K-25, I-K-27, I-K-28 and I-L-3 at a concentration of 200 ppm showed the activity of level 1 or higher.

### Test Example 5.

### Test for control efficacy on rice blast

A formulation prepared from the compound of the present invention according to Formulation Example 1 was diluted with water to a predetermined concentration. 10 ml of the diluted formulation was applied to the foliage of rice plants (variety: Kinmaze) at the 6-7 leaf stage grown in a pot 6 cm in diameter. After the formulation was air-dried, the rice plants were inoculated by spraying a spore suspension of rice blast fungus. The rice plants were kept at 20°C under humid condition for one day, and then transferred to a greenhouse to allow to develop the disease. At 6 days after the inoculation, the control efficacy was evaluated according to the criteria of Test Example 1.

The results of the test revealed that the compounds I-A-1, I-A-2, I-A-3, I-A-4, I-A-5, I-A-7, I-A-10, I-A-13, I-A-16, I-A-17, I-A-25, I-A-35, I-A-36, I-A-37, I-A-38, I-A-39, I-A-41, I-A-43, I-A-49, I-A-52, I-A-54, I-A-55, I-A-56, I-A-57, I-A-58, I-A-59, I-A-62, I-A-63, I-A-64, I-A-68, I-A-70, I-A-71, I-A-75, I-A-76, I-A-77, I-A-80, I-A-81, I-A-82, I-A-89, I-A-94, I-A-98, I-A-99, I-A-100, I-A-101, I-A-102, I-A-103, I-A-113, I-A-114, I-A-125, I-A-127, I-A-148, I-A-150, I-A-155, I-A-164, I-A-165, I-A-168, I-A-171, I-A-172, I-A-173, I-A-175, I-A-178, I-A-180, I-A-186, I-A-195, I-A-196, I-A-197, I-A-198, I-A-199, I-A-200, I-A-206, I-A-214, I-A-217, I-A-218, I-A-219, I-A-222, I-A-226, I-A-238, I-A-246, I-A-250, I-A-251, I-A-252, I-A-253, I-A-254, I-A-255, I-A-256, I-A-257, I-A-258, I-A-259, I-A-260, I-A-261, I-A-262, I-A-263, I-A-264, I-A-265, I-A-266, I-A-267, I-A-268, I-A-269, I-A-270, I-A-271, I-A-272, I-A-273, I-A-274, I-A-275, I-A-276, I-A-277, I-A-278, I-A-279, I-A-281, I-A-282, I-A-284, I-A-285, I-A-286, I-A-287, I-A-288, I-A-289, I-A-290, I-A-291, I-A-292, I-A-293, I-A-294, I-A-295, I-A-296, I-A-297, I-A-298, I-A-299, I-A-300, I-A-301, I-A-302, I-A-303, I-A-304, I-A-347, I-A-348, I-A-349, I-A-350, I-A-351, I-A-352, I-A-353, I-A-354, I-A-355, I-A-357, I-A-359, I-A-362, I-A-363, I-A-365, I-A-366, I-A-368, I-A-375, I-A-378, I-A-381, I-A-384, I-A-385, I-A-386, I-A-387, I-A-388, I-A-389, I-A-390, I-A-391, I-A-392, I-A-393, I-A-398, I-A-399, I-A-400, I-A-401, I-A-403, I-A-404, I-A-405, I-A-406, I-A-407, I-A-408, I-A-409, I-A-410, I-A-411, I-A-412, I-A-413, I-A-414, I-A-415, I-A-416, I-A-417, I-A-418, I-A-419, I-A-420, I-A-421, I-A-422, I-A-425, I-A-426, I-A-427, I-A-428, I-A-429, I-A-430, I-A-432, I-A-437, I-A-441, I-A-442, I-A-443, I-A-444, I-A-445, I-A-446, I-A-447, I-A-449, I-A-452, I-A-456, I-A-457, I-A-459, I-A-460, I-A-461, I-A-462, I-A-463, I-A-464, I-A-465, I-A-466, I-A-468, I-A-470, I-A-471, I-A-472, I-A-473, I-A-474, I-A-475, I-A-476, I-A-477, I-A-478, I-A-480, I-A-481, I-A-482, I-A-483, I-A-485, I-A-486, I-A-487, I-A-488, I-A-489, I-A-490, I-A-491, I-A-499, I-A-500, I-A-501, I-A-502, I-A-503, I-A-504, I-A-505, I-A-507, I-A-508, I-A-509, I-A-510, I-A-511, I-A-512, I-A-513, I-A-514, I-A-515, I-A-516, I-A-517, I-A-518, I-A-519, I-A-520, I-A-521, I-A-522, I-A-524, I-A-525, I-A-526, I-A-527, I-A-528, I-A-529, I-A-530, I-A-531, I-A-532, I-A-533, I-A-534, I-A-535, I-A-536, I-A-537, I-A-538, I-A-539, I-A-540, I-A-541, I-A-542, I-A-543, I-A-545, I-A-546, I-A-547, I-A-548, I-A-549, I-A-550, I-A-551, I-A-555, I-A-556, I-A-557, I-A-558, I-A-559, I-A-560, I-A-561, I-A-562, I-A-563, I-A-564, I-A-565, I-A-568, I-A-569, I-A-570, I-A-571, I-A-572, I-A-573, I-A-574, I-A-575, I-A-576, I-A-577, I-A-578, I-A-579, I-A-580, I-A-581, I-A-582, I-A-583, I-A-584, I-A-585, I-A-586, I-A-587, I-A-588, I-A-589, I-A-590, I-A-591, I-A-592, I-A-593, I-A-594, I-A-595, I-A-596, I-A-597, I-A-598, I-A-599, I-A-600, I-A-601, I-A-602, I-A-603, I-A-604, I-A-605, I-A-606, I-A-607, I-A-608, I-A-609, I-A-610, I-A-611, I-A-612, I-A-613, I-A-614, I-A-615, I-A-619, I-A-620, I-A-621, I-A-622, I-A-623, I-A-624, I-A-625, I-A-626, I-A-627, I-A-628, I-A-629, I-A-630, I-A-632, I-A-634, I-A-635, I-A-636, I-A-637, I-A-638, I-A-639, I-A-643, I-A-644, I-A-646, I-A-647, I-B-2, I-B-16, I-B-17, I-B-18, I-C-13, I-C-14, I-C-15, I-C-16, I-C-17, I-C-18, I-C-19, I-C-20, I-C-21, I-C-22, I-C-23, I-C-24, I-C-25, I-C-26, I-C-27, I-C-28, I-C-29, I-C-30, I-C-31, I-K-7, I-K-8, I-K-9, I-K-10, I-K-11, I-K-13, I-K-15, I-K-17, I-K-18, I-K-19, I-K-20, I-K-21, I-K-22, I-K-23, I-K-24, I-K-25, I-K-26, I-K-27, I-K-28, I-L-2 and I-L-3 at a concentration of 200 ppm showed the activity of level 1 or higher.

### Test Example 6.

### Test for control efficacy on apple scab

A formulation prepared from the compound of the present invention according to Formulation Example 1 was diluted with water to a predetermined concentration. 10 ml of the diluted formulation was applied to the foliage of apple seedlings (variety: ORIN) grown in a pot 5 cm in diameter. After the formulation was air-dried, the apple seedlings were inoculated by spraying a spore suspension of apple scab fungus. The apple seedlings were kept at 20°C under humid condition for two days, and transferred to a room at 15°C, and then appropriately humidified to promote disease development. At 15 days after the inoculation, the control efficacy was evaluated according to the criteria of Test Example 1.

The results of the test revealed that the compounds I-A-2, I-A-3, I-A-4, I-A-5, I-A-7, I-A-10, I-A-13, I-A-16, I-A-25, I-A-35, I-A-36, I-A-37, I-A-38, I-A-40, I-A-41, I-A-43, I-A-49, I-A-54, I-A-55, I-A-56, I-A-57, I-A-58, I-A-63, I-A-68, I-A-71, I-A-75, I-A-77, I-A-78, I-A-80, I-A-81, I-A-89, I-A-94, I-A-96, I-A-97, I-A-98, I-A-100, I-A-101, I-A-102, I-A-103, I-A-113, I-A-114, I-A-127, I-A-132, I-A-133, I-A-148, I-A-150, I-A-152, I-A-157, I-A-159, I-A-160, I-A-165, I-A-168, I-A-171, I-A-175, I-A-180, I-A-186, I-A-190, I-A-192, I-A-196, I-A-198, I-A-199, I-A-200, I-A-206, I-A-218, I-A-219, I-A-220, I-A-222, I-A-226, I-A-238, I-A-239, I-A-250, I-A-251, I-A-252, I-A-253, I-A-254, I-A-257, I-A-258, I-A-259, I-A-260, I-A-261, I-A-262, I-A-263, I-A-264, I-A-265, I-A-266, I-A-267, I-A-269, I-A-270, I-A-271, I-A-272, I-A-273, I-A-274, I-A-275I-A-276, I-A-277, I-A-278, I-A-279, I-A-281, I-A-282, I-A-284, I-A-285, I-A-286, I-A-287, I-A-288, I-A-289, I-A-290, I-A-291, I-A-293, I-A-294, I-A-295, I-A-296, I-A-297, I-A-298, I-A-299, I-A-300, I-A-301, I-A-302, I-A-303, I-A-348, I-A-349, I-A-350, I-A-351, I-A-352, I-A-353, I-A-354, I-A-355, I-A-356, I-A-357, I-A-358, I-A-360, I-A-361, I-A-362, I-A-363, I-A-364, I-A-365, I-A-368, I-A-370, I-A-371, I-A-372, I-A-378, I-A-381, I-A-382, I-A-384, I-A-387, I-A-388, I-A-389, I-A-390, I-A-391, I-A-392, I-A-393, I-A-394, I-A-398, I-A-399, I-A-400, I-A-401, I-A-403, I-A-404, I-A-405, I-A-406, I-A-407, I-A-408, I-A-409, I-A-410, I-A-411, I-A-412, I-A-413, I-A-414, I-A-415, I-A-416, I-A-417, I-A-418, I-A-419, I-A-420, I-A-421, I-A-422, I-A-423, I-A-424, I-A-425, I-A-426, I-A-427, I-A-428, I-A-429, I-A-430, I-A-431, I-A-432, I-A-433, I-A-434, I-A-435, I-A-436, I-A-437, I-A-438, I-A-439, I-A-440, I-A-441, I-A-442, I-A-443, I-A-444, I-A-445, I-A-446, I-A-447, I-A-448, I-A-449, I-A-450, I-A-451, I-A-452, I-A-454, I-A-455, I-A-456, I-A-457, I-A-458, I-A-459, I-A-460, I-A-461, I-A-462, I-A-463, I-A-464, I-A-465, I-A-466, I-A-467, I-A-468, I-A-469, I-A-470, I-A-471, I-A-472, I-A-474, I-A-475, I-A-476, I-A-477, I-A-478, I-A-479, I-A-480, I-A-481, I-A-482, I-A-483, I-A-484, I-A-485, I-A-486, I-A-487, I-A-488, I-A-489, I-A-490, I-A-491, I-A-492, I-A-493, I-A-494, I-A-495, I-A-496, I-A-497, I-A-498, I-A-499, I-A-500, I-A-501, I-A-502, I-A-503, I-A-504, I-A-505, I-A-509, I-A-510, I-A-511, I-A-512, I-A-513, I-A-514, I-A-515, I-A-516, I-A-517, I-A-518, I-A-519, I-A-520, I-A-521, I-A-522, I-A-524, I-A-525, I-A-526, I-A-527, I-A-528, I-A-529, I-A-530, I-A-531, I-A-532, I-A-533, I-A-534, I-A-535, I-A-536, I-A-537, I-A-538, I-A-539, I-A-540, I-A-541, I-A-543, I-A-544, I-A-545, I-A-548, I-A-549, I-A-550, I-A-551, I-A-555, I-A-556, I-A-558, I-A-559, I-A-560, I-A-562, I-A-563, I-A-564, I-A-565, I-A-566, I-A-567, I-A-568, I-A-569, I-A-570, I-A-571, I-A-572, I-A-573, I-A-574, I-A-575, I-A-576, I-A-577, I-A-580, I-A-581, I-A-582, I-A-583, I-A-584, I-A-585, I-A-586, I-A-587, I-A-588, I-A-589, I-A-590, I-A-591, I-A-592, I-A-593, I-A-594, I-A-595, I-A-596, I-A-597, I-A-598, I-A-599, I-A-600, I-A-601, I-A-602, I-A-603, I-A-604, I-A-605, I-A-606, I-A-607, I-A-608, I-A-609, I-A-610, I-A-611, I-A-612, I-A-613, I-A-614, I-A-615, I-A-616, I-A-617, I-A-618, I-A-619, I-A-620, I-A-621, I-A-622, I-A-623, I-A-624, I-A-625, I-A-626, I-A-627, I-A-628, I-A-629, I-A-630, I-A-631, I-A-632, I-A-633, I-A-634, I-A-635, I-A-636, I-A-637, I-A-638, I-A-639, I-A-640, I-A-641, I-A-642, I-A-643, I-A-646, I-A-647, I-B-2, I-B-16, I-B-17, I-B-18, I-C-13, I-C-14, I-C-15, I-C-16, I-C-17, I-C-18, I-C-19, I-C-20, I-C-21, I-C-22, I-C-23, I-C-24, I-C-25, I-C-26, I-C-27, I-C-28, I-C-29, I-C-30, I-C-31, I-K-13, I-K-14, I-K-15, I-K-16, I-K-17, I-K-18, I-K-19, I-K-20, I-K-21, I-K-22, I-K-23, I-K-25, I-K-26, I-K-27, I-K-28 and I-L-1 at a concentration of 200 ppm showed the activity of level 1 or higher.

### Test Example 7.

### Antifungal activity test

The following (a), (b), (c), (d), (e) and (f) were used as test fungus. Each test fungus was mixed with the optimal medium for its growth and the test compound, and placed in a 96-well plate to examine the spore germination inhibitory activity. After growth, the absorbance was measured and the median effective concentration (EC₅₀: ppm) was calculated.
(a) Botryosphaeria berengeriana
(b) Colletotrichum acutatum
(c) Colletotrichum gloeosporioides
(d) Septoria tritici
(e) Fusarium graminearum
(f) Fusarium virguliforme

As a result of the above tests, the compounds with a median effective concentration of 200 ppm or less are listed below:
(a) I-A-36
(b) I-A-36, I-A-100, I-A-101, I-A-155, I-A-199, I-A-250, I-A-252, I-A-360, I-A-379 and I-A-384
(c) I-A-36, I-A-100, I-A-101, I-A-155, I-A-199, I-A-238, I-A-250, I-A-252, I-A-357, I-A-360, I-A-379 and I-A-384
(d) I-A-36, I-A-100, I-A-199, I-A-238, I-A-250, I-A-252, I-A-381 and I-A-384
(e) I-A-36, I-A-100, I-A-101, I-A-199, I-A-200, I-A-238, I-A-250, I-A-252, I-A-357, I-A-360, I-A-379 and I-A-384
(f) I-A-36, I-A-101 and I-A-199

### Test Example 8.

### Barley powdery mildew

A formulation prepared from the compound of the present invention according to Formulation Example 1 was diluted with water to a predetermined concentration. 10 ml of the diluted formulation was applied to the foliage of barley plants (variety: Kanto No. 6) at the one-leaf stage grown in a pot 5 cm in diameter. After the formulation was air-dried, the barley plants were inoculated by dusting with barley powdery mildew fungus, and kept in a greenhouse at 25°C to promote disease development. At 7 days after the inoculation, the control efficacy was evaluated according to the criteria of Test Example 1.

The results of the test revealed that the compounds I-A-2, I-A-3, I-A-4, I-A-5, I-A-10, I-A-13, I-A-16, I-A-25, I-A-35, I-A-36, I-A-43, I-A-55, I-A-56, I-A-63, I-A-71, I-A-77, I-A-79, I-A-95, I-A-97, I-A-99, I-A-100, I-A-103, I-A-127, I-A-132, I-A-133, I-A-135, I-A-139, I-A-147, I-A-148, I-A-152, I-A-155, I-A-167, I-A-169, I-A-190, I-A-192, I-A-197, I-A-198, I-A-199, I-A-200, I-A-206, I-A-214, I-A-217, I-A-218, I-A-219, I-A-220, I-A-222, I-A-226, I-A-238, I-A-239, I-A-246, I-A-250, I-A-251, I-A-252, I-A-254, I-A-255, I-A-256, I-A-257, I-A-259, I-A-260, I-A-261, I-A-262, I-A-264, I-A-265, I-A-266, I-A-269, I-A-271, I-A-272, I-A-273, I-A-274, I-A-275, I-A-277, I-A-278, I-A-279, I-A-280, I-A-281, I-A-283, I-A-286, I-A-288, I-A-289, I-A-290, I-A-291, I-A-292, I-A-293, I-A-294, I-A-295, I-A-296, I-A-299, I-A-300, I-A-301, I-A-302, I-A-304, I-A-346, I-A-347, I-A-348, I-A-351, I-A-352, I-A-353, I-A-355, I-A-357, I-A-358, I-A-359, I-A-361, I-A-363, I-A-370, I-A-379, I-A-383, I-A-384, I-A-387, I-A-388, I-A-389, I-A-390, I-A-391, I-A-392, I-A-393, I-A-394, I-A-398, I-A-400, I-A-401, I-A-403, I-A-404, I-A-405, I-A-407, I-A-408, I-A-409, I-A-410, I-A-411, I-A-413, I-A-414, I-A-416, I-A-417, I-A-420, I-A-421, I-A-422, I-A-436, I-A-440, I-A-474, I-A-475, I-A-480, I-A-485, I-A-488, I-A-492, I-A-524, I-A-525, I-A-527, I-A-530, I-A-558, I-A-576, I-A-577, I-A-606, I-A-619, I-B-2, I-C-2, I-C-5, I-C-15, I-C-16, I-C-17, I-C-18, I-C-26, I-C-27, I-C-28, I-K-7, I-K-8, I-K-9, I-K-10, I-K-13, I-K-14, I-K-15, I-K-16, I-K-17, I-K-18, I-K-19, I-K-20, I-K-22 and I-K-25 at a concentration of 200 ppm showed the activity of level 1 or higher.

### Test Example 9.

### Test for control efficacy on wheat leaf blotch

A formulation prepared from the compound of the present invention according to Formulation Example 1 was diluted with water to a predetermined concentration. 10 ml of the diluted formulation was applied to the foliage of wheat plants at the ten-leaf stage grown in a pot 6 cm in diameter. After the formulation was air-dried, the wheat plants were inoculated by spraying a spore suspension of wheat leaf blotch fungus. The wheat plants were kept at 15°C under constant humidity condition to allow to develop the disease. At 16 days after the inoculation, the control efficacy was evaluated according to the criteria of Test Example 1.

The results of the test revealed that the compounds I-A-5, I-A-37, I-A-39, I-A-75, I-A-81, I-A-89, I-A-94, I-A-106, I-A-124, I-A-132, I-A-135, I-A-139, I-A-152, I-A-159, I-A-175, I-A-196, I-A-206, I-A-218, I-A-220, I-A-222, I-A-239, I-A-250, I-A-253, I-A-264, I-A-269, I-A-287, I-A-289, I-A-290, I-A-291, I-A-349, I-A-355, I-A-359, I-A-386, I-A-390, I-A-392, I-A-410, I-A-432, I-A-433, I-A-434, I-A-444, I-A-520, I-A-524, I-A-525, I-A-542, I-A-545, I-A-546, I-A-561, I-A-562, I-A-563, I-A-572, I-A-574, I-A-578, I-A-589, I-A-591, I-A-595, I-A-596, I-A-608, I-A-609, I-A-619, I-A-621, I-A-622, I-A-624, I-A-635, I-A-636, I-A-637, I-C-15, I-C-17, I-K-13 and I-K-18 at a concentration of 200 ppm showed the activity of level 1 or higher.

### Test Example 10.

### Antifungal activity test

The following (a), (b), (c), (d) and (e) were used as test fungus, and the mycelium growth inhibitory activity of each test compound was examined using PDA medium. After growth, the mycelium colony diameter was measured and the median effective concentration (EC₅₀: ppm) was calculated.
(a) Septoria tritici
(b) Botrytis cinerea
(C) Fusarium graminearum
(d) Sclerotinia sclerotirum
(e) Cochliobolus miyabeanus

As a result of the above tests, the compounds with a median effective concentration of 200 ppm or less are listed below:
(a) I-A-16, I-A-36, I-A-43, I-A-56, I-A-65, I-A-75, I-A-94, I-A-100, I-A-101, I-A-114, I-A-135, I-A-188, I-A-198, I-A-199, I-A-214, I-A-217, I-A-218, I-A-222, I-A-226, I-A-238, I-A-251, I-A-252, I-A-257, I-A-259, I-A-261, I-A-264, I-A-265, I-A-267, I-A-268, I-A-274, I-A-278, I-A-282, I-A-284, I-A-285, I-A-287, I-A-294, I-A-295, I-A-296, I-A-297, I-A-301, I-A-302 and I-A-378
(b) I-A-1, I-A-2, I-A-3, I-A-4, I-A-5, I-A-7, I-A-10, I-A-13, I-A-16, I-A-17, I-A-25, I-A-35, I-A-36, I-A-37, I-A-38, I-A-39, I-A-41, I-A-43, I-A-49, I-A-52, I-A-54, I-A-55, I-A-56, I-A-58, I-A-59, I-A-62, I-A-63, I-A-64, I-A-65, I-A-68, I-A-70, I-A-71, I-A-75, I-A-77, I-A-78, I-A-79, I-A-80, I-A-89, I-A-94, I-A-98, I-A-99, I-A-100, I-A-101, I-A-102, I-A-106, I-A-113, I-A-114, I-A-123, I-A-125, I-A-127, I-A-132, I-A-133, I-A-135, I-A-138, I-A-139, I-A-148, I-A-152, I-A-154, I-A-155, I-A-157, I-A-159, I-A-165, I-A-167, I-A-168, I-A-169, I-A-171, I-A-172, I-A-173, I-A-175, I-A-178, I-A-180, I-A-186, I-A-192, I-A-195, I-A-196, I-A-197, I-A-198, I-A-199, I-A-200, I-A-206, I-A-214, I-A-217, I-A-218, I-A-220, I-A-222, I-A-226, I-A-238, I-A-250, I-A-251, I-A-252, I-A-253, I-A-254, I-A-255, I-A-256, I-A-257, I-A-258, I-A-259, I-A-260, I-A-261, I-A-262, I-A-263, I-A-264, I-A-265, I-A-266, I-A-267, I-A-268, I-A-269, I-A-270, I-A-271, I-A-272, I-A-273, I-A-274, I-A-275, I-A-276, I-A-277, I-A-278, I-A-279, I-A-281, I-A-282, I-A-283, I-A-284, I-A-285, I-A-286, I-A-288, I-A-289, I-A-290, I-A-293, I-A-294, I-A-295, I-A-296, I-A-297, I-A-298, I-A-299, I-A-300, I-A-301, I-A-302, I-A-303, I-A-304, I-A-346, I-A-348, I-A-349, I-A-350, I-A-352, I-A-353, I-A-357, I-A-360, I-A-362, I-A-363, I-A-366, I-A-368, I-A-370, I-A-372, I-A-375, I-A-376, I-A-378, I-A-379, I-A-381, I-A-384, I-C-2, I-C-13 and I-C-14
(c) I-A-1, I-A-2, I-A-3, I-A-4, I-A-5, I-A-7, I-A-10, I-A-13, I-A-16, I-A-17, I-A-25, I-A-35, I-A-36, I-A-39, I-A-41, I-A-43, I-A-49, I-A-52, I-A-54, I-A-55, I-A-56, I-A-57, I-A-58, I-A-59, I-A-62, I-A-63, I-A-64, I-A-68, I-A-70, I-A-71, I-A-75, I-A-77, I-A-79, I-A-80, I-A-81, I-A-89, I-A-94, I-A-97, I-A-98, I-A-99, I-A-100, I-A-101, I-A-102, I-A-103, I-A-106, I-A-113, I-A-114, I-A-123, I-A-125, I-A-127, I-A-132, I-A-133, I-A-135, I-A-138, I-A-139, I-A-148, I-A-152, I-A-154, I-A-155, I-A-157, I-A-160, I-A-165, I-A-167, I-A-168, I-A-169, I-A-171, I-A-172, I-A-173, I-A-175, I-A-178, I-A-180, I-A-186, I-A-188, I-A-190, I-A-192, I-A-194, I-A-195, I-A-196, I-A-197, I-A-198, I-A-199, I-A-200, I-A-206, I-A-214, I-A-217, I-A-218, I-A-220, I-A-222, I-A-226, I-A-238, I-A-250, I-A-251, I-A-252, I-A-253, I-A-254, I-A-255, I-A-256, I-A-257, I-A-258, I-A-259, I-A-260, I-A-261, I-A-262, I-A-263, I-A-264, I-A-265, I-A-266, I-A-267, I-A-268, I-A-269, I-A-270, I-A-271, I-A-272, I-A-273, I-A-274, I-A-275, I-A-276, I-A-277, I-A-278, I-A-279, I-A-280, I-A-281, I-A-282, I-A-283, I-A-284, I-A-285, I-A-286, I-A-287, I-A-288, I-A-289, I-A-290, I-A-293, I-A-294, I-A-295, I-A-296, I-A-297, I-A-298, I-A-299, I-A-300, I-A-301, I-A-302, I-A-303, I-A-348, I-A-349, I-A-350, I-A-352, I-A-353, I-A-356, I-A-357, I-A-358, I-A-359, I-A-360, I-A-362, I-A-363, I-A-364, I-A-365, I-A-368, I-A-370, I-A-371, I-A-372, I-A-376, I-A-378, I-A-379, I-A-381, I-A-384, I-C-2, I-C-13 and I-C-14
(d) I-A-1, I-A-2, I-A-3, I-A-4, I-A-5, I-A-7, I-A-10, I-A-13, I-A-16, I-A-17, I-A-25, I-A-35, I-A-36, I-A-37I-A-38, I-A-39, I-A-40, I-A-41, I-A-43, I-A-49, I-A-52, I-A-54, I-A-55, I-A-56, I-A-57, I-A-58, I-A-59, I-A-62, I-A-63, I-A-64, I-A-65, I-A-68, I-A-70, I-A-71, I-A-75, I-A-76, I-A-77, I-A-78, I-A-79, I-A-80, I-A-81, I-A-82, I-A-89, I-A-94, I-A-95, I-A-97, I-A-98, I-A-99, I-A-100, I-A-101, I-A-102, I-A-103, I-A-106, I-A-113, I-A-114, I-A-123, I-A-125, I-A-127, I-A-130, I-A-131, I-A-132, I-A-133, I-A-135, I-A-138, I-A-139, I-A-148, I-A-152, I-A-154, I-A-155, I-A-157, I-A-159, I-A-160, I-A-162, I-A-163, I-A-165, I-A-167, I-A-168, I-A-169, I-A-171, I-A-172, I-A-173, I-A-175, I-A-178, I-A-180, I-A-186, I-A-192, I-A-194, I-A-195, I-A-196, I-A-197, I-A-198, I-A-199, I-A-200, I-A-206, I-A-214, I-A-217, I-A-218, I-A-219, I-A-220, I-A-222, I-A-226, I-A-238, I-A-239, I-A-250, I-A-251, I-A-252, I-A-253, I-A-254, I-A-255, I-A-256, I-A-257, I-A-258, I-A-259, I-A-260, I-A-261, I-A-262, I-A-263, I-A-264, I-A-265, I-A-266, I-A-267, I-A-268, I-A-269, I-A-270, I-A-271, I-A-272, I-A-273, I-A-274, I-A-275, I-A-276, I-A-277, I-A-278, I-A-279, I-A-280, I-A-281, I-A-282, I-A-283, I-A-284, I-A-285, I-A-286, I-A-287, I-A-288, I-A-289, I-A-290, I-A-293, I-A-294, I-A-295, I-A-296, I-A-297, I-A-298, I-A-299, I-A-300, I-A-301, I-A-302, I-A-303, I-A-304, I-A-346, I-A-348, I-A-349, I-A-350, I-A-352, I-A-353, I-A-356, I-A-357, I-A-358, I-A-359, I-A-360, I-A-362, I-A-363, I-A-364, I-A-365, I-A-366, I-A-368, I-A-369, I-A-370, I-A-371, I-A-372, I-A-373I-A-374, I-A-375, I-A-376, I-A-378, I-A-379, I-A-381, I-A-382, I-A-383, I-A-384, I-C-2, I-C-13 and I-C-14
(e) I-A-1, I-A-2, I-A-3, I-A-4, I-A-5, I-A-7, I-A-10, I-A-13, I-A-16, I-A-17, I-A-25, I-A-35, I-A-36, I-A-37I-A-38, I-A-39, I-A-40, I-A-41, I-A-43, I-A-49, I-A-52, I-A-54, I-A-55, I-A-56, I-A-57, I-A-58, I-A-59, I-A-62, I-A-63, I-A-64, I-A-65, I-A-68, I-A-70, I-A-71, I-A-75, I-A-76, I-A-77, I-A-78, I-A-79, I-A-80, I-A-81, I-A-82, I-A-89, I-A-94, I-A-96, I-A-97, I-A-98, I-A-99, I-A-100, I-A-101, I-A-102, I-A-103, I-A-106, I-A-113, I-A-114, I-A-123, I-A-125, I-A-127, I-A-131, I-A-132, I-A-133, I-A-135, I-A-138, I-A-139, I-A-147, I-A-148, I-A-152, I-A-154, I-A-155, I-A-157, I-A-159, I-A-160, I-A-162, I-A-164, I-A-165, I-A-167, I-A-168, I-A-169, I-A-171, I-A-172, I-A-173, I-A-175, I-A-178, I-A-180, I-A-186, I-A-188, I-A-190, I-A-192, I-A-194, I-A-195, I-A-196, I-A-197, I-A-198, I-A-199, I-A-200, I-A-206, I-A-214, I-A-217, I-A-218, I-A-219, I-A-220, I-A-222, I-A-226, I-A-238, I-A-239, I-A-250, I-A-251, I-A-252, I-A-253, I-A-254, I-A-255, I-A-256, I-A-257, I-A-258, I-A-259, I-A-260, I-A-261, I-A-262, I-A-263, I-A-264, I-A-265, I-A-266, I-A-267, I-A-268, I-A-269, I-A-270, I-A-271, I-A-272, I-A-273, I-A-274, I-A-275, I-A-276, I-A-277, I-A-278, I-A-279, I-A-280, I-A-281, I-A-282, I-A-283, I-A-284, I-A-285, I-A-286, I-A-287, I-A-288, I-A-289, I-A-290, I-A-293, I-A-294, I-A-295, I-A-296, I-A-297, I-A-298, I-A-299, I-A-300, I-A-301, I-A-302, I-A-303, I-A-304, I-A-346, I-A-348, I-A-349, I-A-350, I-A-352, I-A-353, I-A-356, I-A-357, I-A-358, I-A-359, I-A-360, I-A-362, I-A-363, I-A-364, I-A-365, I-A-366, I-A-367, I-A-368, I-A-370, I-A-371, I-A-372, I-A-373, I-A-376, I-A-378, I-A-379, I-A-381, I-A-382, I-A-383, I-A-384, I-C-2, I-C-13 and I-C-14

### Test Example 11.

### Test for control efficacy on cucumber sclerotinia rot

A formulation prepared from the compound of the present invention according to Formulation Example 1 was diluted with water to a predetermined concentration. 10 ml of the diluted formulation was applied to the foliage of cucumber plants (variety: YOTUBA) at the one-leaf stage grown in a pot 9 cm in diameter. After the formulation was air-dried, a 4 mm mycelial disk was prepared from the mycelium of cucumber sclerotinia rot fungus. The cucumber plants were inoculated by placing the mycelial disk in the center of the leave. The cucumber plants were kept at 15°C under constant humidity condition to allow to develop the disease. At 5 or 6 days after the inoculation, the control efficacy was evaluated according to the criteria of Test Example 1.

The results of the test revealed that the compounds I-A-1, I-A-3, I-A-4, I-A-5, I-A-13, I-A-16, I-A-25, I-A-35, I-A-36, I-A-41, I-A-43, I-A-56, I-A-63, I-A-78, I-A-80, I-A-82, I-A-89, I-A-94, I-A-95, I-A-97, I-A-99, I-A-100, I-A-101, I-A-102, I-A-106, I-A-113, I-A-125, I-A-129, I-A-130, I-A-132, I-A-133, I-A-135, I-A-148, I-A-155, I-A-160, I-A-162, I-A-165, I-A-168, I-A-198, I-A-199, I-A-200, I-A-206, I-A-214, I-A-218, I-A-219, I-A-220, I-A-222, I-A-226, I-A-238, I-A-239, I-A-250, I-A-251, I-A-252, I-A-347, I-A-348, I-A-351, I-A-353, I-A-358, I-A-360, I-A-362, I-A-363, I-A-381, I-A-383, I-A-384, I-A-524, I-A-525, I-C-2, I-K-8, I-K-9, I-K-10, I-K-13, I-L-2 and I-L-3 at a concentration of 200 ppm showed the activity of level 1 or higher.

### [Industrial Applicability]

The amide compound of the present invention has an effect of controlling diseases which may infest cereals, fruit trees, vegetables, other crops, and ornamental flowering plants, and is therefore useful as agrichemicals.

This application is based on patent applications No. 2022-182306 filed on November 15, 2022 and No. 2023-057006 filed on March 31, 2023 in Japan, the contents of which are encompassed in full herein.

## Claims

1. A compound represented by the general formula [I] 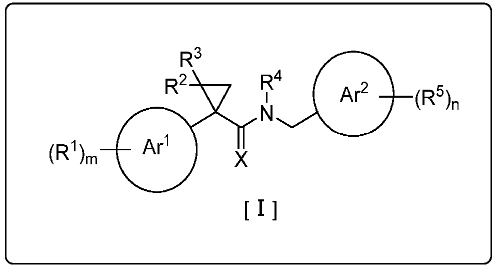 wherein
R¹ and R⁵ may be the same or different and each represents
(a1) a hydrogen atom;
(a2) a halogen atom;
(a3) a cyano group;
(a4) a nitro group;
(a5) a hydroxy group;
(a6) a pentafluorosulfanyl group;
(a7) a (C1-C6) alkyl group;
(a8) a halo (C1-C6) alkyl group;
(a9) a (C2-C6) alkenyl group;
(a10) a halo (C2-C6) alkenyl group;
(a11) a (C2-C6) alkynyl group;
(a12) a halo (C2-C6) alkynyl group;
(a13) a (C3-C6) cycloalkyl group;
(a14) a (C3-C6) cycloalkenyl group;
(a15) a halo (C3-C6) cycloalkyl group;
(a16) a (C3-C6) cycloalkyl (C1-C6) alkyl group;
(a17) a (C3-C6) cycloalkyl (C3-C6) cycloalkyl group;
(a18) a halo (C3-C6) cycloalkyl (C1-C6) alkyl group;
(a19) a halo (C3-C6) cycloalkyl halo (C1-C6) alkyl group;
(a20) a cyano (C1-C6) alkyl group;
(a21) a cyano (C3-C6) cycloalkyl group;
(a22) a hydroxy (C1-C6) alkyl group;
(a23) a (C1-C6) alkoxy (C1-C6) alkyl group;
(a24) a halo (C1-C6) alkoxy (C1-C6) alkyl group;
(a25) a (C3-C6) cycloalkoxy (C1-C6) alkyl group;
(a26) a halo (C3-C6) cycloalkoxy (C1-C6) alkyl group;
(a27) a (C1-C6) alkylsulfanyl (C1-C6) alkyl group;
(a28) a halo (C1-C6) alkylsulfanyl (C1-C6) alkyl group;
(a29) a (C1-C6) alkylsulfanyl halo (C1-C6) alkyl group;
(a30) a (C1-C6) alkylsulfinyl (C1-C6) alkyl group;
(a31) a halo (C1-C6) alkylsulfinyl (C1-C6) alkyl group;
(a32) a (C1-C6) alkylsulfinyl halo (C1-C6) alkyl group;
(a33) a (C1-C6) alkylsulfonyl (C1-C6) alkyl group;
(a34) a halo (C1-C6) alkylsulfonyl (C1-C6) alkyl group;
(a35) a (C1-C6) alkylsulfonyl halo (C1-C6) alkyl group;
(a36) a (C1-C6) alkoxy (C2-C6) alkenyl group;
(a37) a (C1-C6) alkoxy (C2-C6) alkynyl group;
(a38) a (C1-C10) alkoxy group;
(a39) a halo (C1-C10) alkoxy group;
(a40) a (C2-C6) alkenyloxy group;
(a41) a halo (C2-C6) alkenyloxy group;
(a42) a (C2-C6) alkynyloxy group;
(a43) a halo (C2-C6) alkynyloxy group;
(a44) a (C3-C7) cycloalkoxy group;
(a45) a (C3-C6) cycloalkyl (C1-C6) alkoxy group;
(a46) a (C1-C6) alkoxy (C1-C6) alkoxy group;
(a47) a (C1-C6) alkylcarbonyloxy group;
(a48) a (C3-C6) cycloalkylcarbonyloxy group;
(a49) a (C3-C6) cycloalkenyl group;
(a50) a (C1-C6) alkylsulfanyl group;
(a51) a halo (C1-C6) alkylsulfanyl group;
(a52) a (C1-C6) alkylsulfinyl group;
(a53) a halo (C1-C6) alkylsulfinyl group;
(a54) a (C1-C6) alkylsulfonyl group;
(a55) a halo (C1-C6) alkylsulfonyl group;
(a56) a Z group;
(a57) a Z (C1-C6) alkyl group;
(a58) a Z (C1-C6) alkoxy group;
(a59) a Z halo (C1-C6) alkoxy group;
(a60) a Z (C2-C6) alkenyl group;
(a61) a Z halo (C2-C6) alkenyl group;
(a62) a Z (C2-C6) alkynyl group;
(a63) a Z halo (C2-C6) alkynyl group;
(a64) a Z (C3-C6) cycloalkyl group;
(a65) a Z halo (C3-C6) cycloalkyl group;
(a66) a Z carbonyloxy group;
(a67) a Z (C1-C6) alkylsulfanyl group;
(a68) an N-mono-Z (C1-C6) alkylamino group;
(a69) an N-(C1-C6) alkyl-N-Z (C1-C6) alkylamino group;
(a70) an N-Z (C1-C6) alkyl-N-Z (C1-C6) alkylamino group;
(a71) a Z oxy group;
(a72) a Z sulfonylamino group;
(a73) a Z sulfanyl group;
(a74) a Z sulfinyl group;
(a75) a Z sulfonyl group;
(a76) a Z carbonylamino group;
(a77) an N-((C1-C6) alkyl)-N-(Z carbonyl)amino group;
(a78) an N-mono-(C1-C6) alkoxycarbonylamino group;
(a79) an N-((C1-C6) alkoxycarbonyl) -N-((C1-C6) alkyl) amino group;
(a80) an N-(C1-C6) alkyl-N-Z aminocarbonyl group;
(a81) a Z carbonyl group;
(a82) a Z (C1-C6) alkylcarbonylamino group;
(a83) a Z amino group;
(a84) an amino group;
(a85) an N-mono-(C1-C6) alkylamino group;
(a86) an N-(C1-C6) alkyl-N-(C1-C6) alkylamino group;
(a87) an N-mono-(C2-C6) alkenylamino group;
(a88) an N-(C2-C6) alkenyl-N-(C2-C6) alkenylamino group;
(a89) an N-mono-(C2-C6) alkynylamino group;
(a90) an N-(C2-C6) alkynyl-N-(C2-C6) alkynylamino group;
(a91) a (C1-C6) alkylcarbonylamino group;
(a92) a (C3-C6) cycloalkylcarbonylamino group;
(a93) an aminosulfanyl group;
(a94) an aminosulfinyl group;
(a95) an aminosulfonyl group;
(a96) an N-mono-(C1-C6) alkylaminosulfanyl group;
(a97) an N-(C1-C6) alkyl-N-(C1-C6) alkylaminosulfanyl group;
(a98) an N-mono-(C1-C6) alkylaminosulfinyl group;
(a99) an N-(C1-C6) alkyl-N-(C1-C6) alkylaminosulfinyl group;
(a100) an N-mono-(C1-C6) alkylaminosulfonyl group;
(a101) an N-(C1-C6) alkyl-N-(C1-C6) alkylaminosulfonyl group;
(a102) an aminocarbonyl group;
(a103) an N-mono-(C1-C6) alkylaminocarbonyl group;
(a104) an N-(C1-C6) alkyl-N-(C1-C6) alkylaminocarbonyl group;
(a105) a (C1-C6) alkylcarbonyl group;
(a106) a (C3-C6) cycloalkylcarbonyl group;
(a107) a (C1-C6) alkoxycarbonyl group;
(a108) an aminooxy group;
(a109) a hydroxyamino group;
(a110) a (C3-C6) cycloalkoxycarbonyl group;
(alll) a formyl group;
(a112) a carboxy group;
(a113) an oxido group;
(a114) a (C1-C12) alkylsilyl group;
(a115) a halo (C3-C6) cycloalkoxy group;
(a116) a Z halo (C1-C6) alkyl group;
(a117) an N-Z aminosulfonyl group;
(a118) a Z,Z (C1-C6) alkoxy group;
(a119) a (C1-C6) alkoxy (C1-C6) alkoxy (C1-C6) alkoxy group;
(a120) an N-((C1-C6) alkoxycarbonyl)-N-Z amino group;
(a121) a (C3-C6) cycloalkoxy group;
(a122) a halo (C1-C6) alkylcarbonylamino group;
(a123) a (C3-C6) cycloalkylamino group;
(a124) a Z aminocarbonyl group;
(a125) a (C3-C6) cycloalkylaminocarbonyl group; or
(a126) a group represented by the following formula A1, A2, A3, A4 or A5 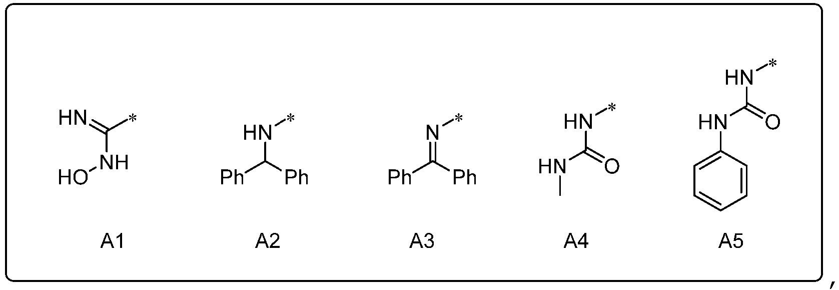
Z represents
(b1) a phenyl group;
(b2) a substituted phenyl group having, on a ring, 1 to 5 substituents independently selected from the group consisting of a halogen atom, a nitro group, a cyano group, a hydroxy group, an amino group, a (C1-C6) alkyl group, a halo (C1-C6) alkyl group, a (C1-C6) alkoxy group, a halo (C1-C6) alkoxy group, a (C2-C6) alkenyl group, a halo (C2-C6) alkenyl group, a (C2-C6) alkynyl group, a (C3-C6) cycloalkyl group, a (C2-C6) alkenyloxy group, a halo (C2-C6) alkenyloxy group, a (C1-C6) alkylsulfanyl group, a halo (C1-C6) alkylsulfanyl group, a (C1-C6) alkylsulfonyloxy group, a halo (C1-C6) alkylsulfonyloxy group, a (C1-C6) alkylsulfinyl group, a halo (C1-C6) alkylsulfinyl group, a (C1-C6) alkylsulfonyl group, a halo (C1-C6) alkylsulfonyl group, a (C2-C6) alkenylsulfanyl group, a halo (C2-C6) alkenylsulfanyl group, a (C2-C6) alkenylsulfinyl group, a halo (C2-C6) alkenylsulfinyl group, a (C2-C6) alkenylsulfonyl group, a halo (C2-C6) alkenylsulfonyl group, an N-mono-(C1-C6) alkylamino group, an N,N-di-(C1-C6) alkylamino group in which the alkyl groups may be the same or different, a (C1-C6) alkylsulfonylamino group, a halo (C1-C6) alkylsulfonylamino group, a (C1-C6) alkylcarbonyl group, a formyl group, a halo (C1-C6) alkylcarbonyl group, a hydroxy (C1-C6) alkyl group, a (C1-C6) alkoxycarbonyl group, an aminocarbonyl group, a (C1-C12) alkylsilyl group, a phenoxy group and a phenyl group;
(b3) a heterocyclic group;
(b4) a heterocyclic group having, on a ring, 1 to 3 substituents independently selected from the group consisting of a halogen atom, a hydroxy group, a (C1-C6) alkyl group, a halo (C1-C6) alkyl group, a (C1-C6) alkoxy group, a halo (C1-C6) alkoxy group, a (C1-C6) alkylsulfanyl group, a halo (C1-C6) alkylsulfanyl group, a (C1-C6) alkylsulfinyl group, a halo (C1-C6) alkylsulfinyl group, a (C1-C6) alkylsulfonyl group, a halo (C1-C6) alkylsulfonyl group and a (C1-C6) alkoxycarbonyl group;
(b5) a naphthyl group;
(b6) a dihydroindenyl group; or
(b7) an indenyl group,
R² and R³ may be the same or different and each represents
(c1) a (C1-C6) alkyl group; or
(c2) a halogen atom,
R₄ represents
(d1) a hydrogen atom;
(d2) a (C1-C6) alkyl group;
(d3) a halo (C1-C6) alkyl group;
(d4) a (C1-C6) alkoxy group;
(d5) a halo (C1-C6) alkoxy group;
(d6) a (C2-C6) alkenyl group;
(d7) a (C2-C6) alkynyl group;
(d8) a (C3-C6) cycloalkyl group;
(d9) a (C3-C6) cycloalkyl (C1-C6) alkyl group;
(d10) a (C1-C6) alkoxy (C1-C6) alkyl group;
(d11) a (C1-C6) alkylcarbonyl group;
(d12) a (C1-C6) alkoxycarbonyl group;
(d13) a (C3-C6) cycloalkylcarbonyl group;
(d14) a (C1-C6) alkoxy (C1-C6) alkylcarbonyl group;
(d15) a halo (C1-C6) alkylcarbonyl group;
(d16) a (C1-C6) alkoxycarbonyl (C1-C6) alkyl group;
(d17) an amino (C1-C6) alkylcarbonyl group;
(d18) an N-mono-(C1-C6) alkylamino (C1-C6) alkylcarbonyl group;
(d19) an N,N-di-(C1-C6) alkylamino (C1-C6) alkylcarbonyl group;
(d20) a (C1-C6) alkylsulfanyl group;
(d21) a halo (C1-C6) alkylsulfanyl group;
(d22) a (C1-C6) alkylsulfinyl group;
(d23) a halo (C1-C6) alkylsulfinyl group;
(d24) a (C1-C6) alkylsulfonyl group;
(d25) a halo (C1-C6) alkylsulfonyl group;
(d26) a (C3-C6) cycloalkylsulfanyl group;
(d27) a (C3-C6) cycloalkylsulfinyl group;
(d28) a (C3-C6) cycloalkylsulfonyl group;
(d29) an amino (C1-C6) alkyl group;
(d30) an N-mono-(C1-C6) alkylamino (C1-C6) alkyl group;
(d31) an N,N-di-(C1-C6) alkylamino (C1-C6) alkyl group;
(d32) a Z (C1-C6) alkyl group;
(d33) a Z group;
(d34) a Z oxy group;
(d35) a Z carbonyl group;
(d36) a (C2-C6) alkenyloxycarbonyl group;
(d37) a (C2-C6) alkynyloxycarbonyl group;
(d38) a (C2-C6) alkenylcarbonyl group;
(d39) an amino (C1-C6) alkylcarbonyl group;
(d40) an N-mono-(C1-C6) alkylamino (C1-C6) alkylcarbonyl group;
(d41) an N,N-di-(C1-C6) alkylamino (C1-C6) alkylcarbonyl group;
(d42) a Z (C1-C6) alkylcarbonyl group; or
(d43) a Z (C1-C6) alkoxycarbonyl group,
two R¹ may be bonded to each other to form a ring structure represented by the following formula R¹⁻¹, R¹⁻², R¹⁻³ or R¹⁻⁴, each containing the carbon atoms to which they are bonded 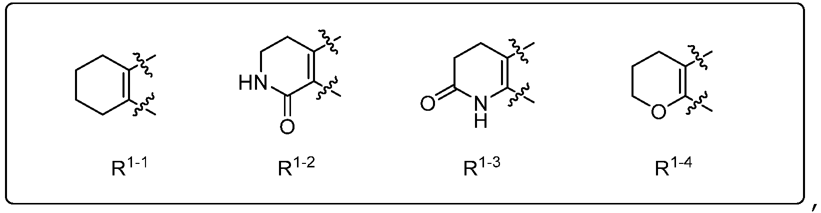
two R⁵ may be bonded to each other to form a ring structure represented by the following formula R⁵⁻¹ or R⁵⁻², each containing the carbon atoms to which they are bonded 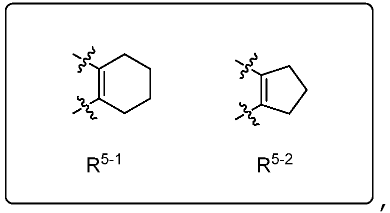
R⁴ and R⁵ may be bonded to each other to form a ring structure containing a partial structure represented by the following formula R⁴⁻⁵⁻¹ or R⁴⁻⁵⁻² and the carbon atom and the nitrogen atom to which they are bonded 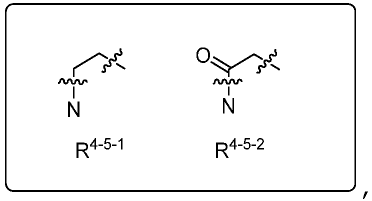
X represents an oxygen atom or a sulfur atom,
Ar¹ represents phenyl, pyridyl, pyridazinyl, pyrimidinyl, pyrazinyl, thienyl, thiazolyl or a group represented by the following formula #1, #2 or #3 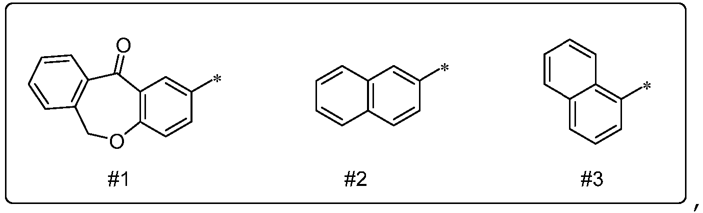
Ar² represents a group represented by the following formula Ar²⁻¹, Ar²⁻², Ar²⁻³, Ar²⁻⁴, Ar²⁻⁵, Ar²⁻⁶, Ar²⁻¹, Ar²⁻¹, Ar^{2-9,} Ar²⁻¹⁰, Ar²⁻¹¹, Ar²⁻¹², Ar²⁻¹³, Ar²⁻¹⁴, Ar²⁻¹⁵, Ar²⁻¹⁶, Ar²⁻¹⁷, Ar²⁻¹⁸, Ar²⁻¹⁹, Ar²⁻²⁰, Ar²⁻²¹, Ar²⁻²² or Ar²⁻²³ 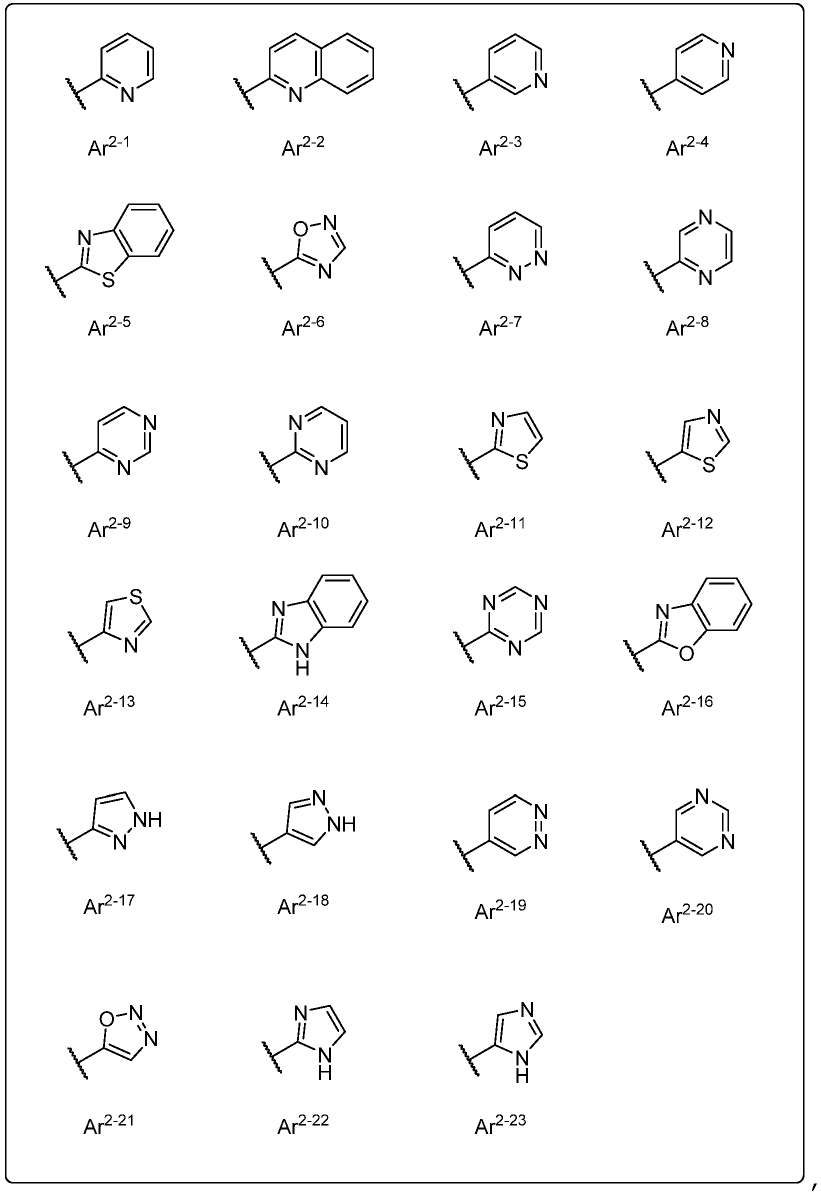
m represents 1, 2, 3, 4 or 5, and
n represents 1, 2, 3, 4 or 5,
or a salt thereof,
provided that a compound represented by the following formula DC1 and a salt thereof are excluded

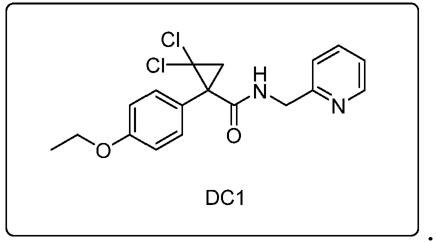

2. The compound or salt thereof according to claim 1,
wherein
R¹ and R⁵ may be the same or different and each is
(a1) a hydrogen atom;
(a2) a halogen atom;
(a3) a cyano group;
(a4) a nitro group;
(a5) a hydroxy group;
(a7) a (C1-C6) alkyl group;
(a8) a halo (C1-C6) alkyl group;
(a9) a (C2-C6) alkenyl group;
(a10) a halo (C2-C6) alkenyl group;
(a11) a (C2-C6) alkynyl group;
(a12) a halo (C2-C6) alkynyl group;
(a13) a (C3-C6) cycloalkyl group;
(a14) a (C3-C6) cycloalkenyl group;
(a15) a halo (C3-C6) cycloalkyl group;
(a16) a (C3-C6) cycloalkyl (C1-C6) alkyl group;
(a17) a (C3-C6) cycloalkyl (C3-C6) cycloalkyl group;
(a22) a hydroxy (C1-C6) alkyl group;
(a23) a (C1-C6) alkoxy (C1-C6) alkyl group;
(a24) a halo (C1-C6) alkoxy (C1-C6) alkyl group;
(a25) a (C3-C6) cycloalkoxy (C1-C6) alkyl group;
(a26) a halo (C3-C6) cycloalkoxy (C1-C6) alkyl group;
(a27) a (C1-C6) alkylsulfanyl (C1-C6) alkyl group;
(a36) a (C1-C6) alkoxy (C2-C6) alkenyl group;
(a37) a (C1-C6) alkoxy (C2-C6) alkynyl group;
(a38) a (C1-C10) alkoxy group;
(a39) a halo (C1-C10) alkoxy group;
(a40) a (C2-C6) alkenyloxy group;
(a41) a halo (C2-C6) alkenyloxy group;
(a42) a (C2-C6) alkynyloxy group;
(a43) a halo (C2-C6) alkynyloxy group;
(a44) a (C3-C7) cycloalkoxy group;
(a45) a (C3-C6) cycloalkyl (C1-C6) alkoxy group;
(a46) a (C1-C6) alkoxy (C1-C6) alkoxy group;
(a47) a (C1-C6) alkylcarbonyloxy group;
(a48) a (C3-C6) cycloalkylcarbonyloxy group;
(a49) a (C3-C6) cycloalkenyl group;
(a50) a (C1-C6) alkylsulfanyl group;
(a51) a halo (C1-C6) alkylsulfanyl group;
(a52) a (C1-C6) alkylsulfinyl group;
(a53) a halo (C1-C6) alkylsulfinyl group;
(a54) a (C1-C6) alkylsulfonyl group;
(a55) a halo (C1-C6) alkylsulfonyl group;
(a56) a Z group;
(a57) a Z (C1-C6) alkyl group;
(a58) a Z (C1-C6) alkoxy group;
(a59) a Z halo (C1-C6) alkoxy group;
(a62) a Z (C2-C6) alkynyl group;
(a66) a Z carbonyloxy group;
(a67) a Z (C1-C6) alkylsulfanyl group;
(a68) an N-mono-Z (C1-C6) alkylamino group;
(a69) an N-(C1-C6) alkyl-N-Z (C1-C6) alkylamino group;
(a70) an N-Z (C1-C6) alkyl-N-Z (C1-C6) alkylamino group;
(a71) a Z oxy group;
(a72) a Z sulfonylamino group;
(a73) a Z sulfanyl group;
(a74) a Z sulfinyl group;
(a75) a Z sulfonyl group;
(a76) a Z carbonylamino group;
(a77) an N-((C1-C6) alkyl)-N-(Z carbonyl)amino group;
(a78) an N-mono-(C1-C6) alkoxycarbonylamino group;
(a79) an N-((C1-C6) alkoxycarbonyl)-N-((C1-C6) alkyl) amino group;
(a80) an N-(C1-C6) alkyl-N-Z aminocarbonyl group;
(a81) a Z carbonyl group;
(a82) a Z (C1-C6) alkylcarbonylamino group;
(a83) a Z amino group;
(a84) an amino group;
(a85) an N-mono-(C1-C6) alkylamino group;
(a86) an N-(C1-C6) alkyl-N-(C1-C6) alkylamino group;
(a87) an N-mono-(C2-C6) alkenylamino group;
(a88) an N-(C2-C6) alkenyl-N-(C2-C6) alkenylamino group;
(a89) an N-mono-(C2-C6) alkynylamino group;
(a90) an N-(C2-C6) alkynyl-N-(C2-C6) alkynylamino group;
(a91) a (C1-C6) alkylcarbonylamino group;
(a92) a (C3-C6) cycloalkylcarbonylamino group;
(a93) an aminosulfanyl group;
(a94) an aminosulfinyl group;
(a95) an aminosulfonyl group;
(a96) an N-mono-(C1-C6) alkylaminosulfanyl group;
(a97) an N-(C1-C6) alkyl-N-(C1-C6) alkylaminosulfanyl group;
(a98) an N-mono-(C1-C6) alkylaminosulfinyl group;
(a99) an N-(C1-C6) alkyl-N-(C1-C6) alkylaminosulfinyl group;
(a100) an N-mono-(C1-C6) alkylaminosulfonyl group;
(a101) an N-(C1-C6) alkyl-N-(C1-C6) alkylaminosulfonyl group;
(a102) an aminocarbonyl group;
(a103) an N-mono-(C1-C6) alkylaminocarbonyl group;
(a104) an N-(C1-C6) alkyl-N-(C1-C6) alkylaminocarbonyl group;
(a105) a (C1-C6) alkylcarbonyl group;
(a106) a (C3-C6) cycloalkylcarbonyl group;
(a107) a (C1-C6) alkoxycarbonyl group;
(a108) an aminooxy group;
(a109) a hydroxyamino group;
(a110) a (C3-C6) cycloalkoxycarbonyl group;
(alll) a formyl group;
(a112) a carboxy group;
(a113) an oxido group;
(a114) a (C1-C12) alkylsilyl group;
(a120) an N-((C1-C6) alkoxycarbonyl)-N-Z amino group;
(a121) a (C3-C6) cycloalkoxy group;
(a122) a halo (C1-C6) alkylcarbonylamino group;
(a123) a (C3-C6) cycloalkylamino group;
(a124) a Z aminocarbonyl group;
(a125) a (C3-C6) cycloalkylaminocarbonyl group; or
(a126) a group represented by the following formula A1, A2, A3, A4 or A5 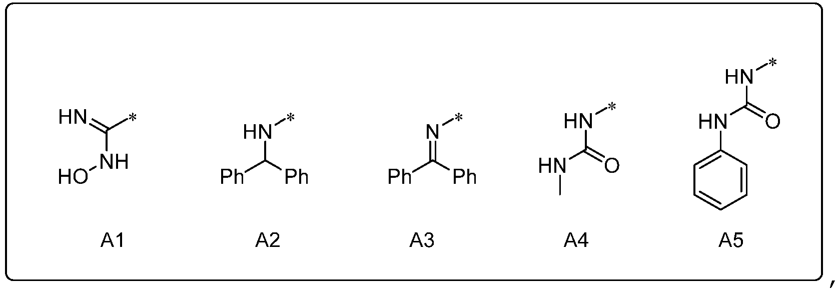
R² and R³ may be the same or different and each is a halogen atom,
R⁴ is
(d1) a hydrogen atom;
(d2) a (C1-C6) alkyl group;
(d4) a (C1-C6) alkoxy group;
(d6) a (C2-C6) alkenyl group;
(d9) a (C3-C6) cycloalkyl (C1-C6) alkyl group;
(d10) a (C1-C6) alkoxy (C1-C6) alkyl group;
(d11) a (C1-C6) alkylcarbonyl group;
(d12) a (C1-C6) alkoxycarbonyl group;
(d14) a (C1-C6) alkoxy (C1-C6) alkylcarbonyl group;
(d16) a (C1-C6) alkoxycarbonyl (C1-C6) alkyl group;
(d20) a (C1-C6) alkylsulfanyl group;
(d22) a (C1-C6) alkylsulfinyl group;
(d24) a (C1-C6) alkylsulfonyl group;
(d28) a (C3-C6) cycloalkylsulfonyl group;
(d29) an amino (C1-C6) alkyl group;
(d30) an N-mono-(C1-C6) alkylamino (C1-C6) alkyl group;
(d31) an N,N-di-(C1-C6) alkylamino (C1-C6) alkyl group;
(d32) a Z (C1-C6) alkyl group;
(d33) a Z group;
(d35) a Z carbonyl group;
(d36) a (C2-C6) alkenyloxycarbonyl group;
(d37) a (C2-C6) alkynyloxycarbonyl group;
(d38) a (C2-C6) alkenylcarbonyl group;
(d39) an amino (C1-C6) alkylcarbonyl group;
(d40) an N-mono-(C1-C6) alkylamino (C1-C6) alkylcarbonyl group; or
(d41) an N,N-di-(C1-C6) alkylamino (C1-C6) alkylcarbonyl group,
R⁴ and R⁵ may be bonded to each other to form a ring structure containing a partial structure represented by the following formula R⁴⁻⁵⁻¹ and the carbon atom and the nitrogen atom to which they are bonded 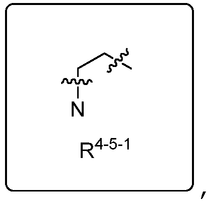 and
Ar² is a group represented by the following formula Ar²⁻¹, Ar²⁻², Ar²⁻³, Ar²⁻⁴, Ar²⁻⁵, Ar²⁻⁶, Ar²⁻⁷, Ar²⁻⁸, Ar²⁻⁹, Ar²⁻¹⁰, Ar²⁻¹¹, Ar²⁻¹², Ar²⁻¹³, Ar²⁻¹⁴, Ar²⁻¹⁷, Ar²⁻¹⁸, Ar²⁻¹⁹ or Ar²⁻²⁰

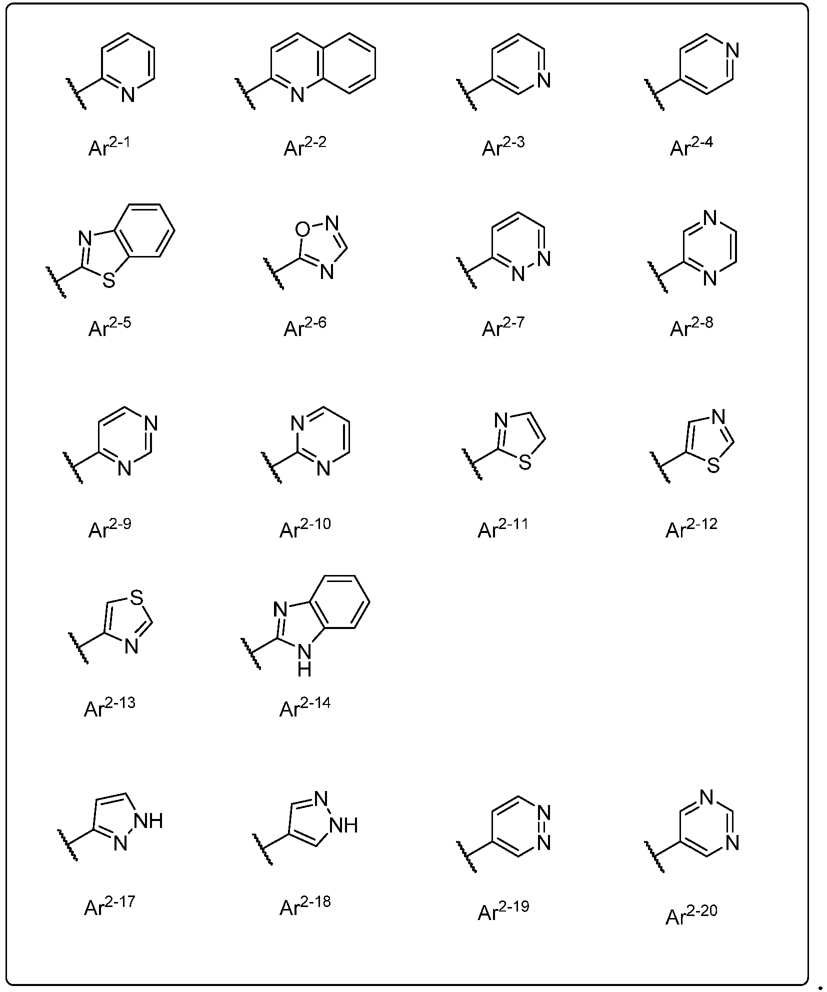

3. The compound or salt thereof according to claim 2, wherein
R¹ and R⁵ may be the same or different and each is
(a1) a hydrogen atom;
(a2) a halogen atom;
(a3) a cyano group;
(a4) a nitro group;
(a5) a hydroxy group;
(a7) a (C1-C6) alkyl group;
(a8) a halo (C1-C6) alkyl group;
(a9) a (C2-C6) alkenyl group;
(a11) a (C2-C6) alkynyl group;
(a13) a (C3-C6) cycloalkyl group;
(a14) a (C3-C6) cycloalkenyl group;
(a22) a hydroxy (C1-C6) alkyl group;
(a36) a (C1-C6) alkoxy (C2-C6) alkenyl group;
(a38) a (C1-C10) alkoxy group;
(a39) a halo (C1-C10) alkoxy group;
(a40) a (C2-C6) alkenyloxy group;
(a44) a (C3-C7) cycloalkoxy group;
(a45) a (C3-C6) cycloalkyl (C1-C6) alkoxy group;
(a46) a (C1-C6) alkoxy (C1-C6) alkoxy group;
(a47) a (C1-C6) alkylcarbonyloxy group;
(a48) a (C3-C6) cycloalkylcarbonyloxy group;
(a50) a (C1-C6) alkylsulfanyl group;
(a51) a halo (C1-C6) alkylsulfanyl group;
(a52) a (C1-C6) alkylsulfinyl group;
(a54) a (C1-C6) alkylsulfonyl group;
(a56) a Z group;
(a57) a Z (C1-C6) alkyl group;
(a58) a Z (C1-C6) alkoxy group;
(a62) a Z (C2-C6) alkynyl group;
(a66) a Z carbonyloxy group;
(a67) a Z (C1-C6) alkylsulfanyl group;
(a68) an N-mono-Z (C1-C6) alkylamino group;
(a69) an N-(C1-C6) alkyl-N-Z (C1-C6) alkylamino group;
(a70) an N-Z (C1-C6) alkyl-N-Z (C1-C6) alkylamino group;
(a71) a Z oxy group;
(a72) a Z sulfonylamino group;
(a76) a Z carbonylamino group;
(a77) an N-((C1-C6) alkyl)-N-(Z carbonyl)amino group;
(a78) an N-mono-(C1-C6) alkoxycarbonylamino group;
(a79) an N-((C1-C6) alkoxycarbonyl) -N-((C1-C6) alkyl) amino group;
(a81) a Z carbonyl group;
(a82) a Z (C1-C6) alkylcarbonylamino group;
(a83) a Z amino group;
(a84) an amino group;
(a85) an N-mono-(C1-C6) alkylamino group;
(a86) an N-(C1-C6) alkyl-N-(C1-C6) alkylamino group;
(a87) an N-mono-(C2-C6) alkenylamino group;
(a91) a (C1-C6) alkylcarbonylamino group;
(a92) a (C3-C6) cycloalkylcarbonylamino group;
(a95) an aminosulfonyl group;
(a100) an N-mono-(C1-C6) alkylaminosulfonyl group;
(a101) an N-(C1-C6) alkyl-N-(C1-C6) alkylaminosulfonyl group;
(a102) an aminocarbonyl group;
(a103) an N-mono-(C1-C6) alkylaminocarbonyl group;
(a104) an N-(C1-C6) alkyl-N-(C1-C6) alkylaminocarbonyl group;
(a105) a (C1-C6) alkylcarbonyl group;
(a106) a (C3-C6) cycloalkylcarbonyl group;
(a107) a (C1-C6) alkoxycarbonyl group;
(alll) a formyl group;
(a112) a carboxy group;
(a113) an oxido group;
(a114) a (C1-C12) alkylsilyl group;
(a120) an N-((C1-C6) alkoxycarbonyl)-N-Z amino group;
(a121) a (C3-C6) cycloalkoxy group;
(a122) a halo (C1-C6) alkylcarbonylamino group;
(a123) a (C3-C6) cycloalkylamino group;
(a124) a Z aminocarbonyl group;
(a125) a (C3-C6) cycloalkylaminocarbonyl group; or
(a126) a group represented by the following formula A1, A2, A3, A4 or A5 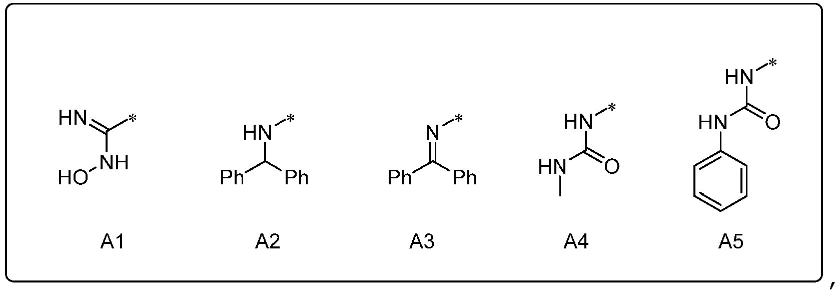
R⁴ is
(d1) a hydrogen atom;
(d2) a (C1-C6) alkyl group;
(d4) a (C1-C6) alkoxy group;
(d6) a (C2-C6) alkenyl group;
(d10) a (C1-C6) alkoxy (C1-C6) alkyl group;
(d11) a (C1-C6) alkylcarbonyl group;
(d12) a (C1-C6) alkoxycarbonyl group;
(d14) a (C1-C6) alkoxy (C1-C6) alkylcarbonyl group;
(d16) a (C1-C6) alkoxycarbonyl (C1-C6) alkyl group;
(d22) a (C1-C6) alkylsulfinyl group;
(d24) a (C1-C6) alkylsulfonyl group;
(d28) a (C3-C6) cycloalkylsulfonyl group;
(d31) an N,N-di-(C1-C6) alkylamino (C1-C6) alkyl group;
(d32) a Z (C1-C6) alkyl group;
(d36) a (C2-C6) alkenyloxycarbonyl group;
(d38) a (C2-C6) alkenylcarbonyl group;
(d39) an amino (C1-C6) alkylcarbonyl group; or
(d41) an N,N-di-(C1-C6) alkylamino (C1-C6) alkylcarbonyl group, and
X is an oxygen atom.

4. An agricultural and horticultural fungicide comprising the compound or salt thereof according to claim 1 as an active ingredient.

5. An agricultural and horticultural fungicide comprising the compound or salt thereof according to claim 2 as an active ingredient.

6. An agricultural and horticultural fungicide comprising the compound or salt thereof according to claim 3 as an active ingredient.

7. A method for controlling an agricultural and horticultural disease, comprising treating a plant or soil with an effective amount of the agricultural and horticultural fungicide according to claim **4.**

8. A method for controlling an agricultural and horticultural disease, comprising treating a plant or soil with an effective amount of the agricultural and horticultural fungicide according to claim **5.**

9. A method for controlling an agricultural and horticultural disease, comprising treating a plant or soil with an effective amount of the agricultural and horticultural fungicide according to claim **6.**

10. Use of the compound or salt thereof according to claim 1 as a fungicide.

11. Use of the compound or salt thereof according to claim 2 as a fungicide.

12. Use of the compound or salt thereof according to claim 3 as a fungicide.
